# EUROPEAN PATENT SPECIFICATION

(11) **EP 2 875 023 B1**
(45) Date of publication and mention of the grant of the patent: **05.04.2017**
(21) Application number: 13742344.8
(22) Date of filing: 16.07.2013
(51) Int. Cl.: C07D 405/14, C07D 405/12, C07D 471/04, A61K 31/4375, A61P 3/00, A61P 11/00, A61P 35/00, A61K 31/403, A61K 31/5377, A61K 31/4725

(54) **OCTAHYDRO-CYCLOPENTAPYRROLYL ANTAGONISTS OF CCR2**
OCTAHYDROCYCLOPENTAPYRROLYL ANTAGONISTEN VON CCR2
DERIVES D'OCTAHYDROCYCLOPENTAPYRROLE COMME ANTAGONISTES DE RCC2

(30) Priority: 19.07.2012 US 201261673383 P
(43) Date of publication of application: 27.05.2015
(73) Proprietor: Janssen Pharmaceutica NV, 2340 Beerse (BE)
(72) Inventor: CAI, Chaozhong, North Wales, Pennsylvania 19454 (US); MCCOMSEY, David F., Warminster, Pennsylvania 18974 (US); SUI, Zhihua, Norristown, Pennsylvania 19403 (US); KANG, Fu An, Collegeville, Pennsylvania 19426 (US)
(74) Representative: Carpmaels & Ransford LLP
(86) International application number: PCT/US2013/050665
(87) International publication number: WO 2014/014901

(56) References cited:
- WO-A2-2005/079496
- WO-A2-2005/120505

## Description

### FIELD OF THE INVENTION

The invention is directed to substituted fused cyclopentyl compounds, which are antagonists to the chemoattractant cytokine receptor 2 (CCR2), pharmaceutical compositions, and them for use in methods for use. More particularly, the CCR2 antagonists are compounds useful for preventing, treating or ameliorating a CCR2 mediated syndrome, disorder or disease.

### BACKGROUND OF THE INVENTION

CCR2 is a member of the GPCR family of receptors, as are all known chemokine receptors, and are expressed by monocytes and memory T-lymphocytes. The CCR2 signaling cascade involves activation of phospholipases (PLCβ2), protein kinases (PKC), and lipid kinases (PI-3 kinase).

Chemoattractant cytokines (i.e., chemokines) are relatively small proteins (8-10 kD), which stimulate the migration of cells. The chemokine family is divided into four subfamilies based on the number of amino acid residues between the first and second highly conserved cysteines.

Monocyte chemotactic protein-1 (MCP-1) is a member of the CC chemokine subfamily (wherein CC represents the subfamily having adjacent first and second cysteines) and binds to the cell-surface chemokine receptor 2 (CCR2). MCP-1 is a potent chemotactic factor, which, after binding to CCR2, mediates monocyte and lymphocyte migration (i.e., chemotaxis) toward a site of inflammation. MCP-1 is also expressed by cardiac muscle cells, blood vessel endothelial cells, fibroblasts, chondrocytes, smooth muscle cells, mesangial cells, alveolar cells, T-lymphocytes, macrophages, and the like.

After monocytes enter the inflammatory tissue and differentiate into macrophages, monocyte differentiation provides a secondary source of several proinflammatory modulators, including tumor necrosis factor-α (TNF-α), interleukin-1 (IL-1), IL-8 (a member of the CXC chemokine subfamily, wherein CXC represents one amino acid residue between the first and second cysteines), IL-12, arachidonic acid metabolites (e.g., PGE₂ and LTB₄), oxygen-derived free radicals, matrix metalloproteinases, and complement components.

Animal model studies of chronic inflammatory diseases have demonstrated that inhibition of binding between MCP-1 and CCR2 by an antagonist suppresses the inflammatory response. The interaction between MCP-1 and CCR2 has been implicated (see

Rollins B J, Monocyte chemoattractant protein 1: a potential regulator of monocyte recruitment in inflammatory disease, Mol. Med. Today, 1996, 2:198; and Dawson J, et al., Targeting monocyte chemoattractant protein-1 signaling in disease, Expert Opin. Ther. Targets, 2003 Feb. 7 (1):35-48) in inflammatory disease pathologies such as psoriasis, uveitis, atherosclerosis, rheumatoid arthritis (RA), multiple sclerosis, Crohn's Disease, nephritis, organ allograft rejection, fibroid lung, renal insufficiency, type II diabetes and diabetic complications, diabetic nephropathy, diabetic retinopathy, diabetic retinitis, diabetic microangiopathy, tuberculosis, sarcoidosis, invasive *staphylococcia,* inflammation after cataract surgery, allergic rhinitis, allergic conjunctivitis, chronic urticaria, Chronic Obstructive Pulmonary Disease (COPD), allergic asthma, periodontal diseases, periodonitis, gingivitis, gum disease, diastolic cardiomyopathies, cardiac infarction, myocarditis, chronic heart failure, angiostenosis, restenosis, reperfusion disorders, glomerulonephritis, solid tumors and cancers, chronic lymphocytic leukemia, chronic myelocytic leukemia, multiple myeloma, malignant myeloma, Hodgkin's disease, and carcinomas of the bladder, breast, cervix, colon, lung, prostate, and stomach.

Monocyte migration is inhibited by MCP-1 antagonists (either antibodies or soluble, inactive fragments of MCP-1), which have been shown to inhibit the development of arthritis, asthma, and uveitis. Both MCP-1 and CCR2 knockout (KO) mice have demonstrated that monocyte infiltration into inflammatory lesions is significantly decreased. In addition, such KO mice are resistant to the development of experimental allergic encephalomyelitis (EAE, a model of human MS), cockroach allergen-induced asthma, atherosclerosis, and uveitis. Rheumatoid arthritis and Crohn's Disease patients have improved during treatment with TNF-α antagonists (e.g., monoclonal antibodies and soluble receptors) at dose levels correlated with decreases in MCP-1 expression and the number of infiltrating macrophages.

MCP-1 has been implicated in the pathogenesis of seasonal and chronic allergic rhinitis, having been found in the nasal mucosa of most patients with dust mite allergies. MCP-1 has also been found to induce histamine release from basophils in vitro. During allergic conditions, both allergens and histamines have been shown to trigger (i.e. to up-regulate) the expression of MCP-1 and other chemokines in the nasal mucosa of people with allergic rhinitis, suggesting the presence of a positive feedback loop in such patients.

International Patent Publication No. WO-2005/120505 discusses compounds which are modulators of chemokine receptor activity and may be useful in treating inflammatory and immunoregulatory diseases. These compounds have the following structure of Formula (I), wherein n, R¹, R³-R¹⁰, R¹⁵, R¹⁶, Y and Z are defined therein: 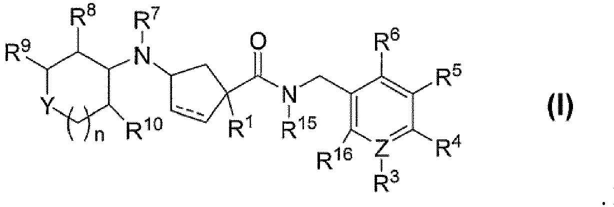 International Patent Publication No. WO-2005/079496 discusses compounds which are modulators of MCP-1 and may be useful in treating inflammatory and autoimmune conditions. These compounds have the following structure of Formula (I) wherein X, Z, a-d, n, R¹-R⁵, R¹⁰, R^{10a} and R¹² are defined therein:

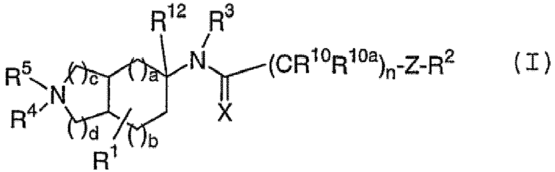

There remains a need for small molecule CCR2 antagonists for preventing, treating or ameliorating a CCR2 mediated inflammatory syndrome, disorder or disease resulting from MCP-1 induced monocyte and lymphocyte migration to a site of inflammation.

### SUMMARY OF THE INVENTION

The present invention relates to the compounds of Formula (I) 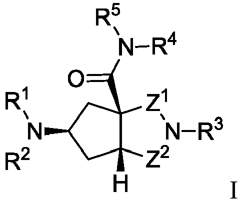 wherein
R¹ is C₍₁₋₄₎alkylOC₍₁₋₄₎alkyl, cyclohexyl, or tetrahydropyranyl, wherein said cyclohexyl or tetrahydropyranyl may be optionally substituted with one substituent selected from the group consisting of: OC₍₁₋₄₎alkyl, OH, CH₂CH₃, -CN, NH₂, NH(CH₃), N(CH₃)₂, or OCF₃;
R² is H, C(S)NHCH₂CH(CH₃)₂, or C(S)NHCH₃;
R³ is 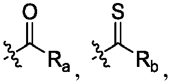 -SO₂-R_{c}, 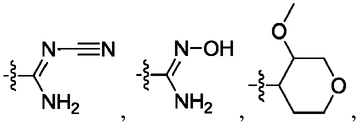 H, -CN, C₍₁₋₄₎alkyl, C₍₁₋₄₎alkylNA¹A², C₍₁₋₃₎alkylC(O)NA¹A², C₍₃₋₆₎cycloalkyl, oxetan-3-yl, -(CH₂)ₙPh-Rₐₐ, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl; wherein said 4,5 dihydro thiazolyl, 4,5 dihydro oxazolyl, thiazolyl, oxazolyl, pyrimidinyl, pyridyl, pyrazyl, and furyl may be optionally substituted with up to two substituent independently selected from Rₐₐ.
   n is 0, 1, 2, or 3;
   Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl,-CH₂NHBoc, -OCH₂CH=CH₂, -OCH₂CH₂CF₃, -OCH₂CH₂C(CH₃)₂OH, -OCH₂CH₂OC₍₁₋₄₎alkyl, -OCH₂CH₂CN, -OPh-Rₐₐ, 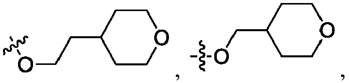 -OC₍₁₋₄₎alkyl-Ph-Rₐₐ, 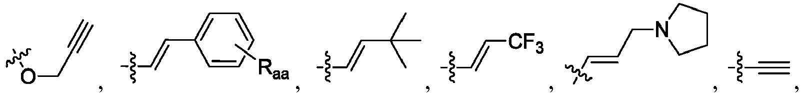 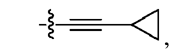 phenyl-Rₐₐ, oxazol-2-yl, oxazol-4-yl, isoxazol-5-yl, or thiazol-2-yl;
   Rₐₐ is H, OC₍₁₋₄₎alkyl, OCF₃, -CO₂H, Cl, Br, F, or -CN;
   R_{b} is NA¹A²;
   R_{c} is NA¹A², CH₂Ph, CH₂CH₂Ph, or C₍₁₋₄₎alkyl;
   A¹ is H, C₍₁₋₆₎alkyl, Ph-Rₐₐ, C(O)CH₃, CH₂Ph-Rₐₐ, or C₍₁₋₄₎alkylOC₍₁₋₄₎alkyl;
   A² is H, C₍₁₋₆₎alkyl; or
   A¹ and A² may be taken together with the nitrogen to which they are attached to form a ring selected from the group consisting of: 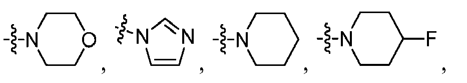 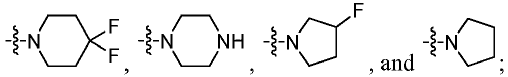
R⁴ is CH₂Ph, wherein said Ph is optionally substituted with up to two groups selected from CF₃, OCF₃, and F;
R⁵ is H; or R⁴ and R⁵ are taken together with their attached nitrogen to form a pair of fused rings selected from the group consisting of: 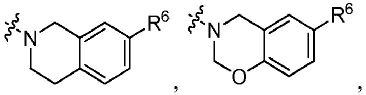 and 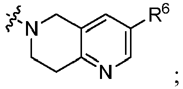 R⁶ is CF₃, or OCF₃;
Z¹ is CH₂ or C=O;
Z² is CH₂ or Z² may be C=O provided that Z¹ and Z² are not both simultaneously C=O; and pharmaceutically acceptable salts thereof.

### DETAILED DESCRIPTION OF THE INVENTION

The present invention comprises compounds of Formula (I) 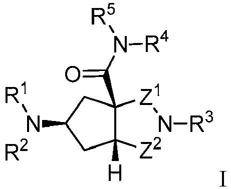 wherein
R¹, R², R³, R⁴, R⁵; Z¹ and Z² are as defined above.

In an embodiment of the invention:
R¹ is C₍₁₋₄₎alkylOCH₃, cyclohexyl, 1-methoxy cyclohex-2-yl, tetrahydropyran-4-yl, or 3-C₍₁₋₄₎alkoxy tetrahydropyran-4-yl;
R³ is 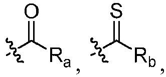 -SO₂-R_{c}, 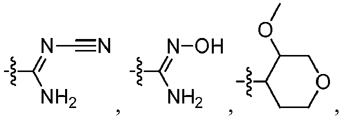 H, -CN, C₍₁₋₄₎alkyl, C₍₁₋₄₎alkylNA¹A², C₍₁₋₃₎alkylC(O)N(C₍₁₋₂₎alkyl)₂, C₍₃₋₆₎cycloalkyl, oxetan-3-yl, -(CH₂)ₙPh, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl;
   n is 0, 1, 2, or 3;
   Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl, -CH₂NHBoc,-OCH₂CH=CH₂, -OCH₂CH₂CF₃, -OCH₂CH₂C(CH₃)₂OH, -OCH₂CH₂OC₍₁₋₄₎alkyl,-OCH₂CH₂CN, -OPh, 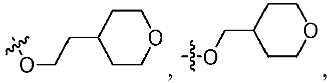 -OC₍₁₋₄₎alkyl-Ph-Rₐₐ, 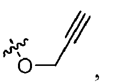 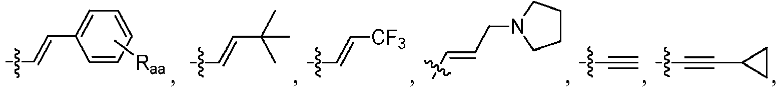 phenyl, oxazol-2-yl, oxazol-4-yl, isoxazol-5-yl, or thiazol-2-yl;
   Rₐₐ is H, OC₍₁₋₄₎alkyl, -CO₂H, Cl, Br, F, or -CN;
   R_{b} is NA¹A²;
   R_{c} is NH₂, NHCH₂Ph, CH₂Ph, CH₂CH₂Ph, or C₍₁₋₄₎alkyl;
   A¹ is H, C₍₁₋₆₎alkyl, Ph-Rₐₐ, or C(O)CH₃, CH₂Ph-Rₐₐ, C₍₁₋₄₎alkylOC₍₁₋₄₎alkyl; A² is H, C₍₁₋₆₎alkyl; or A¹ and A² may be taken together with the nitrogen to which they are attached to form a ring selected from the group consisting of: 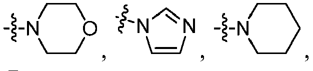 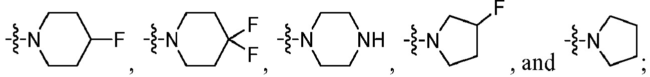
R⁴ is CH₂Ph, wherein said Ph is optionally substituted with up to two groups selected from CF₃, OCF₃, and F;
R⁵ is H; or
R⁴ and R⁵ are taken together with their attached nitrogen to form a pair of fused rings selected from the group consisting of: 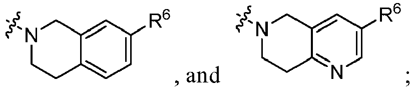
and pharmaceutically acceptable salts thereof.

In another embodiment of the invention:
R¹ is C₍₁₋₄₎alkylOCH₃, 3-C₍₁₋₄₎alkoxy tetrahydropyran-4-yl, or tetrahydropyran-4-yl;
R² is H, C(S)NHCH₂CH(CH₃)₂, or C(S)NHCH₃;
R³ is 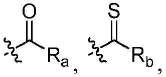 -SO₂-R_{c}, 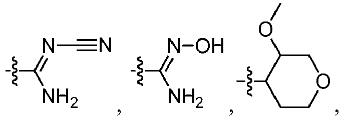 H, -CN, C₍₁₋₄₎alkyl, -C₍₁₋₃₎alkylC(O)N(C₍₁₋₂₎alkyl)₂, C₍₃₋₆₎cycloalkyl, oxetan-3-yl, -(CH₂)ₙPh, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl;
   n is 0, 1, 2, or 3;
   Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl, -CH₂NHBoc,-OCH₂CH=CH₂, -OCH₂CH₂CF₃, -OCH₂CH₂C(CH₃)₂OH, -OCH₂CH₂OCH₃, -OCH₂CH₂CN,-OPh, 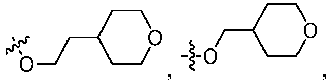 -OC₍₁₋₄₎alkyl-Ph-Rₐₐ, 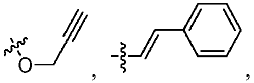 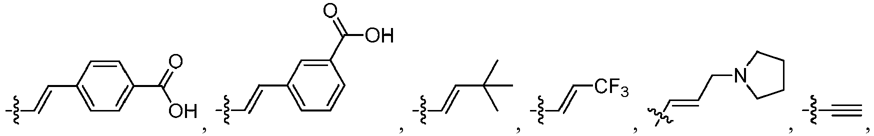 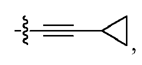 phenyl, oxazol-2-yl, oxazol-4-yl, isoxazol-5-yl, or thiazol-2-yl;
   Rₐₐ is H, OC(₁₋₄)alkyl, or -CN;
   R_{b} is 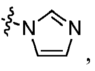 NH₂, NHCH₃, NHCH₂Ph, or NHCH₂CH(CH₃)₂;
   R_{c} is NH₂, NHCH₂Ph, CH₂Ph, CH₂CH₂Ph, or CH₃;
   A¹ is H, C₍₁₋₆₎alkyl, Ph, C(O)CH₃, CH₂Ph, or C₍₁₋₄₎alkylOC₍₁₋₄₎alkyl;
   A² is H, C₍₁₋₆₎alkyl; or
   A¹ and A² may be taken together with the nitrogen to which they are attached to form a ring selected from the group consisting of: 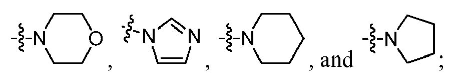
R⁴ is CH₂Ph, wherein said Ph is optionally substituted with up to two groups selected from CF₃, OCF₃, and F;
R⁵ is H; or R⁴ and R⁵ are taken together with their attached nitrogen to form a pair of fused rings selected from the group consisting of: 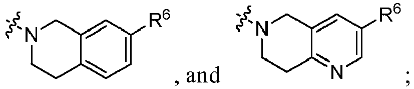 R⁶ is CF₃, or OCF₃;
Z¹ is CH₂ or C=O;
Z² is CH₂ or Z² may be C=O provided that Z¹ and Z² are not both simultaneously C=O; and pharmaceutically acceptable salts thereof.

In another embodiment of the invention:
R¹ is CH₂CH₂OCH₃, 3-C₍₁₋₄₎alkoxy tetrahydropyran-4-yl, or tetrahydropyran-4-yl;
R² is H, C(S)NHCH₂CH(CH₃)₂, or C(S)NHCH₃;
R³ is 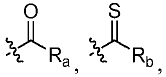 -SO₂-R_{c}, 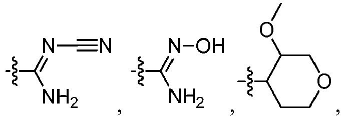 H, -CN, C₍₁₋₄₎alkyl, -C₍₁₋₃₎alkylC(O)N(C₍₁₋₂₎alkyl)₂, cyclopropyl, cyclobutanyl, oxetan-3-yl, -(CH₂)ₙPh, -C₍₁₋₄₎alkylCO₂C₍₁₋₄₎alkyl, 4,5 dihydro thiazol-2-yl, 4,5 dihydro oxazol-2-yl, thiazol-2-yl, pyrimidin-2-yl, or 3-methyl 1,2,4 oxadiazol-5-yl;
   n is 0, 1, 2, or 3;
   Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂NHBoc, -OCH₂Ph, -CH₂OC(O)C₍₁₋₄₎alkyl,-CH₂OC₍₁₋₄₎alkyl, -OCH₂Ph-CN, -OCH₂Ph-OCH₃, -OCH₂CH=CH₂, -OCH₂CH₂CF₃,-OCH₂CH₂C(CH₃)₂OH, -OCH₂CH₂OCH₃, -OCH₂CH₂CN, -OPh, 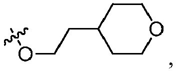 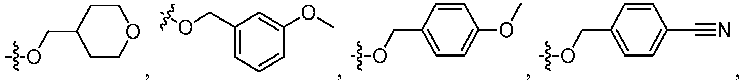 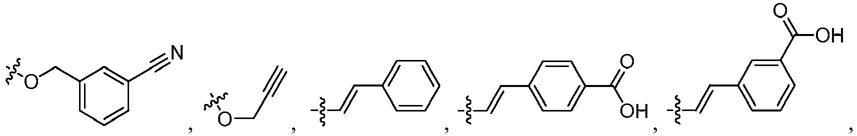 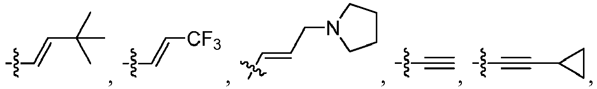 phenyl, oxazol-2-yl, oxazol-4-yl, isoxazol-5-yl, or thiazol-2-yl;
   R_{b} is 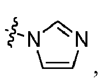 NH₂, NHCH₃, NHCH₂Ph, or NHCH₂CH(CH₃)₂;
   R_{c} is NH₂, NHCH₂Ph, CH₂Ph, CH₂CH₂Ph, or CH₃;
   A¹ is H, C₍₁₋₆₎alkyl, Ph, or C(O)CH₃, CH₂Ph, C₍₁₋₄₎alkylOC₍₁₋₄₎alkyl;
   A² is H, C₍₁₋₆₎alkyl; or
   A¹ and A² may be taken together with the nitrogen to which they are attached to form a ring selected from the group consisting of: 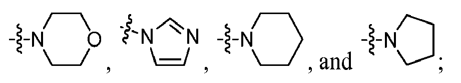
R⁴ is CH₂Ph, wherein said Ph is optionally substituted with up to two groups selected from CF₃, OCF₃, and F;
R⁵ is H; or R⁴ and R⁵ are taken together with their attached nitrogen to form a pair of fused rings selected from the group consisting of: 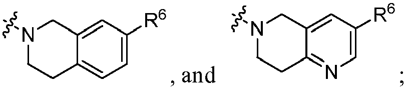 R⁶ is CF₃, or OCF₃;
Z¹ is CH₂ or C=O;
Z² is CH₂ or Z² may be C=O provided that Z¹ and Z² are not both simultaneously C=O; and pharmaceutically acceptable salts thereof.

In another embodiment of the invention:
R¹ is CH₂CH₂OCH₃, 3-C₍₁₋₂₎alkoxy tetrahydropyran-4-yl, or tetrahydropyran-4-yl;
R² is H, C(S)NHCH₂CH(CH₃)₂, or C(S)NHCH₃;
R³ is 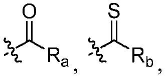 -SO₂-R_{c}, 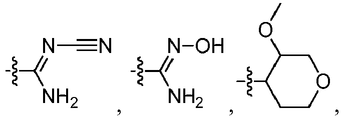 H, -CN, C₍₁₋₃₎alkyl,-CH₂C(O)N(CH₃)₂, cyclopropyl, oxetan-3-yl, -(CH₂)ₙPh, -CH₂CO₂CH₃, 4,5 dihydro thiazol-2-yl, 4,5 dihydro oxazol-2-yl, thiazol-2-yl, pyrimidin-2-yl, or 3-methyl 1,2,4 oxadiazol-5-yl;
   n is 0, 1, 2, or 3;
   Rₐ is H, NH₂, NHCH₃, N(CH₃)₂, NHCH₂CH(CH₃)₂, NHCH(CH₃)₂, NHCH₂C(CH₃)₃, NHCH₂CH₂OCH₃, NHCH₂CH₂NA₁A₂, NHPh, NHCH₂Ph, CH₂CH₂N(CH₃)₂, CH₂CH₂CH₂N(CH₃)₂, -CN, -CH₃, -CH₂CH₂Ph, -CH₂OPh, -CH₂OC(O)CH₃, -CH₂OCH₃,-CH₂OC(CH₃)₃, -CH₂NHC(O)CH₃, -CH₂N(CH₃)₂, -CH₂NHBoc, -OCH₃, -OCH₂C(CH₃)₃,-OCH₂CH(CH₃)₂, -OCH₂CH₂CH₃, -OCH₂Ph, -OCH₂Ph-CN, -OCH₂Ph-OCH₃,-OCH₂CH=CH₂, -OCH₂CH₂CF₃, -OCH₂CH₂C(CH₃)₂OH, -OCH₂CH₂OCH₃,-OCH₂CH₂CN, -OCH₂CH₂N(C₍₁₋₂₎alkyl)₂, -OCH₂CH₂NHC(O)CH₃, -OPh, 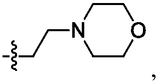 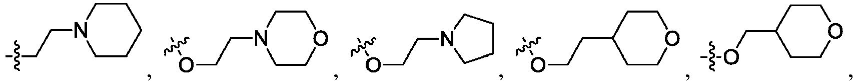 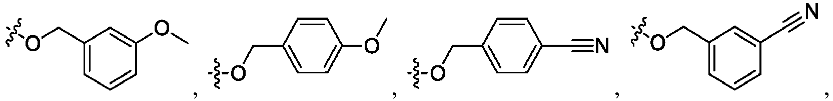 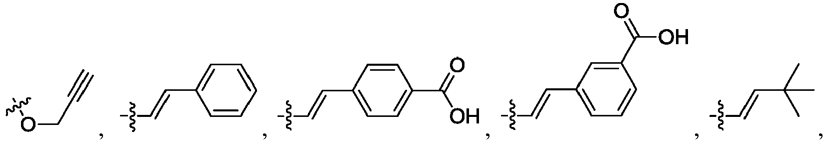 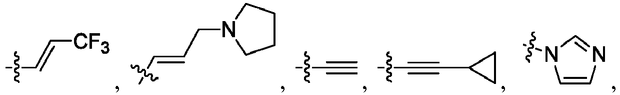 phenyl, oxazol-2-yl, oxazol-4-yl, isoxazol-5-yl, thiazol-2-yl, 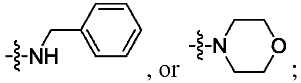 R_{b} is 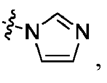 NH₂, NHCH₃, NHCH₂Ph, or NHCH₂CH(CH₃)₂;
   R_{c} is NH₂, NHCH₂Ph, CH₂Ph, CH₂CH₂Ph, or CH₃;
R⁴ is 1,3-bis(trifluoromethyl)benz-5-yl, 1-fluoro-3-(trifluoromethyl)benz-5-yl, or 1-(trifluoromethyl)benz-5-yl;
R⁵ is H; or R⁴ and R⁵ are taken together with their attached nitrogen to form a pair of fused rings selected from the group consisting of: 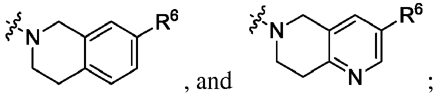 R⁶ is CF₃, or OCF₃;
Z¹ is CH₂ or C=O;
Z² is CH₂ or Z² may be C=O provided that Z¹ and Z² are not both simultaneously C=O; and pharmaceutically acceptable salts thereof.

Another embodiment of the invention comprises a compound of Formula (I), selected from the group consisting of: 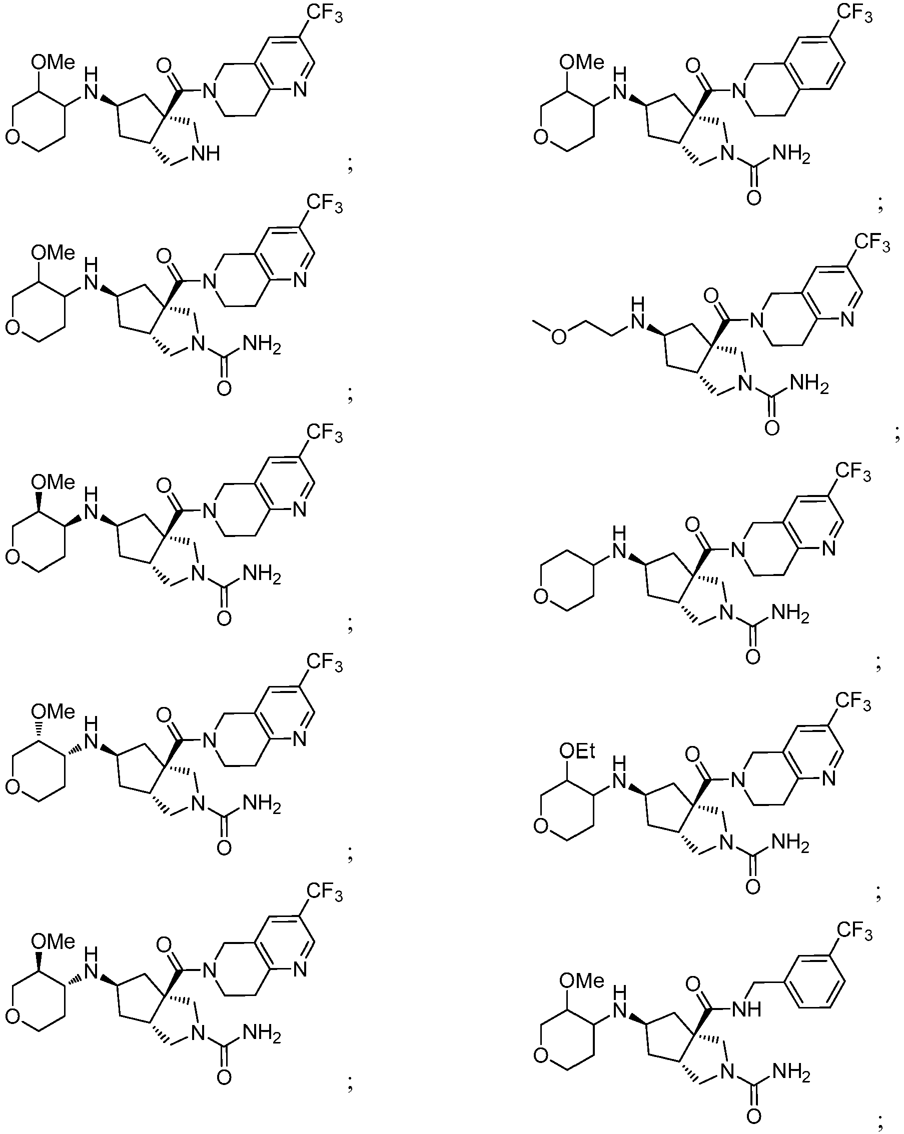 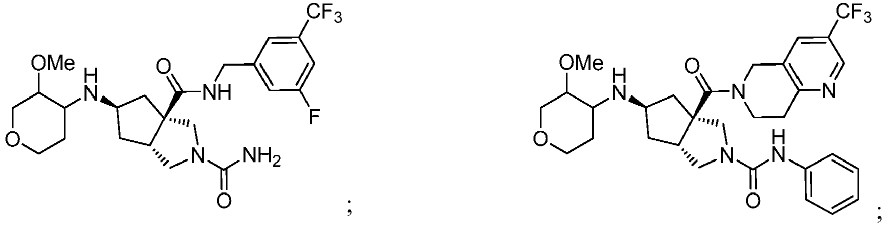 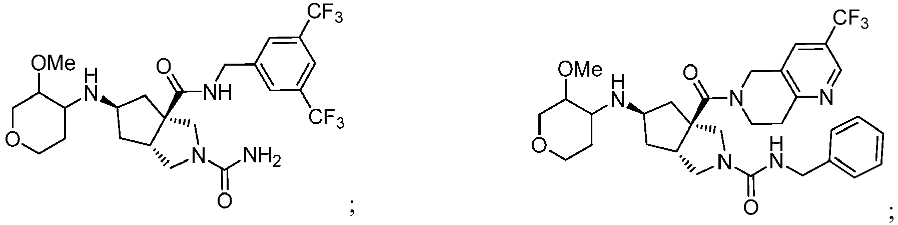 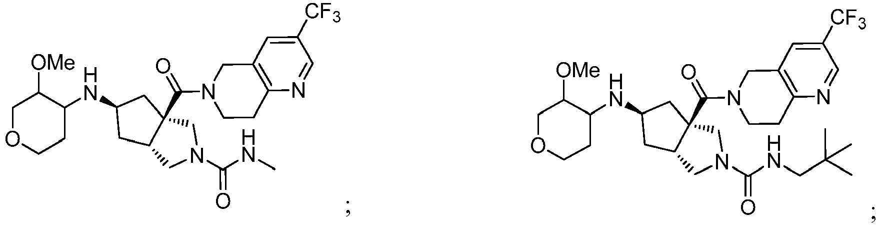 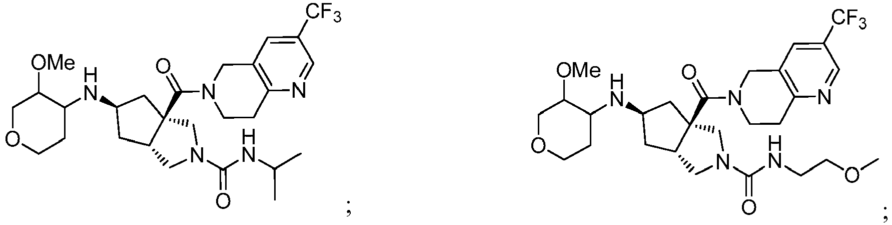 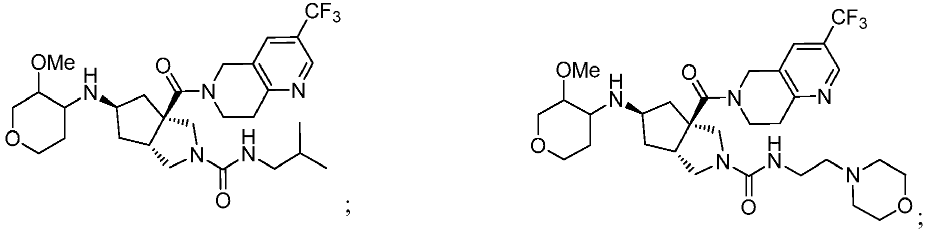 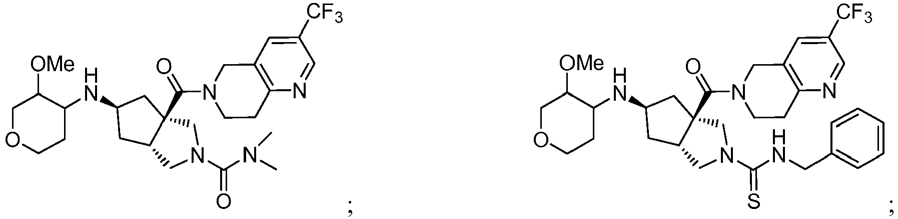 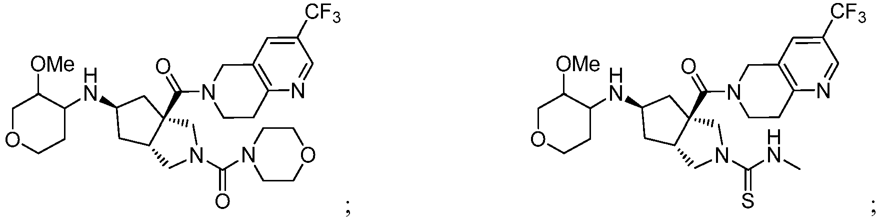 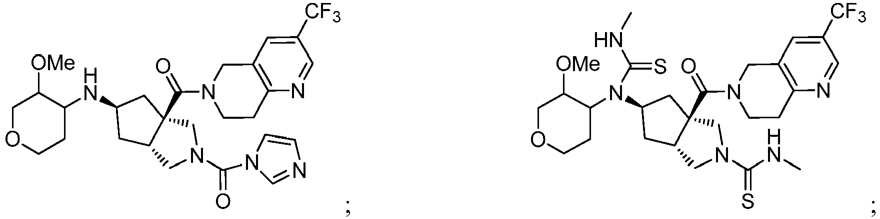 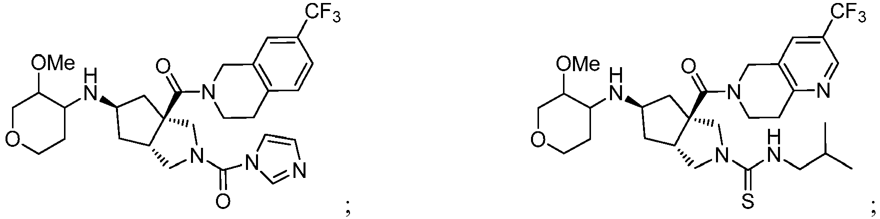 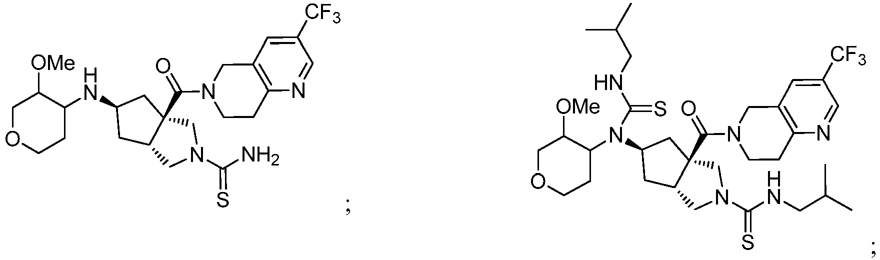 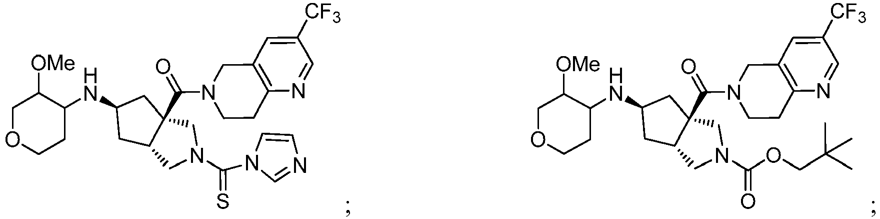 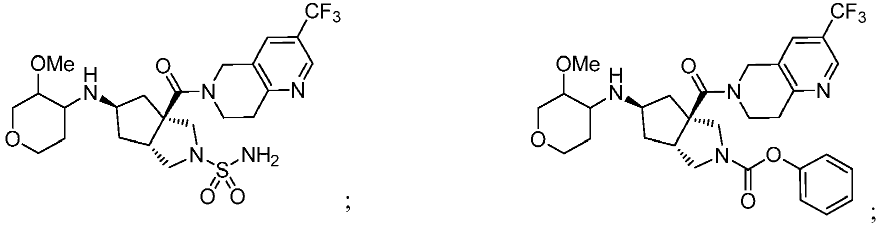 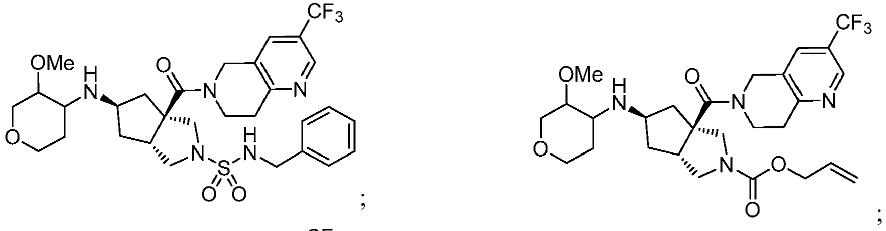 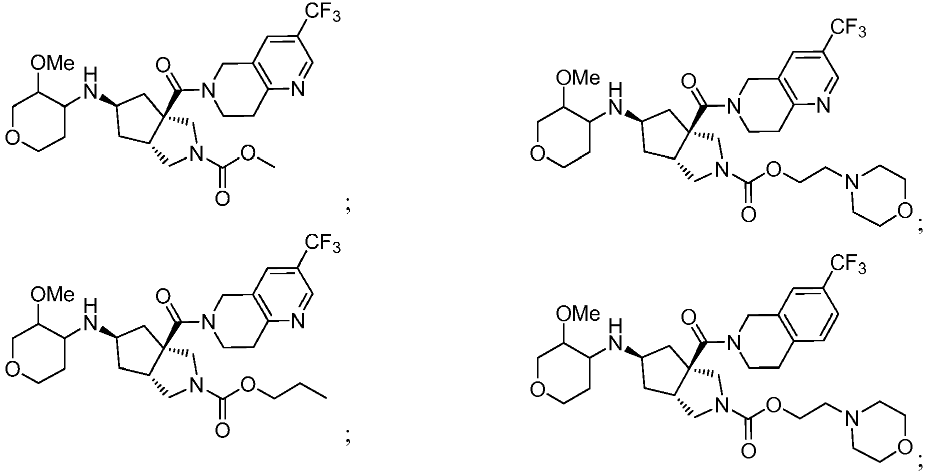 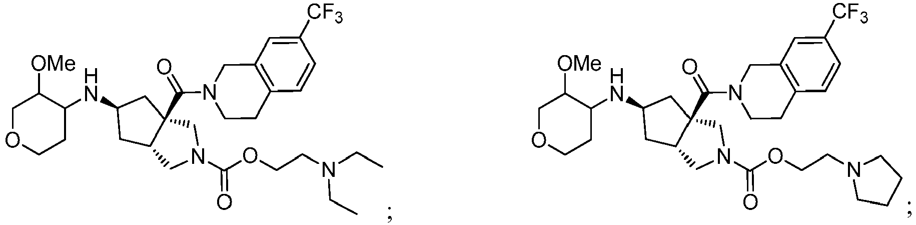 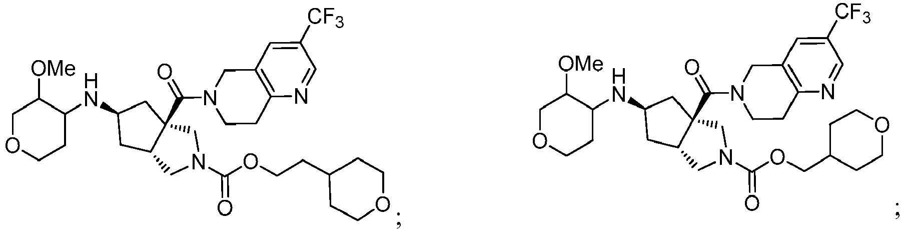 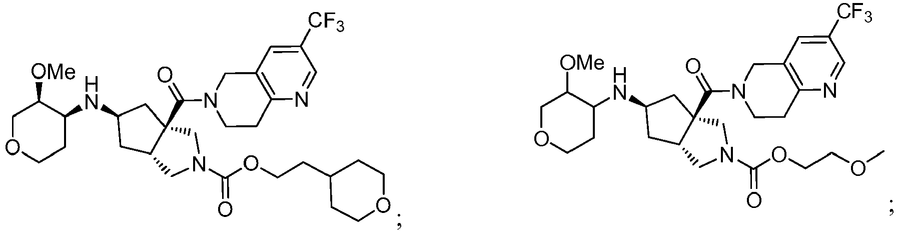 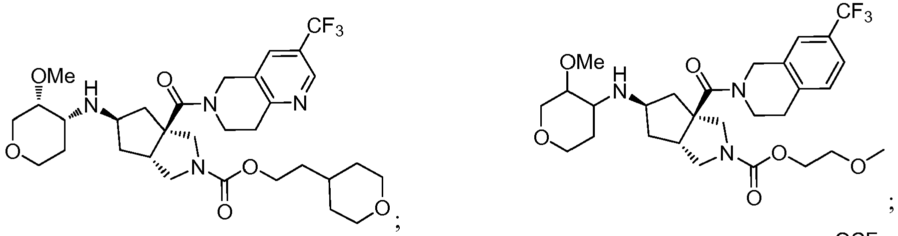 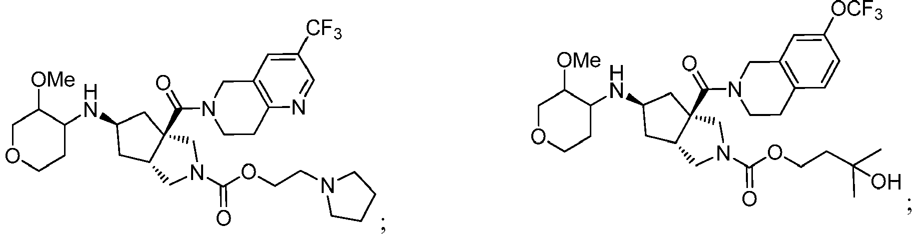 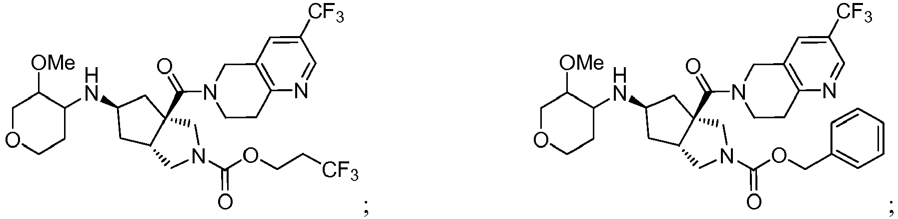 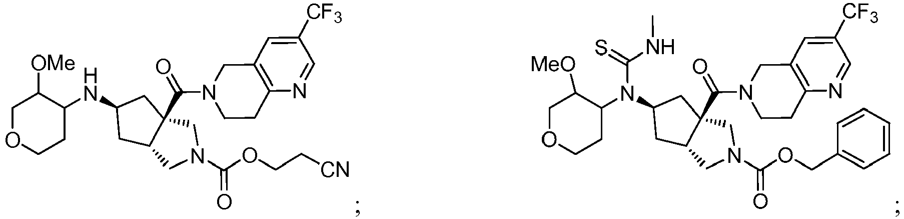 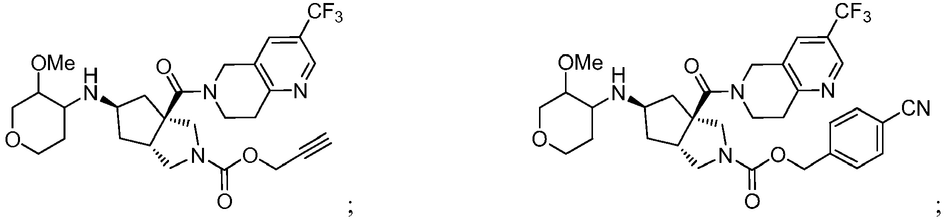 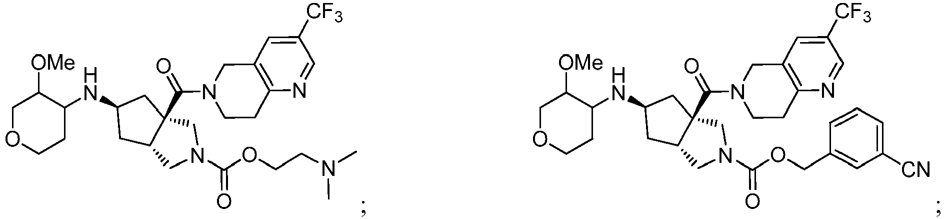 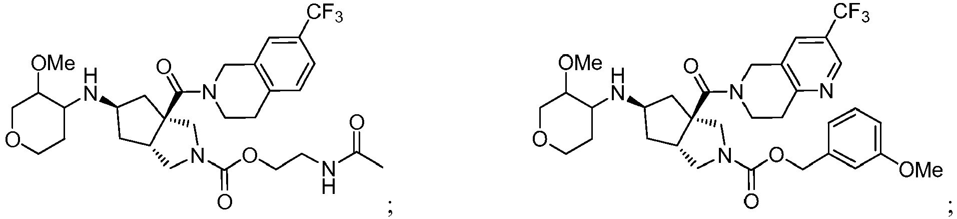 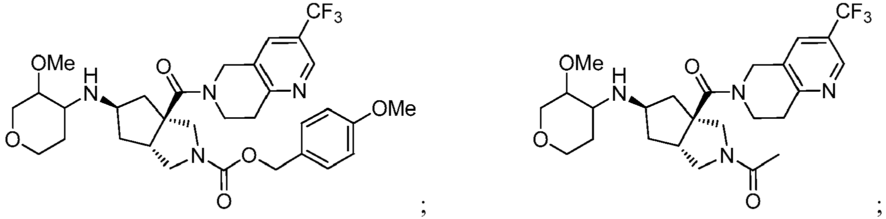 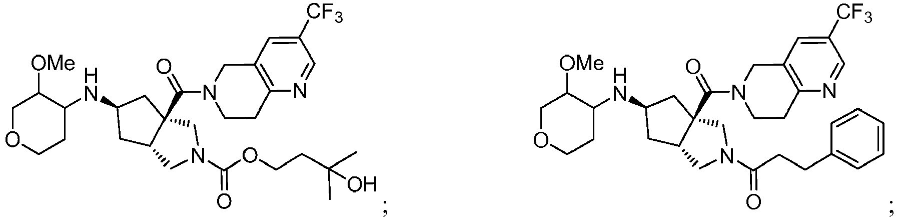 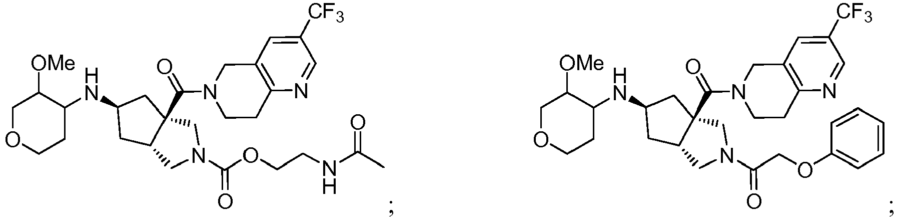 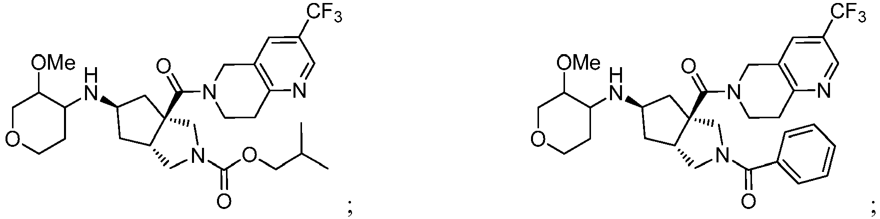 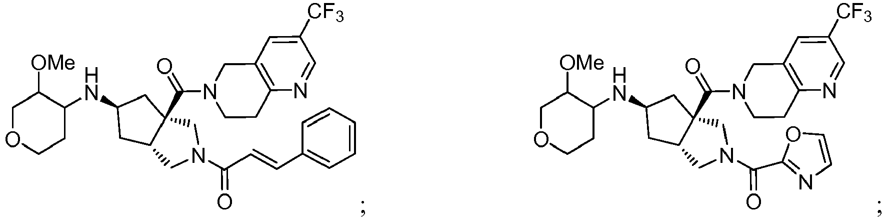 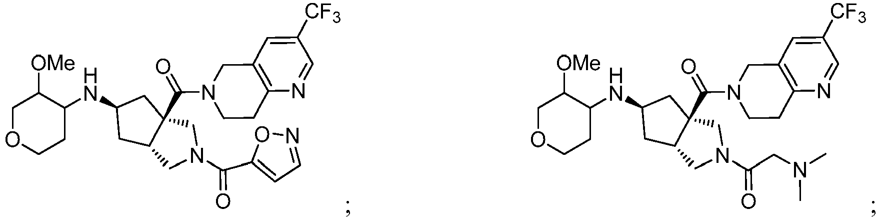 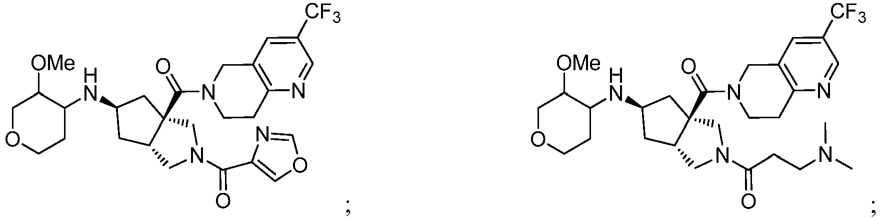 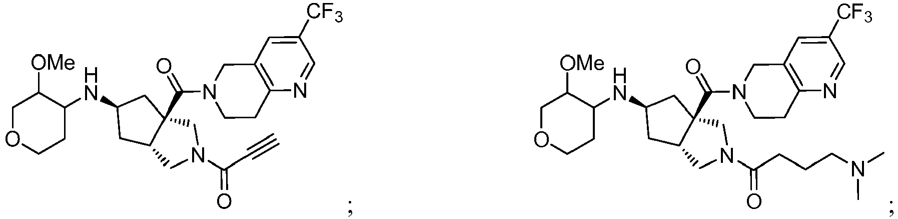 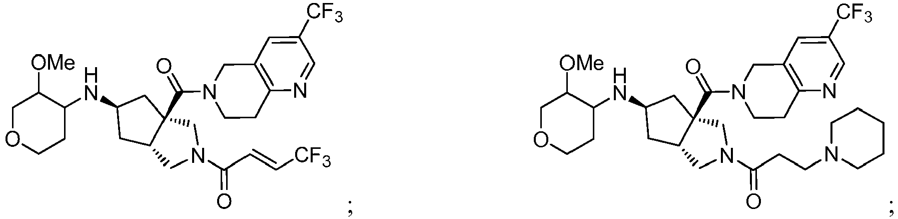 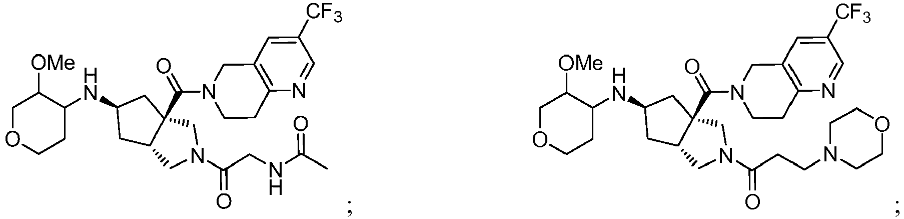 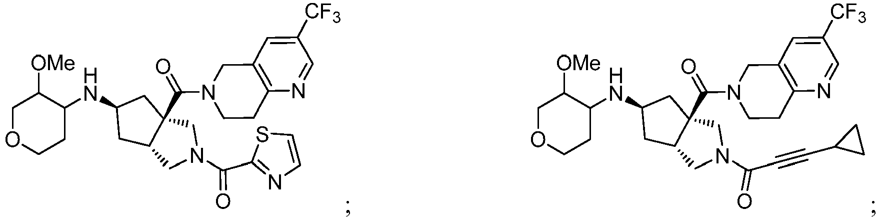 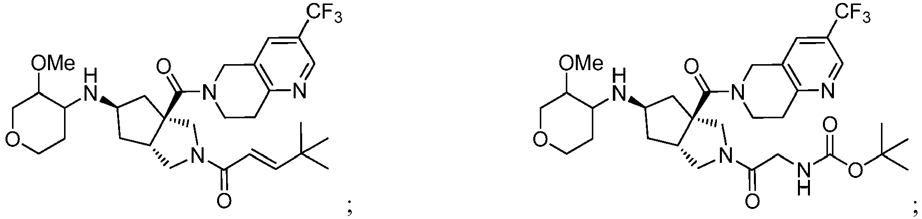 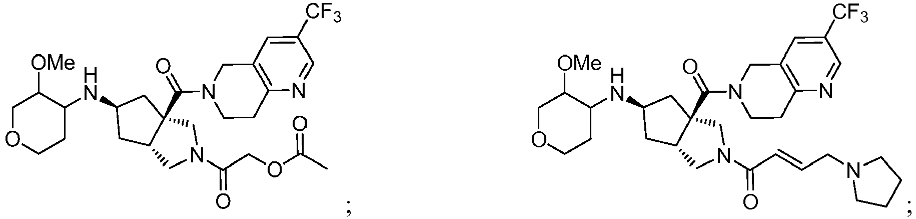 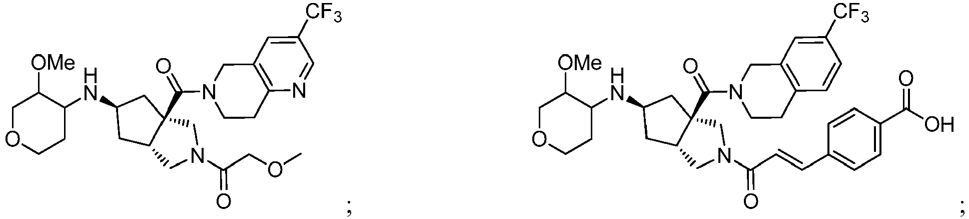 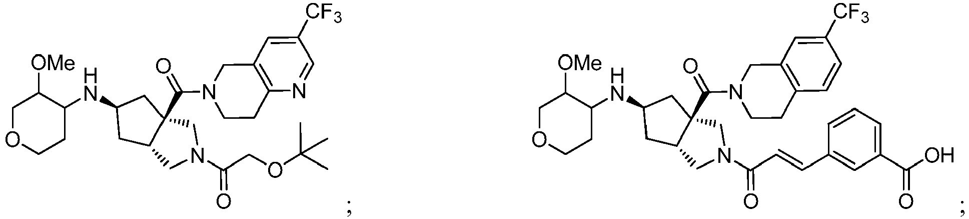 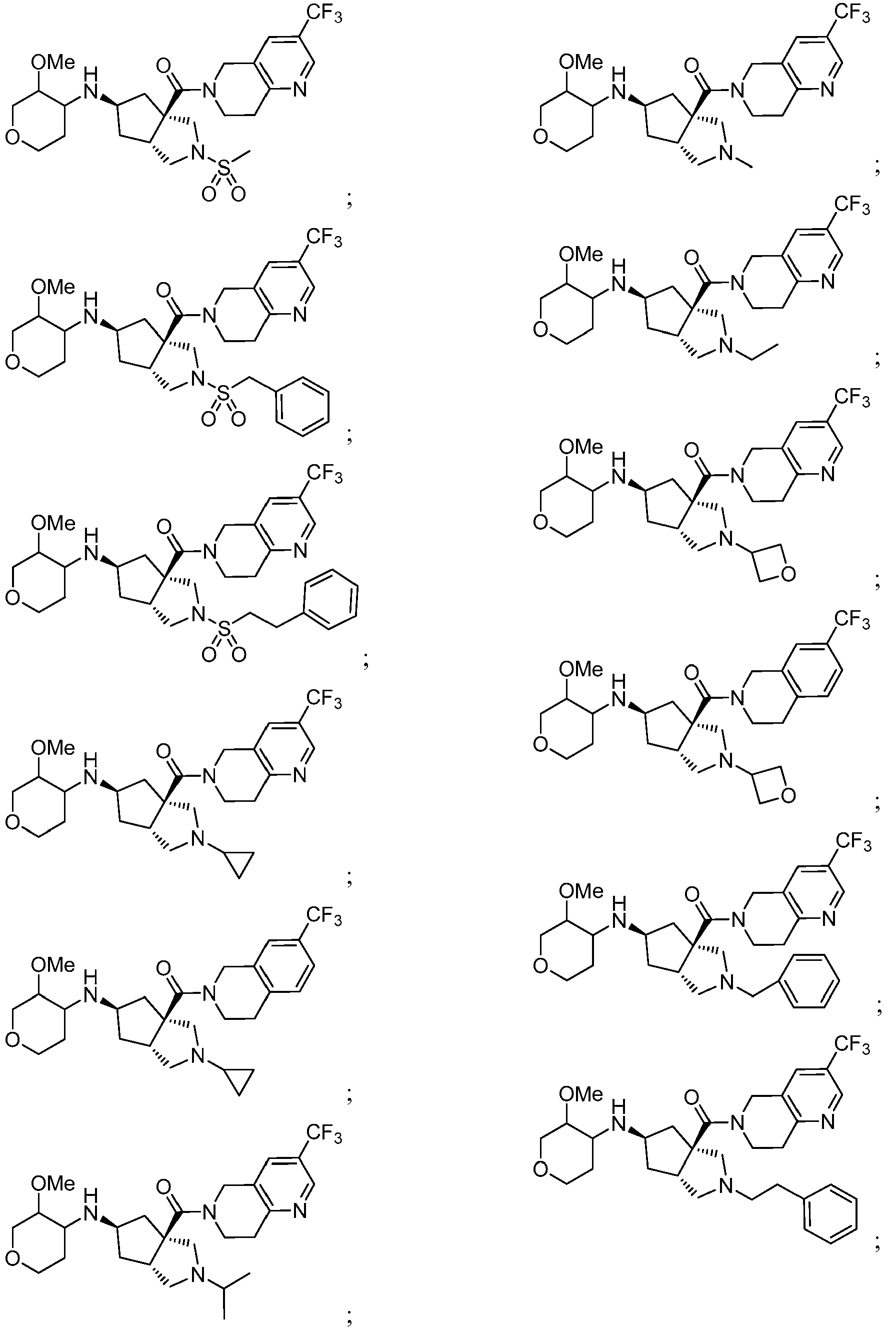 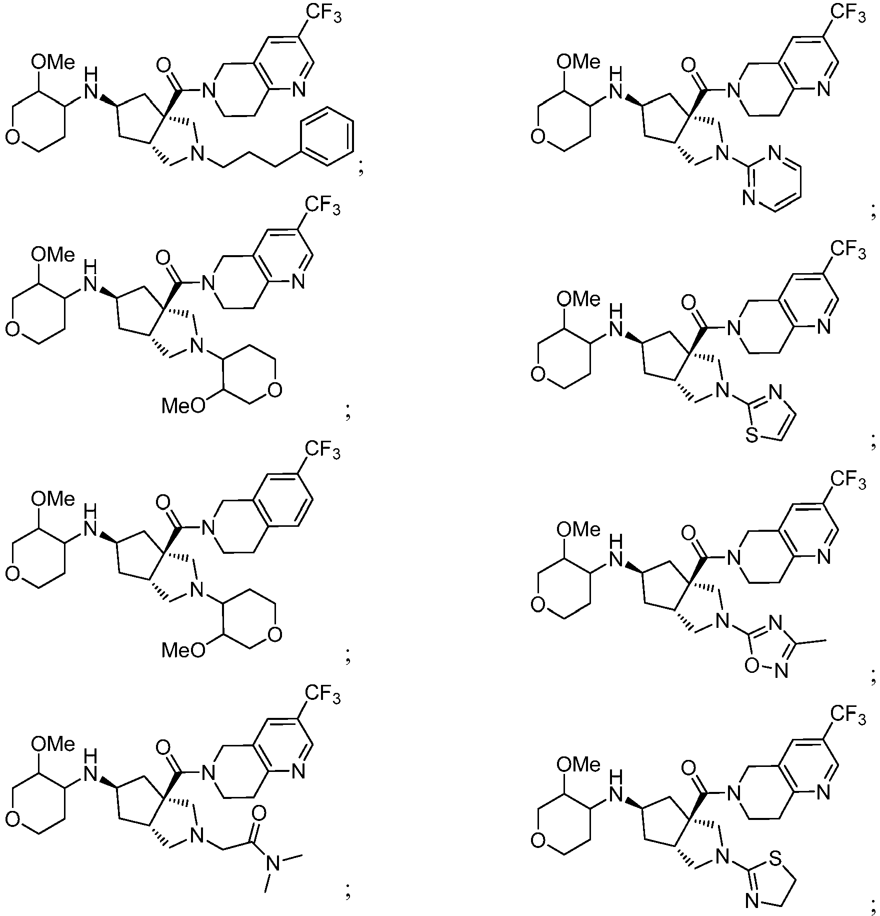 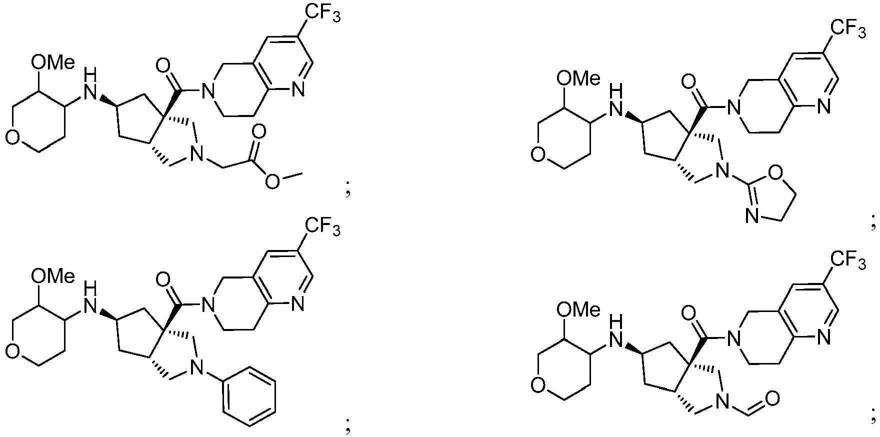 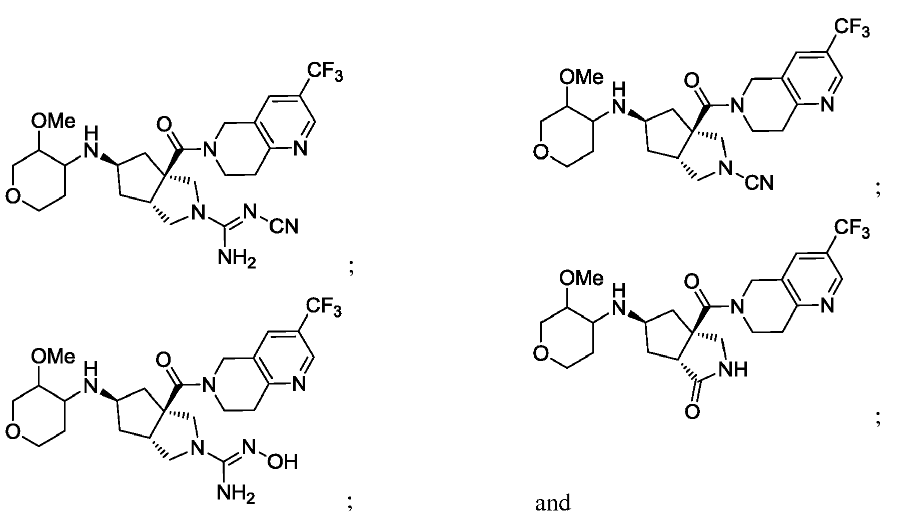 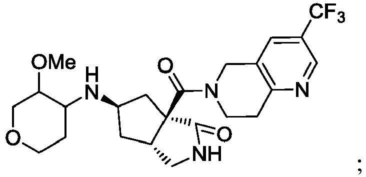 and pharmaceutically acceptable salts thereof.

In another embodiment, the Invention relates to a pharmaceutical composition, comprising a compound of formula (I) and a pharmaceutically acceptable carrier.

In another embodiment, the invention relates to a pharmaceutical composition made by mixing a compound of formula (I) and a pharmaceutically acceptable carrier.

In another embodiment, the invention relates to a process for making a pharmaceutical composition comprising mixing a compound of formula (I) and a pharmaceutically acceptable carrier.

The present invention is further directed to a product prepared according to any of the processes described herein.

In another embodiment, the present disclosure is directed to a process for the preparation of a compound of formula (I), as described in more detail in the Schemes and Examples which follow herein.

In another embodiment, the disclosure relates to a method for preventing, treating or ameliorating a CCR2 mediated syndrome, disorder or disease comprising administering to a subject in need thereof a therapeutically effective amount of a compound of formula (I).

In another embodiment, the disclosure relates to a method for preventing, treating or ameliorating a CCR2 mediated inflammatory syndrome, disorder or disease wherein the syndrome, disorder or disease is associated with elevated MCP-1 expression or MCP-1 overexpression, or is an inflammatory condition that accompanies syndromes, disorders or diseases associated with elevated MCP-1 expression or MCP-1 overexpression comprising administering to a subject in need thereof an effective amount of a compound of claim 1.

In another embodiment, the invention relates to a compound of formula (I) for use in a method of preventing, treating or ameliorating a syndrome, disorder or disease, wherein said syndrome, disorder or disease is selected from the group consisting of: Chronic Obstructive Pulmonary Disease (COPD), ophthalmic disorders, uveitis, atherosclerosis, rheumatoid arthritis, psoriasis, psoriatic arthritis, atopic dermatitis, multiple sclerosis, Crohn's Disease, ulcerative colitis, nephritis, organ allograft rejection, fibroid lung, renal insufficiency, type-I diabetes, type II diabetes, diabetic complications, diabetic nephropathy, diabetic retinopathy, diabetic retinitis, diabetic microangiopathy, overweight, obesity, obesity-associated insulin resistance, metabolic syndrome, tuberculosis, sarcoidosis, invasive *staphyloccocia,* inflammation after cataract surgery, allergic rhinitis, allergic conjunctivitis, chronic urticaria, asthma, allergic asthma, periodontal diseases, periodonitis, gingivitis, gum disease, diastolic cardiomyopathies, cardiac infarction, myocarditis, chronic heart failure, angiostenosis, restenosis, reperfusion disorders, aortic abdominal aneurism, glomerulonephritis, solid tumors and cancers, chronic lymphocytic leukemia, chronic myelocytic leukemia, multiple myeloma, malignant myeloma, Hodgkin's disease, and carcinomas of the bladder, breast, cervix, colon, lung, prostate, or stomach and chronic neuroinflammatory disorders including, but not limited to, Alzheimer's disease, ischemic stroke, spinal cord injury, nerve crush injury and traumatic brain injury wherein said method comprises administering to a subject in need thereof an effective amount of a compound of formula (I).

In another embodiment, the invention relates to a compound of formula (I) for use in a method of preventing, treating or ameliorating a syndrome, disorder or disease, wherein said syndrome, disorder or disease is selected from the group consisting of: type I diabetes, type II diabetes, diabetic complications, diabetic nephropathy, diabetic retinopathy, diabetic retinitis, diabetic microangiopathy, obesity, obesity-associated insulin resistance, metabolic syndrome, asthma, and allergic asthma, wherein said method comprises administering to a subject in need thereof a therapeutically effective amount of a compound of formula (I).

In another embodiment, the invention relates to a compound of formula (I) for use in a method of treating a disorder selected from the group consisting of type II diabetes, obesity and asthma, wherein said method comprises administering to a subject in need thereof a therapeutically effective amount of a compound of formula (I).

### DEFINITIONS

The term "alkyl" refers to both linear and branched chain radicals of up to 12 carbon atoms, preferably up to 6 carbon atoms, unless otherwise indicated, and includes, but is not limited to, methyl, ethyl, propyl, isopropyl, butyl, isobutyl, sec-butyl, tert-butyl, pentyl, isopentyl, hexyl, isohexyl, heptyl, octyl, 2,2,4-trimethylpentyl, nonyl, decyl, undecyl and dodecyl.

The term "C_{(*a*-*b*)}" (where *a* and *b* are integers referring to a designated number of carbon atoms) refers to an alkyl, alkenyl, alkynyl, alkoxy or cycloalkyl radical or to the alkyl portion of a radical in which alkyl appears as the prefix root containing from *a to b* carbon atoms inclusive. For example, C₍₁₋₄₎ denotes a radical containing 1, 2, 3 or 4 carbon atoms.
The term "cycloalkyl" refers to a saturated or partially unsaturated monocyclic or bicyclic hydrocarbon ring radical derived by the removal of one hydrogen atom from a single ring carbon atom. Examples of cycloalkyl radicals include, but are not limited to cyclopropyl, cyclobutyl, cyclopentyl, cyclopentenyl, cyclohexyl, cyclohexenyl, cycloheptyl and cyclooctyl. Additional examples include C₍₃₋₈₎cycloalkyl, C₍₅₋₈₎cycloalkyl, C₍₃₋₁₂₎cycloalkyl, C₍₃₋₂₀₎cycloalkyl, decahydronaphthalenyl, and 2,3,4,5,6,7-hexahydro-1H-indenyl.

The term "heteroaryl" refers to a radical derived by the removal of one hydrogen atom from a ring carbon atom of a heteroaromatic ring system. A heteroaromatic ring system shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure. Examples of heteroaryl radicals include, but are not limited to, furyl, thienyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, indolizinyl, indolyl, isoindolyl, benzo[*b*]furyl, benzo[*b*]thienyl, indazolyl, benzimidazolyl, benzthiazolyl, purinyl, 4*H-*quinolizinyl, quinolinyl, isoquinolinyl, cinnolinyl, phthalzinyl, quinazolinyl, quinoxalinyl, 1,8-naphthyridinyl, and pteridinyl.

For use in medicines, the salts of the compounds of this invention refer to non-toxic "pharmaceutically acceptable salts." FDA approved pharmaceutically acceptable salt forms (Ref. International J. Pharm. 1986, 33, 201-217; J. Pharm. Sci., 1977, Jan, 66(1), p1) include pharmaceutically acceptable acidic/anionic or basic/cationic salts.

Throughout this specification, compounds are described as being separated, usually by silica gel column, although preparatory thin layer chromatography, or high or low pressure liquid chromatography may also be used. It is generally accepted that when eluting compounds through a silica gel-type separation medium, that the least polar compounds elute before the more polar compounds. Therefore, the term "less polar isomer", refers to the isomer that will elute first from a silica gel type separation medium.

### ABBREVIATIONS

Herein and throughout this application, the following abbreviations may be used.
- ACN: acetonitrile
- BOC or Boc: tert-butyloxycarbonyl
- BOP: (Benzotriazole-1-yl-oxy-tris-(dimethylamino)-phosphonium hexafluorophosphate)
- DBU: diazabicycloundecene
- DCC: dicyclohexylcarbodiimide
- DCM: dichloromethane
- DIAD: diisopropylazodicarboxylate
- DIPEA: diisopropylethylamine
- DMAP: dimethylaminopyridine
- DME: dimethoxyethane
- DMF: dimethylformamide
- DMSO: dimethylsulfoxide
- EDAC: 1-Ethyl-3-[3-dimethylaminopropyl]carbodiimide hydrochloride
- Et: ethyl
- EtOAc: ethyl acetate
- eq: equivalents
- HOBt: hydroxybenzotriazole
- M: moles/liter
- Me: methyl
- min.: minutes
- OAc: acetate
- Ph: phenyl
- PyBop: Benzotriazol-1-yloxy)tripyrrolidinophosphonium Hexafluorophosphate
- PyBrop: bromo-tris-pyrrolidinophosphonium hexafluorophosphate
- rt: room temperature
- TEA: triethylamine
- TFA: trifluoroacetic acid
- THF: tetrahydrofuran
- TLC: thin layer chromatography
- TPP: triphenyl phosphine

Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate and triethiodide. Organic or inorganic acids also include, and are not limited to, hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic or trifluoroacetic acid.

Pharmaceutically acceptable basic/cationic salts include, and are not limited to aluminum, 2-amino-2-hydroxymethyl-propane-1,3-diol (also known as tris(hydroxymethyl)aminomethane, tromethane or "TRIS"), ammonia, benzathine, *t*-butylamine, calcium, calcium gluconate, calcium hydroxide, chloroprocaine, choline, choline bicarbonate, choline chloride, cyclohexylamine, diethanolamine, ethylenediamine, lithium, LiOMe, L-lysine, magnesium, meglumine, NH₃, NH₄OH, N-methyl-D-glucamine, piperidine, potassium, potassium-*t*-butoxide, potassium hydroxide (aqueous), procaine, quinine, sodium, sodium carbonate, sodium-2-ethylhexanoate (SEH), sodium hydroxide, triethanolamine or zinc.

### METHODS OF USE

Disclosed is a method for preventing, treating or ameliorating a CCR2 mediated syndrome, disorder or disease comprising administering to a subject in need thereof an effective amount of a compound of Formula (I) or a form, composition or medicament thereof.

Examples of a CCR2 mediated syndrome, disorder or disease for which the compounds of Formula (I) are useful include chronic obstructive pulmonary disorder (COPD), ophthalmic disorders, uveitis, atherosclerosis, rheumatoid arthritis, psoriasis, psoriatic arthritis, atopic dermatitis, multiple sclerosis, Crohn's Disease, ulcerative colitis, nephritis, organ allograft rejection, fibroid lung, renal insufficiency, type-I diabetes, type II diabetes, diabetic complications, diabetic nephropathy, diabetic retinopathy, diabetic retinitis, diabetic microangiopathy, overweight, obesity, obesity-associated insulin resistance, metabolic syndrome, tuberculosis, chronic obstructive pulmonary disease, sarcoidosis, invasive *staphyloccocia,* inflammation after cataract surgery, allergic rhinitis, allergic conjunctivitis, chronic urticaria, asthma, allergic asthma, periodontal diseases, periodonitis, gingivitis, gum disease, diastolic cardiomyopathies, cardiac infarction, myocarditis, chronic heart failure, angiostenosis, restenosis, reperfusion disorders, aortic abdominal aneurism, multiple sclerosis, glomerulonephritis, solid tumors and cancers, chronic lymphocytic leukemia, chronic myelocytic leukemia, multiple myeloma, malignant myeloma, Hodgkin's disease, carcinomas of the bladder, breast, cervix, colon, lung, prostate, or stomach, and chronic neuroinflammatory disorders including, but not limited to, Alzheimer's disease, ischemic stroke, spinal cord injury, nerve crush injury and traumatic brain injury.

Some of the quantitative expressions given herein are qualified with the term "about". It is understood that whether the term "about" is used explicitly or not, every quantity given herein is meant to refer to both the actual given value and the approximation to such given value that would reasonably be inferred based on the ordinary skill in the art, including approximations due to the experimental and/or measurement conditions for such given value. In addition, some of the quantitative expressions herein are recited as a range from about amount X to about amount Y. It is understood that wherein a range is recited, the range is not limited to the recited upper and lower bounds, but rather includes the full range from about amount X through about amount Y, or any range therein.

The term "administering" with respect to the methods of the invention, means a method for therapeutically or prophylactically preventing, treating or ameliorating a syndrome, disorder or disease as described herein by using a compound of Formula (I) or a form, composition or medicament thereof. Such methods include administering an effective amount of said compound, compound form, composition or medicament at different times during the course of a therapy or concurrently in a combination form. The methods of the invention are to be understood as embracing all known therapeutic treatment regimens.

The term "subject" refers to a patient, which may be animal, typically a mammal, typically a human, which has been the object of treatment, observation or experiment. In one aspect of the invention, the subject is at risk of (or susceptible to) developing a syndrome, disorder or disease that is associated with elevated MCP-1 expression or MCP-1 overexpression, or a patient with an inflammatory condition that accompanies syndromes, disorders or diseases associated with elevated MCP-1 expression or MCP-1 overexpression.

The term "therapeutically effective amount" means that amount of active compound or pharmaceutical agent that elicits the biological or medicinal response in a tissue system, animal or human, that is being sought by a researcher, veterinarian, medical doctor, or other clinician, which includes preventing, treating or ameliorating the symptoms of a syndrome, disorder or disease being treated.

The term "uveitis" generically refers to any inflammatory disease involving the eye. Uveitis can be divided into clinically distinct subtypes based on the part of the eye in which the inflammation is present (percentages correspond to patients known to fit these categories): anterior (51%), intermediate (13%), posterior (20%), or panuveitis (16%) and, according to the course of the disease, as either acute (16%), recurring (26%), or chronic (58%). Those with anterior uveitis (·19%) eventually develop irreparable vision damage despite aggressive treatment such as unilateral blindness (9%), bilateral blindness (2%), or unilateral or bilateral vision impairment (8%). Most cases of uveitis are idiopathic, but known causes include infection (e.g., toxoplasmosis, cytomegalovirus, and the like) or development as a component of a systemic inflammatory and/or autoimmune disorder (e.g., juvenile RA, HLA-B27 associated spondyloarthropathies, sarcoidosis, and the like). (HLA-B27: Human Leukocyte Antigen B*27- is a class I surface antigen encoded by the B locus in the major histocompatibility complex (MHC) on chromosome 6 and presents microbial antigens to T cells. HLA-B27 is strongly associated with a certain set of autoimmune diseases referred to as the seronegative spondyloarthropathies.)

When employed as CCR2 inhibitors, the compounds of the invention may be administered in an effective amount within the dosage range of about 0.5 mg to about 10 g, or any amount or range therein, preferably between about 0.5 mg to about 5 g, or any amount or range therein, in single or divided daily doses. The dosage administered will be affected by factors such as the route of administration, the health, weight and age of the recipient, the frequency of the treatment and the presence of concurrent and unrelated treatments.

It is also apparent to one skilled in the art that the therapeutically effective dose for compounds of the present invention or a pharmaceutical composition thereof will vary according to the desired effect. Therefore, optimal dosages to be administered may be readily determined by one skilled in the art and will vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level. The above dosages are thus exemplary of the average case. There can, of course, be individual instances where higher or lower dosage ranges are merited, and such are within the scope of this invention.

The compounds of Formula (I) may be formulated into pharmaceutical compositions comprising any known pharmaceutically acceptable carriers. Exemplary carriers include, but are not limited to, any suitable solvents, dispersion media, coatings, antibacterial and antifungal agents and isotonic agents. Exemplary excipients that may also be components of the formulation include fillers, binders, disintegrating agents and lubricants.

The pharmaceutically-acceptable salts of the compounds of Formula (I) include the conventional non-toxic salts or the quaternary ammonium salts which are formed from inorganic or organic acids or bases. Examples of such acid addition salts include acetate, adipate, benzoate, benzenesulfonate, citrate, camphorate, dodecylsulfate, hydrochloride, hydrobromide, lactate, maleate, methanesulfonate, nitrate, oxalate, pivalate, propionate, succinate, sulfate and tartrate. Base salts include ammonium salts, alkali metal salts such as sodium and potassium salts, alkaline earth metal salts such as calcium and magnesium salts, salts with organic bases such as dicyclohexylamino salts and salts with amino acids such as arginine. Also, the basic nitrogen-containing groups may be quaternized with, for example, alkyl halides.

The pharmaceutical compositions of the invention may be administered by any means that accomplish their intended purpose. Examples include administration by parenteral, subcutaneous, intravenous, intramuscular, intraperitoneal, transdermal, buccal or ocular routes. Alternatively or concurrently, administration may be by the oral route. Suitable formulations for parenteral administration include aqueous solutions of the active compounds in water-soluble form, for example, water-soluble salts, acidic solutions, alkaline solutions, dextrose-water solutions, isotonic carbohydrate solutions and cyclodextrin inclusion complexes.

The present invention also encompasses a method of making a pharmaceutical composition comprising mixing a pharmaceutically acceptable carrier with any of the compounds of the present invention. Additionally, the present invention includes pharmaceutical compositions made by mixing a pharmaceutically acceptable carrier with any of the compounds of the present invention. As used herein, the term "composition" is intended to encompass a product comprising the specified ingredients in the specified amounts, as well as any product which results, directly or indirectly, from combinations of the specified ingredients in the specified amounts.

### POLYMORPHS AND SOLVATES

Furthermore, the compounds of the present invention may have one or more polymorph or amorphous crystalline forms and as such are intended to be included in the scope of the invention. In addition, the compounds may form solvates, for example with water (i.e., hydrates) or common organic solvents. As used herein, the term "solvate" means a physical association of the compounds of the present invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. The term "solvate" is intended to encompass both solution-phase and isolatable solvates. Non-limiting examples of suitable solvates include ethanolates, methanolates, and the like.

It is intended that the present invention include within its scope polymorphs and solvates of the compounds of the present invention. Thus, in the disclosed methods of treatment, the term "administering" shall encompass the means for treating, ameliorating or preventing a syndrome, disorder or disease described herein with the compounds of the present invention or a polymorph or solvate thereof, which would obviously be included within the scope of the invention albeit not specifically disclosed.

In another embodiment, the invention relates to a compound as described in the Examples of Formula (I) for use as a medicament.

In another embodiment, the disclosure relates to the use of a compound as described in the Examples of Formula (I) for the preparation of a medicament for the treatment of a disease associated with an elevated or inappropriate CCR2 activity.

Further disclosed are prodrugs of the compounds of this invention. In general, such prodrugs will be functional derivatives of the compounds which are readily convertible *in vivo* into the required compound. Conventional procedures for the selection and preparation of suitable prodrug derivatives are described, for example, in "Design of Prodrugs", Ed. H. Bundgaard, Elsevier, 1985.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

### GENERAL REACTION SCHEME

Representative compounds of the present invention can be synthesized in accordance with the general synthetic methods described below and are illustrated more particularly in the schemes that follow. Since the schemes are an illustration, the invention should not be construed as being limited by the chemical reactions and conditions expressed. The preparation of the various starting materials used in the schemes is well within the skill of persons versed in the art. The following schemes describe general synthetic methods whereby intermediate and target compounds of the present invention may be prepared. Additional representative compounds and stereoisomers, racemic mixtures, diastereomers and enantiomers thereof can be synthesized using the intermediates prepared in accordance to the general schemes and other materials, compounds and reagents known to those skilled in the art. All such compounds, stereoisomers, racemic mixtures, diastereomers and enantiomers thereof are intended to be encompassed within the scope of the present invention.

Certain intermediates may be prepared according to the process outlined in Schemes A-FF below.

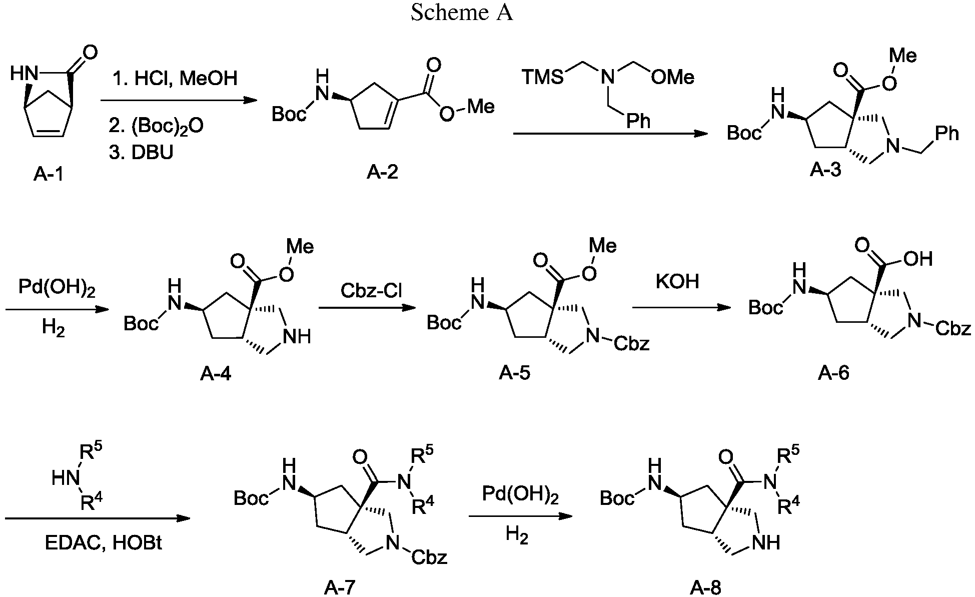

Ester A-2 may be made from commercially available A-1 (Aldrich) by a three step procedure described by Smith, M. E. B. et. al., Tetrahedron Lett. 2001, 42(7), 1347-50. Using the procedure described in PCT Int. Appl., 2006012396, 02 Feb 2006, pp 22-23, a solution of A-2 and commercially available *N*-(methoxymethyl)-*N-*(trimethylsilylmethyl)benzylamine in an organic solvent such as DCM may be treated with a dilute (for example 0.5%) solution of TFA in the same solvent at a temperature in the range of about 0 °C to about 25 °C to give compound A-3. Hydrogenation of A-3 in the present of a catalyst such as Pd(OH)₂ or 5-10% Pd/C in an organic solvent such as methanol or ethanol at a temperature in the range of about 25 °C to about 50 °C at the pressure in the range of about 101.3 kPa (1 atm) to 304.0 kPa (3 atm) yielded amine A-4. The amine A-4 may be then treated with commercially available benzyl chloroformate in the presence of an organic base such as triethylamine or diethylpropylamine, in an organic solvent such as dichloromethane or THF at a temperature in the range of about 0 °C to about 25 °C to give Compound A-5. The carboxylic acid A-5 may be obtained by saponification of A-6 with an aqueous inorganic base such as KOH or NaOH in an organic solvent such methanol or THF at a temperature in the range of about 0 °C to about 25 °C and then coupled with suitable, commercially available amines HNR⁴R⁵ in the presence of coupling reagents such as EDAC/HOBt, PyBOP, PyBrop, or DCC in an organic solvent such as THF, dichloromethane or 1,2-dichloroethane, at a temperature in the range of about 0 °C to about 25 °C to give the amide A-7. The resulting amide A-7 may be treated with hydrogen gas under pressure from 101.3kPa (1 atm) to 304.0kPa (3 atm), catalyzed by a catalyst such as 5-10% Pd/C, in an organic solvent such as methanol, ethanol, ethyl acetate or THF, at a temperature in the range of about 0 °C to about 25 °C, to yield intermediate A-8.

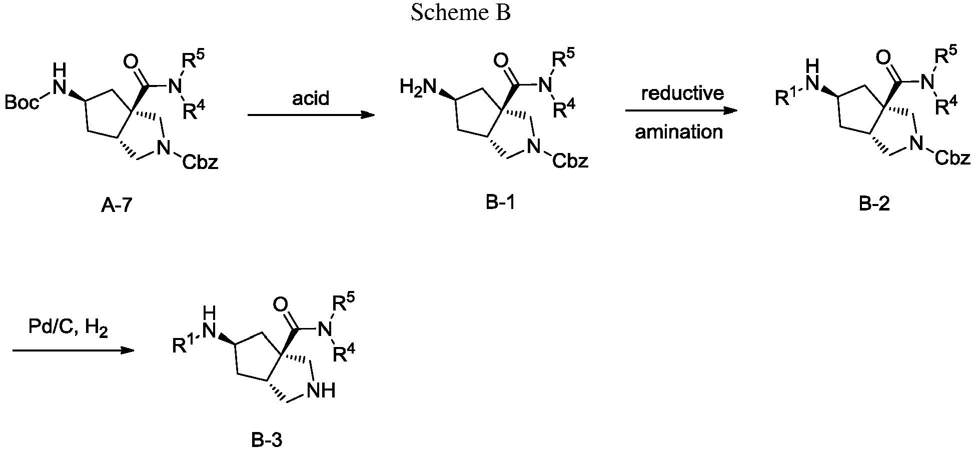

The Boc protecting group on compound A-7 may be removed using acids such as TFA or HCl according to reported protocols in the scientific literature to form a corresponding salt of compound B-1, which may be substituted using reductive amination with an appropriately substituted aldehyde or ketone in the presence of a hydride source, such as sodium borohydride or sodium triacetoxyborohydride, to provide compounds of formula B-2. A Cbz protecting in formula B-2 may be removed by, for instance, hydrogenation in the presence of a palladium catalyst, to afford a required intermediate amine of formula B-3.

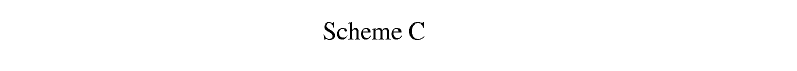

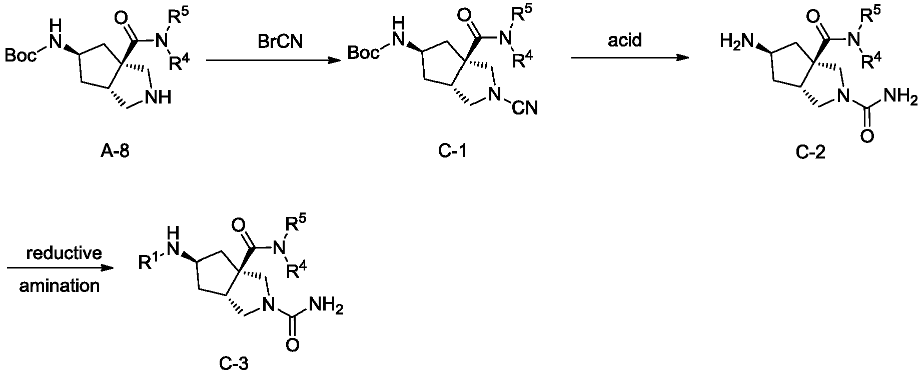

Compound A-8 may be cyanated by suitable cyanogen reagents such as cyanogen bromide to give a compound of formula C-1(Garbrecht, W. L.; Herbst, R. M. J. Org. Chem., 1953, 18, 1003-1013). Treatment with an acid such as TFA will remove the Boc protecting group and also convert the cyano group on N to urea as shown in compound C-2. A compound of formula C-2 may be further elaborated via a reductive amination as described above to provide compounds of formula C-3.

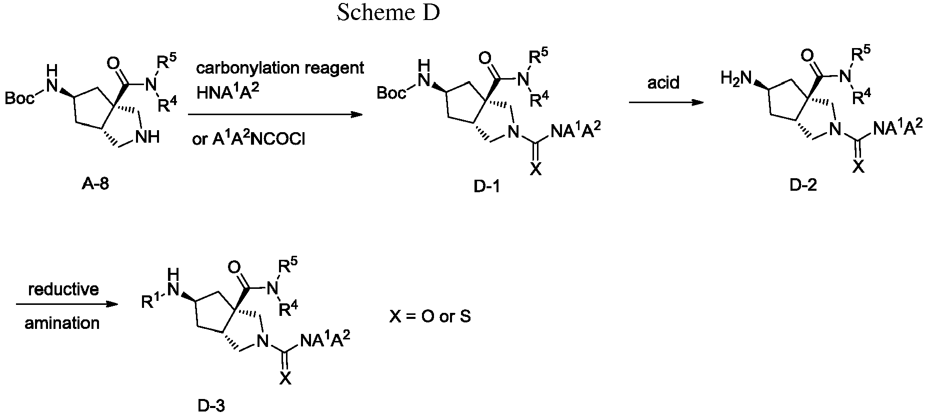

Compound A-8 may be reacted with a carbonylation reagent, either commercially or prepared by reported protocols in the scientific literature, such as *N*,*N'*-carbonydiimidazole, phosgene, trichlorophosgene (for urea) or *N*,*N'*-thiocarbonydiimidazole, di-2-pyridyl thionocarbonate (for thiourea). Further reaction with ammonia (when A¹ and A² are H) or a suitably substituted
amine, or with suitable carbamoyl chloride (for urea) or thiocarbamoyl chloride (for thiourea) gives urea or thiourea D-1. Compound D-1 may be further deprotected to give compound D-2 and substituted to afford compound D-3 by using the methods described above.

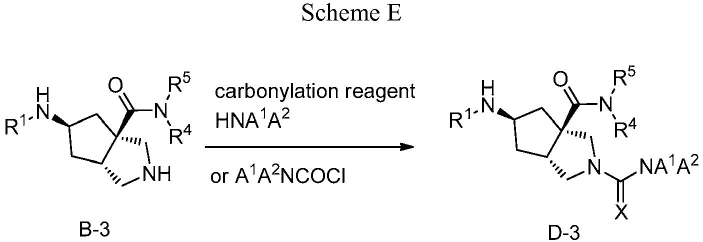

Alternatively, Scheme E shows how a compound of formula D-3 may be directly prepared from the intermediate B-3 in a similar manner described in the first step in Scheme D.

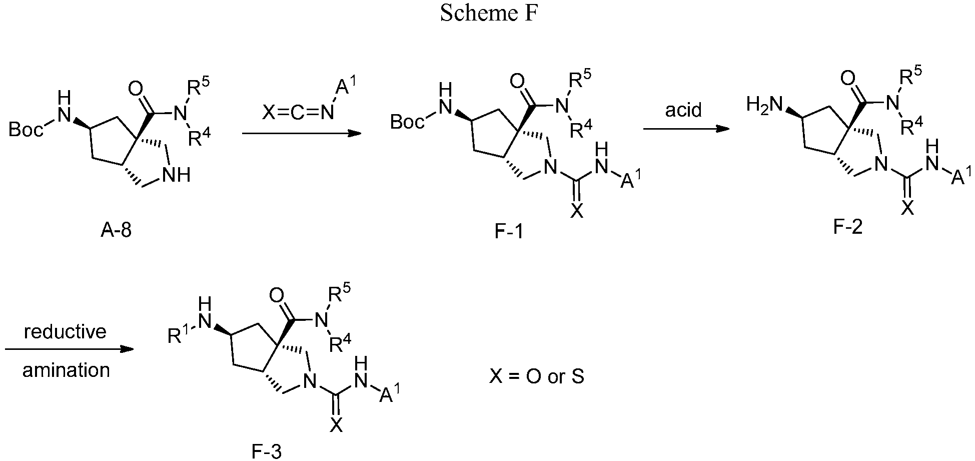

Scheme F demonstrates an alternative method for preparation of certain compound F-3. A compound of formula A-8 may be treated with a commercially available isocyanate or isothiocyanate in an aprotic solvent such as DCM or THF at ambient temperature to generate compound F-1. Compound F-3 may be obtained via the deprotection of the Boc group in compound F-1 and subsequent reductive amination of compound F-2 by using the methods described above.

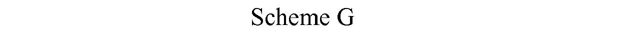

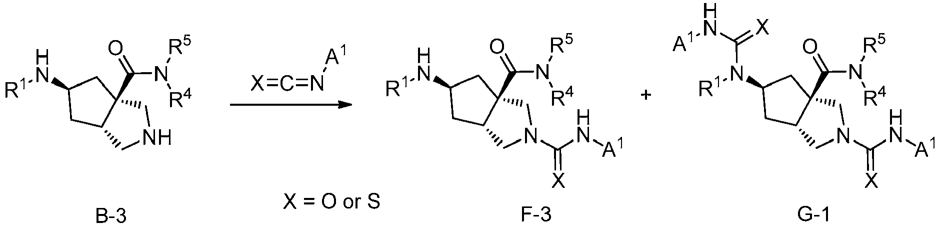

A compound of formula F-3 may be synthesized directly from compound B-3 by treatment of a commercially available isocyanate or isothiocyanate in an aprotic solvent such as DCM or THF at ambient temperature. When an excess amount of cyanate or thiocyanate is used or reaction is processed in a longer time, a by-product of formula G-1 may be produced.

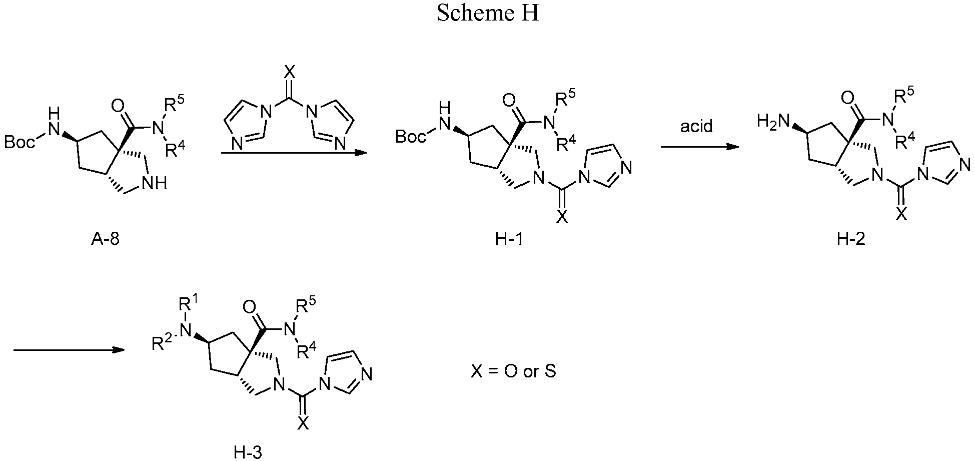

Compound A-8 may be treated with *N*,*N'*-carbonydiimidazole or *N*,*N'*-thiocarbonydiimidazole in an aprotic medium such as DCM or THF at ambient temperature to give a compound of formula H-1, which may be further converted to compound H-3 in a similar manner described previously.

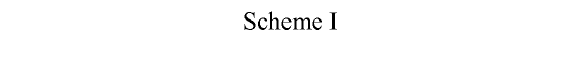

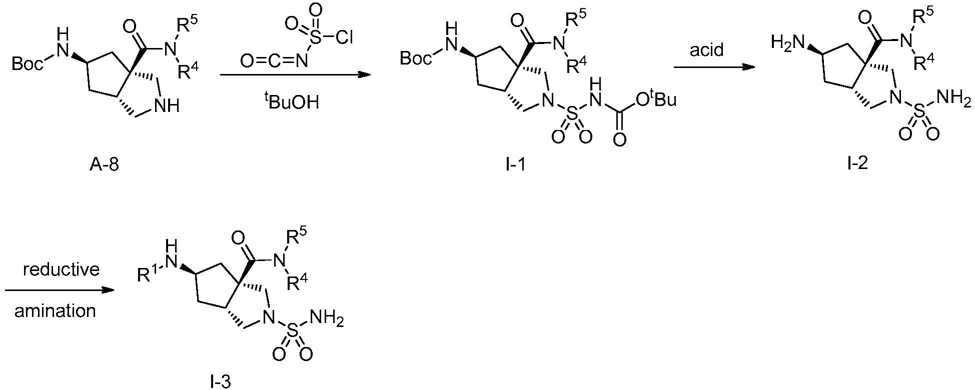

Compound A-8 may be treated with chlorosulfonyl isocyanate and *tert*-butanol in an aprotic solvent such as DCM or THF at a range of 0 °C to ambient temperature (Regainia, Z. et al. Tetrahedron 2000, 56(3), 381-7) to generate compound I-1. Compound I-3 may be obtained via the deprotection of the Boc groups in compound I-1 and subsequent reductive amination of compound I-2 by using the methods described above.

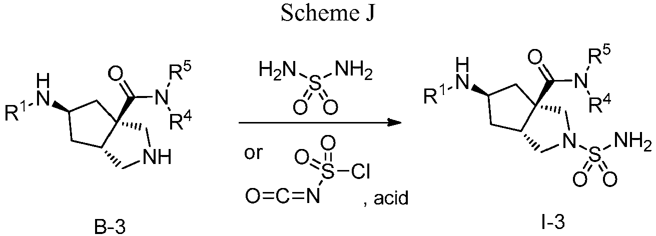

Compound of formula B-3 may be condensed with sulfamide upon heating to afford a compound of formula I-3 as reported in (Sarges, R.; et al. J. Med. Chem. 1976, 19(5), 695-709). Alternatively, compound I-3 may be generated by treating B-3 with chlorosulfonyl isocyanate and *tert*-butanol in an aprotic solvent such as DCM or THF at a range of 0 °C to ambient temperature (Regainia, Z. et al. Tetrahedron 2000, 56(3), 381-7).

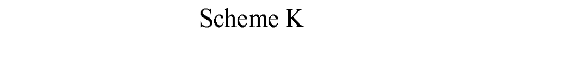

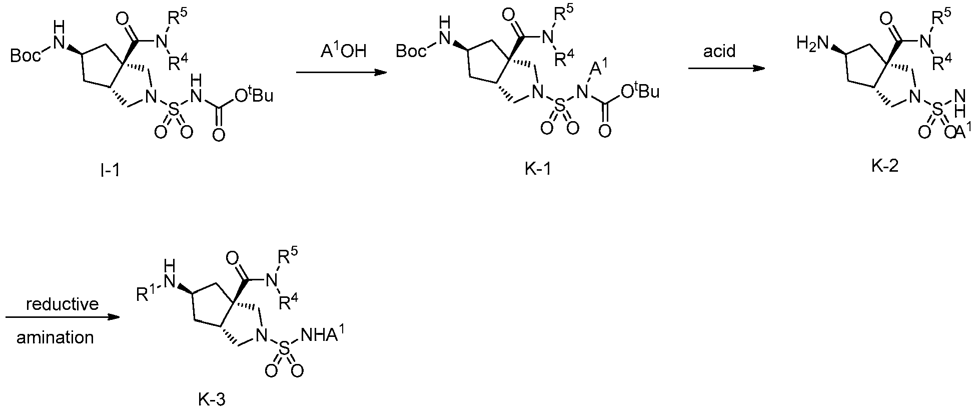

An intermediate of formula I-1 in Scheme I may be coupled to a suitable alcohol with a coupling agent such as DIAD and an additive such as TPP at a range of 0 °C to ambient temperature (Abdaoui, M.; et al. Tetrahedron 2000, 56(16), 2427-35) to provide a compound of formula K-1. Similarly, Compound K-3 may be obtained via the deprotection of the Boc groups in compound K-1 and subsequent reductive amination of compound K-2 by using the methods described previously.

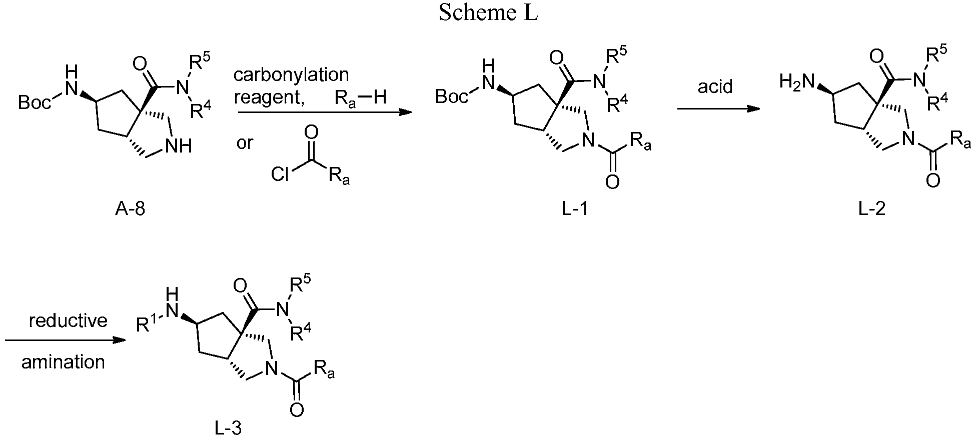

Compounds of Formula I wherein R³ is C(O)Rₐ, and Rₐ is alkoxyl or phenoxyl may be made according to Schemes L, and M. Compound A-8 may be treated with an available carbonylation reagent such as *N*,*N'*-disuccinimidyl carbonate and an additive such as DMAP, or with an available chloroformate and a base such as TEA, DIPEA in aprotic mediums such as DCM, acetonitrile, DMF at ambient temperature to give a carbamate of formula L-1. Compound L-1 may be further deprotected to give compound L-2 and substituted to afford compound L-3 by using the methods described previously.

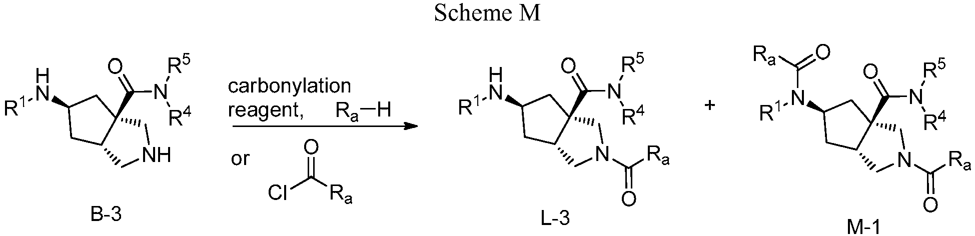

Alternatively, one skilled in the art will recognize that a compound of formula L-3 may be directly made from a compound of formula B-3 by using known methods presented in Scheme L. However, a second product of formula M-1 may be obtained an excess amount of chloroformate is used or reaction is processed in a longer time.

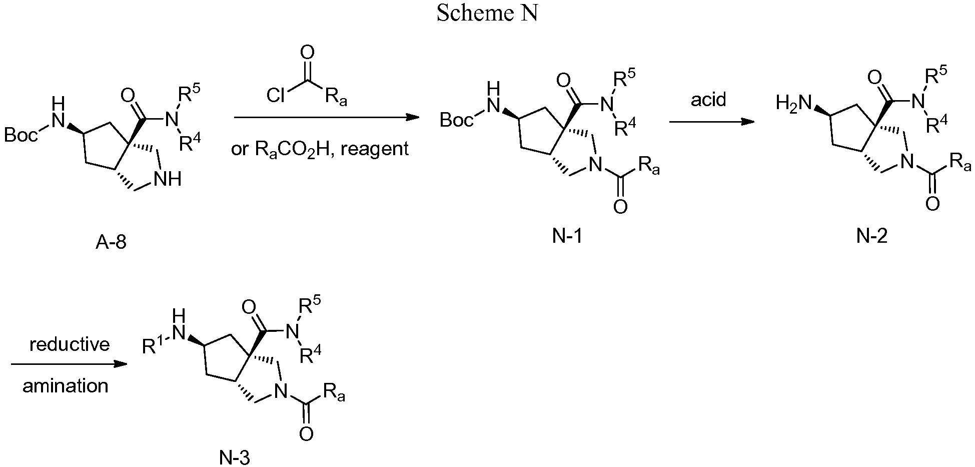

Schemes N and O show the synthesis of N-3, wherein Rₐ is as defined in Formula I. A compound of formula A-8 may be coupled to a carbonyl chloride, available either commercially or prepared according to reported protocols in the scientific literature, or to a carboxylic acid with a coupling agent such as EDAC, BOP and an additive such as HOBt, in the presence of a base such as TEA, DIPEA to provide the amide of formula N-1. Compound N-1 may be finally elaborated to N-3 by removal of the protecting Boc group and subsequent substitution.

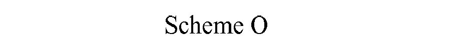

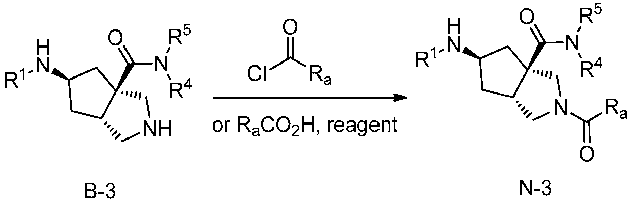

Alternatively, the compound N-3 may also be prepared from the compound B-3 by coupling to a carbonyl chloride or a carboxylic acid in a same method presented in Scheme N.

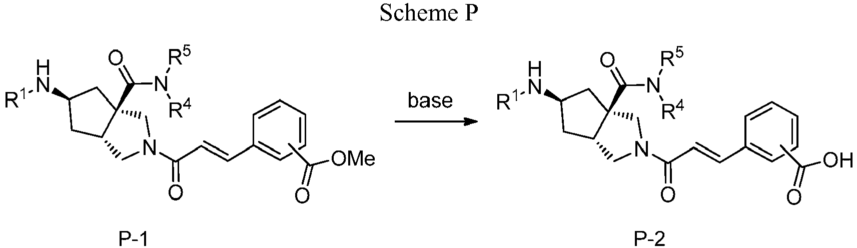

A compound of formula P-1 with a carboxylic ester on the phenyl ring, which may be made either via the method presented in Scheme N or Scheme O, may be further hydrolyzed to compound P-2 by using an aqueous inorganic base such as NaOH, LiOH, KOH in a solvent such as methanol, THF at a range of 0 °C to room temperature.

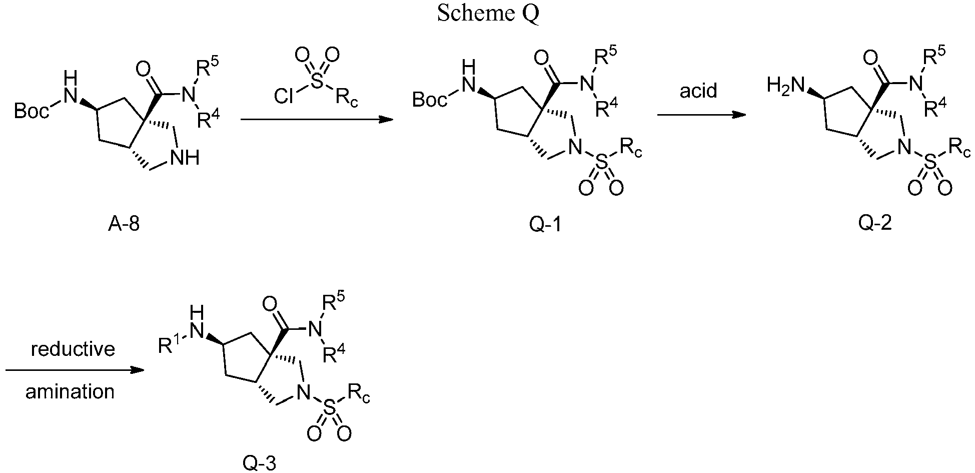

Scheme Q demonstrates a method for preparation of the sulfonamide of formula Q-3 by similar methods to those described in Scheme N by using a sulfonyl chloride instead of a carbonyl chloride.

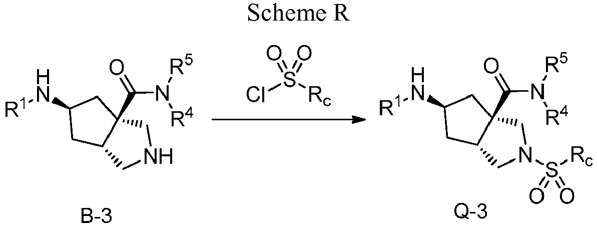

Alternatively, the compound Q-3 may also be prepared from the compound B-3 by coupling to a carbonyl chloride in the same method presented in Scheme Q.

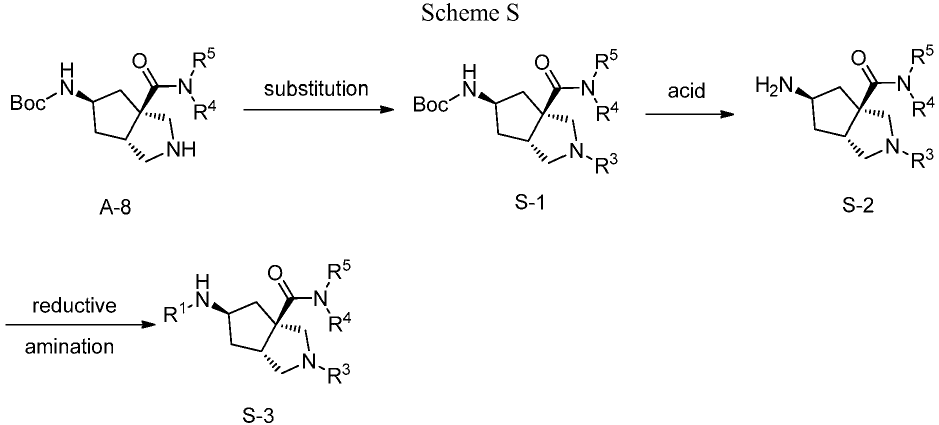

Compound A-8 may be converted to a compound of formula S-1 by a variety chemical routes which utilize conventional chemical methods known to those skilled in the art. For example, a reductive amination of compound A-8 with an aldehyde or ketone in the presence of a hydride source, such as sodium borohydride or sodium triacetoxyborohydride, to provide compounds of formula S-1. As demonstrated previously, Compound S-1 may be elaborated to S-3 by removal of the protecting Boc group and subsequent substitution.

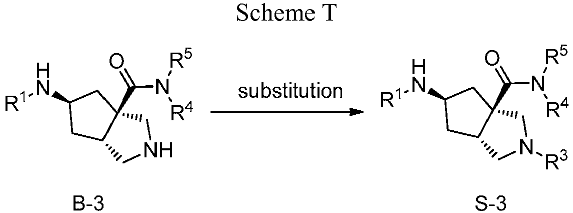

Alternatively, a compound of formula S-3 may be directly prepared via a substitution such as reductive amination from a compound of formula B-3.

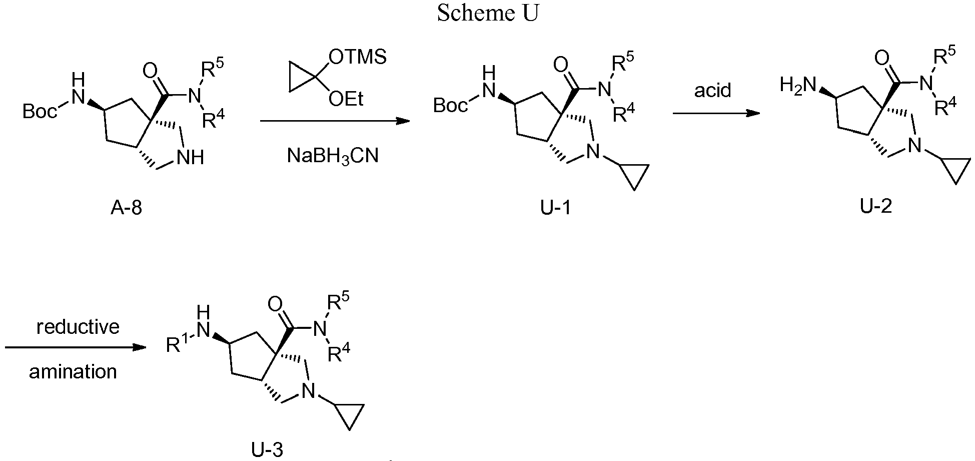

A compound of formula U-1 (where R³ is a cyclopropyl group) may be made by using (1-ethoxycyclopropoxy)-trimethylsilane in the presence of a hydride source such as sodium cyanoborohydride and an acid such as acetic acid in a solvent such as methanol at a elevated temperature around 80 °C (Gillaspy, M. et al. Tetrahedron Lett. 1995, 36(41), 7399-402). Subsequent deprotection and reductive amination may convert compound U-1 to compound U-3 as described previously.

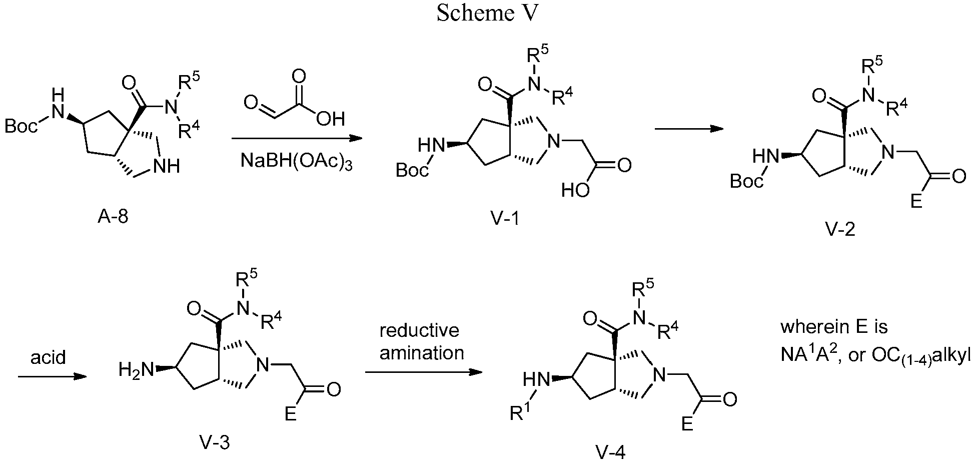

Compounds of Formula I wherein R₃ is C₍₁₋₃₎alkylC(O)NA¹A² or C₍₁₋₄₎alkylCO₂C₍₁₋₄₎alkyl may be synthesized according to Scheme V. A compound of formula A-8 may participate in a reductive amination with glyoxylic acid (or other alkyl group substituted with both aldehyde and acid moieties) in the presence of a hydride source such as sodium triacetoxyborohydride at an ambient temperature to afford an acid of formula V-1. One skilled in the art will recognize that the transformation of compound V-1 to the corresponding ester or the corresponding amide (where X is N) of formula V-2 using standard literature protocols. Compound V-4 may be generated by following previously described method.

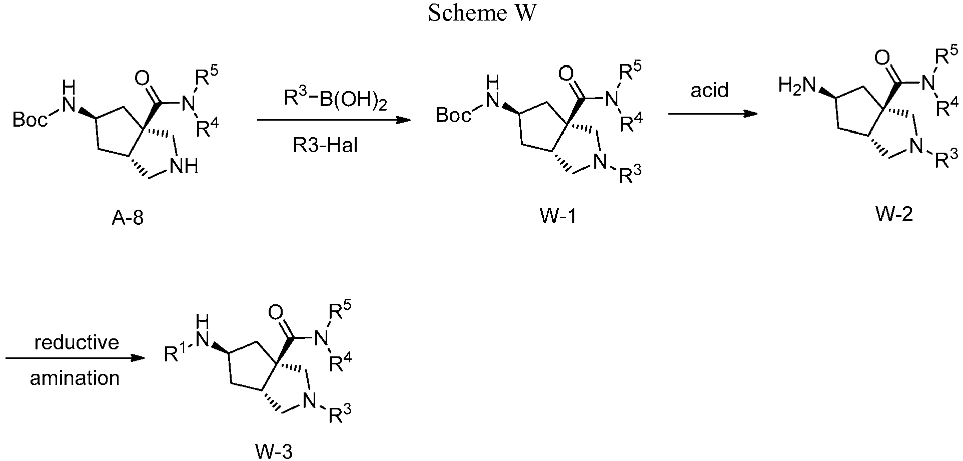

Certain R³ substituents of the present invention where R³ is an aromatic or heteroaromatic group may be introduced into a compound of formula A-8 through a transition metal-catalyzed cross coupling reaction (Lyons, T. W.; Sanford, M. S. Chem. Rev. 2010, 110(2), 1147-69) to afford compounds of formula W-1. Suitable precursors include boronic acids or heteroaromatic halides. Suitable palladium catalysts include palladium tetrakis triphenylphosphine. Finally, compound W-1 may be converted to compound W-3 by removal of the protecting Boc group and subsequent substitution.

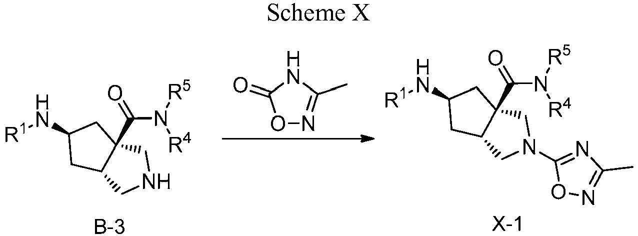

A compound of formula X-1 where R3 is an oxadiazole or substituted oxadiazole may be obtained via a phosphonium-mediated cross-coupling from a compound of formula B-3 and the corresponding oxadiazol-2-one as described in Levins, C.; Wan, Z-K. Org. Lett. 2008, 10(9), 1755-1758.

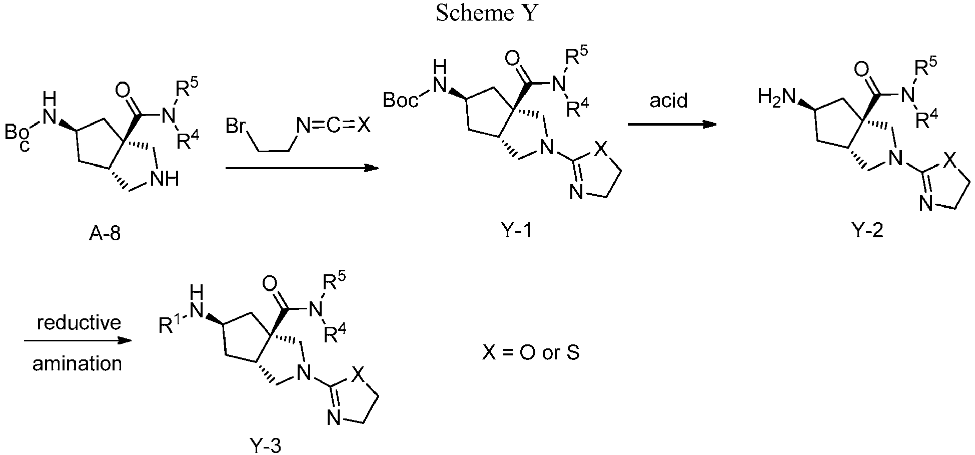

A compound of formula Y-1 may be formed by treatment of compound A-8 with 2-bromoethyl isocyanate (where X is O)(Hiltmann, R. et al. Eur. J. Med. Chem. 1977, 12(1), 63-8) or 2-bromoethyl isothiocyanate (where X is S)(Hackler, R. E.; Balko, T. W. Syn. Comm. 1975, 5(2), 143-6) in the presence of a base such as TEA in an aprotic solvent such as DCM at an ambient temperature. Subsequent deprotection and substitution with reductive amination may lead to a compound of formula Y-3.

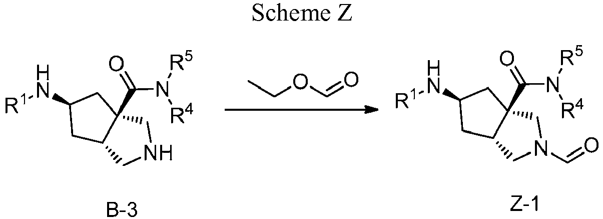

Using the procedure described in King, J. A.; McMillan, F. H. J. Amer. Chem. Soc. 1950, 72, 1236-40, a compound of formula Z-1 may be formed by condensation of a compound B-3 with ethyl formate at an elevated temperature around 70 °C.

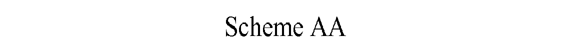

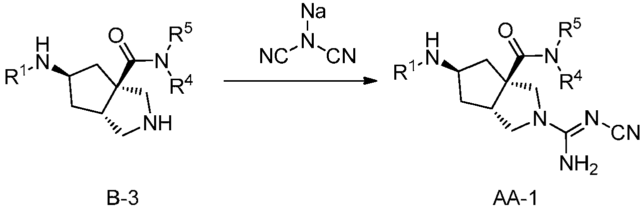

Formation of certain compounds of formula AA-1 may be achieved by heating a mixture of B-3 and sodium dicyanamide in 5% water in isopropyl alcohol at an elevated temperature around 120 °C under an inert atmosphere (Rembarz, G. et al. J. fuer Prak. Chem, 1964, 26(5-6), 314-8).

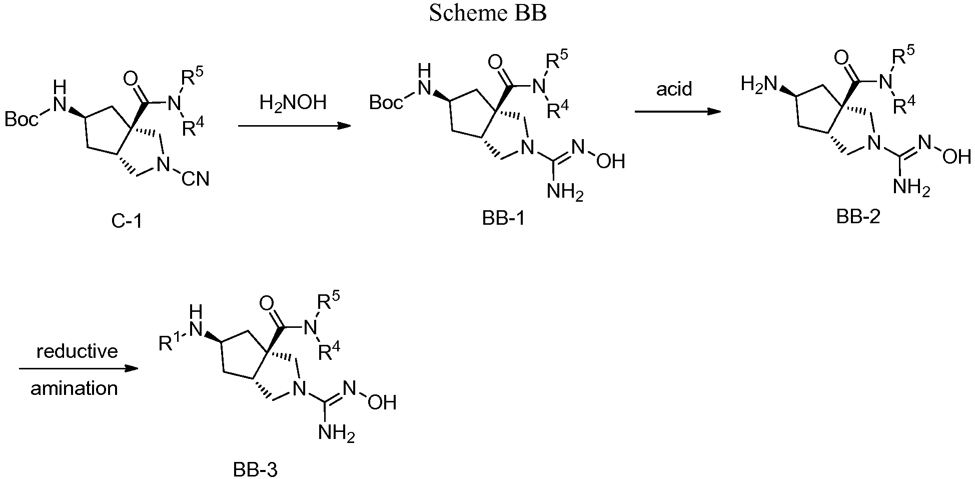

A mixture of compound C-1 with hydroxylamine in a solvent such as ethanol at an elevated temperature around 80 °C under an inert atmosphere may be condensed to a compound of formula BB-1 as described in Nordmann, R.; Loosli, H. R. Helv. Chim. Acta, 1985, 68(4), 1025-32. Subsequent deprotection compound of BB-1 and substitution of the resulting compound BB-2 with reductive amination may lead to a compound of formula BB-3 as described previously.

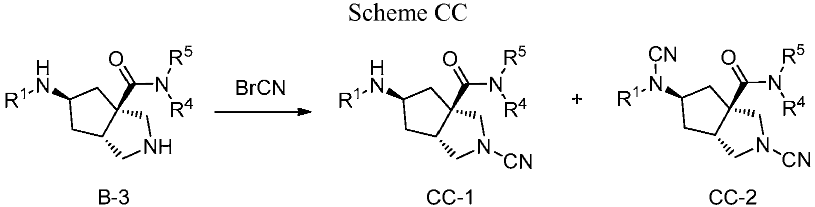

A compound of formula B-3 may undergo a cyanation reaction as described in Scheme C to generate mono-cyanated compound CC-1 or/and dicyanated compound CC-2 where an excess amount of cyanating agent is used.

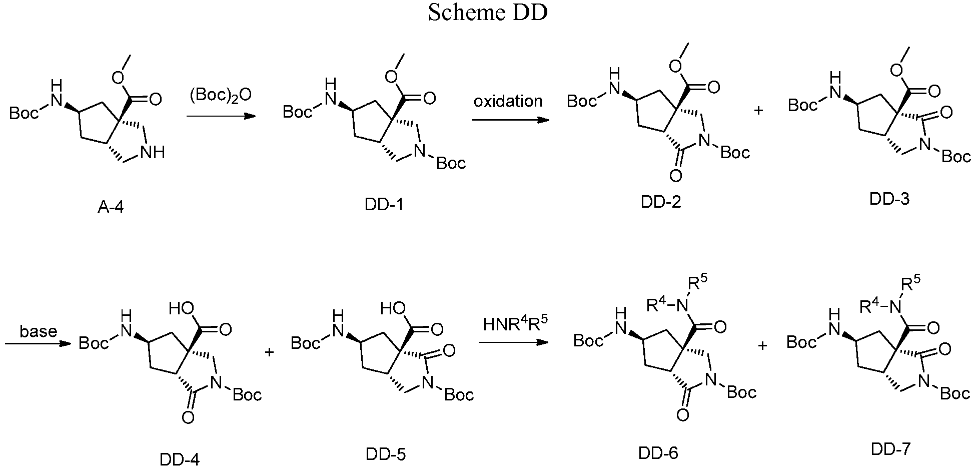

A compound of formula A-4 may be protected by a Boc group to lead a compound of formula DD-1, which may be further oxidized to a mixture of two isomers DD-2 and DD-3 by a suitable oxidizing agent such as sodium bromate and in the presence of an additive such as ruthenium(III) chloride (Tanaka, K.; Yoshifuji, S.; Nitta, Y. Chem. Pharm. Bull. 1986, 34(9), 3879-84). One skilled in the art will recognize that the mixture of formulas DD-2 and DD-3 may be hydrolyzed to their corresponding carboxylic acids of formulas DD-4 and DD-5, followed by coupling to certain amines to afford separable amides of formulas DD-6 and DD-7.

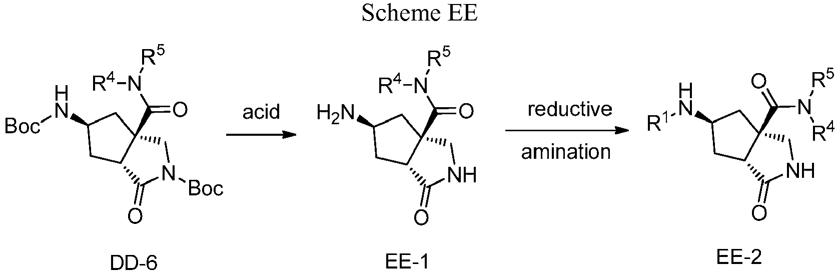

Both Boc protecting groups in the compound of formula DD-6, prepared in Scheme DD, may be removed by an acid such as TFA to generate a compound of formula EE-1. A compound of formula EE-1 may then be substituted by reductive amination to a compound EE-2 as described previously.

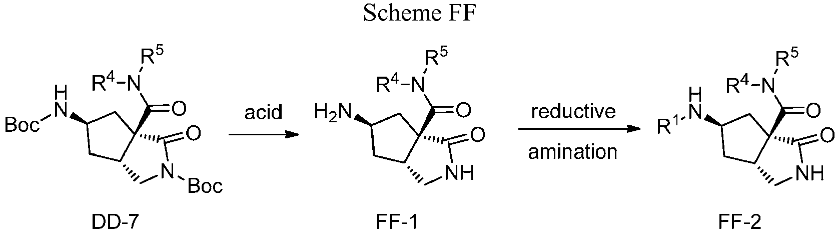

In a similar manner, transformation of a compound of formula FF-2 from a compound DD-7 may be achieved using conditions described in Scheme EE.

### Specific Examples

Specific compounds which are representative of this invention were prepared as per the following examples and reactions sequences; the examples and the diagrams depicting the reaction sequences are offered by the way of illustration, to aid in the understanding of the invention and should not be construed to limit in any way the invention set forth in the claims which follow thereafter. The instant compounds may also be used as intermediates in subsequent examples to produce additional compounds of the present invention. No attempt has been made to optimize the yields obtained in any of the reactions. One skilled in the art would know how to increase such yields through routine variations in reaction times, temperatures, solvents and/or reagents.

### Intermediate 1

### tert-Butyl ((3aR,5R,6aR)-3a-(3-(trifluoromethyl)-5,6,7,8-tetrahydro-1,6-naphthyridine-6-carbonyl)octahydrocyclopenta[c]pyrrol-5-yl)carbamate.

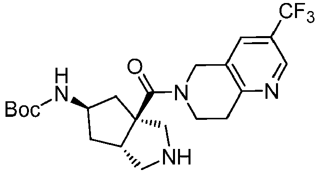

**Step A. (1*R*,4*S*)-Methyl 4-((*tert*-butoxycarbonyl)amino)cyclopent-2-enecarboxylate.** A solution of (1*S*,4*R*)-2-azabicyclo[2.2.1]hept-5-en-3-one (5 g, 98%, 45.8 mmol, Aldrich) and concentrated hydrochloric acid (9.2 mL) in methanol (115 mL) in a sealed tube was heated with stirring for 16 h. After cooling to rt, the solvents were removed by rotary evaporation. The residue was triturated with Et₂O and stirred. Filtration and evaporation to dryness gave the HCl salt as a white solid, which was dissolved in DCM (228 mL) and cooled to 0 °C. To the cooled solution was added di-*tert*-butyl dicarbonate (11.14 g, 49.5 mmol), followed by addition of TEA (6.95 mL, 49.5 mmol) over 1 h. The reaction mixture was stirred at rt overnight and quenched by the addition of aqueous NH₄Cl solution. The organic phase was washed with cool 0.5 *N* HCl solution and dried over Na₂SO₄. Evaporation and purification by column chromatography (eluent: 20% EtOAc in hexanes to 40%) gave the product as a colorless oil. 10.2 g, 92.3%. LC/MS: C₁₂H₁₉NO₄: m/z 264.0 (M+Na).

**Step B. (*R*)-Methyl 4-((*tert*-butoxycarbonyl)amino)cyclopent-1-enecarboxylate.** A mixture of the product from Step **A** (9.8 g, 40.6 mmol) and DBU (9 mL, 60 mmol) in DCM (70 mL) was stirred at rt overnight. The mixture was cooled to 0 °C and washed with 1N HCl aqueous solution and brine, dried over Na₂SO₄. Evaporation and purification by column chromatography (eluent: 20% EtOAc in hexanes to 40%) gave the product as a white solid. ¹H-NMR (400 MHz, CDCl₃): δ 1.44 (s, 9 H), 2.36-2.47 (m, 2 H), 2.88-2.99 (m, 2 H), 3.74 (s, 3 H), 4.36 (br s, 1 H), 4.71 (br s, 1 H), 6.71-6.72 (m, 1 H). LC/MS: C₁₂H₁₉NO₄: m/z 264.0 (M+Na).

**Step C. (3a*R*,5*R*,6a*R*)-Methyl 2-benzyl-5-((*tert*-butoxycarbonyl)amino)octahydro-cyclopenta[*c*]pyrrole-3a-carboxylate.** To a solution of the product from Step **B** (5.51 g, 20 mmol) and *N-*(methoxymethyl)-*N*-(trimethylsilylmethyl)benzylamine (6.4 mL, 24 mmol, Aldrich) in DCM (40 mL) was added 1*M* TFA in DCM (2.4 mL, 2.4 mmol) slowly. The resulting mixture was stirred at rt overnight and quenched by the addition of saturated aqueous NaHCO₃ solution. The organic phase was washed with brine, dried over Na₂SO₄. Evaporation and purification by column chromatography (eluent: 10% EtOAc in hexanes to 20%) gave the desired isomer as a colorless gel. ¹H-NMR (400 MHz, CDCl₃) of the desired isomer: δ 1.38 (s, 9 H), 1.66 - 1.86 (m, 2 H), 1.86 - 1.96 (m, 1 H), 1.96 - 2.08 (m, 1 H), 2.29 - 2.63 (m, 3 H), 2.71-2.93 (m, 2 H), 3.43 - 3.56 (m, 2 H), 3.58 - 3.74 (s, 3 H), 4.08 - 4.29 (m, 1 H), 4.78 (d, *J*=6.6 Hz, 1 H), 7.11 - 7.31 (m, 5 H). LC/MS: C₂₄H₂₈N₂O₄: m/z 375.2 (M+H).

**Step D. (3a*R*,5*R*,6a*R*)-Methyl 5-((*tert*-butoxycarbonyl)amino)octahydrocyclopenta[*c*]-pyrrole-3a-carboxylate.** To a solution of the product from Step **C** (28.6 g, 76.4 mmol) in methanol (200 mL) was added 5% palladium hydroxide on carbon (0.26 g). Hydrogenation proceeded overnight in Parr shaker at rt at 344.7 kPa (50 psi). Filtration and evaporation to dryness gave the product as a yellow gel. ¹H-NMR (400 MHz, CDCl₃): δ 1.44 (s, 9 H), 1.72 - 1.90 (m, 2 H), 1.93 - 2.05 (m, 1 H), 2.05 - 2.17 (m, 1 H), 2.67 (dd, *J*=11.6, 5.8 Hz, 1 H), 2.77 (d, *J*=12.1 Hz, 1 H), 2.82 - 2.94 (m, 1 H), 3.30 (dd, *J*=11.6, 8.1 Hz, 1 H), 3.44 - 3.57 (m, 2 H), 3.68 - 3.79 (m, 3 H), 4.14 (d, *J*=6.3 Hz, 1 H), 4.85 (d, *J*=5.8 Hz, 1 H). LC/MS: C₁₄H₂₄N₂O₄: m/z 285.2 (M+H).

**Step E. (3a*R*,5*R*,6a*R*)-2-Benzyl 3a-methyl 5-((*tert*-butoxycarbonyl)amino)-hexahydrocyclopenta[*c*]pyrrole-2,3a(1*H*)-dicarboxylate.** To a solution of the product from Step **D** (21.5 g, 75.6 mmol) in DCM (400 mL) was added TEA (31.6 mL, 226.8 mmol) and benzyl chloroformate (14.5 mL, 95%, 98.3 mmol) at 0 °C under Ar. The mixture was stirred at rt for 3 h and quenched by the addition of aqueous NaHCO₃ solution. The aqueous phase was extracted with DCM (3 x) and the combined organic phases were dried over Na₂SO_{4.} Evaporation and purification by column chromatography (eluent: 20% EtOAc in hexanes to 30%) gave the product as a colorless gel. ¹H-NMR (400 MHz, CDCl₃): δ 1.43 (s, 9 H), 1.89 (br. s., 2 H), 2.13 (br. s., 2 H), 2.99 (d, *J*=1.0 Hz, 1 H), 3.22-3.57 (m, 2 H), 3.59 - 3.83 (m, 4 H), 3.99 (d, *J*=11.6 Hz, 1 H), 4.23 (d, *J*=5.8 Hz, 1 H), 4.85 (br. s., 1 H), 5.12 (d, *J=*3.0 Hz, 2 H), 7.28 - 7.42 (m, 5 H). LC/MS: C₂₂H₃₀N₂O₆: m/z 441.2 (M+Na).

**Step F. (3a*R*,5*R*,6a*R*)-2-((Benzyloxy)carbonyl)-5-((*tert*-butoxycarbonyl)amino)-octahydrocyclopenta[*c*]pyrrole-3a-carboxylic acid.** To a mixture of the product from Step **E** (10.8 g, 25.81 mmol) in THF (40 mL) was added 6*N* KOH aqueous solution (12.9 mL). After being stirred at rt overnight, the reaction mixture was condensed. The residue was acidified by a cooled 1*N* HCl solution to pH ~3 and extracted with EtOAc, dried over Na₂SO₄. Filtration and evaporation to dryness gave the product as a white foam. LC/MS: C₂₁H₂₈N₂O₆: m/z 427.2 (M +Na).

**Step G. (3a*R*,5*R*,6a*R*)-Benzyl 5-((*tert*-butoxycarbonyl)amino)-3a-(3-(trifluoromethyl)-5,6,7,8-tetrahydro-1,6-naphthyridine-6-carbonyl)hexahydrocyclopenta-[*c*]pyrrole-2(1*H*)-carboxylate.** To a solution of the product from Step **F** (9.25 g, 22.87 mmol)
and 3-(trifluoromethyl)-5,6,7,8-tetrahydro-1,6-naphthyridine dihydrochloride (7.55 g, 27.44 mmol) in THF (80 mL) was added TEA (19.13 mL, 137.2 mmol), EDAC (5.7 g, 29.73 mol) and HOBt (4.02 g, 29.73 mmol). The resulting mixture was stirred at rt overnight. The reaction was quenched by addition of brine, extracted with EtOAc, and dried over Na₂SO₄. Evaporation and purification by column chromatography (eluent: 80% EtOAc in hexanes to 100%) gave the product as a yellow foam. ¹H-NMR (400 MHz, CDCl₃): δ 1.31 - 1.40 (s, 9 H), 1.61- 1.70 (m, 1 H), 1.84 (br. s., 3 H), 2.40 (br. s., 1 H), 3.13 (br. s., 2 H), 3.30 (br. s., 1 H), 3.58 - 3.89 (m, 5 H), 4.24 (br. s., 1 H), 4.53 - 4.78 (m, 2 H), 4.89 (br. s., 1 H), 5.05 - 5.16 (m, 2 H), 7.31 - 7.42 (m, 5 H), 7.69 (s, 1 H), 8.72 (s, 1 H); LC/MS: C₃₀H_{35F3}N₄O₅: m/z 589.3 (M+H).

**Step H. *tert*-Butyl ((3a*R*,5*R*,6a*R*)-3a-(3-(trifluoromethyl)-5,6,7,8-tetrahydro-1,6-naphthyridine-6-carbonyl)octahydrocyclopenta[*c*]pyrrol-5-yl)carbamate.** A solution of the product from Step **G** (9.2 g, mmol) in methanol (60 mL) was bubbled with Ar for 15 min, and 10% Palladium on carbon (1 g) was added. The mixture was hydrogenated at 172.4 kPa (25 psi) in Parr shaker overnight. Filtration and evaporation to dryness gave the product as yellow foam. ¹H-NMR (400 MHz, CDCl₃): δ 1.33 - 1.44 (m, 9 H), 1.65 - 2.07 (m, 6 H), 2.28 (dd, *J*=12.5, 5.7 Hz, 1 H), 2.61 (dd, *J*=11.2, 5.7 Hz, 1 H), 2.95 - 3.30 (m, 5 H), 3.58 - 3.70 (m, 1 H), 3.88 (br. s., 2 H), 4.18 (d, *J*=6.3 Hz, 1 H), 4.58-4.92 (m, 3 H), 7.70 (s, 1 H), 8.71 (s, 1 H); LC/MS: C₂₂H₂₉F₃N₄O₃: m/z 455.3 (M+H).

### Intermediate 2

### tert-Butyl ((3aR,5R,6aR)-3a-(7-(trifluoromethyl)-1,2,3,4-tetrahydroisoquinoline-2-carbonyl)octahydrocyclopenta[c]pyrrol-5-yl)carbamate

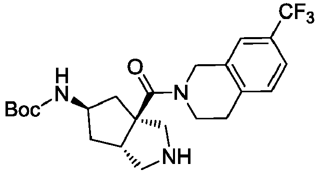

Prepared according to the procedure of Intermediate **1,** using 7-(trifluoromethyl)-1,2,3,4-tetrahydroisoquinoline hydrochloride instead of 3-(trifluoromethyl)-5,6,7,8-tetrahydro-1,6-naphthyridine dihydrochloride in Step **G.** ¹H-NMR (400 MHz, CDCl₃): δ 1.34 - 1.43 (s, 9 H), 1.66 - 1.98 (m, 2 H), 2.25 (dd, *J*=12.9, 5.6 Hz, 3 H), 2.62 (dd, *J*=11.4, 5.6 Hz, 1 H), 2.86 - 3.10 (m, 3 H), 3.16 (dd, *J*=11.1, 8.1 Hz, 2 H), 3.59 - 3.91 (m, 3 H), 4.18 (d, *J*=6.6 Hz, 1 H), 4.57-4.89 (m, 3 H), 7.21 - 7.27 (m, 1 H), 7.35 - 7.49 (m, 2 H); LC/MS: C₂₃H₃₀F₃N₃O₃: m/z 454.2 (M+H).

### Intermediate 3

### tert-Butyl ((3aR,5R,6aR)-3a-(7-(trifluoromethoxy)-1,2,3,4-tetrahydroisoquinoline-2-carbonyl)octahydrocyclopenta[c]pyrrol-5-yl)carbamate

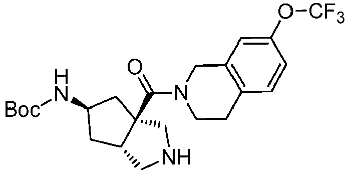

Prepared according to the procedure of Intermediate **1,** using 7-(trifluoromethoxy)-1,2,3,4-tetrahydroisoquinoline hydrochloride instead of 3-(trifluoromethyl)-5,6,7,8-tetrahydro-1,6-naphthyridine dihydrochloride in Step **G.** ¹H-NMR (400 MHz, CDCl₃): δ 1.32 (br. s., 9 H), 1.89 - 2.29 (m, 4 H), 2.48 (dd, *J*=13.4, 5.3 Hz, 1 H), 2.90 (br. s., 2 H), 3.21 (d, *J*=10.4 Hz, 1 H), 3.32 - 3.50 (m, 2 H), 3.69 (m, 3 H), 3.96 (br. s., 1 H), 4.24 (br. s., 1 H), 4.63 (m, 2 H), 4.80 (br. s., 1 H), 7.06 (d, *J*=10.1 Hz, 2 H), 7.17 (d, *J*=8.3 Hz, 1 H); LC/MS: C₂₃H₃₀F₃N₃O₄: m/z 470.2 (M+H).

### Intermediate 4

### tert-Butyl ((3aR,5R,6aR)-3a-((3-(trifluoromethyl)benzyl)carbamoyl)octahydrocyclopenta[c]pyrrol-5-yl)carbamate

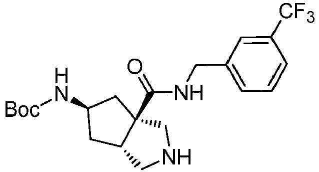

Prepared according to the procedure of Intermediate **1,** using 3-(trifluoromethyl)benzylamine instead of 3-(trifluoromethyl)-5,6,7,8-tetrahydro-1,6-naphthyridine dihydrochloride in Step **G.** LC/MS: C₂₁H₂₈F₃N₃O₃: m/z 428.5 (M+H).

### Intermediate 5

### tert-Butyl ((3aR,5R,6aR)-3a-((3-fluoro-5-(trifluoromethyl)benzyl)carbamoyl)-octahydrocyclopenta[c]pyrrol-5-yl)carbamate

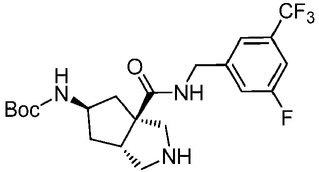

Prepared according to the procedure of Intermediate **1,** using 3-fluoro-5-(trifluoromethyl)benzylamine instead of 3-(trifluoromethyl)-5,6,7,8-tetrahydro-1,6-naphthyridine dihydrochloride in Step **G.** LC/MS: C₂₁H₂₇F₄N₃O₃: m/z 446.5 (M+H).

### Intermediate 6

### tert-Butyl ((3aR,5R,6aR)-3a-((3,5-bis(trifluoromethyl)benzyl)carbamoyl)octahydrocyclopenta[c]pyrrol-5-yl)carbamate

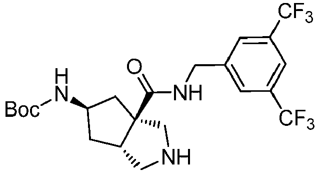

Prepared according to the procedure of Intermediate **1,** using 3,5-bis(trifluoromethyl)benzyl)amine instead of 3-(trifluoromethyl)-5,6,7,8-tetrahydro-1,6-naphthyridine dihydrochloride in Step **G.** LC/MS: C₂₂H₂₇F₆N₃O₃: m/z 496.5 (M+H).

### Example 1

### ((3aR,5R,6aR)-5-(((3S*,4S*)-3-Methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

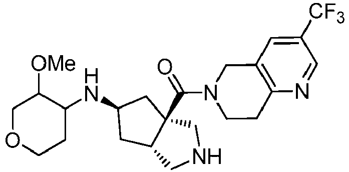

**Step A. (3a*R*,5*R*,6a*R*)-Benzyl 5-amino-3a-(3-(trifluoromethyl)-5,6,7,8-tetrahydro-1,6-naphthyridine-6-carbonyl)hexahydrocyclopenta[*c*]pyrrole-2(1*H*)-carboxylate.** A solution of the product from Step **G** in Intermediate **1** (80 mg, 0.136 mmol) in TFA (1 mL) and DCM (1 mL) was stirred at rt for 1.5 h. Concentration by rotary evaporation was followed by dilution with DCM and additional evaporation to give the product as a TFA salt. LC/MS: C₂₅H₂₇F₃N₄O₃: m/z 498.3 (M+H).

**Step B. (3a*R*,5*R*,6a*R*)-Benzyl 5-((3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrole-2(1*H*)-carboxylate.** A mixture of the product from Step **A** (difluoroacetate, 97.45 mg, 0.136 mmol), 3-methoxydihydro-*2H*-pyran-4(*3H*)-one (35.4 mg, 0.272 mmol), 4Å molecular sieves (60 mg) and TEA (0.19 mL, 1.36 mmol) in DCM (4 mL) was stirred at rt for 2 h, followed by addition of sodium triacetoxyborohydride (46.12 mg, 0.218 mmol). The resulting mixture was stirred at rt overnight. The reaction was quenched by addition of saturated NaHCO₃ aqueous solution, extracted with DCM, dried over Na₂SO₄. After removal of solvent, the residue was purified by column chromatography (eluent: 5% 7N NH₃ in MeOH in DCM) to give the product as a yellow foam. ¹H-NMR (400 MHz, CDCl₃): δ 1.56 - 2.12 (m, 7 H), 2.31 (br. s., 1 H), 2.55 - 2.67 (m, 1 H), 3.06 - 3.21 (m, 3 H), 3.24 - 4.16 (m, 14 H), 4.71 (br. s., 2 H), 5.12 (s, 2 H), 7.29 - 7.44 (m, 5 H), 7.69 (br. s., 1 H), 8.72 (br. s., 1 H); LC/MS: C₃₁H₃₇F₃N₄O₅: m/z 603.0 (M+H).

**Step C. ((3a*R*,5*R*,6a*R*)-5-(((3*S**,4*S**)-3-Methoxytetrahydro-2*H*-pyran-4-yl)amino)octahydro-cyclopenta[*c*]pyrrol-3a-yl)(3-(trifluoromethyl)-7,8-dihydro-1,6-naphthyridin-6(5*H*)-yl)methanone.** A solution of the product from Step **B** (1.53 g, 2.55 mmol) in methanol (18 mL) was bubbled with Ar for 15 min, and 10% Palladium on carbon (0.3 g) was added. The mixture was hydrogenated at 172.4 kPa (25 psi) in Parr shaker overnight. Filtration and evaporation to dryness gave the product as yellow foam. ¹H-NMR (400 MHz, CDCl₃): δ 1.37 - 2.04 (m, 7 H), 2.21 (d, *J*=5.8 Hz, 1 H), 2.47 - 2.63 (m, 1 H), 2.66 - 2.86 (m, 1 H), 2.86 - 3.54 (m, 12 H), 3.60 (br. s., 1 H), 3.66 - 4.21 (m, 4 H), 4.61 - 5.05 (m, 2 H), 7.70 (br. s., 1 H), 8.71 (br. s., 1 H); LC/MS: C₂₃H₃₁F₃N₄O₃: m/z 469.2 (M+H).

### Example 2

### (3aR,5R,6aR)-5-(((3S*,4S*)-(3-Methoxytetrahydro-2H-pyran-4-yl)amino)-3a-(3-(trifluoromethyl)-5,6,7,8-tetrahydro-1,6-naphthyridine-6-carbonyl)hexahydrocyclo-penta[c]pyrrole-2(1H)-carboxamide.

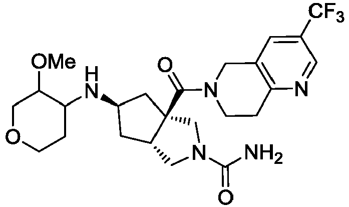

**Step A. *tert*-Butyl ((3a*R*,5*R*,6a*R*)-2-cyano-3a-(3-(trifluoromethyl)-5,6,7,8-tetrahydro-1,6-naphthyridine-6-carbonyl)octahydrocyclopenta[*c*]pyrrol-5-yl)carbamate.** To a mixture of the product from Step **H** in Intermediate **1** (3.26 g, 7.17 mmol) and K₂CO₃ (1.19 g, 8.61 mmol) in acetonitrile (15 mL) was added cyanogen bromide (1.44 mL, 7.17 mmol). After being stirred at rt overnight, the mixture was filtered and the filtrate was condensed *in vacuo*. The residue was purified by column chromatography (eluent: 80% EtOAc in hexanes to 100%) gave the product as a white foam. ¹H-NMR (400 MHz, CDCl₃): δ 1.31 - 1.44 (m, 9 H), 1.77 - 1.93 (m, 2 H), 2.05 (s, 1 H), 2.37 (d, *J*=5.8 Hz, 1 H), 3.14 (br. s., 2 H), 3.24 (dd, *J*=9.9, 4.0 Hz, 1 H), 3.50 - 3.89 (m, 6 H), 4.24 (d, *J*=6.1 Hz, 1 H), 4.49 - 5.02 (m, 3 H), 7.71 (s, 1 H), 8.73 (s, 1 H); LC/MS: C₂₃H₂₈F₃N₅O₃: m/z 480.2 (M+H).

**Step B. (3a*R*,5*R*,6a*R*)-5-amino-3a-(3-(trifluoromethyl)-5,6,7,8-tetrahydro-1,6-naphthyridine-6-carbonyl)hexahydrocyclopenta[*c*]pyrrol-2(*1H*)-carboxamide trifluoroacetate.** A solution of the product from Step **A** (0.19 g, 0.396 mmol) in TFA (1.5 mL) and DCM (1.5 mL) was stirred at rt for 1.5 h. Concentration by rotary evaporation was followed by dilution with DCM and additional evaporation to give the product as a TFA salt. LC/MS: C₁₈H₂₂F₃N₅O₂: m/z 398.3 (M+H).

**Step C. (3a*R*,5*R*,6a*R*)-5-(((3*S**,4*S**)-(3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrole-2(*1H*)-carboxamide.** A mixture of the product from Step **B** (247.7 mg, 0.396 mmol), 3-methoxydihydro-*2H*-pyran-4(*3H*)-one (154.6 mg, 1.188 mmol), 4Å molecular sieves (0.2 g) and TEA (0.165 mL, 1.188 mmol) in DCM (5 mL) was stirred at rt for 2 h, followed by addition of sodium triacetoxyborohydride (0.168 g, 0.792 mmol). The resulting mixture was stirred at rt overnight. The reaction was quenched by addition of saturated NaHCO₃ aqueous solution, extracted with DCM, dried over Na₂SO₄. After removal of solvent, the residue was purified by column chromatography (eluent: 5% 7*N* NH₃ in methanol in DCM) to give the product as a white foam. ¹H-NMR (400 MHz, CDCl₃): δ 1.51 - 1.95 (m, 4 H), 2.07 - 2.58 (m, 3 H), 2.72 (br. s., 1 H), 3.06 - 4.13 (m, 18H), 4.57 - 5.04 (m, 4 H), 7.71 (br. s., 1 H), 8.71 (br. s., 1 H); LC/MS: C₂₄H₃₂F₃N₅O₄: m/z 512.3 (M+H).

### Example 3

### (3aR,5R,6aR)-5-(((3S,4S)-(3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide.

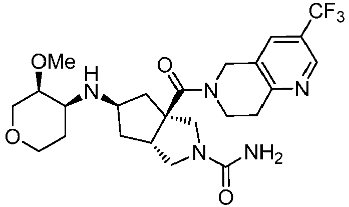

The 1^{st} fraction of Example 2 from chiral HPLC (Kromasil K40813, 10-Amycoat, 30 x 250 mm; eluent: alcohol by EMD). ¹H-NMR (400 MHz, CDCl₃): δ 0.79 0.96 (m, 1 H), 1.14-1.23 (m, 1 H), 1.60 - 2.03 (m, 5 H), 2.40 (d, *J*=5.6 Hz, 1 H), 2.79 (br. s., 1 H), 3.07 - 4.19 (m, 17 H), 4.56 - 5.19 (m, 4 H), 7.74 (s, 1 H), 8.70 (br. s., 1 H); LC/MS: C₂₄H₃₂F₃N₅O₄: m/z 512.0 (M+H).

### Example 4

### (3aR,5R,6aR)-5-(((3R,4R)-(3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide.

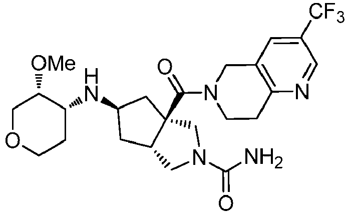

The 3^{rd} fraction of Example 2 from chiral HPLC (Kromasil K40813, 10-Amycoat, 30 x 250 mm; eluent: alcohol by EMD). ¹H-NMR (400 MHz, CDCl₃): δ 1.16 - 1.29 (m, 2 H), 1.58-2.02 (m, 5 H), 2.39 (br. s., 1 H), 2.81 (br. s., 1 H), 3.06 - 3.22 (m, 3 H), 3.25 - 3.47 (m, 6 H), 3.52 - 4.14 (m, 8 H), 4.60 - 5.10 (m, 4 H), 7.72 (s, 1 H), 8.71 (br. s., 1 H); LC/MS: C₂₄H₃₂F₃N₅O₄: m/z 512.0 (M+H).

### Example 5

### (3aR,5R,6aR)-5-(((3S*,4R*)-(3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide.

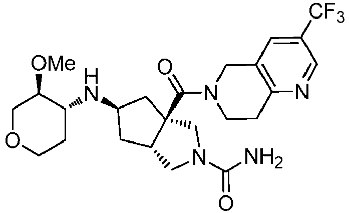

The 2^{nd} fraction of Example 2 (the minor product, a diastereoisomeric mixture) from chiral HPLC (Kromasil K40813, 10-Amycoat, 30 x 250 mm; eluent: alcohol by EMD). ¹H-NMR (400 MHz, CDCl₃): δ 1.19 - 1.48 (m, 2 H), 1.75 - 2.03 (m, 5 H), 2.29 - 2.56 (m, 2 H), 2.94 - 3.41 (m, 9 H), 3.46 - 4.13 (m, 8 H), 4.40 - 4.57 (m, 2 H), 4.63 - 5.03 (m, 2 H), 7.70 (br. s., 1 H), 8.72 (br. s., 1 H); LC/MS: C₂₄H₃₂F₃N₅O₄: m/z 512.0 (M+H).

### Example 6

### (3aR,5R,6aR)-5-(((3S*,4S*)-3-Methoxytetrahydro-2H-pyran-4-yl)amino)-3a-(7-(trifluoromethyl)-1,2,3,4-tetrahydroisoquinoline-2-carbonyl)hexahydrocyclopenta-[c]pyrrole-2(1H)-carboxamide

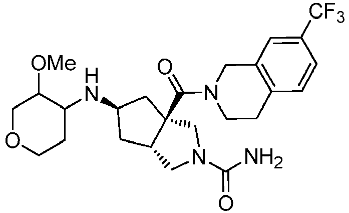

**Step A. *tert*-Butyl ((3a*R*,5*R*,6a*R*)-2-Cyano-3a-(7-(trifluoromethyl)-1,2,3,4-tetrahydroisoquinoline-2-carbonyl)octahydrocyclopenta[*c*]pyrrol-5-yl)carbamate.** To a mixture of Intermediate **2** (0.46 g, 1.014 mmol) and K₂CO₃ (0.168 g, 1.217 mmol) in acetonitrile (2.6 mL) was added cyanogen bromide (0.203 mL, 1.014 mmol, 5M in acetonitrile) at rt. After being stirred at rt overnight, the mixture was filtered and the filtrate was condensed *in vacuo*. The residue was purified by column chromatography (eluent: 80% EtOAc in hexanes to 100%) to give the product as a colorless gel. ¹H-NMR (400 MHz, CDCl₃): δ 1.31 - 1.46 (s, 9 H), 1.87 (t, *J*=6.6 Hz, 2 H), 2.36 (dd, *J*=13.4, 6.1 Hz, 1 H), 2.85 - 3.08 (m, 2 H), 3.23 (dd, *J*=9.6, 4.0 Hz, 1 H), 3.44 - 3.99 (m, 6 H), 4.23 (d, *J*=6.3 Hz, 1 H), 4.45 - 4.80 (m, 3 H), 7.28 - 7.34 (m, 1 H), 7.35 - 7.44 (m, 1 H), 7.46 (d, *J*=7.6 Hz, 1 H); LC/MS: C₂₄H₂₉F₃N₄O₃: m/z 479.2 (M+H).

**Step B. (3a*R*,5*R*,6a*R*)-5-Amino-3a-(7-(trifluoromethyl)-1,2,3,4-tetrahydroisoquinoline-2-carbonyl)hexahydrocyclopenta[*c*]pyrrole-2(1*H*)-carboxamide.** A solution of the product from Step **A** (0.03 g, 0.627mmol) in TFA (1 mL) and DCM (1 mL) was stirred at rt for 1.5 h. The volatile organic compounds were removed by evaporation, and the residue was diluted with DCM and evaporated again to give the product as a TFA salt. LC/MS: C₁₉H₂₃F₃N₄O₂: m/z 397.2 (M+H).

**Step C. (3a*R*,5*R*,6a*R*)-5-(((3*S**,4*S**)-3-Methoxytetrahydro-2*H*-pyran-4-yl)amino)-3a-(7-(trifluoromethyl)-1,2,3,4-tetrahydroisoquinoline-2-carbonyl)hexahydrocyclopenta-[*c*]pyrrole-2(1*H*)-carboxamide.** A mixture of the product from Step **B** (316 mg, 0.62 mmol), 3-methoxydihydro-*2H*-pyran-4(*3H*)-one (242 mg, 1.86 mmol), 4Å molecular sieves (0.3 g) and TEA (0.259mL, 1.86 mmol) in DCM (8 mL) was stirred at rt for 2 h, followed by addition of sodium triacetoxyborohydride (263 mg, 1.24 mmol). The resulting mixture was stirred at rt overnight. The reaction was quenched by the addition of saturated NaHCO₃ aqueous solution, extracted with DCM, and dried over Na₂SO₄. After removal of the solvent, the residue was purified by column chromatography (eluent: EtOAc to 15% 7N NH₃ in methanol in EtOAc) to give the product as a white foam. ¹H-NMR (400 MHz, CDCl₃): δ 1.76 (br. s., 1 H), 1.89 - 2.41 (m, 4 H), 2.53 (br. s., 1 H), 2.85 - 2.99 (m, 2 H), 3.06 - 4.02 (m, 18 H), 4.12 (q, *J*=7.1 Hz, 1 H), 4.69 (br. s., 2 H), 5.17 - 5.31 (m, 1 H), 7.25 (d, *J*=7.8 Hz, 1 H), 7.34 - 7.49 (m, 2 H); LC/MS: C₂₅H₃₃F₃N₄O₄: m/z 511.0 (M+H).

### Example 7

### (3aR,5R,6aR)-5-((2-Methoxyethyl)amino)-3a-(3-(trifluoromethyl)-5,6,7,8-tetrahydro-1,6-naphthyridine-6-carbonyl)hexahydrocyclopenta[c]pyrrole-2(1H)-carboxamide

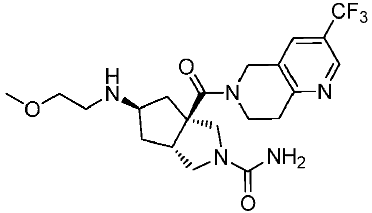

Prepared analogously to Example **6.** ¹H-NMR (400 MHz, CDCl₃): δ 1.70 - 1.98 (m, 4 H), 2.38 (br. s., 1 H), 2.58 - 2.81 (m, 2 H), 3.04 - 3.21 (m, 3 H), 3.26 - 3.35 (m, 3 H), 3.36 - 3.51 (m, 3 H), 3.59 (t, *J*=8.6 Hz, 1 H), 3.64 - 3.92 (m, 5 H), 4.55 - 4.73 (m, 3 H), 4.89 (br. s., 1 H), 7.70 (br. s., 1 H), 8.71 (s, 1 H); LC/MS: C₂₁H₂₈F₃N₅O₃: m/z 456.2 (M+H).

### Example 8

### (3aR,5R,6aR)-5-((Tetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxamide

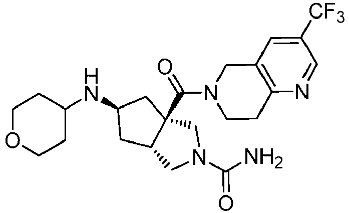

Prepared analogously to Example **6**. ¹H-NMR (400 MHz, CDCl₃): δ 1.29 - 1.92 (m, 8 H), 2.37 (br. s., 1 H), 2.64 (br. s., 1 H), 3.03 - 3.25 (m, 3 H), 3.26 - 3.46 (m, 2 H), 3.47 - 3.67 (m, 2 H), 3.65 - 4.03 (m, 7 H), 4.44 - 5.04 (m, 4 H), 7.69 (br. s., 1 H), 8.72 (s, 1 H); LC/MS: C₂₃H₃₀F₃N₅O₃: m/z 482.2 (M+H).

### Example 9

### (3aR,5R,6aR)-5-(((3S*,4S*)-3-Ethoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide

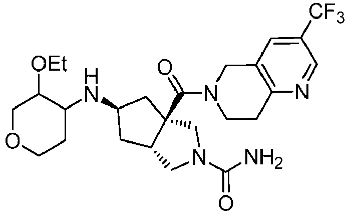

Prepared analogously to Example 6. ¹H-NMR (400 MHz, CDCl₃): δ 1.11 - 1.22 (m, 3 H), 1.53-1.94 (m, 5 H), 2.09 - 2.47 (m, 2 H), 2.63 - 2.81 (m, 1 H), 3.06 - 4.04 (m, 16 H), 4.63 - 5.01 (m, 4 H), 5.64 - 6.10 (m, 1 H), 7.71 (s, 1 H), 8.71 (br. s., 1 H); LC/MS: C₂₅H₃₄F₃N₅O₄: m/z 526.0 (M+H).

### Example 10

### (3aR,5R,6aR)-5-(((3S*,4S*)-3-Methoxytetrahydro-2H-pyran-4-yl)amino)-N^{3a}-(3-(trifluoromethyl)benzyl)hexahydrocyclopenta[c]pyrrole-2,3a(1H)-dicarboxamide

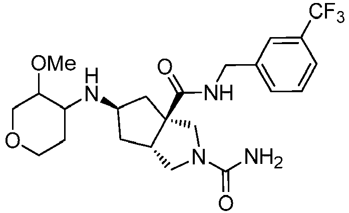

Prepared analogously to Example **6.** ¹H-NMR (400 MHz, CDCl₃): δ 1.00 - 1.05 (m, 1H), 1.35-2.04 (m, 5 H), 2.19 (dd, *J*=14.1, 9.9 Hz, 1 H), 2.39 - 2.66 (m, 2 H), 2.91 - 3.20 (m, 3 H), 3.20-3.41 (m, 5 H), 3.49 - 3.70 (m, 2 H), 3.69 - 3.91 (m, 1 H), 3.92 - 4.19 (m, 1 H), 4.29 - 4.47 (m, 2 H), 4.46 - 4.63 (m, 1 H), 4.85 (s, 2 H), 7.38 - 7.62 (m, 4 H), 9.60 - 9.86 (m, 1 H); LC/MS: C₂₃H₃₁F₃N₄O₄: m/z 485.2 (M+H).

### Example 11

### (3aR,5R,6aR)-N^{3a}-(3-Fluoro-5-(trifluoromethyl)benzyl)-5-(((3S*,4S*)-3-methoxytetrahydro-2H-pyran-4-yl)amino)hexahydrocyclopenta[c]pyrrole-2,3a(1H)-dicarboxamide

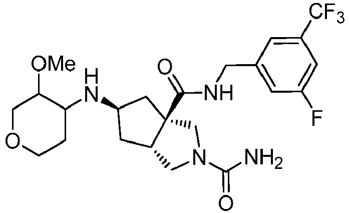

Prepared analogously to Example **6.** ¹H-NMR (400 MHz, CDCl₃): δ 0.97 - 1.18 (m, 1 H), 1.51-1.75 (m, 3 H), 1.79 - 2.05 (m, 3 H), 2.12 - 2.27 (m, 1 H), 2.59 (dt, *J*=11.2, 4.1 Hz, 1 H), 2.97-3.09 (m, 1 H), 3.08 - 3.41 (m, 7 H), 3.57 (ddd, *J*=10.9, 7.6, 3.7 Hz, 1 H), 3.67 (d, *J*=15.2 Hz, 1 H), 3.77 - 3.95 (m, 1 H), 3.99 - 4.21 (m, 1 H), 4.29 - 4.61 (m, 5 H), 7.12 - 7.34 (m, 3 H), 9.78-10.01 (m, 1 H); LC/MS: C₂₃H₃₀F₄N₄O₄: m/z 503.0 (M+H).

### Example 12

### (3aR,5R,6aR)-N^{3a}-(3,5-Bis(trifluoromethyl)benzyl)-5-(((3S*,4S*)-3-methoxytetrahydro-2H-pyran-4-yl)amino)hexahydrocyclopenta[c]pyrrole-2,3a(1H)-dicarboxamide

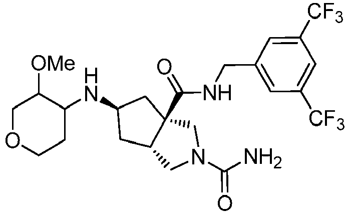

Prepared analogously to Example 6. ¹H-NMR (400 MHz, CDCl₃): δ 0.98 - 1.20 (m, 1 H), 1.39-2.11 (m, 5 H), 2.15 - 2.29 (m, 1 H), 2.50 - 2.66 (m, 1 H), 2.90 - 3.41 (m, 9 H), 3.48 - 3.59 (m, 1 H), 3.60 - 3.72 (m, 1 H), 3.75 - 3.95 (m, 1 H), 3.99 - 4.20 (m, 1 H), 4.30 - 4.68 (m, 3 H), 4.96 (br. s., 2 H), 7.62 - 7.87 (m, 3 H), 9.82 - 10.13 (m, 1 H); LC/MS: C₂₄H₃₀F₆N₄O₄: m/z 553.3 (M+H).

### Example 13

### (3aR,5R,6aR)-5-(((3S*,4S*)-(3-Methoxytetrahydro-2H-pyran-4-yl)amino)-N-methyl-3a-(3-(trifluoromethyl)-5,6,7,8-tetrahydro-1,6-naphthyridine-6-carbonyl)hexahydrocyclopenta-[c]pyrrole-2(1H)-carboxamide

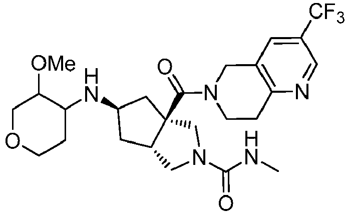

**Step A. *tert-*Butyl ((3a*R*,5*R*,6a*R*)-2-(methylcarbamoyl)-3a-(3-(trifluoromethyl)-5,6,7,8-tetrahydro-1,6-naphthyridine-6-carbonyl)octahydrocyclopenta[*c*]pyrrol-5-yl)carbamate.** To a solution of intermediate **1**(0.11 g, 0.242 mmol) in DCM (2 mL) or THF (2 mL) was added methyl isocyanate (16.9 mg, 0.29 mmol). The mixture was stirred at rt overnight. Aqueous workup and purification by CombiFlash (eluent: 8% methanol in DCM) gave the product as a colorless gel. ¹H-NMR (400 MHz, CDCl₃): δ 1.33 - 1.47 (m, 9 H), 1.72-2.44 (m, 3 H), 2.44 (br. s., 1 H), 2.67 - 2.88 (m, 4 H), 3.04 - 3.24 (m, 3 H), 3.54 (t, *J*=8.6 Hz, 1 H), 3.60 - 3.95 (m, 4 H), 4.25 (br. s., 1 H), 4.36 - 4.56 (m, 1 H), 4.61 - 5.04 (m, 3 H), 7.72 (s, 1 H), 8.71 (br. s., 1 H); LC/MS: C₂₄H₃₂F₃N₅O₄: m/z 512.3 (M+H).

**Step B. (3a*R*,5*R*,6a*R*)-5-Amino-*N*-methyl-3a-(3-(trifluoromethyl)-5,6,7,8-tetrahydro-1,6-naphthyridine-6-carbonyl)hexahydrocyclopenta[*c*]pyrrole-2(1*H*)-carboxamide.** A solution of the product from Step **A** (0.08 g, 0.156mmol) in TFA (1.5 mL) and DCM (1.5 mL) was stirred at rt for 1.5 h. The volatile organic compounds were removed by evaporation, and the residue was diluted with DCM and evaporated again to give the product as a TFA salt. LC/MS: C₁₉H₂₄F₃N₅O₂: m/z 412.5 (M+H).

**Step C. (3a*R*,5*R*,6a*R*)-5-(((3*S******,4*S******)-(3-Methoxytetrahydro-2*H*-pyran-4-yl)amino)-*N-*methyl-3a-(3-(trifluoromethyl)-5,6,7,8-tetrahydro-1,6-naphthyridine-6-carbonyl)hexahydrocyclopenta-[*c*]pyrrole-2(1*H*)-carboxamide.** A mixture of the product from Step **B** (99.8 mg, 0.156 mmol), 3-methoxydihydro-2*H*-pyran-4(3*H*)-one (60.91 mg, 0.468 mmol), 4Å molecular sieves (0.2 g) and TEA (0.0651mL, 0.468 mmol) in DCM (2 mL) was stirred at rt for 2 h, followed by addition of sodium triacetoxyborohydride (66.13 mg, 0.312 mmol). The resulting mixture was stirred at rt overnight. The reaction was quenched by addition of saturated NaHCO₃ aqueous solution, extracted with DCM, and dried over Na₂SO₄. After removal of the solvent, the residue was purified by column chromatography (eluent: 5% 7N NH₃ in methanol in DCM) to give the product as a yellowish gel. ¹H-NMR (400 MHz, CDCl₃): δ 1.52 - 1.95 (m, 4H), 2.34 (br. s., 1 H), 2.61 - 2.89 (m, 6 H), 3.03 - 3.61 (m, 11 H), 3.63 - 4.13 (m, 7 H), 4.27 (br. s., 1 H), 4.57 - 5.11 (m, 2 H), 7.69 (br. s., 1 H), 8.71 (br. s., 1 H); LC/MS: C₂₅H₃₄F₃N₅O₄: m/z 526.2 (M+H).

The following title compounds were synthesized using a similar procedure:

### Example 14

### (3aR,5R,6aR)-N-Isopropyl-5-(((3S*,4S*)-(3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxamide

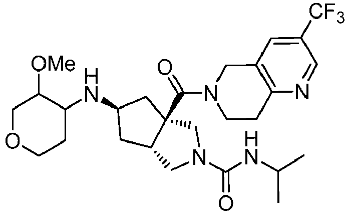

Prepared analogously to Example **13**. ¹H-NMR (400 MHz, CDCl₃): δ 1.15 (t, *J*=5.8 Hz, 6 H), 1.57 - 2.18 (m, 3 H), 2.40 (dd, *J*=12.6, 6.3 Hz, 1 H), 2.81 - 4.19 (m, 24 H), 4.57 - 5.02 (m, 2 H), 7.70 (s, 1 H), 8.71 (s, 1 H); LC/MS: C₂₇H₃₈F₃N₅O₄: m/z 554.2 (M+H).

### Example 15

### (3aR,5R,6aR)-N-Isobutyl-5-(((3S*4S*)-(3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide

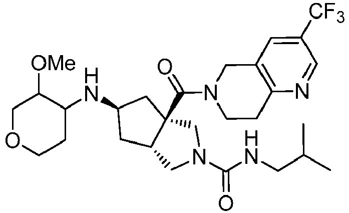

Prepared analogously to Example **13**. ¹H-NMR (400 MHz, CD₃OD): δ 0.87 (br. s., 6 H), 1.48-1.98 (m, 6 H), 2.29 - 4.28 (m, 26 H), 8.05 (br. s., 1 H), 8.69 (br. s., 1 H); LC/MS: C₂₈H₄₀F₃N₅O₄: m/z 568.5 (M+H).

### Example 16

### (3aR,5R,6aR)-5-(((3S*,4S*)-(3-methoxytetrahydro-2H-pyran-4-yl)amino)-N-phenyl-3a-(3-(trifluoromethyl)-5,6,7,8-tetrahydro-1,6-naphthyridine-6-carbonyl)hexahydrocyclopenta-[c]pyrrole-2(1H)-carboxamide

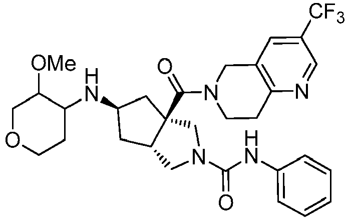

Prepared analogously to Example **13.** ¹H-NMR (400 MHz, CDCl₃): δ 1.58 - 1.94 (m, 6 H), 2.36 (br. s., 1 H), 2.72 (br. s., 1 H), 3.09 - 3.56 (m, 10 H), 3.68 - 4.08 (m, 8 H), 4.57 - 5.11 (m, 2 H), 6.39 (d, *J*=14.9 Hz, 1 H), 6.97 - 7.11 (m, 1 H), 7.21 - 7.33 (m, 2 H), 7.38 (d, *J*=7.8 Hz, 2 H), 7.68 (br. s., 1 H), 8.71 (br. s., 1 H); LC/MS: C₃₀H₃₆F₃N₅O₄: m/z 588.3 (M+H).

### Example 17

### (3aR,5R,6aR)-N-benzyl-5-(((3S*,4S*)-(3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxamide

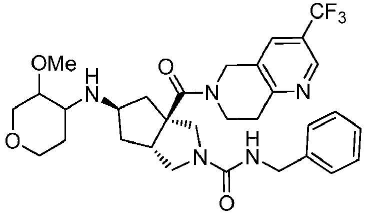

Prepared analogously to Example **13**. ¹H-NMR (400 MHz, CDCl₃): δ 1.59 - 1.94 (m, 6 H), 2.34 (br. s., 1 H), 2.72 (br. s., 1 H), 3.11 - 4.07 (m, 18 H), 4.40 (d, *J*=5.1 Hz, 2 H), 4.63 - 5.03 (m, 3 H), 7.26 - 7.39 (m, 5 H), 7.69 (br. s., 1 H), 8.71 (br. s., 1 H); LC/MS: C₃₁H₃₈F₃N₅O₄: m/z 602.5 (M+H).

### Example 18

### (3aR,5R,6aR)-5-(((3S*,4S*)-3-Methoxytetrahydro-2H-pyran-4-yl)amino)-N-neopentyl-3a-(3-(trifluoromethyl)-5,6,7,8-tetrahydro-1,6-naphthyridine-6-carbonyl)hexahydrocyclopenta[c]pyrrole-2(1H)-carboxamide

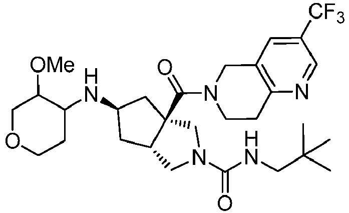

**Step A. *tert*-Butyl ((3a*R*,5*R*,6a*R*)-2-(neopentylcarbamoyl)-3a-(3-(tritluoromethyl)-5**,**6**,**7**,**8**-**tetrahydro**-**1**,**6**-**naphthyridine**-**6**-**carbonyl)octahydrocyclopenta[*c*]pyrrol-5-yl)carbamate.** A mixture of the intermediate **1** (0.10 g, 0.22 mmol) and TEA (0.128 mL, 0.924 mmol) in DCM (6 mL) was added dropwise to a solution of triphosgene (24.65 mg, 0.0814 mmol) in DCM (4 mL) at 0 °C. The mixture was stirred at 0 °C for 30 min and treated with a solution of neopentylamine (23.73 mg, 0.264 mmol) in DCM (2 mL). The mixture was then stirred at rt overnight and quenched by the addition of saturated aqueous NaHCO₃ solution, extracted with DCM, and dried over Na₂SO₄. Filtration and purification by CombiFlash (eluent: 5% methanol in DCM) gave the product as a colorless gel. ¹H-NMR (400 MHz, CDCl₃): δ 0.90 (d, *J*=3.8 Hz, 9H), 1.27 - 1.54 (m, 9 H), 1.74 - 2.10 (m, 3 H), 2.43 (br. s., 1 H), 2.86 - 3.38 (m, 5 H), 3.45 - 3.94 (m, 5H), 4.30 (t, *J*=5.9 Hz, 2 H), 4.45 - 5.07 (m, 3 H), 7.33 - 7.46 (m, 1 H), 7.69 (br. s., 1 H), 8.71 (br. s., 1 H); LC/MS: C₂₈H₄₀F₃N₅O₄: m/z 568.2 (M+H).

**Step B. (3a*R*,5*R*,6a*R*)-5-Amino-*N*-neopentyl-3a-(3-(trifluoromethyl)-5,6,7,8-tetrahydro-1,6-naphthyridine-6-carbonyl)hexahydrocyclopenta[*c*]pyrrole-2(1*H*)-carboxamide.** The title compound was prepared analogously to Step **B** in Example **13** as a TFA salt. LC/MS: C₂₃H₃₂F₃N₅O₂: m/z 468.2 (M+H).

**Step C. (3a*R*,5*R*,6a*R*)-5-(((3*S******,4*S******)-3-Methoxytetrahydro-2*H*-pyran-4-yl)amino)-*N-*neopentyl-3a-(3-(trifluoromethyl)-5,6,7,8-tetrahydro-1,6-naphthyridine-6-carbonyl)hexahydrocyclopenta[*c*]pyrrole-2(1*H*)-carboxamide.** The title compound was prepared analogously to Step **C** in Example **13.** ¹H-NMR (400 MHz, CDCl₃): δ 0.79 - 0.97 (m, 9 H), 1.07 - 1.35 (m, 2 H), 1.54 - 2.07 (m, 5 H), 2.36 (br. s., 1 H), 2.72 (br. s., 1 H), 2.95 - 3.22 (m, 4 H), 3.22 - 3.65 (m, 8 H), 3.68 - 4.00 (m, 6 H), 4.00 - 4.15 (m, 1 H), 4.28 (t, *J*=5.6 Hz, 1 H), 4.60 - 4.78 (m, 1 H), 4.90 (br. s., 1 H), 7.69 (br. s., 1 H), 8.71 (br. s., 1 H); LC/MS: C₂₉H₄₂F₃N₅O₄: m/z 582.2 (M+H).

### Example 19

### (3aR,5R,6aR)-N-(2-Methoxyethyl)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxamide

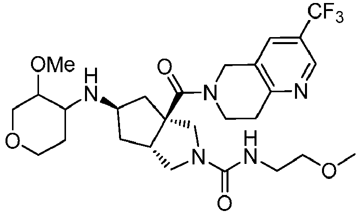

Prepared analogously to Example **18.** ¹H-NMR (400 MHz, CDCl₃): δ 0.77 - 0.94 (m, 1 H), 1.14 - 1.34 (m, 3 H), 1.53 - 1.90 (m, 7 H), 2.33 (br. s., 1 H), 2.70 (br. s., 1 H), 3.12 - 3.50 (m, 13 H), 3.62 - 3.95 (m, 7 H), 4.05 (ddd, *J*=11.6, 7.6, 3.3 Hz, 1 H), 4.57 - 5.04 (m, 2 H), 7.69 (s, 1 H), 8.71 (s, 1 H); LC/MS: C₂₇H₃₈F₃N₅O₅: m/z 570.2 (M+H).

### Example 20

### (3aR,5R,6aR)-5-(((3S*,4S*)-3-Methoxytetrahydro-2H-pyran-4-yl)amino)-N-(2-morpholinoethyl)-3a-(3-(trifluoromethyl)-5,6,7,8-tetrahydro-1,6-naphthyridine-6-carbonyl)hexahydrocyclopenta[c]pyrrole-2(1H)-carboxamide

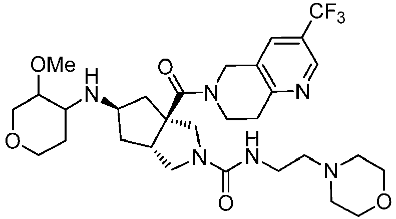

Prepared analogously to Example **18.** ¹H-NMR (400 MHz, CDCl₃): δ 0.77 - 0.94 (m, 3 H), 1.06 - 1.35 (m, 3 H), 1.51 - 1.88 (m,7 H), 2.49 (d, *J*=11.1 Hz, 5 H), 2.72 (br. s., 1 H), 3.01 - 3.20 (m, 2 H), 3.21 - 3.60 (m, 9 H), 3.64 - 3.97 (m, 8 H), 4.00 - 4.15 (m, 1 H), 4.63 - 5.02 (m, 2 H), 7.69 (s, 1 H), 8.71 (s, 1 H); LC/MS: C₃₀H₄₃F₃N₆O₅: m/z 625.5 (M+H).

### Example 21

### (3aR,5R,6aR)-5-(((3S*,4S*)-3-Methoxytetrahydro-2H-pyran-4-yl)amino)-N,N-dimethyl-3a-(3-(trifluoromethyl)-5,6,7,8-tetrahydro-1,6-naphthyridine-6-carbonyl)hexahydrocyclopenta[c]pyrrole-2(1H)-carboxamide

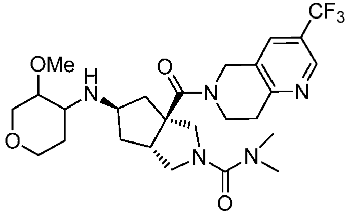

**Step A. *tert*-Butyl ((3a*R*,5*R*,6a*R*)-2-(dimethylcarbamoyl)-3a-(3-(trifluoromethyl)-5,6,7,8-tetrahydro-1,6-naphthyridine-6-carbonyl)octahydrocyclopenta[*c*]pyrrol-5-yl)carbamate.** To a solution of Intermediate **1** (0.11 g, 0.242 mmol) in DCM (10 mL) at 0 °C were added DIPEA (0.101 mL, 0.581 mmol) and *N,N*-dimethylcarbamoyl chloride (0.0544 mL, 0.581 mmol). The mixture was stirred at 0 °C for lh and at rt overnight. Aqueous workup and purification by CombiFlash (eluent: 8% methanol in DCM) gave the product as a colorless gel. ¹H-NMR (400 MHz, CDCl₃): δ 1.32 - 1.51 (m, 9 H), 1.74 - 2.06 (m, 2 H), 2.42 (dd, *J*=12.3, 5.7 Hz, 1 H), 2.76 - 2.90 (m, 7 H), 2.94 - 3.28 (m, 4 H), 3.49 - 3.93 (m, 5 H), 4.23 (br. s., 1 H), 4.61 - 5.00 (m, 3 H), 7.71 (s, 1 H), 8.71 (br. s., 1 H); LC/MS: C₂₅H₃₄F₃N₅O₄: m/z 526.5 (M+H).

**Step B. (3a*R*,5*R*,6a*R*)-5-Amino-*N*,*N*-dimethyl-3a-(3-(trifluoromethyl)-5,6,7,8-tetrahydro-1,6-naphthyridine-6-carbonyl)hexahydrocyclopenta[*c*]pyrrole-2(1*H*)-carboxamide.** The title compound was prepared analogously to Step **B** in Example **13** as a TFA salt. LC/MS: C₂₀H₂₆F₃N₅O₂: m/z 426.5 (M+H).

**Step C. (3a*R*,5*R*,6a*R*)-5-(((3*S******,4*S******)-3-Methoxytetrahydro-2*H*-pyran-4-yl)amino)-*N,N-*dimethyl-3a-(3-(trifluoromethyl)-5,6,7,8-tetrahydro-1,6-naphthyridine-6-carbonyl)hexahydrocyclopenta[*c*]pyrrole-2(1*H*)-carboxamide.** The title compound was prepared analogously to Step **C** in Example **13.** ¹H-NMR (400 MHz, CDCl₃): δ 1.59 - 2.11 (m, 5 H), 2.34 - 2.51 (m, 1 H), 2.74 - 2.95 (m, 7 H), 3.06 - 3.23 (m, 5 H), 3.24 - 3.47 (m, 7 H), 3.47 - 3.70 (m, 4 H), 3.76 - 4.00 (m, 4 H), 4.10 (d, *J*=12.1 Hz, 1 H), 4.79 (br. s., 2 H), 7.71 (s, 1 H), 8.71 (br. s., 1 H); LC/MS: C₂₆H₃₆F₃N₅O₄: m/z 540.3 (M+H).

### Example 22

### ((3aR,5R,6aR)-5-(((3S*,4S*)-3-Methoxytetrahydro-2H-pyran-4-yl)amino)-2-(morpholine-4-carbonyl)octahydrocyclopenta[c]pyrrol-3a-yl)(3-(trifluoromethyl)-7,8-dihydro-1,6-naphthyridin-6(5H)-yl)methanone

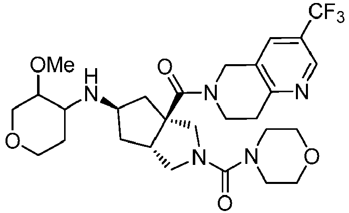

Prepared analogously to Example **21.** ¹H-NMR (400 MHz, CDCl₃): δ 1.84 - 2.14 (m, 4 H), 2.33 (br. s., 1 H), 3.01 - 3.35 (m, 11 H), 3.51 - 3.96 (m, 14 H), 4.45 (br. s., 1 H), 4.61 - 4.97 (m, 2 H), 7.71 (s, 1 H), 8.73 (s, 1 H); LC/MS: C₂₈H₃₅F₃N₅O₅: m/z 581.3 (M+H).

### Example 23

### ((3aR,5R,6aR)-2-(1H-Imidazole-1-carbonyl)-5-(((3S*,4S*)-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

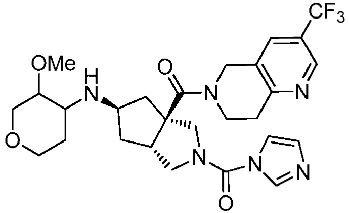

**Step A. *tert-*Butyl ((3a*R*,5*R*,6a*R*)-2-(1H-imidazole-1-carbonyl)-3a-(3-(trifluoromethyl)-5,6,7,8-tetrahydro-1,6-naphthyridine-6-carbonyl)octahydrocyclopenta[*c*]pyrrol-5-yl)carbamate.** To a solution of Intermediate **1** (135 mg, 0.297 mmol) in THF (5 mL) was added carbonyl diimidazole (57.8 mg, 0.356 mmol). The mixture was stirred at rt under Ar for 1.5 h and evaporated to give an oil, which was partitioned between water and DCM. The DCM solution was washed with brine (3 x), dried over Na₂SO₄. Filtration and evaporation to dryness gave the product as a white solid. ¹H-NMR (400 MHz, CDCl₃): δ 1.35 - 1.45 (m, 9 H), 1.73 - 2.13 (m, 2 H), 2.27 (t, *J*=7.7 Hz, 1 H), 2.46 - 2.56 (m, 1 H), 3.17 (br. s., 2 H), 3.41 - 3.55 (m, 1 H), 3.76 (d, *J*=5.9 Hz, 3 H), 3.89 - 4.10 (m, 3 H), 4.23 - 4.32 (m, 1 H), 4.49 - 5.02 (m, 3 H), 7.10 (br. s., 1 H), 7.29 - 7.35 (m, 1 H), 7.72 (s, 1 H), 7.98 (br. s., 1 H), 8.74 (br. s., 1 H); LC/MS: C₂₆H₃₁F₃N₆O₄: m/z 549.0 (M+H).

**Step B. ((3a*R*,5*R*,6a*R*)-5-Amino-2-(1H-imidazole-1-carbonyl)octahydrocyclopenta-[*c*]pyrrol-3a-yl)(3-(trifluoromethyl)-7,8-dihydro-1,6-naphthyridin-6(5*H*)-yl)methanone.** The title compound was prepared analogously to Step **B** in Example **13** as a TFA salt. LC/MS: C₂₁H₂₃F₃N₆O₂: m/z 449.0 (M+H).

**Step C. ((3a*R*,5*R*,6a*R*)-2-(1*H*-Imidazole-1-carbonyl)-5-(((3*S******,4*S******)-3-methoxytetrahydro-2*H*-pyran-4-yl)amino)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **13.** ¹H-NMR (400 MHz, CD₃OD): δ 1.76 - 2.41 (m, 5 H), 3.03 (br. s., 1 H), 3.36 - 3.83 (m, 13 H), 3.85 - 4.38 (m, 7 H), 4.98 (br. s., 2 H), 7.70 (br. s., 1 H), 8.08 (br. s., 1 H), 8.66 (br. s., 1 H), 9.09 (br. s., 1 H), 9.53 (s, 1 H); LC/MS: C₂₇H₃₃F₃N₆O₄: m/z 563.3 (M+H).

### Example 24

### ((3aR,5R,6aR)-2-(1H-Imidazole-1-carbonyl)-5-(((3S*,4S*)-3-methoxytetrahydro-2H-pyran-4-yl)amino)octahydrocyclopenta[c]pyrrol-3a-yl)(7-(trifluoromethyl)-3,4-dihydroisoquinolin-2(1H)-yl)methanone

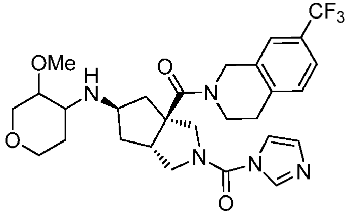

Prepared analogously to Example **23** from Intermediate **2.** ¹H-NMR (400 MHz, CD₃OD): δ 1.80 - 2.40 (m, 5 H), 3.01 (br. s., 3 H), 3.24 - 4.33 (m, 18 H), 4.66 - 4.84 (m, 2 H), 7.40 (br. s., 1 H), 7.45 - 7.80 (m, 3 H), 8.06 (br. s., 1 H), 9.50 (br. s., 1 H); LC/MS: C₂₈H₃₄F₃N₅O₄: m/z 562 (M+H).

### Example 25

### (3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carbothioamide

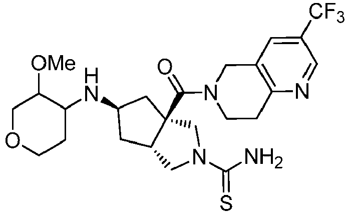

**Step A. *tert-* Butyl ((3a*R*,5*R*,6a*R*)-2-carbamothioyl-3a-(3-(trifluoromethyl)-5,6,7,8-tetrahydro**-**1**,**6**-**naphthyridine**-**6**-**carbonyl**)**octahydrocyclopenta[*c*]pyrrol**-**5**-**yl)carbamate**. To a solution of Intermediate **1** (136.3 mg, 0.3 mmol) in THF (5 mL) was added 1,1'-thiocarbonyldiimidazole (64.2 mg, 0.36 mmol), and the mixture was stirred at rt overnight. To the mixture was added ammonium hydroxide (5.11 mg, 0.3 mmol) and the resolution was heated at 65 °C overnight. Aqueous workup and purification by CombiFlash (eluent: 5% methanol in DCM) gave the product as a white solid. ¹H-NMR (400 MHz, CDCl₃): δ 1.31 - 1.46 (m, 9 H), 1.82 - 2.09 (m, 4 H), 2.42 - 2.63 (m, 1 H), 3.16 (br. s., 3 H), 3.59 - 3.88 (m, 4 H), 3.98 - 4.41 (m, 3 H), 4.57 - 4.98 (m, 3 H), 5.59 (br. s., 1 H), 7.72 (s, 1 H), 8.72 (br. s., 1 H); LC/MS: C₂₃H₃₀F₃N₅O₃S: m/z 514 (M+H).

**Step B. (3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrole-2(1*H*)-carbothioamide.** The title compound was prepared analogously to Step **B** in Example **13** as a TFA salt as a TFA salt. LC/MS: C₁₈H₂₂F₃N₅OS: m/z 414 (M+H).

**Step C. (3a*R*,5*R*,6a*R*)-5-(((3*S**,4*S**)-3-Methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrole-2(1*H*)-carbothioamide.** The title compound was prepared analogously to Step **C** in Example **13.** ¹H-NMR (400 MHz, DMSO-*d₆*): δ 1.87 - 2.35 (m, 9 H), 2.59 - 2.91 (m, 3 H), 3.29 - 4.73 (m, 14 H), 4.90 - 5.22 (m, 4 H), 7.90 (s, 1 H), 8.48 (s, 1 H); LC/MS: C₂₄H₃₂F₃N₅O₃S: m/z 528 (M+H).

### Example 26

### (3aR,5R,6aR)-N-Benzyl-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carbothioamide

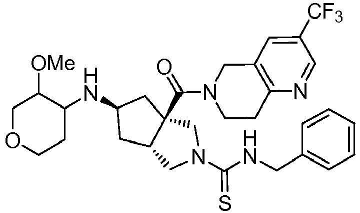

**Step A. *tert*-Butyl ((3a*R*,5*R*,6a*R*)-2-(benzylcarbamothioyl)-3a-(3-(trifluoromethyl)-5**,**6**,**7**,**8**-**tetrahydro**-**1**,**6**-**naphthyridine**-**6**-**carbonyl)octahydrocyclopenta[*c*]pyrrol-5-yl)carbamate.** To a solution of the Intermediate **1** (90.0 mg, 0.2 mmol) in THF (2 mL) was added a solution of benzyl isothiocyanate (29.84 mg, 0.2 mmol) in THF (1 mL). The mixture was stirred at rt for 1 h. Aqueous workup and purification by CombiFlash (eluent: 8% methanol in DCM) gave the product as a colorless oil. ¹H-NMR (400 MHz, CDCl₃): δ 1.32 - 1.44 (m, 9 H), 1.63 (br. s., 2 H), 1.80 - 2.06 (m, 5 H), 2.50 (t, *J*=8.3 Hz, 1 H), 3.09 - 3.32 (m, 3 H), 3.62 - 3.90 (m, 4 H), 4.11 (d, *J*=12.0 Hz, 1 H), 4.36 (t, *J*=7.1 Hz, 2 H), 4.56 - 4.94 (m, 2 H), 7.33 - 7.41 (m, 5 H), 7.71 (s, 1 H), 8.72 (s, 1 H); LC/MS: C₃₀H₃₆F₃N₅O₃S: m/z 604.5 (M+H).

**Step B. (3a*R*,5*R*,6a*R*)-5-Amino-*N*-benzyl-3a-(3-(trifluoromethyl)-5,6,7,8-tetrahydro-1,6-naphthyridine-6-carbonyl)hexahydrocyclopenta[*c*]pyrrole-2(1*H*)-carbothioamide.** The title compound was prepared analogously to Step **B** in Example **13** as a TFA salt. LC/MS: C₂₅H₂₈F₃N₅OS: m/z 504 (M+H).

**Step C. (3a*R*,5*R*,6a*R*)-*N*-Benzyl-5-(((3*****S******,4*S******)-3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrole-2(1*H*)-carbothioamide.** The title compound was prepared analogously to Step **C** in Example **13.** ¹H-NMR (400 MHz, CDCl₃): δ 1.66 (br. s., 5 H), 2.06 (d, *J*=12.7 Hz, 2 H), 2.33 (br. s., 1 H), 2.46 - 2.76 (m, 2 H), 3.13 - 3.55 (m, 9 H), 3.79 (d, *J*=6.4 Hz, 4 H), 4.00 (br. s., 1 H), 4.17 (d, *J*=12.0 Hz, 2 H), 4.68 - 4.94 (m, 5 H), 7.31 - 7.40 (m, 5 H), 7.69 (br. s., 1 H), 8.71 (s, 1 H); LC/MS: C₃₁H₃₈F₃N₅O₃S: m/z 618 (M+H).

### Example 27

### (3aR,5R,6aR)-5-(((3S*,4S*)-3-Methoxytetrahydro-2H-pyran-4-yl)amino)-N-methyl-3a-(3-(trifluoromethyl)-5,6,7,8-tetrahydro-1,6-naphthyridine-6-carbonyl)hexahydrocyclopenta[c]pyrrole-2(1H)-carbothioamide

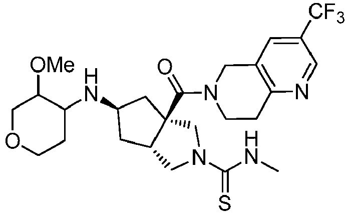

A solution of Example **1** (0.1 g, 0.213 mmol) in DCM (1 mL) was added methyl isothiocyanate (19.31 mg, 0.256 mmol) and the mixture was stirred at rt overnight. The reaction was quenched by addition of saturated aqueous NaHCO₃ solution, extracted with DCM, dried over Na₂SO₄. Filtration and purification by CombiFlash (eluent: 5% methanol in DCM) gave the product Example **27** as a white solid, ¹H-NMR (400 MHz, CDCl₃): δ 1.56 - 1.89 (m, 6 H), 2.41 (br. s., 1 H), 2.71 (br. s., 1 H), 3.08 - 3.57 (m, 13 H), 3.64 - 4.12 (m, 7 H), 4.33 (br. s., 1 H), 4.62 - 5.02 (m, 2 H), 5.46 (d, *J*=2.5 Hz, 1 H), 7.71 (s, 1 H), 8.71 (br. s., 1 H); LC/MS: C₂₅H₃₄F₃N₅O₃S: m/z 542.3 (M+H).

### Example 28

### (3aR,5R,6aR)-5-(1-((3S*,4S*)-3-Methoxytetrahydro-2H-pyran-4-yl)-3-methylthioureido)-N-methyl-3a-(3-(trifluoromethyl)-5,6,7,8-tetrahydro-1,6-naphthyridine-6-carbonyl)hexahydrocyclopenta[c]pyrrole-2(1H)-carbothioamide

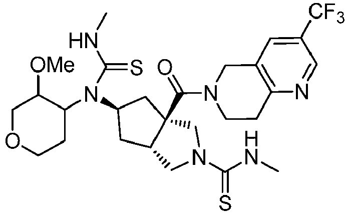

Prepared as a byproduct of the procedure used in Example **27,** the title compound was isolated as a white solid. LC/MS: C₂₇H₃₇F₃N₆O₃S₂: m/z 615.2 (M+H).

### Example 29

### (3aR,5R,6aR)-N-Isobutyl-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carbothioamide

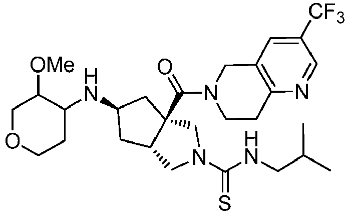

Prepared analogously to Example **27,** a white solid. ¹H-NMR (400 MHz, CDCl₃): δ 0.89 - 0.99 (m, 6 H), 1.56 - 1.99 (m, 6 H), 2.31 - 2.53 (m, 1 H), 2.71 (br. s., 1 H), 3.08 - 3.58 (m, 13 H), 3.63 - 4.17 (m, 7 H), 4.34 (br. s., 1 H), 4.61 - 5.02 (m, 2 H), 5.31 (br. s., 1 H), 7.71 (s, 1 H), 8.72 (br. s., 1 H); LC/MS: C₂₈H₄₀F₃N₅O₃S: m/z 584.2 (M+H).

### Example 30

### (3aR,5R,6aR)-N-Isobutyl-5-((3S*,4S*)-3-isobutyl-1-(3-methoxytetrahydro-2H-pyran-4-yl)thioureido)-3a-(3-(trifluoromethyl)-5,6,7,8-tetrahydro-1,6-naphthyridine-6-carbonyl)hexahydrocyclopenta[c]pyrrole-2(1)-carbothioamide

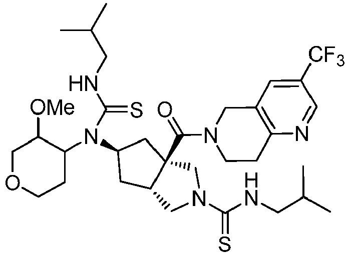

Prepared analogously to Example **28,** a white solid. LC/MS: C₃₃H₄₉F₃N₆O₃S₂: m/z 699.5 (M+H).

### Example 31

### ((3aR,5R,6aR)-2-(1H-Imidazole-1-carbonothioyl)-5-(((3S*,4S*)-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

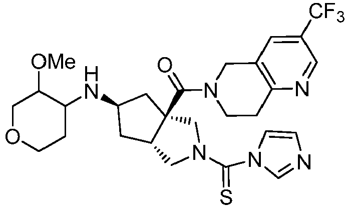

**Step A. *tert-*Butyl((3a*R*,5*R*,6a*R*)-2-(1*H*-imidazole-1-carbonothioyl)-3a-(3-(trifluoromethyl)**-**5**,**6**,**7**,**8**-**tetrahydro**-**1**,**6**-**naphthyridine**-**6**-**carbonyl)octahydrocyclopenta[*c*]pyrrol-5-yl)carbamate.** To a solution of Intermediate **1** (409 mg, 0.9 mmol) in THF (15 mL) was added 1,1'-thiocarbonyldiimidazole (176.2 mg, 0.99 mmol), and the mixture was stirred at rt overnight. A little solid was filtered and the filtrate was evaporated. The residue was purified by CombiFlash (eluent: 5% methanol in DCM) gave the product as a colorless oil. ¹H-NMR (400 MHz, CDCl₃): δ 1.30 - 1.44 (m, 9 H), 1.78 - 2.15 (m, 2 H), 2.50 (s, 1 H), 3.18 (br. s., 1H), 3.39-3.89 (m, 6 H), 3.96 (d, *J*=7.1 Hz, 1 H), 4.35 (d, *J*=7.1 Hz, 3 H), 4.71 (s, 3 H), 7.08 (s, 1 H), 7.38 (d, *J*=4.6 Hz, 1 H), 7.75 (s, 1 H), 7.94 (s, 1 H), 8.74 (s, 1 H); LC/MS: C₂₆H₃₁F₃N₆O₃S: m/z 565.5 (M+H).

**Step B. ((3a*R*,5*R*,6a*R*)-5-Amino-2-(1*H*-imidazole-1-carbonothioyl)octahydrocyclopenta[*c*]pyrrol-3a-yl)(3-(trifluoromethyl)-7,8-dihydro-1,6-naphthyridin-6(5*H*)-yl)methanone tritrifluoroacetate.** The title compound was prepared analogously to Step **B** in Example **13** as a TFA salt. LC/MS: C₂₁H₂₃F₃N₆OS: m/z 465.5 (M+H).

**Step C. ((3a*R*,5*R*,6a*R*)-2-(1*H*-Imidazole-1-carbonothioyl)-5-(((3*S******,4*S******)-3-methoxytetrahydro-2H-pyran-4-yl)amino)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **13** as an oil. ¹H-NMR (400 MHz, CD₃OD): δ 1.79 - 2.45 (m, 5 H), 2.97 - 4.50 (m, 22 H), 4.96 (s, 1 H), 7.70 (s, 1 H), 8.11 (br. s., 1 H), 8.43 (br. s., 1 H), 8.96 (br. s., 1 H), 9.54 (s, 1 H); LC/MS: C₂₇H₃₃F₃N₆O₃S: m/z 579 (M+H).

### Example 32

### (3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-sulfonamide

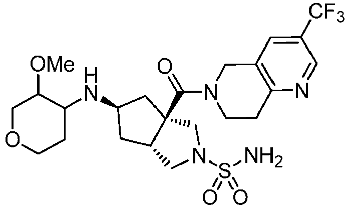

**Step A. *tert*-Butyl ((3a*R*,5*R*,6a*R*)-5-((*tert*-butoxycarbonyl)amino)-3a-(3-(trifluoromethyl)-5,6,7,8-tetrahydro-1,6-naphthyridine-6-carbonyl)hexahydrocyclopenta[*c*]pyrrol-2(1*H*)-yl)sulfonylcarbamate.** To a cool (0 °C) solution of chlorosulfonyl isocyanate (0.1 mL, 1.129 mol) in DCM (2 mL) was added a solution of *tert*-butanol (83.7 mg, 1.129 mmol) in DCM (2 mL). After being stirred at 0 °C for 30 min, the resulting solution and TEA (0.318 mL, 2.257 mmol) were added dropwise to a solution of the Intermediate 1 (513 mg, 1.129 mmol) in DCM (3 mL) while keeping the temperature below 5 °C. The reaction mixture was stirred at rt for 2 h, quenched by addition of brine. Aqueous workup and purification by CombiFlash (eluent: 5% methanol in DCM) gave the product as a yellow oil. ¹H-NMR (400 MHz, CDCl₃): δ 1.33 - 1.49 (m, 18 H), 1.72 - 1.95 (m, 3 H), 2.42 (br. s., 1 H), 3.02 - 3.33 (m, 4 H), 3.48 - 3.60 (m, 1 H), 3.69 - 3.98 (m, 4 H), 4.25 (br. s., 1 H), 4.55 - 4.95 (m, 3 H), 7.41 - 7.62 (m, 1 H), 7.71 (s, 1 H), 8.72 (s, 1 H); LC/MS: C₂₇H₃₈F₃N₅O₇S: m/z 634.2 (M+H).

**Step B. (3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrole-2(1*H*)-sulfonamide.** The title compound was prepared analogously to Step **B** in Example **13** as a TFA salt. LC/MS: C₁₇H₂₂F₃N₅O₃S: m/z 434.5 (M+H).

**Step C. (3a*R*,5*R*,6a*R*)-5-(((3*S******,4*S******)-3-Methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrole-2(1*H*)-sulfonamide.** The title compound was prepared analogously to Step **C** in Example **13** as a yellow gel. ¹H-NMR (400 MHz, CDCl₃): δ 1.54 - 1.91 (m, 4 H), 2.32 (d, *J*=6.6 Hz, 1 H), 2.73 (br. s., 1 H), 3.04 - 3.18 (m, 3 H), 3.22-3.45 (m, 8 H), 3.47 - 3.60 (m, 2 H), 3.72 - 3.99 (m, 3 H), 4.06 (ddd, *J*=15.9, 7.4, 3.5 Hz, 1 H), 4.78 (br. s., 2 H), 5.30 (d, *J*=10.4 Hz, 2 H), 5.59 - 5.88 (m, 3 H), 7.72 (s, 1 H), 8.71 (br. s., 1 H); LC/MS: C₂₃H₃₂F₃N₅O₅S: m/z 548.0 (M+H).

An alternative way to make Example **32:**
A mixture of Example **1** (42 mg, 0.0896 mmol) and sulfamide (15 mg, 0.155 mmol) in DME (1 mL) in a sealed tube was heated at 90 °C overnight. After condensation, the residue was purified by CombiFlash (eluent: 5% 7*N* NH₃ in methanol in DCM) to give the product as a yellow oil. LC/MS: C₂₃H₃₂F₃N₅O₅S: m/z 548.2 (M+H).

### Example 33

### (3aR,5R,6aR)-N-Benzyl-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-sulfonamide

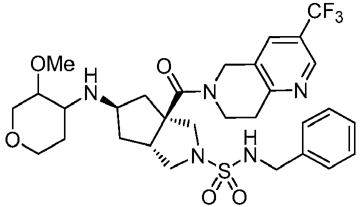

**Step A. *tert*-Butyl benzyl(((3a*R*,5*R*,6a*R*)-5-((*tert*-butoxycarbonyl)amino)-3a-(3-(trifluoromethyl)-5,6,7,8-tetrahydro-1,6-naphthyridine-6-carbonyl)hexahydrocyclopenta-[*c*]pyrrol-2(1*H*)-yl)sulfonyl)carbamate.** To a cool (0 °C) solution of **34**A (90 mg, 0.142mol), TPP (37 mg, 0.141 mmol) and benzyl alcohol (15.1 mg, 0.14 mmol) in THF (3 mL) under Ar was added dropwise a solution of DIAD (29 mg, 0.143 mmol) in THF(0.5 mL). After being stirred at rt overnight, the mixture was condensed and dissolved in DMSO and purified by HPLC to give the product as a clear oil.

**Step B. (3a*R*,5*R*,6a*R*)-5-Amino-*N*-benzyl-3a-(3-(trifluoromethyl)-5,6,7,8-tetrahydro-1,6-naphthyridine-6-carbonyl)hexahydrocyclopenta[*c*]pyrrole-2(1*H*)-sulfonamide.** The title compound was prepared analogously to Step **B** in Example **13** as a TFA salt. LC/MS: C₂₄H₂₈F₃N₅O₃S: m/z 524 (M+H).

**Step C. (3a*R*,5*R*,6a*R*)-*N*-Benzyl-5-(((3*S******,4*S******)-3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrole-2(1*H*)-sulfonamide.** The title compound was prepared analogously to Step **C** in Example **13** as a yellow gel. ¹H-NMR (400 MHz, CDCl₃): δ 1.70 - 2.19 (m, 5 H), 2.65 (s, 1 H), 2.97 - 3.62 (m, 15 H), 3.64 - 4.03 (m, 5 H), 4.19 (br. s., 3 H), 4.74 (br. s., 2 H), 7.08 - 7.38 (m, 5 H), 8.05 (br. s., 1 H), 8.73 (br. s., 1 H); LC/MS: C₃₀H₃₈F₃N₅O₅S: m/z 638 (M+H).

### Example 34

### (3aR,5R,6aR)-Methyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

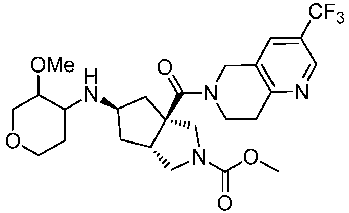

**Step A. (3a*R*,5*R*,6a*R*)-Methyl 5-((*tert*-butoxycarbonyl)amino)-3a-(3-(trifluoromethyl)-5,6,7,8-tetrahydro-1,6-naphthyridine-6-carbonyl)hexahydrocyclopenta[*c*]pyrrole-2(1*H*)-carboxylate.** To a cool (0 °C) solution of Intermediate **1** (0.11 g, 0.242 mol), TEA (0.132, 0.944 mmol) in DCM (3 mL) under Ar was added methyl chloroformate (0.0564 mL, 0.726 mmol) dropwise. The mixture was then stirred at 0 °C for 30 min and rt overnight. The reaction was quenched by addition of saturated NaHCO₃, separated, extracted with DCM, and dried over Na₂SO₄. Purification by CombiFlash (eluent: EtOAc) gave the product as a white foam. ¹H-NMR (400 MHz, CDCl₃): δ 1.32 - 1.46 (m, 9 H), 1.73 - 2.03 (m, 3 H), 2.31 - 2.52 (m, 1 H), 2.93 - 3.36 (m, 3 H), 3.55 - 3.88 (m, 9 H), 4.24 (d, *J*=4.5 Hz, 1 H), 4.48 - 5.01 (m, 3 H), 7.71 (br. s., 1 H), 8.72 (br. s., 1 H); LC/MS: C₂₄H₃₁F₃N₄O₅: m/z 513.5 (M+H).

**Step B. (3a*R*,5*R*,6a*R*)-Methyl 5-(methylamino)-3a-(3-(trifluoromethyl)-5,6,7,8-tetrahydro-1,6-naphthyridine-6-carbonyl)hexahydrocyclopenta[*c*]pyrrole-2(1*H*)-carboxylate.** The title compound was prepared analogously to Step **B** in Example **13** as a TFA salt. LC/MS: C₁₉H₂₃F₃N₄O₂: m/z 413.5 (M+H).

**Step C. (3a*R*,5*R*,6a*R*)-Methyl 5-(((3*S******,4*S******)-3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrole-2(1*H*)-carboxylate.** The title compound was prepared analogously to Step **C** in Example **13** as a yellow gel. ¹H-NMR (400 MHz, CDCl₃): δ 1.54 - 1.92 (m, 5 H), 2.21 - 2.38 (m, 2 H), 2.53 - 2.65 (m, 3 H), 2.66 - 2.78 (m, 1 H), 3.09 - 4.28 (m, 18 H), 4.65 - 4.95 (m, 2 H), 7.70 (br. s., 1 H), 8.72 (br. s., 1 H); LC/MS: C₂₅H₃₃F₃N₄O₅: m/z 527.2 (M+H).

The following title compounds were synthesized using a similar procedure:

### Example 35

### (3aR,5R,6aR)-Propyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrole-2(1H)-carboxylate

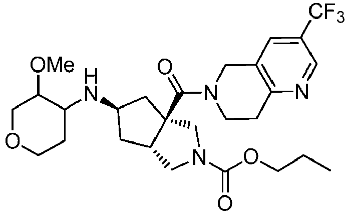

Prepared analogously to Example **34.** ¹H-NMR (400 MHz, CDCl₃): δ 0.93 (d, *J*=6.3 Hz, 3 H), 1.53 - 2.44 (m, 6 H), 2.75 (br. s., 1 H), 3.05 - 4.17 (m, 23 H), 4.61 - 5.06 (m, 2 H), 7.71 (br. s., 1 H), 8.72 (br. s., 1 H); LC/MS: C₂₇H₃₇F₃N₄O₅: m/z 555.2 (M+H).

### Example 36

### (3aR,5R,6aR)-Neopentyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrole-2(1H)-carboxylate

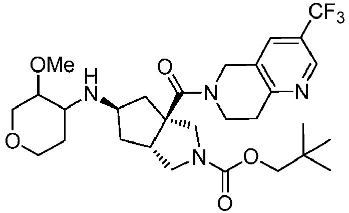

Prepared analogously to Example **34.** ¹H-NMR (400 MHz, CDCl₃): δ 0.93 (br. s., 9 H), 1.53 - 2.04 (m, 3 H), 2.32 (br. s., 1 H), 2.74 (br. s., 1 H), 3.05 - 4.14 (m, 23 H), 4.60 - 5.03 (m, 2 H), 7.69 (br. s., 1 H), 8.71 (br. s., 1 H); LC/MS: C₂₉H₄₁F₃N₄O₅: m/z 583.3 (M+H).

### Example 37

### (3aR,5R,6aR)-Phenyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrole-2(1H)-carboxylate

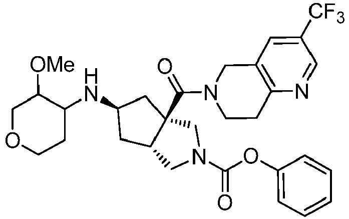

Prepared analogously to Example **34.** ¹H-NMR (400 MHz, CDCl₃): δ 1.59 - 2.12 (m, 3 H), 2.38 (br. s., 1 H), 2.76 (br. s., 1 H), 3.06 - 4.19 (m, 21 H), 4.61 - 5.06 (m, 2 H), 7.04 - 7.23 (m, 3 H), 7.30 - 7.43 (m, 2 H), 7.71 (br. s., 1 H), 8.72 (br. s., 1 H); LC/MS: C₃₀H₃₅F₃N₄O₅: m/z 589.3 (M+H).

### Example 38

### (3aR,5R,6aR)-Allyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

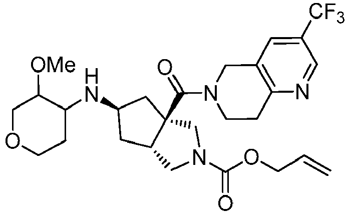

Prepared analogously to Example **34.** ¹H-NMR (400 MHz, CDCl₃): δ 1.54 - 1.93 (m, 3 H), 2.31 (br. s., 1 H), 2.73 (br. s., 1 H), 3.06 - 4.12 (m, 21 H), 4.58 (d, *J*=5.6 Hz, 2 H), 4.65 - 5.00 (m, 2 H), 5.16 - 5.36 (m, 2 H), 5.83 - 6.00 (m, 1 H), 7.70 (br. s., 1 H), 8.72 (s, 1 H); LC/MS: C₂₇H₃₅F₃N₄O₅: m/z 553.3 (M+H).

### Example 39

### (3aR,5R,6aR)-2-Morpholinoethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

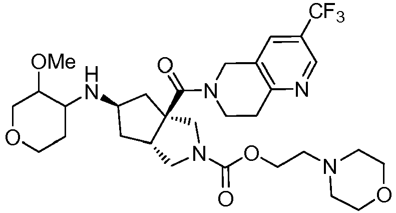

**A. (3a*R*,5*R*,6a*R*)-2-Morpholinoethyl 5-((3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrole-2(1*H*)-carboxylate.** To a mixture of 4-(2-hydroxyethyl)morpholine (0.0299 mL, 0.242 mmol), *N,N'*-disuccinimidyl carbonate (62.0 mg, 0.242 mmol) in acetonitrile (1.2 mL) and DCM (1.2 mL) was added DMAP (9.462 mg, 0.0774 mmol). The mixture was stirred at rt for 2 h and treated with intermediate **1** (110 mg, 0.242 mmol) and DMF (0.2 mL). After being stirred at rt overnight, the reaction mixture was concentrated and partitioned between aqueous NaHCO₃ solution and DCM, separated, and dried over Na₂SO₄. Purification by CombiFlash (eluent: 8% methanol in DCM) gave the product as a colorless gel. ¹H-NMR (400 MHz, CDCl₃): δ 1.20 - 1.33 (m, 1 H), 1.86 (br. s., 3 H), 2.45 (br. s., 1 H), 2.60 - 3.05 (m, 16 H), 3.08 - 3.34 (m, 3 H), 3.56 - 3.89 (m, 9 H), 4.13 - 4.41 (m, 3 H), 4.70 - 5.01 (m, 2 H), 8.01 (s, 1 H), 8.71 (br. s., 1 H); LC/MS: C₂₉H₄₀F₃N₅O₆: m/z 612.3 (M+H).

**B. (3a*R*,5*R*,6a*R*)-2-Morpholinoethyl 5-amino-3a-(3-(trifluoromethyl)-5,6,7,8-tetrahydro-1,6-naphthyridine-6-carbonyl)hexahydrocyclopenta[*c*]pyrrole-2(1*H*)-carboxylate.** The title compound was prepared analogously to Step **B** in Example **13** as a TFA salt. LC/MS: C₂₄H₃₂F₃N₅O₄: m/z 512.5 (M+H).

**C. (3a*R*,5*R*,6a*R*)-2-Morpholinoethyl 5-(((3*S**,4*S******)-3-methoxytetrahydro-2*H-*pyran-4-yl)amino)-3a-(3-(trifluoromethyl)-5,6,7,8-tetrahydro-1,6-naphthyridine-6-carbonyl)hexahydrocyclopenta[*c*]pyrrole-2(1*H*)-carboxylate.** The title compound was prepared analogously to Step **C** in Example **13** as a yellow gel. ¹H-NMR (400 MHz, CDCl₃): δ 1.51 - 1.97 (m, 5 H) 2.21 - 2.79 (m, 6 H) 3.06 - 4.30 (m, 27 H) 4.60 - 5.03 (m, 2 H) 7.70 (br. s., 1 H) 8.72 (br. s., 1 H); LC/MS: C₃₀H₄₂F₃N₅O₆: m/z 626.2 (M+H).

The following title compounds were synthesized using a similar procedure:

### Example 40

### (3aR,5R,6aR)-2-Morpholinoethyl 5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydro-cyclopenta[c]pyrrole-2(1H)-carboxylate

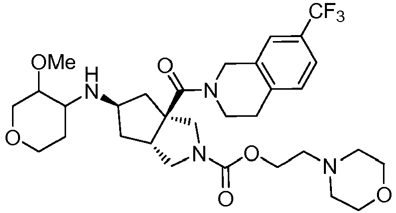

Prepared analogously to Example **39.** ¹H-NMR (400 MHz, CDCl₃): δ 1.50 - 2.01 (m, 5 H), 2.24 - 4.29 (m, 33 H), 4.68 - 4.89 (m, 2 H), 7.29 (br. s., 1 H), 7.40 (br. s., 2 H); LC/MS: C₃₁H₄₃F₃N₄O₆: m/z 625.3 (M+H).

### Example 41

### (3aR,5R,6aR)-2-(Diethylamino)ethyl 5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydrocyclopenta[c]pyrrole-2(1H)-carboxylate.

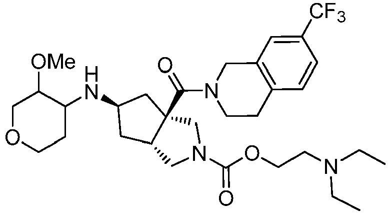

Prepared analogously to Example **39.** ¹H-NMR (400 MHz, CDCl₃): δ 1.05 (t, *J*=6.7 Hz, 6 H), 1.55 - 1.91 (m, 6 H), 2.29 (br. s., 1 H), 2.54 - 2.80 (m, 7 H), 2.95 (br. s., 2 H), 3.19 - 3.50 (m, 8 H), 3.57 - 3.81 (m, 6 H), 3.86 - 3.97 (m, 1 H), 4.00 - 4.23 (m, 3 H), 4.71 (br. s., 2 H), 7.26 (br. s., 1 H), 7.33 - 7.53 (m, 2 H); LC/MS: C₃₁H₄₅F₃N₄O₅: m/z 611.2 (M+H).

### Example 42

### (3aR,5R,6aR)-2-(Tetrahydro-2H-pyran-4-yl)ethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

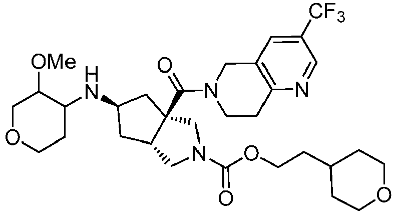

Prepared analogously to Example **39.** ¹H-NMR (400 MHz, CDCl₃): δ 1.14 - 1.33 (m, 2 H), 1.45 - 1.89 (m, 10 H), 2.25 (br. s., 1 H), 2.67 (br. s., 1 H), 2.98 - 4.13 (m, 25 H), 4.51 - 5.01 (m, 2 H), 7.63 (br. s., 1 H), 8.65 (br. s., 1 H); LC/MS: C₃₁H₄₃F₃N₄O₆: m/z 625.3 (M+H).

### Example 43

### (3aR,5R,6aR)-2-(Tetrahydro-2H-pyran-4-yl)ethyl 5-(((3S,4S)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

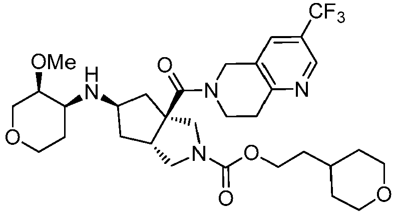

Isolated by chiral HPLC (Kromasil K40813, 10-Amycoat, 30 x 250 mm; eluent: 50% isopropyl alcohol in heptanes) from the product of Example **39.** The first fraction. ¹H-NMR (400 MHz, CDCl₃): δ 1.13 - 1.40 (m, 2 H), 1.52 - 1.98 (m, 10 H), 2.33 (br. s., 1 H), 2.72 (br. s., 1 H), 3.05 - 4.22 (m, 25 H), 4.60 - 5.04 (m, 2 H), 7.73 (br. s., 1 H), 8.72 (br. s., 1 H).

### Example 44

### (3aR,5R,6aR)-2-(Tetrahydro-2H-pyran-4-yl)ethyl 5-(((3R,4R)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

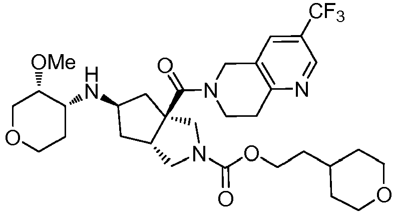

Isolated by chiral HPLC (Kromasil K40813, 10-Amycoat, 30 x 250 mm; eluent: 50% isopropyl alcohol in heptanes) from the product of Example **39.** The second fraction. ¹H-NMR (400 MHz, CDCl₃): δ 1.12 - 1.40 (m, 2 H), 1.45 - 1.98 (m, 10 H), 2.32 (br. s., 1 H), 2.74 (br. s., 1 H), 3.01 - 4.21 (m, 25 H), 4.59 - 5.04 (m, 2 H), 7.70 (br. s., 1 H), 8.70 (br. s., 1 H).

### Example 45

### (3aR,5R,6aR)-2-(Pyrrolidin-1-yl)ethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxyl

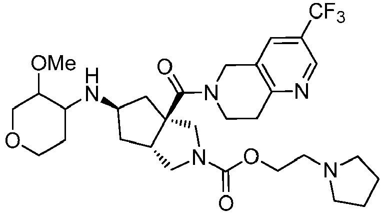

Prepared analogously to Example **39.** ¹H-NMR (400 MHz, CDCl₃): δ 1.53 - 1.96 (m, 6 H), 2.12 - 2.85 (m, 10 H), 2.92 - 4.32 (m, 22 H), 4.59 - 5.05 (m, 2 H), 7.71 (d, *J*=7.8 Hz, 1 H), 8.72 (br. s., 1 H); LC/MS: C₃₀H₄₂F₃N₅O₅: m/z 610.3 (M+H).

### Example 46

### (3aR,5R,6aR)-2-(Pyrrolidin-1-yl)ethyl 5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydrocyclopenta[c]pyrrole-2(1H)-carboxylate

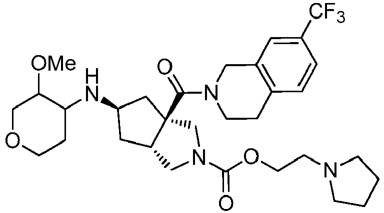

Prepared analogously to Example **39.** ¹H-NMR (400 MHz, CDCl₃): δ 1.41 - 1.86 (m, 9 H), 2.05 - 2.36 (m, 1 H), 2.39 - 2.98 (m, 9 H), 3.05 - 4.04 (m, 17 H), 4.07 - 4.25 (m, 2 H), 4.64 (br. s., 2 H), 7.14 - 7.23 (m, 1 H), 7.24 - 7.44 (m, 2 H); LC/MS: C₃₁H₄₃F₃N₄O₅: m/z 609.2 (M+H).

### Example 47

### (3aR,5R,6aR)-(Tetrahydro-2H-pyran-4-yl)methyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate.

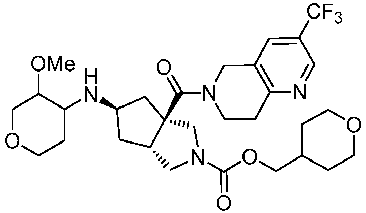

Prepared analogously to Example 39. ¹H-NMR (400 MHz, CDCl₃): δ 1.21 - 1.46 (m, 2 H), 1.50 - 2.17 (m, 5 H), 2.32 (br. s., 1 H), 2.74 (br. s., 1 H), 3.03 - 4.14 (m, 28 H), 4.60 - 5.06 (m, 2 H), 7.71 (br. s., 1 H), 8.72 (br. s., 1 H); LC/MS: C₃₀H₄₁F₃N₄O₆: m/z 611.2 (M+H).

### Example 48

### (3aR,5R,6aR)-2-Methoxyethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclo-penta[c]pyrrole-2(1H)-carboxylate.

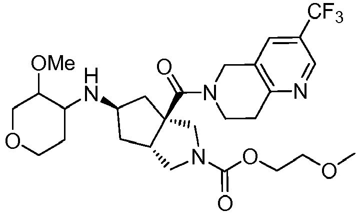

Prepared analogously to Example **39.** ¹H-NMR (400 MHz, CDCl₃): δ 1.50 - 1.92 (m, 5 H), 2.32 (br. s., 1 H), 2.73 (br. s., 1 H), 2.92 - 4.15 (m, 24 H), 4.23 (br. s., 2 H), 4.72 (m, 2 H), 7.71 (br. s., 1 H), 8.72 (br. s., 1 H); LC/MS: C₂₇H₃₇F₃N₄O₆: m/z 571.3 (M+H).

### Example 49

### (3aR,5R,6aR)-2-Methoxyethyl 5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydrocyclopenta[c]pyrrole-2(1H)-carboxylate.

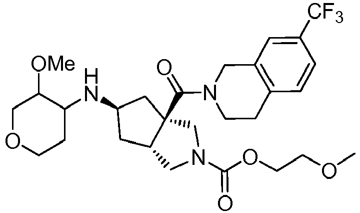

Prepared analogously to Example **39.** ¹H-NMR (400 MHz, CDCl₃): δ 1.48 - 1.97 (m, 6 H), 2.30 (br. s., 1 H), 2.72 (br. s., 1 H), 2.96 (br. s., 2 H), 3.19 - 3.53 (m, 11 H), 3.53 - 3.82 (m, 8 H), 3.85 - 3.98 (m, 1 H), 4.00 - 4.10 (m, 1 H), 4.16 - 4.29 (m, 2 H), 4.46 - 4.49 (m, 2 H), 7.22 - 7.31 (m, 1 H), 7.33 - 7.50 (m, 2 H); LC/MS: C₂₈H₃₈F₃N₃O₆: m/z 571.3 (M+H).

### Example 50

### (3aR,5R,6aR)-3-Hydroxy-3-methylbutyl 5-(((3S*,4S*)-3-methoxytetrahydro-2H-pyran-4-yl)amino)-3a-(7-(trifluoromethoxy)-1,2,3,4-tetrahydroisoquinoline-2-carbonyl)hexahydrocyclopenta[c]pyrrole-2(1H)-carboxylate.

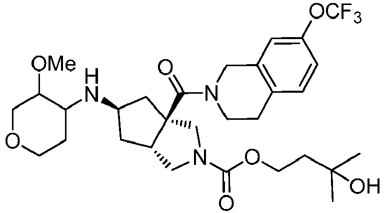

Prepared analogously to Example **39.** ¹H-NMR (400 MHz, CDCl₃): δ 1.26 (s, 6 H), 1.53 - 2.05 (m, 9 H), 2.28 (br. s., 1 H), 2.72 (br. s., 1 H), 2.89 (d, *J*=4.0 Hz, 2 H), 3.16 - 3.52 (m, 8 H), 3.51 - 3.83 (m, 6 H), 3.83 - 3.98 (m, 1 H), 3.99 - 4.11 (m, 1 H), 4.25 (t, *J*=6.6 Hz, 2 H), 4.66 (br. s., 2 H), 6.91 - 7.11 (m, 2 H), 7.17 (d, *J*=8.1 Hz, 1 H); LC/MS: C₃₀H₄₂F₃N₃O₇: m/z 614.2 (M+H).

### Example 51

### (3aR,5R,6aR)-3,3,3-Trifluoropropyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclo-penta[c]pyrrole-2(1H)-carboxylate

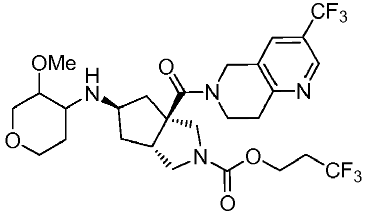

Prepared analogously to Example **39.** ¹H-NMR (400 MHz, CDCl₃): δ 1.53 - 2.12 (m, 5 H), 2.25 - 2.79 (m, 1 H), 2.93 - 4.36 (m, 24 H), 4.63 - 5.03 (m, 2 H), 7.71 (br. s., 1 H), 8.72 (br. s., 1 H); LC/MS: C₂₇H₃₄F₆N₄O₅: m/z 609.2 (M+H).

### Example 52

### (3aR,5R,6aR)-2-Cyanoethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate.

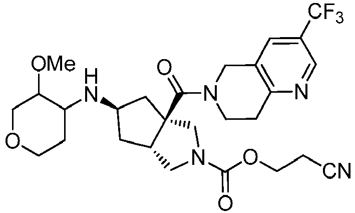

Prepared analogously to Example **39.** ¹H-NMR (400 MHz, CDCl₃): δ 1.55 - 1.90 (m, 5 H), 2.33 (br. s., 1 H), 2.62 - 2.80 (m, 3 H), 3.07 - 3.57 (m, 11 H), 3.60 - 4.12 (m, 8 H), 4.18 - 4.37 (m, 2 H), 4.62 - 5.02 (m, 2 H), 7.71 (d, *J*=6.8 Hz, 1 H), 8.72 (br. s., 1 H); LC/MS: C₂₇H₃₄F₃N₅O₅: m/z 566.2 (M+H).

### Example 53

### (3aR,5R,6aR)-Prop-2-yn-1-yl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate.

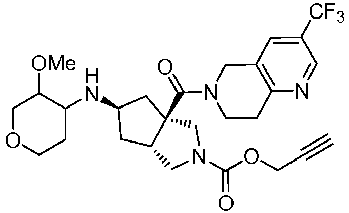

Prepared analogously to Example **39.** ¹H-NMR (400 MHz, CDCl₃): δ 1.56 - 2.03 (m, 3 H), 2.23 - 2.80 (m, 3 H), 3.07 - 4.29 (m, 21 H), 4.65 - 4.95 (m, 4 H), 7.71 (br. s., 1 H), 8.72 (br. s., 1 H); LC/MS: C₂₇H₃₃F₃N₄O₅: m/z 551.2 (M+H).

### Example 54

### (3aR,5R,6aR)-2-(Dimethylamino)ethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)-hexahydrocyclopenta[c]pyrrole-2(1H)-carboxylate.

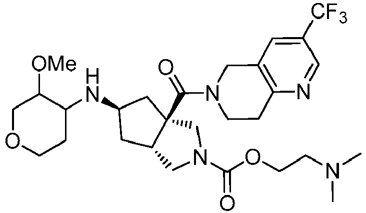

Prepared analogously to Example **39.** ¹H-NMR (400 MHz, CDCl₃): δ 1.55 - 1.98 (m, 5 H), 2.25 - 2.36 (m, 7 H), 2.51 - 2.81 (m, 3 H), 3.05 - 3.55 (m, 11 H), 3.59 - 4.28 (m, 10 H), 4.60 - 5.00 (m, 2 H), 7.62 - 7.77 (m, 1 H), 8.72 (s, 1 H); LC/MS: C₂₈H₄₀F₃N₅O₅: m/z 584.2 (M+H).

### Example 55

### (3aR,5R,6aR)-2-Acetamidoethyl 5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydrocyclopenta[c]pyrrole-2(1H)-carboxylate.

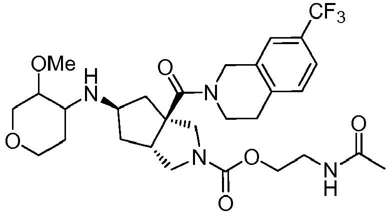

Prepared analogously to Example **39.** ¹H-NMR (400 MHz, CDCl₃): δ 1.45 - 1.91 (m, 6 H), 1.98 (br. s., 3 H), 2.31 (br. s., 1 H), 2.72 (br. s., 1 H), 2.97 (br. s., 2 H), 3.18 - 4.28 (m, 20 H), 4.70 (d, *J*=15.7 Hz, 2 H), 6.08 - 6.46 (m, 1 H), 7.30 (d, *J*=6.6 Hz, 1 H), 7.43 (d, *J*=10.6 Hz, 2 H); LC/MS: C₂₉H₃₉F₃N₄O₆: m/z 597.3 (M+H).

### Example 56

### (3aR,5R,6aR)-Benzyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carboxylate

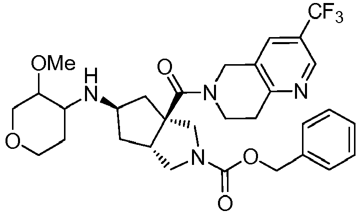

Prepared analogously to Example **39.** ¹H-NMR (400 MHz, CDCl₃): δ 1.51 - 2.44 (m, 4 H), 2.88 - 4.23 (m, 22 H), 4.73 (br. s., 2 H), 5.08 - 5.33 (m, 2 H), 7.35 (br. s., 5 H), 7.69 (br. s., 1 H), 8.72 (br. s., 1 H); LC/MS: C₃₁H₃₇F₃N₅O₅: m/z 603.3 (M+H).

### Example 57

### (3aR,5R,6aR)-Benzyl 5-(1-((3S*,4S*)-3-methoxytetrahydro-2H-pyran-4-yl)-3-methylthioureido)-3a-(3-(trifluoromethyl)-5,6,7,8-tetrahydro-1,6-naphthyridine-6-carbonyl)hexahydrocyclopenta[c]pyrrole-2(1H)-carboxylate

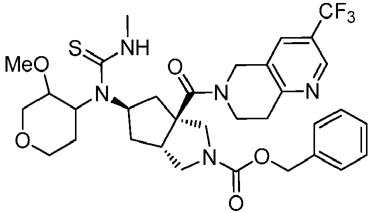

Prepared analogously to Example **28** from the product of Example **56.** ¹H-NMR (400 MHz, CDCl₃): δ 1.26 (br. s., 1 H), 1.57 - 4.28 (m, 27 H), 4.79 (br. s., 2 H), 5.13 (br. s., 2 H), 5.81 (br. s., 1 H), 7.36 (br. s., 5 H), 7.75 - 7.76 (br. s., 1 H), 8.71 (br. s., 1 H); LC/MS: C₃₃H₄₀F₃N₅O₅S: m/z 676.2 (M+H).

### Example 58

### (3aR,5R,6aR)-4-Cyanobenzyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxylate.

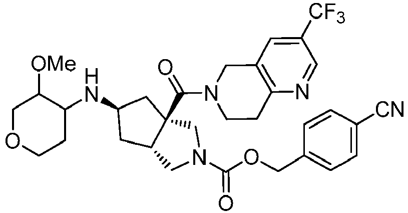

Prepared analogously to Example **39.** ¹H-NMR (400 MHz, CDCl₃): δ 1.55 - 1.91 (m, 2 H), 2.33 (br. s., 1 H), 2.73 (br. s., 1 H), 3.08 - 4.28 (m, 22 H), 4.60 - 5.03 (m, 2 H), 5.08 - 5.25 (m, 2 H), 7.38 - 7.51 (m, 2 H), 7.58 - 7.75 (m, 3 H), 8.72 (br. s., 1 H); LC/MS: C₃₂H₃₆F₃N₅O₅: m/z 628.3 (M+H).

### Example 59

### (3aR,5R,6aR)-3-Cyanobenzyl 5-((3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxylate.

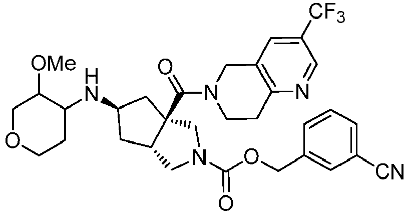

Prepared analogously to Example **39.** ¹H-NMR (400 MHz, CDCl₃): δ 1.53 - 1.99 (m, 2 H), 2.24 - 2.43 (m, 1 H), 2.56 - 2.81 (m, 1 H), 3.12 - 4.28 (m, 22 H), 4.67 - 4.96 (m, 2 H), 5.14 (br. s., 2 H), 7.49 - 7.75 (m, 5 H), 8.71 (br. s., 1 H); LC/MS: C₃₂H₃₆F₃N₅O₅: m/z 628.3 (M+H).

### Example 60

### (3aR,5R,6aR)-3-Methoxybenzyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxylate

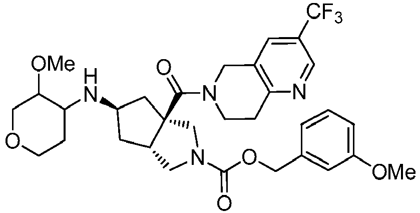

Prepared analogously to Example **39.** ¹H-NMR (400 MHz, CDCl₃): δ 1.55 - 2.41 (m, 3 H), 2.64 - 2.79 (m, 1 H), 3.07 - 4.13 (m, 25 H), 4.63 - 4.96 (m, 2 H), 5.09 (d, *J*=4.0 Hz, 2 H), 6.80 - 6.97 (m, 3 H), 7.24 - 7.32 (m, 1 H), 7.69 (br. s., 1 H), 8.71 (s, 1 H); LC/MS: C₃₂H₃₉F₃N₄O₅: m/z 633.3 (M+H).

### Example 61

### (3aR,5R,6aR)-4-Methoxybenzyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboxylate

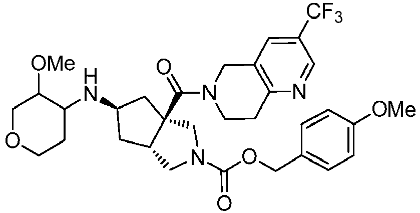

To a mixture of 4-methoxybenzyl alcohol (7.05 mg, 0.05 mmol), *N*,*N'*-disuccinimidyl carbonate (12.8 mg, 0.05 mmol) in acetonitrile (0.3 mL) and DCM (0.3 mL) was added DMAP (1.955 mg, 0.016 mmol). The mixture was stirred at rt for 2 h and treated with Example **1** (23.43 mg, 0.05 mmol) and DMF (0.05 mL). After being stirred at rt overnight, the reaction mixture was concentrated and partitioned between aqueous NaHCO₃ solution and DCM, separated, and dried over Na₂SO₄. Purification by CombiFlash (eluent: 8% MeOH in DCM) gave the product as a colorless gel. ¹H-NMR (400 MHz, CDCl₃): δ 1.64 - 1.99 (m, 2 H), 2.21 (br. s., 1 H), 2.40 (br. s., 1 H), 2.94 - 3.45 (m, 8 H), 3.55 - 4.20 (m, 17 H), 4.76 (br. s., 2 H), 5.04 (br. s., 2 H), 6.88 (d, *J*=8.1 Hz, 2 H), 7.24 - 7.35 (m, 2 H), 7.71 (s, 1 H), 8.70 (br. s., 1 H); LC/MS: C₃₂H₃₉F₃N₄O₆: m/z 633.3 (M+H).

### Example 62

### (3aR,5R,6aR)-3-Hydroxy-3-methylbutyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)-hexahydrocyclopenta[c]pyrrole-2(1H)-carboxylate

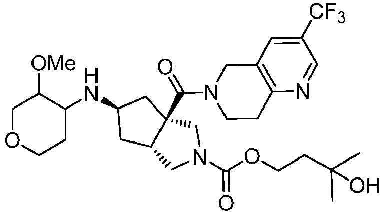

Prepared analogously to Example **61.** ¹H-NMR (400 MHz, CDCl₃): δ 1.26 (s, 6 H), 1.55 - 1.91 (m, 3 H), 2.12 - 2.42 (m, 3 H), 2.73 (br. s., 1 H), 2.93 - 4.34 (m, 24 H), 4.63 - 5.03 (m, 2 H), 7.71 (br. s., 1 H), 8.71 (br. s., 1 H); LC/MS: C₂₉H₄₁F₃N₄O₆: m/z 599.2 (M+H).

### Example 63

### (3aR,5R,6aR)-2-Acetamidoethyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclo-penta[c]pyrrole-2(1H)-carboxylate.

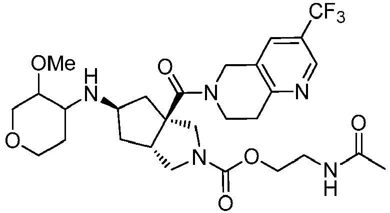

Prepared analogously to Example **61.** ¹H-NMR (400 MHz, CDCl₃): δ 1.56 - 2.04 (m, 7 H), 2.33 (br. s., 1 H), 2.73 (br. s., 1 H), 3.10 - 4.27 (m, 24 H), 4.61 - 5.04 (m, 2 H), 6.13 - 6.45 (m, 1 H), 7.64 - 7.83 (m, 1 H), 8.72 (br. s., 1 H); LC/MS: C₂₈H₃₈F₃N₅O₆: m/z 598.2 (M+H).

### Example 64

### (3aR,5R,6aR)-Isobutyl 5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrole-2(1H)-carboxylate

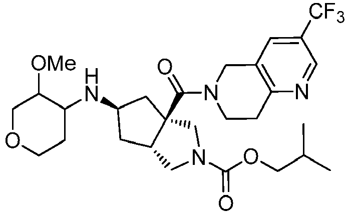

To a solution of Example 1 (0.1 g, 0.213 mmol) and TEA (0.12 mL, 0.854 mmol) in DCM (2.6 mL) at 0 °C was added isobutyl chloroformate (0.086 mL, 0.64 mmol). The mixture was stirred at 0 °C for 30 min and at rt overnight. The reaction was quenched by addition of saturated aqueous NaHCO₃ solution, extracted with DCM, dried over Na₂SO₄. Purification by CombiFlash (eluent: 5% methanol in DCM, then 5% 7*N* NH₃ in methanol in DCM) gave the title compound as a white solid. ¹H-NMR (400 MHz, CDCl₃): δ 0.93 (d, *J*=6.3 Hz, 6 H), 1.57 - 1.99 (m, 7 H), 2.32 (br. s., 1 H), 2.74 (br. s., 1 H), 3.08 - 3.56 (m, 10 H), 3.56 - 4.00 (m, 9 H), 4.01 - 4.11 (m, 1 H), 4.57 - 5.07 (m, 2 H), 7.70 (br. s., 1 H), 8.72 (br. s., 1 H); LC/MS: C₂₈H₃₉F₃N₄O₅: m/z 569.3 (M+H).

### Example 65

### (E)-1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-3-phenylprop-2-en-1-one

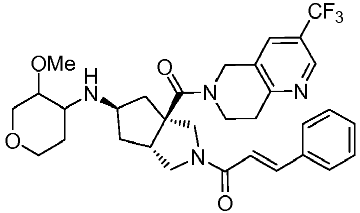

**Step A**. ***tert-*Butyl ((3a*R*,5*R*,6a*R*)-2-cinnamoyl-3a-(3-(trifluoromethyl)-5,6,7,8-tetrahydro-1,6-naphthyridine-6-carbonyl)octahydrocyclopenta[*c*]pyrrol-5-yl)carbamate.** To a solution of Intermediate **1** (0.11 g, 0.242 mmol) and TEA (0.0538 mL, 0.387 mmol) in DCM (2 mL) at 0 °C was added cinnamoyl chloride (45.26 mg, 0.266 mol). The mixture was stirred at 0 °C for 30 min and at rt for 2 days. The reaction mixture was quenched by addition of brine, extracted with DCM, and dried over Na₂SO₄. Purification by CombiFlash (eluent: 60% EtOAc in hexanes) gave the product as a yellow gel. ¹H-NMR (400 MHz, CDCl₃): δ 1.32 - 1.44 (m, 9 H), 1.85 - 1.97 (m, 3 H), 2.47 (br. s., 1 H), 3.17 (br. s., 2 H), 3.55 (dd, *J*=10.1, 4.0 Hz, 1 H), 3.70 - 4.36 (m, 7 H), 4.63 - 5.05 (m, 3 H), 6.68 (d, *J*=15.7 Hz, 1 H), 7.31 - 7.43 (m, 3 H), 7.52 (d, *J*=4.3 Hz, 2 H), 7.63 - 7.77 (m, 2 H), 8.72 (br. s., 1 H); LC/MS: C₃₁H₃₅F₃N₄O₄: m/z 585.5 (M+H).

**Step B. (*E*)-1-((3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrol-2(1*H*)-yl)-3-phenylprop-2-en-1-one.** The title compound was prepared analogously to Step **B** in Example **13** as a TFA salt. LC/MS: C₂₆H₂₇F₃N₄O₂: m/z 485.5 (M+H).

**Step C. (*E*)-1-((3a*R*,5*R*,6a*R*)-5-(((3*S**,4*S**)-3-Methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrol-2(1*H*)-yl)-3-phenylprop-2-en-1-one.** The title compound was prepared analogously to Step **C** in Example **13** as a yellow gel. ¹H-NMR (400 MHz, CDCl₃): δ 1.53 - 2.15 (m, 5 H), 2.39 (br. s., 1 H), 2.73 (br. s., 1 H), 3.07 - 4.15 (m, 19 H), 4.61 - 5.05 (m, 2 H), 6.70 (d, *J*=15.4 Hz, 1 H), 7.32 - 7.43 (m, 3 H), 7.52 (d, *J*=4.8 Hz, 2 H), 7.63 - 7.76 (m, 2 H), 8.72 (br. s., 1 H); LC/MS: C₃₂H₃₇F₃N₄O₄: m/z 599.2 (M+H).

The following title compounds were synthesized using a similar procedure:

### Example 66

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)ethanone

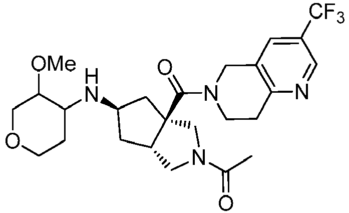

Prepared analogously to Example **65.** ¹H-NMR (400 MHz, CDCl₃): δ 1.53 - 1.96 (m, 5 H), 2.04 (s, 3 H), 2.35 (d, *J*=2.3 Hz, 1 H), 2.72 (br. s., 1 H), 3.05 - 4.17 (m, 19 H), 4.58 - 5.06 (m, 2 H), 7.58 - 7.79 (m, 1 H), 8.72 (br. s., 1 H); LC/MS: C₂₅H₃₃F₃N₄O₄: m/z 511.5 (M+H).

### Example 67

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-3-phenylpropan-1-one.

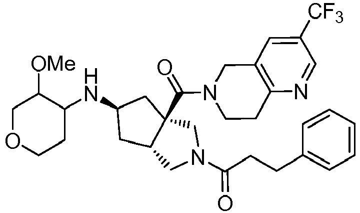

Prepared analogously to Example **65.** ¹H-NMR (400 MHz, CDCl₃): δ 1.54 - 1.91 (m, 5 H), 2.31 (br. s., 1 H), 2.46 - 2.60 (m, 2 H), 2.70 (br. s., 1 H), 2.87 - 4.15 (m, 21 H), 4.59 - 5.04 (m, 2 H), 7.17 - 7.33 (m, 5 H), 7.69 (br. s., 1 H), 8.72 (br. s., 1 H); LC/MS: C₃₂H₃₉F₃N₄O₄: m/z 601.3 (M+H).

### Example 68

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-2-phenoxyethanone

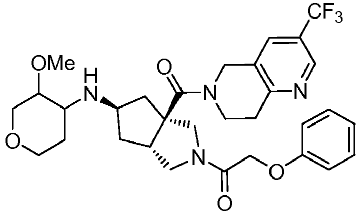

Prepared analogously to Example **65.** ¹H-NMR (400 MHz, CDCl₃): δ 1.49 - 1.92 (m, 5 H), 2.13 - 2.39 (m, 1 H), 2.67 (br. s., 1 H), 3.04 - 4.10 (m, 19 H), 4.53 - 5.06 (m, 4 H), 6.82 - 7.04 (m, 3 H), 7.29 (t, *J*=7.6 Hz, 2 H), 7.69 (br. s., 1 H), 8.72 (br. s., 1 H); LC/MS: C₃₁H₃₇F₃N₄O₅: m/z 603.3 (M+H).

### Example 69

### ((3aR,5R,6aR)-2-Benzoyl-5-(((3S*,4S*)-3-methoxytetrahydro-2H-pyran-4-yl)amino)-octahydrocyclopenta[c]pyrrol-3a-yl)(3-(trifluoromethyl)-7,8-dihydro-1,6-naphthyridin-6(5H)-yl)methanone.

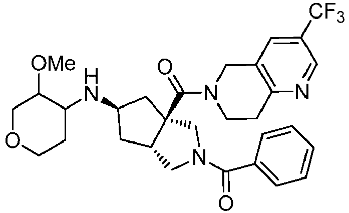

Prepared analogously to Example **65.** ¹H-NMR (400 MHz, CD₃OD): δ 1.79 - 2.32 (m, 5 H), 2.75 - 3.16 (m, 2 H), 3.25 - 4.20 (m, 17 H), 4.27 (dd, *J*=13.3, 5.5 Hz, 2 H), 4.75 - 5.05 (m, 2 H), 7.49 (d, *J*=8.8 Hz, 5 H), 8.56 (br. s., 1 H), 9.05 (br. s., 1 H); LC/MS: C₃₀H₃₅F₃N₄O₄: m/z 573 (M+H).

### Example 70

### ((3aR,5R,6aR)-5-(((3S*,4S*)-3-Methoxytetrahydro-2H-pyran-4-yl)amino)-2-(oxazole-2-carbonyl)octahydrocyclopenta[c]pyrrol-3a-yl)(3-(trifluoromethyl)-7,8-dihydro-1,6-naphthyridin-6(5H)-yl)methanone.

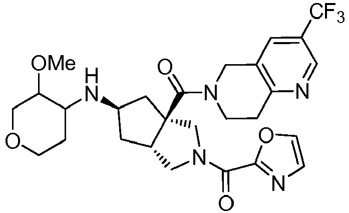

Prepared analogously to Example **65.** ¹H-NMR (400 MHz, CD₃OD): δ 1.75 - 2.29 (m, 5 H), 2.86 (br. s., 1 H), 3.09 - 3.60 (m, 11 H), 3.79 (m, 1 H), 3.88 - 4.62 (m, 8 H), 4.80 - 4.90 (m, 2 H), 7.39 (s, 1 H), 7.97 - 8.15 (m, 2 H), 8.73 (s, 1 H); LC/MS: C₂₇H₃₂F₃N₅O₅: m/z 564 (M+H).

### Example 71

### Isoxazol-5-yl((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)methanone.

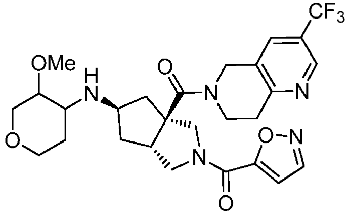

Prepared analogously to Example **65.** ¹H-NMR (400 MHz, CD₃OD): δ 1.80 - 2.35 (m, 5 H), 2.85 - 3.00 (m, 1 H), 3.22 - 3.85 (m, 11 H), 3.92 - 4.40 (m, 9 H), 4.84 - 5.05 (m, 2 H), 6.97 (d, *J*=2.0 Hz, 1 H), 8.53 (d, *J*=2.0 Hz, 2 H), 9.07 (br. s., 1 H); LC/MS: C₂₇H₃₂F₃N₅O₅: m/z 564 (M+H).

### Example 72

### ((3aR,5R,6aR)-5-(((3S*,4S*)-3-Methoxytetrahydro-2H-pyran-4-yl)amino)-2-(oxazole-4-carbonyl)octahydrocyclopenta[c]pyrrol-3a-yl)(3-(trifluoromethyl)-7,8-dihydro-1,6-naphthyridin-6(5H)-yl)methanone.

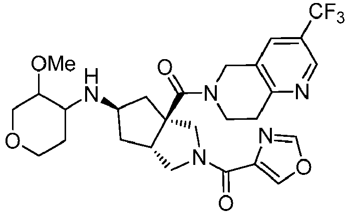

Prepared analogously to Example **65.** ¹H-NMR (400 MHz, CD₃OD): δ 1.84 - 2.38 (m, 5 H), 2.93 (dd, *J*=13.8, 8.2 Hz, 1 H), 3.28 - 4.57 (m, 20 H), 4.88 - 5.12 (m, 2 H), 8.28 (s, 1 H), 8.47 (br. s., 1 H), 8.65 (br. s., 1 H), 9.09 (d, *J*=16.6 Hz, 1 H); LC/MS: C₂₇H₃₂F₃N₅O₅: m/z 564 (M+H).

### Example 73

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)prop-2-yn-1-one

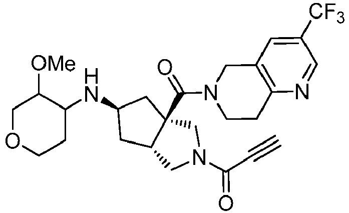

**Step A. *tert*-Butyl ((3a*R*,5*R*,6a*R*)-2-propioloyl-3a-(3-(trifluoromethyl)-5,6,7,8-tetrahydro-1,6-naphthyridine-6-carbonyl)octahydrocyclopenta[*c*]pyrrol-5-yl)carbamate.** To a solution of Intermediate **1** (91 mg, 0.2 mmol) in acetonitrile (5 mL) was added propiolic acid (23.46 mg, 0.22 mmol), HOBt (27.06 mg, 0.2 mmol), TEA (22.29 mg, 0.22 mmol), and EDAC (42.22 mg, 0.22 mmol). The resulting mixture was stirred at rt overnight. The reaction was quenched by addition of brine, extracted with EtOAc, dried over Na₂SO₄. Evaporation and purification by column chromatography (eluent: 80% EtOAc in hexanes to 100%) gave the product. LC/MS: C₂₅H₂₉F₃N₄O₄: m/z 507 (M+H).

**Step B. 1-((3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrol-2(1*H*)-yl)prop-2-yn-1-one.** The title compound was prepared analogously to Step **B** in Example **13** as a TFA salt. LC/MS: C₂₀H₂₁F₃N₄O₂: m/z 407 (M+H).

**Step C. 1-((3a*R*,5*R*,6a*R*)-5-(((3*S**,4*S**)-3-Methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta-[*c*]pyrrol-2(1*H*)-yl)prop-2-yn-1-one.** The title compound was prepared analogously to Step **C** in Example **13.** ¹H-NMR (400 MHz, CD₃OD): δ 1.75 - 2.25 (m, 5 H), 2.83 (br. s., 1 H), 3.08 - 4.33 (m, 21 H), 4.74 - 4.94 (m, 2 H), 8.06 (br. s., 1 H), 8.72 (br. s., 1 H); LC/MS: C₂₆H₃₁F₃N₄O₄: m/z 521 (M+H).

The following title compounds were synthesized using a similar procedure:

### Example 74

### (E)-4,4,4-Trifluoro-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)but-2-en-1-one

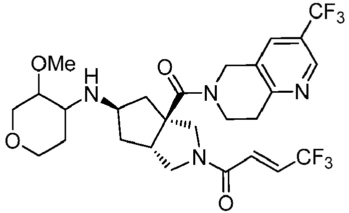

Prepared analogously to Example **73.** ¹H-NMR (400 MHz, CDCl₃): δ 1.52 - 1.96 (m, 5 H), 2.37 (br. s., 1 H), 2.72 (br. s., 1 H), 3.09 - 3.60 (m, 10 H), 3.61 - 4.18 (m, 9 H), 4.58 - 5.08 (m, 2 H), 6.70 - 6.86 (m, 2 H), 7.60 - 7.77 (m, 1 H), 8.73 (br. s., 1 H); LC/MS: C₂₇H₃₂F₆N₄O₄: m/z 591.2 (M+H).

### Example 75

### N-(2-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-2-oxoethyl)acetamide

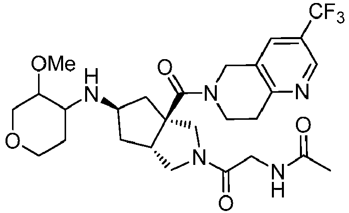

Prepared analogously to Example **73.** ¹H-NMR (400 MHz, CD₃OD): δ 0.25 - 0.78 (m, 5 H), 1.22 - 1.42 (m, 1 H), 1.67 - 2.76 (m, 26 H), 3.29 - 3.52 (m, 2 H), 7.15 (br. s., 1 H), 7.59 (br. s., 1 H); LC/MS: C₂₇H₃₆F₃N₅O₅: m/z 568 (M+H).

### Example 76

### 2-(Dimethylamino)-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)ethanone

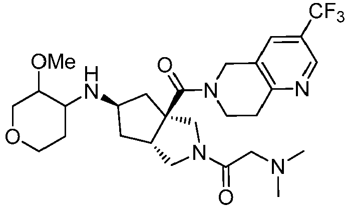

Prepared analogously to Example **73.** ¹H-NMR (400 MHz, CD₃OD): δ 0.27 - 0.81 (m, 5 H), 1.29 - 1.57 (m, 7 H), 1.67 - 3.03 (m, 22 H), 3.30 - 3.57 (m, 2 H), 7.00 (br. s., 1 H), 7.49 (d, *J*=8.1 Hz, 1 H); LC/MS: C₂₇H₃₈F₃N₅O₄: m/z 554 (M+H).

### Example 77

### 3-(Dimethylamino)-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)propan-1-one

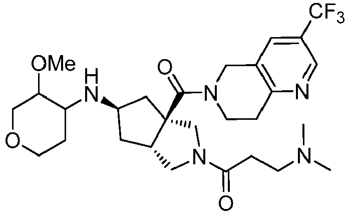

Prepared analogously to Example **73.** ¹H-NMR (400 MHz, CD₃OD): δ 0.27 - 0.79 (m, 5 H), 1.23 - 2.81 (m, 31 H), 3.29 - 3.55 (m, 2 H), 7.07 (br. s., 1 H), 7.53 (br. s., 1 H); LC/MS: C₂₈H₄₀F₃N₅O₄: m/z 568 (M+H).

### Example 78

### 4-(Dimethylamino)-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)-hexahydrocyclopenta[c]pyrrol-2(1H)-yl)butan-1-one

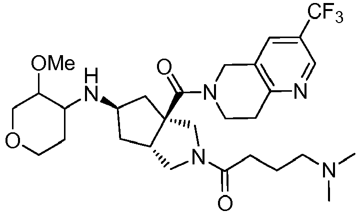

Prepared analogously to Example **73.** ¹H-NMR (400 MHz, CD₃OD): δ 1.89 - 2.33 (m, 5 H), 2.46 - 2.77 (m, 2 H), 2.89 (s, 7 H), 3.13 - 4.32 (m, 24 H), 4.94 - 5.08 (m, 2 H), 8.73 (br. s., 1 H), 9.16 (br. s., 1 H); LC/MS: C₂₉H₄₂F₃N₅O₄: m/z 582 (M+H).

### Example 79

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-3-(piperidin-1-yl)propan-1-one

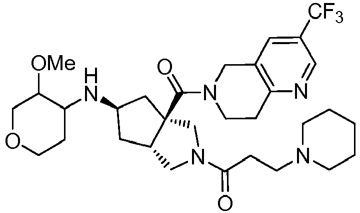

Prepared analogously to Example **73.** ¹H-NMR (400 MHz, CD₃OD): δ 1.53 (d, *J*=12.5 Hz, 1 H), 1.75 - 2.36 (m, 10 H), 2.81 - 3.14 (m, 5 H), 3.34 - 4.34 (m, 24 H), 5.01 (br. s., 2 H), 8.85 (br. s., 1 H), 9.22 (s, 1 H); LC/MS: C₃₁H₄₄F₃N₅O₄: m/z 608 (M+H).

### Example 80

### 1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-3-morpholinopropan-1-one

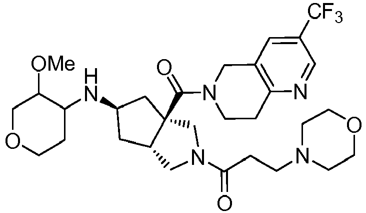

Prepared analogously to Example **73.** ¹H-NMR (400 MHz, CD₃OD): δ 1.82 - 2.35 (m, 5 H), 2.81 - 4.34 (m, 33 H), 4.97 (br. s., 2 H), 8.70 (br. s., 1 H), 9.14 (br. s., 1 H); LC/MS: C₃₀H₄₂F₃N₅O₅: m/z 610 (M+H).

### Example 81

### ((3aR,5R,6aR)-5-(((3S*,4S*)-3-Methoxytetrahydro-2H-pyran-4-yl)amino)-2-(thiazole-2-carbonyl)octahydrocyclopenta[c]pyrrol-3a-yl)(3-(trifluoromethyl)-7,8-dihydro-1,6-naphthyridin-6(5H)-yl)methanone

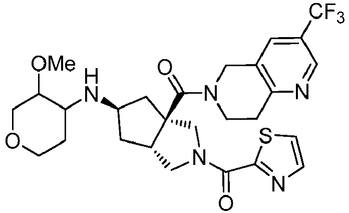

Prepared analogously to Example **73** while PyBOP was used instead of EDAC. ¹H-NMR (400 MHz, CD₃OD): δ 1.78 - 2.05 (m, 2 H), 2.08 - 2.36 (m, 3 H), 2.81 - 2.98 (m, 1 H), 3.19 - 3.84 (m, 12 H), 3.91 - 4.31 (m, 7 H), 4.45 - 4.77 (m, 1 H), 4.84 - 5.06 (m, 2 H), 7.79 - 8.05 (m, 2 H), 8.50 (br. s., 1 H), 9.02 (d, *J*=18.1 Hz, 1 H); LC/MS: C₂₇H₃₂F₃N₅O₄S: m/z 580 (M+H).

### Example 82

### (E)-1-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-4,4-dimethylpent-2-en-1-one

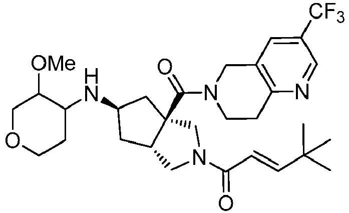

Prepared analogously to Step A in Example **65,** using the product of Example **1** as the starting material. ¹H-NMR (400 MHz, CDCl₃): δ 1.08 (s, 9 H), 1.52 - 1.97 (m, 6 H), 2.36 (br. s., 1 H), 2.72 (br. s., 1 H), 3.08 - 4.20 (m, 18 H), 4.57 - 5.05 (m, 2 H), 5.95 (d, *J*=15.4 Hz, 1 H), 6.93 (d, *J*=15.4 Hz, 1 H), 7.70 (br. s., 1 H), 8.72 (br. s., 1 H); LC/MS: C₃₀H₄₁F₃N₄O₄: m/z 579.2 (M+H).

The following title compounds were synthesized using a similar procedure:

### Example 83

### 2-((3aR,5R,6aR)-5-(((3S*,4S*)-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)-2-oxoethyl acetate

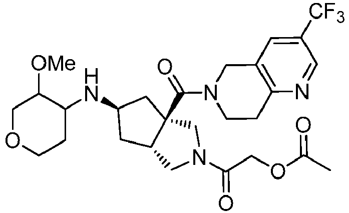

Prepared analogously to Example **82.** ¹H-NMR (400 MHz, CDCl₃): δ 1.51 - 2.01 (m, 5 H), 2.10 - 2.43 (m, 4 H), 2.45 - 2.84 (m, 2 H), 2.90 - 4.16 (m, 19 H), 4.49 - 5.04 (m, 3 H), 7.70 (br. s., 1 H), 8.72 (br. s., 1 H); LC/MS: C₂₇H₃₅F₃N₄O₆: m/z 569.3 (M+H).

### Example 84

### 2-Methoxy-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrol-2(1H)-yl)ethanone

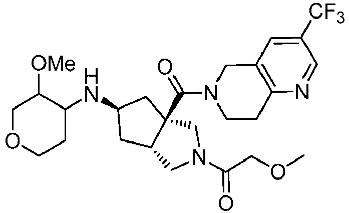

Prepared analogously to Example **82.** ¹H-NMR (400 MHz, CDCl₃): δ 1.53 - 1.93 (m, 5 H), 2.14 - 2.83 (m, 4 H), 3.02 - 4.17 (m, 22 H), 4.58 - 5.07 (m, 2 H), 7.70 (br. s., 1 H), 8.72 (br. s., 1 H); LC/MS: C₂₆H₃₅F₃N₄O₅: m/z 541.3 (M+H).

### Example 85

### 2-(tert-Butoxy)-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrol-2(1H)-yl)ethanone

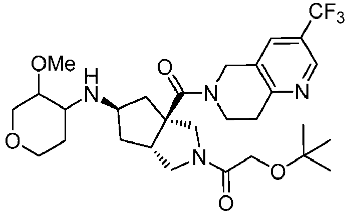

Prepared analogously to Example **82.** ¹H-NMR (400 MHz, CDCl₃): δ 1.13 - 1.32 (m, 9 H), 1.55 - 1.95 (m, 6 H), 2.33 (br. s., 1 H), 2.72 (br. s., 1 H), 3.07 - 3.57 (m, 10 H), 3.67 - 4.13 (m, 10 H), 4.61 - 5.04 (m, 2 H), 7.69 (br. s., 1 H), 8.72 (br. s., 1 H); LC/MS: C₂₉H₄₁F₃N₄O₅: m/z 583.3 (M+H).

### Example 86

### 3-Cyclopropyl-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrol-2(1H)-yl)prop-2-yn-1-one.

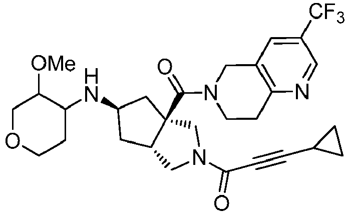

Prepared analogously to Step A in Example **73.** ¹H-NMR (400 MHz, CDCl₃): δ 0.78 - 0.99 (m, 4 H), 1.31 - 1.53 (m, 2 H), 1.58 - 2.44 (m, 6 H), 2.80 - 4.19 (m, 19 H), 4.72 - 4.98 (m, 2 H), 7.64 - 7.78 (m, 1 H), 8.71 (br. s., 1 H); LC/MS: C₂₉H₃₅F₃N₄O₄: m/z 561.3 (M+H).

The following were synthesized using a similar procedure:

### Example 87

### tert-Butyl (2-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)-2-oxoethyl)carbamate

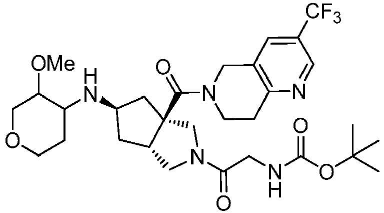

Prepared analogously to Example **82.** LC/MS: C₃₀H₄₂F₃N₅O₆: m/z 626.2 (M+H).

### Example 88

### (E)-1-((3aR,5R,6aR)-5-(((3S*,4S*)-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrol-2(1H)-yl)-4-(pyrrolidin-1-yl)but-2-en-1-one.

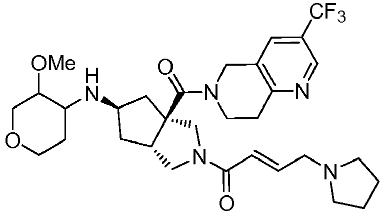

Prepared analogously to Example **82.** ¹H-NMR (400 MHz, CD₃OD): δ 1.76 - 2.27 (m, 8 H), 2.85 (s, 1 H), 3.06 - 4.08 (m, 26 H), 4.25 (m, 1 H), 4.78 - 5.00 (m, 2 H), 6.68 - 6.79 (m, 2 H), 8.06 (br. s., 1 H), 8.73 (br. s., 1 H); LC/MS: C₃₁H₄₂F₃N₅O₄: m/z 606 (M+H).

### Intermediate 7

### (E)-3-(4-Methoxyphenyl)-1-((3aR,5R,6aR)-5-((3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)prop-2-en-1-one

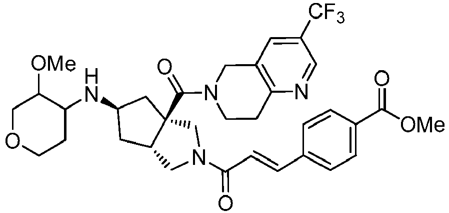

Prepared analogously to Example **73.** LC/MS: C₃₅H₄₀F₃N₃O₆: m/z 656 (M+H).

### Intermediate 8

### (E)-3-(3-Methoxyphenyl)-1-((3aR,5R,6aR)-5-((3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)prop-2-en-1-one

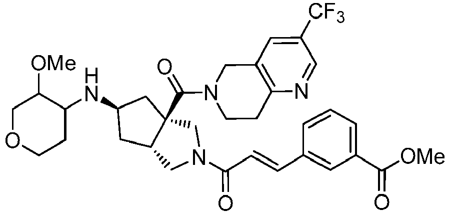

Prepared analogously to Example **73.** LC/MS: C₃₅H₄₀F₃N₃O₆: m/z 656 (M+H).

### Example 89

### 4-((E)-3-((3aR,5R,6aR)-5-(((3S*,4S*)-3-methoxytetrahydro-2H-pyran-4-yl)amino)-3a-(7-(trifluoromethyl)-1,2,3,4-tetrahydroisoquinoline-2-carbonyl)hexahydrocyclopenta-[c]pyrrol-2(1H)-yl)-3-oxoprop-1-en-1-yl)benzoic acid

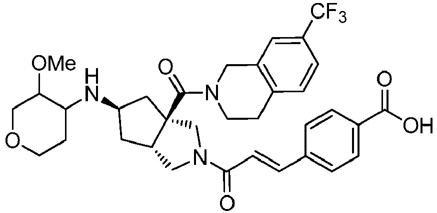

To a solution of Intermediate **7** (120 mg, 0.183 mmol) in methanol (20 mL) was added a 0.1 M LiOH aqueous solution (2 mL). The mixture was heated at gentle reflux for 5 h. After cooling to rt, the solvent was removed by evaporation and the residue was purified by HPLC to give a TFA solid, which was then converted to a HCl salt by adding 1N aqueous HCl solution and evaporating to dryness for three cycles. ¹H-NMR (400 MHz, CD₃OD): δ 1.81 (m, 1 H), 1.89 - 2.29 (m, 4 H), 2.84 (m, 1 H), 3.05 (m., 2 H), 3.23 - 3.59 (m, 10 H), 3.64 - 4.13 (m, 8 H), 4.28 (m, 1 H), 4.75 - 4.96 (m, 2 H), 7.09 (d, *J*=16 Hz, 1 H), 7.41 (d, *J*=6.8 Hz, 1 H), 7.73 (m, 5 H), 8.04 (d, *J*=7.8 Hz, 2 H); LC/MS: C₃₄H₃₈F₃N₃O₆: m/z 642 (M+H).

### Example 90

### 3-((E)-3-((3aR,5R,6aR)-5-(((3S^{*},4S^{*})-3-methoxytetrahydro-2H-pyran-4-yl)amino)-3a-(7-(trifluoromethyl)-1,2,3,4-tetrahydroisoquinoline-2-carbonyl)hexahydrocyclopenta-[c]pyrrol-2(1H)-yl)-3-oxoprop-1-en-1-yl)benzoic acid

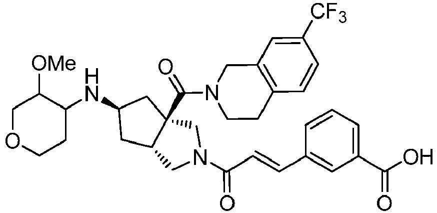

The title compound was prepared analogously to Example **89** using Intermediate **8**. ¹H-NMR (400 MHz, CD₃OD): δ 1.74 - 2.30 (m, 5 H), 2.79 - 2.94 (m, 1 H), 3.05 (m, 2 H), 3.26 - 4.37 (m, 19 H), 4.70 - 4.97 (m, 2 H), 7.03 (d, *J*=15.4 Hz, 1 H), 7.40 (d, *J*=6.8 Hz, 1 H), 7.45 - 7.75 (m, 4 H), 7.86 (d, *J*=7.6 Hz, 1 H), 8.04 (d, *J*=7.8 Hz, 1 H), 8.25 (s, 1 H); LC/MS: C₃₄H₃₈F₃N₃O₆: m/z 642 (M+H).

### Example 91

### ((3aR,5R,6aR)-5-(((3S^{*},4S^{*})-3-Methoxytetrahydro-2H-pyran-4-yl)amino)-2-(methylsulfonyl)octahydrocyclopenta[c]pyrrol-3a-yl)(3-(trifluoromethyl)-7,8-dihydro-1,6-naphthyridin-6(5H)-yl)methanone

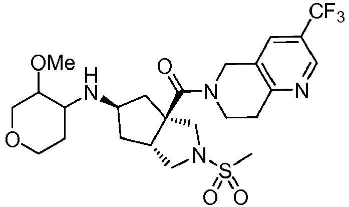

**Step A**. ***tert***-**Butyl ((3a*R*,5*R*,6a*R*)-2-(methylsulfonyl)-3a-(3-(trifluoromethyl)-5,6,7,8-tetrahydro-1,6-naphthyridine-6-carbonyl)octahydrocyclopenta[*c*] pyrrol-5-yl)carbamate.**

To a solution of Intermediate 1 (0.11 g, 0.242 mmol) and TEA (0.0344 mL, 0.247 mmol) in DCM (3 mL) at 0 °C was added methanesulfonyl chloride (0.0188 mL, 0.242 mol). The mixture was stirred at 0 °C for 30 min and at rt overnight. The reaction mixture was quenched by addition of brine, extracted with DCM, dried over Na₂SO₄. Purification by CombiFlash (eluent: 60% EtOAc in hexanes) gave the product as a yellow gel. ¹H-NMR (400 MHz, CDCl₃): δ 1.14 - 1.55 (m, 9 H), 1.67 - 2.16 (m, 4 H), 2.27 - 2.47 (m, 1 H), 2.76 - 2.94 (m, 1 H), 3.06 - 3.93 (m, 10 H), 4.25 (d, *J*=5.1 Hz, 1 H), 4.54 - 5.01 (m, 3 H), 7.73 (s, 1 H), 8.72 (s, 1 H); LC/MS: C₂₃H₃₁F₃N₄O₅S: m/z 555.5 (M+Na).

**Step B. ((3a*R*,5*R*,6a*R*)-5-Amino-2-(methylsulfonyl)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **B** in Example **13** as a TFA salt. LC/MS: C₁₈H₂₃F₃N₄O₃S: m/z 433.5 (M+H).

**Step C. ((3a*R*,5*R*,6a*R*)-5-(((3*S**,4*S**)-3-Methoxytetrahydro-2*H*-pyran-4-yl)amino)-2-(methylsulfonyl)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step C in Example **13.** ¹H-NMR (400 MHz, CDCl₃): δ 1.51 - 2.04 (m, 5 H), 2.16 - 2.43 (m, 2 H), 2.53 - 2.88 (m, 6 H), 3.05 - 4.32 (m, 16 H), 4.65 - 5.01 (m, 2 H), 7.70 (s, 1 H), 8.73 (br. s., 1 H); LC/MS: C₂₄H₃₃F₃N₄O₅S: m/z 547.5 (M+H).

The following title compounds were synthesized using a similar procedure:

### Example 92

### ((3aR,5R,6aR)-2-(Benzylsulfonyl)-5-(((3S*,4S*)-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone.

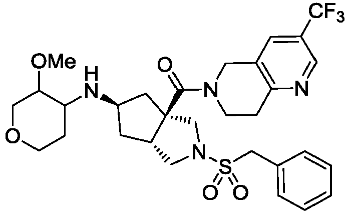

The title compound was prepared analogously to Example **91.** ¹H-NMR (400 MHz, CDCl₃): δ 1.54 - 1.88 (m, 5 H), 2.18 (br. s., 2 H), 2.71 (br. s., 1 H), 2.83 - 3.14 (m, 3 H), 3.19 - 3.96 (m, 14 H), 3.99 - 4.12 (m, 1 H), 4.16 - 4.35 (m, 2 H), 4.45 - 4.96 (m, 2 H), 7.09 - 7.42 (m, 5 H), 7.70 (br. s., 1 H), 8.74 (s, 1 H); LC/MS: C₃₀H₃₇F₃N₄O₅S: m/z 623.2 (M+H).

### Example 93

### ((3aR,5R,6aR)-5-(((3S^{*},4S^{*})-3-Methoxytetrahydro-2H-pyran-4-yl)amino)-2-(phenethylsulfonyl)octahydrocyclopenta[c]pyrrol-3a-yl)(3-(trifluoromethyl)-7,8-dihydro-1,6-naphthyridin-6(5H)-yl)methanone

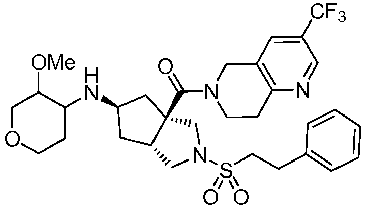

The title compound was prepared analogously to Example **91.** ¹H-NMR (400 MHz, CDCl₃): δ 1.59 - 1.98 (m, 5 H), 2.31 (d, *J*=6.6 Hz, 2 H), 2.75 (br. s., 1 H), 3.04 - 4.13 (m, 22 H), 4.63 - 4.92 (m, 2 H), 7.16 - 7.32 (m, 5 H), 7.69 (s, 1 H), 8.72 (s, 1 H); LC/MS: C₃₁H₃₉F₃N₄O₅S: m/z 637.3 (M+H).

### Example 94

### ((3aR,5R,6aR)-2-Cyclopropyl-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

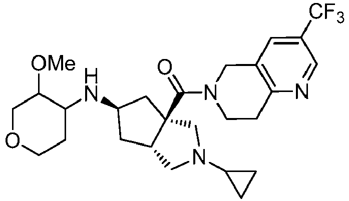

**Step A. *tert-*Butyl ((3a*R*,5*R*,6a*R*)-2-cyclopropyl-3a-(3-(tritluoromethyl)-5,6,7,8-tetrahydro-1,6-naphthyridine-6-carbonyl)octahydrocyclopenta[*c*]pyrrol-5-yl)carbamate.** A solution of Intermediate **1** (0.1213 g, 0.267 mmol) in methanol (2 mL) was added (1-ethoxycyclopropoxy)-trimethylsilane (0.0813 mL, 0.4 mol), sodium cyanoborohydride (70.62 mg, 1.068 mmol) and acetic acid (0.153 mL, 2.669 mmol). The mixture was heated at reflux overnight. The reaction mixture was quenched by addition of saturated aqueous NaHCO₃ solution, extracted with EtOAc, and dried over Na₂SO₄. Purification by CombiFlash (eluent: 5% methanol in DCM) gave the product as a colorless gel. ¹H-NMR (400 MHz, CDCl₃): δ 0.39 (d, *J*=6.1 Hz, 4 H), 1.31 - 1.46 (m, 11 H), 1.65 - 1.98 (m, 3 H), 2.15 - 2.31 (m, 1 H), 2.55 (dd, *J*=9.0, 3.2 Hz, 1 H), 2.66 - 2.81 (m, 2 H), 2.94 - 3.19 (m, 3 H), 3.61 (br. s., 1 H), 3.77 - 3.92 (m, 1 H), 4.20 (d, *J*=6.1 Hz, 1 H), 4.64 - 4.84 (m, 3 H), 7.70 (s, 1 H), 8.70 (br. s., 1 H); LC/MS: C₂₅H₃₃F₃N₄O₃: m/z 495.2 (M+H).

**Step B. ((3a*R*,5*R*,6a*R*)-5-Amino-2-cyclopropyloctahydrocyclopenta[*c*]pyrrol-3a-yl)(3-(tritluoromethyl)-7,8-dihydro-1,6-naphthyridin-6(5*H*)-yl)methanone.** The title compound was prepared analogously to Step **B** in Example **13** as a TFA salt. LC/MS: C₂₀H₂₅F₃N₄O: m/z 395.2 (M+H).

**Step C. ((3a*R*,5*R*,6a*R*)-2-Cyclopropyl-5-(((3*S*^{*},4*S*^{*})-3-methoxytetrahydro-2*H*-pyran-4-yl)amino)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **13.** ¹H-NMR (400 MHz, CDCl₃): δ 0.26 - 0.47 (m, 4 H), 0.98 - 1.10 (m, 1 H), 1.46 - 1.80 (m, 6 H), 2.16 (br. s., 1 H), 2.43 - 4.15 (m, 19 H), 4.79 (br. s., 2 H), 7.69 (br. s., 1 H), 8.70 (br. s., 1 H); LC/MS: C₂₆H₃₅F₃N₄O₃: m/z 509.2 (M+H).

### Example 95

### ((3aR,5R,6aR)-2-Cyclopropyl-5-(((3S^{*},4S^{*})-3-methoxytetrahydro-2H-pyran-4-yl)amino)octahydrocyclopenta[c]pyrrol-3a-yl)(7-(trifluoromethyl)-3,4-dihydroisoquinolin-2(1H)-yl)methanone

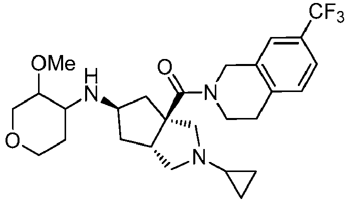

The title compound was prepared analogously to Example **94.** ¹H-NMR (400 MHz, CDCl₃): δ 0.19 - 0.40 (m, 4 H), 1.38 - 1.78 (m, 7 H), 2.05 (br. s., 1 H), 2.39 (dt, *J*=9.0, 4.4 Hz, 1 H), 2.60 - 2.91 (m, 6 H), 3.12 - 3.44 (m, 7 H), 3.52 - 4.01 (m, 5 H), 4.66 (br. s., 2 H), 7.13 - 7.23 (m, 1 H), 7.25 - 7.41 (m, 2 H); LC/MS: C₂₇H₃₆F₃N₃O₃: m/z 508.3 (M+H).

### Example 96

### ((3aR,5R,6aR)-2-Isopropyl-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

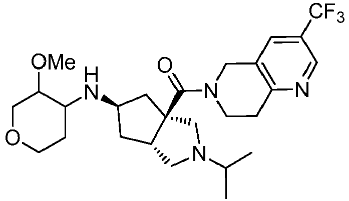

**Step A. *tert*-Butyl ((3a*R*,5*R*,6a*R*)-2-Isopropyl-3a-(3-(trifluoromethyl)-5,6,7,8-tetrahydro-1,6-naphthyridine-6-carbonyl)octahydrocyclopenta[*c*]pyrrol-5-yl)carbamate.** A mixture of Intermediate **1** (0.11 g, 0.242 mmol) and acetone (19.5 µL, 0.266 mmol) in DCM (2 mL) was stirred at rt for 20 min, followed by addition of NaBH(OAc)₃ (076.9 mg, 0.363 mmol). The mixture was stirred at rt overnight and quenched by addition of saturated aqueous NaHCO₃ solution, extracted with DCM, dried over Na₂SO₄. Purification by CombiFlash (eluent: 8% MeOH in DCM) gave the product as a colorless gel. ¹H-NMR (400 MHz, CDCl₃): δ 0.95 - 1.12 (m, 6 H), 1.31 - 1.47 (m, 9 H), 1.68 - 1.78 (m, 1 H), 1.78 - 1.99 (m, 2 H), 2.20 - 2.39 (m, 2 H), 2.46 - 2.74 (m, 3 H), 2.93 (br. s., 1 H), 3.12 (br. s., 2 H), 3.61 (br. s., 1 H), 3.85 (br. s., 2 H), 4.25 (d, *J*=6.1 Hz, 1 H), 4.58 - 4.93 (m, 3 H), 7.70 (s, 1 H), 8.71 (s, 1 H); LC/MS: C₂₅H₃₅F₃N₄O₃: m/z 497.2 (M+H).

**Step B. ((3a*R*,5*R*,6a*R*)-5-Amino-2-isopropyloctahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **B** in Example **93** as a TFA salt. LC/MS: C₂₀H₂₇F₃N₄O: m/z 397.2 (M+H).

**Step C. ((3a*R*,5*R*,6a*R*)-2-Isopropyl-5-(((3*S*^{*},4*S*^{*})-3-methoxytetrahydro-2*H*-pyran-4-yl)amino)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **93** as a colorless gel. ¹H-NMR (400 MHz, CDCl₃): δ 0.93 - 1.14 (m, 6 H), 1.52 - 1.87 (m, 5 H), 2.12 - 2.41 (m, 3 H), 2.49 - 2.88 (m, 5 H), 3.02 - 3.67 (m, 10 H), 3.75 - 4.13 (m, 4 H), 4.80 (br. s., 2 H), 7.69 (br. s., 1 H), 8.70 (s, 1 H); LC/MS: C₂₆H₃₇F₃N₄O₃: m/z 511.2 (M+H).

### Example 97

### ((3aR,5R,6aR)-5-(((3S^{*},4S^{*})-3-Methoxytetrahydro-2H-pyran-4-yl)amino)-2-methyloctahydrocyclopenta[c]pyrrol-3a-yl)(3-(trifluoromethyl)-7,8-dihydro-1,6-naphthyridin-6(5H)-yl)methanone.

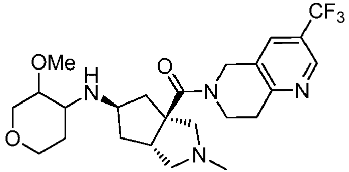

The title compound was prepared analogously to Example **96.** ¹H-NMR (400 MHz, CDCl₃): δ 1.49 - 1.88 (m, 5 H), 2.15 - 2.42 (m, 5 H), 2.56 - 2.85 (m, 5 H), 2.93 - 3.58 (m, 9 H), 3.62 - 4.12 (m, 5 H), 4.78 (br. s., 2 H), 7.69 (br. s., 1 H), 8.70 (br. s., 1 H); LC/MS: C₂₄H₃₃F₃N₄O₃: m/z 483.3 (M+H).

### Example 98

### ((3aR,5R,6aR)-2-Ethyl-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone.

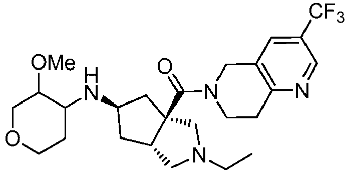

The title compound was prepared analogously to Example **96.** ¹H-NMR (400 MHz, CDCl₃): δ 0.98 - 1.21 (m, 3 H), 1.48 - 1.97 (m, 5 H), 2.12 - 2.53 (m, 4 H), 2.58 - 2.92 (m, 4 H), 3.04 - 4.21 (m, 15 H), 4.79 (br. s., 2 H), 7.69 (br. s., 1 H), 8.71 (br. s., 1 H); LC/MS: C₂₅H₃₅F₃N₄O₃: m/z 497.2 (M+H).

### Example 99

### ((3aR,5R,6aR)-5-(((3S^{*},4S^{*})-3-methoxytetrahydro-2H-pyran-4-yl)amino)-2-(oxetan-3-yl)octahydrocyclopenta[c]pyrrol-3a-yl)(3-(trifluoromethyl)-7,8-dihydro-1,6-naphthyridin-6(5H)-yl)methanone

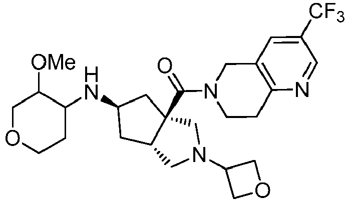

The title compound was prepared analogously to Example **96.** ¹H-NMR (400 MHz, CDCl₃): δ 1.54 - 1.89 (m, 5 H), 1.91 - 2.32 (m, 2 H), 2.32 - 2.46 (m, 1 H), 2.48 - 2.67 (m, 2 H), 2.67 - 3.21 (m, 4 H), 3.21 - 4.12 (m, 13 H), 4.50 - 5.00 (m, 6 H), 7.71 (s, 1 H), 8.71 (s, 1 H); LC/MS: C₂₆H₃₅F₃N₄O₄: m/z 525.3 (M+H).

### Example 100

### ((3aR,5R,6aR)-5-(((3S^{*},4S^{*})-3-Methoxytetrahydro-2H-pyran-4-yl)amino)-2-(oxetan-3-yl)octahydrocyclopenta[c]pyrrol-3a-yl)(7-(trifluoromethyl)-3,4-dihydroisoquinolin-2(1H)-yl)methanone.

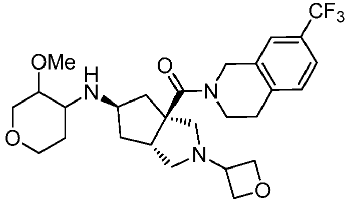

The title compound was prepared analogously to Example **96.** ¹H-NMR (400 MHz, CDCl₃): δ 1.51 - 1.93 (m, 6 H), 2.11 - 2.26 (m, 1 H), 2.34 - 2.45 (m, 1 H), 2.48 - 2.62 (m, 2 H), 2.78 (d, *J*=8.8 Hz, 2 H), 2.92 (br. s., 2 H), 3.16 - 3.45 (m, 6 H), 3.47 - 3.62 (m, 2 H), 3.62 - 3.83 (m, 3 H), 3.85 - 4.00 (m, 1 H), 3.99 - 4.09 (m, 1 H), 4.48 - 4.95 (m, 6 H), 7.22 - 7.30 (m, 1 H), 7.34 - 7.51 (m, 2 H); LC/MS: C₂₇H₃₆F₃N₃O₄: m/z 524.3 (M+H).

### Example 101

### ((3aR,5R,6aR)-2-Benzyl-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone.

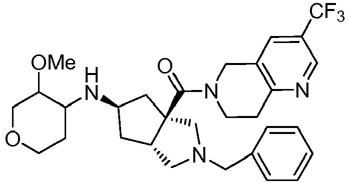

The title compound was prepared analogously to Example **96.** ¹H-NMR (400 MHz, CDCl₃): δ 1.47 - 2.06 (m, 6 H), 2.21 (d, *J*=5.8 Hz, 1 H), 2.39 - 2.68 (m, 3 H), 2.71 - 2.90 (m, 2 H), 2.93 - 3.18 (m, 2 H), 3.23 - 3.73 (m, 10 H), 3.74 - 4.01 (m, 3 H), 4.02 - 4.16 (m, 1 H), 4.76 (br. s., 2 H), 7.18 - 7.37 (m, 5 H), 7.66 (br. s., 1 H), 8.70 (br. s., 1 H); LC/MS: C₃₀H₃₇F₃N₄O₃: m/z 559.2 (M+H).

### Example 102

### ((3aR,5R,6aR)-5-(((3S^{*},4S^{*})-3-Methoxytetrahydro-2H-pyran-4-yl)amino)-2-phenethyloctahydrocyclopenta[c]pyrrol-3a-yl)(3-(trifluoromethyl)-7,8-dihydro-1,6-naphthyridin-6(5H)-yl)methanone.

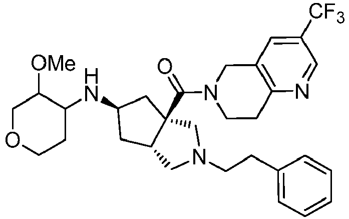

The title compound was prepared analogously to Example **96.** ¹H-NMR (400 MHz, CDCl₃): δ 1.53 - 1.90 (m, 5 H), 2.18 (br. s., 1 H), 2.30 - 2.96 (m, 10 H), 2.96 - 3.71 (m, 10 H), 3.76 - 4.22 (m, 4 H), 4.76 (br. s., 2 H), 7.13 - 7.32 (m, 5 H), 7.66 (br. s., 1 H), 8.69 (br. s., 1 H); LC/MS: C₃₁H₃₉F₃N₄O₃: m/z 573.3 (M+H).

### Example 103

### ((3aR,5R,6aR)-5-(((3S^{*},4S^{*})-3-Methoxytetrahydro-2H-pyran-4-yl)amino)-2-(3-phenylpropyl)octahydrocyclopenta[c]pyrrol-3a-yl)(3-(trifluoromethyl)-7,8-dihydro-1,6-naphthyridin-6(5H)-yl)methanone.

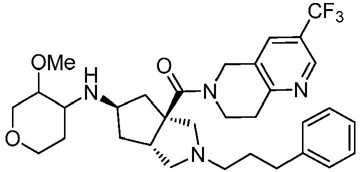

The title compound was prepared analogously to Example **96.** ¹H-NMR (400 MHz, CDCl₃): δ 1.52 - 1.90 (m, 8 H), 2.20 (br. s., 1 H), 2.30 - 2.49 (m, 3 H), 2.49 - 2.69 (m, 4 H), 2.78 (d, *J=3.5* Hz, 2 H), 3.00 - 3.16 (m, 2 H), 3.20 - 3.41 (m, 6 H), 3.50 - 3.71 (m, 2 H), 3.79 - 4.13 (m, 4 H), 4.78 (br. s., 2 H), 7.15 - 7.32 (m, 5 H), 7.68 (s, 1 H), 8.70 (s, 1 H); LC/MS: C₃₂H₄₁F₃N₄O₃: m/z 587.2 (M+H).

### Example 104

### ((3aR,5R,6aR)-2-((3S^{*},4S^{*})-3-Methoxytetrahydro-2H-pyran-4-yl)-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone.

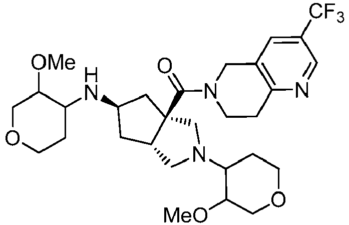

The title compound was prepared analogously to Example **96,** a mixture of at least four diastereoisomers. ¹H-NMR (400 MHz, CDCl₃): δ 1.36 - 2.07 (m, 8 H), 2.14 - 2.38 (m, 2 H), 2.45 - 2.60 (m, 1 H), 2.67 - 3.20 (m, 6 H), 3.23 - 3.69 (m, 14 H), 3.82 - 4.16 (m, 6 H), 4.68 - 4.96 (m, 2 H), 7.71 (d, *J*=4.8 Hz, 1 H), 8.70 (s, 1 H); LC/MS: C₂₉H₄₁F₃N₄O₅: m/z 583.3 (M+H).

### Example 105

### ((3aR,5R,6aR)-2-((3S^{*},4S^{*})-3-Methoxytetrahydro-2H-pyran-4-yl)-5-(((3S^{*},4S^{*})-3-methoxytetrahydro-2H-pyran-4-yl)amino)octahydrocyclopenta[c]pyrrol-3a-yl)(7-(trifluoromethyl)-3,4-dihydroisoquinolin-2(1H)-yl)methanone.

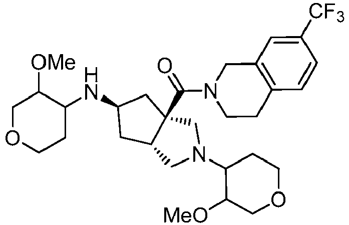

The title compound was prepared analogously to Example **96,** a mixture of at least four diastereoisomers. ¹H-NMR (400 MHz, CDCl₃): δ 1.48 - 2.05 (m, 8 H), 2.20 (dd, *J*=11.2, 4.9 Hz, 2 H), 2.45 - 2.59 (m, 1 H), 2.65 - 3.05 (m, 6 H), 3.20 - 4.16 (m, 20 H), 4.73 (br. s., 2 H), 7.20 - 7.30 (m, 1 H), 7.33 - 7.50 (m, 2 H); LC/MS: C₃₀H₄₂F₃N₃O₅: m/z 582.2 (M+H).

### Example 106

### 2-((3aR,5R,6aR)-5-(((3S^{*},4S^{*})-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)-N,N-dimethylacetamide

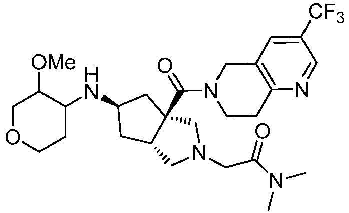

**Step A. 2-((3a*R*,5*R*,6a*R*)-5-((*tert*-Butoxycarbonyl)amino)-3a-(3-(trifluoromethyl)-5,6,7,8-tetrahydro-1,6-naphthyridine-6-carbonyl)hexahydrocyclopenta[*c*]pyrrol-2(1*H*)-yl)acetic acid.** A solution of Intermediate **1** (0.22 g, 0.484 mmol), glyoxylic acid monohydrate (0.136 g, 1.452 mmol) and 4Å molecular sieves (0.8 g) in DCM (8 ml) at 0 °C was added NaBH(OAc)₃ (0.216 g, 0.968 mol). The mixture was stirred at 0 °C for 30 min and at rt overnight. The reaction was quenched by addition of saturated NaHCO₃ solution, separated, extracted with DCM, and dried over Na₂SO₄. The crude product was obtained by evaporation. LC/MS: C₂₄H₃₁F₃N₄O₅: m/z 513.3 (M+H).

**Step B. *tert-*Butyl ((3a*R*,5*R*,6a*R*)-2-(2-(dimethylamino)-2-oxoethyl)-3a-(3-(trifluoromethyl)-5,6,7,8-tetrahydro-1,6-naphthyridine-6-carbonyl)octahydrocyclopenta-[*c*]pyrrol-5-yl)carbamate.** A solution the product from Step **A** (37 mg, 0.072 mmol), dimethylamine (0.0433 mL, 0.0866 mmol, 2 M in THF), EDAC (18 mg, 0.0938 mmol), HOBt (19.51 mg, 0.144 mmol) and DIPEA (0.0252 mL, 0.144 mmol) in DMF (3 mL) was stirred at rt over the weekend. The reaction was quenched by addition of brine, extracted with EtOAc, dried over Na₂SO₄ and purified by CombiFlash (eluent: 8% methanol in DCM) to give the product as a yellowish gel. ¹H-NMR (400 MHz, CDCl₃): δ 1.29 - 1.43 (m, 9 H), 1.75 - 2.09 (m, 4 H), 2.20 - 2.35 (m, 1 H), 2.55 - 2.71 (m, 3 H), 2.86 - 3.00 (m, 4 H), 3.05 - 3.22 (m, 6 H), 3.60 - 3.93 (m, 2 H), 4.25 (d, *J*=5.6 Hz, 1 H), 4.59 - 4.92 (m, 3 H), 7.70 (s, 1 H), 8.70 (s, 1 H); LC/MS: C₂₆H₃₆F₃N₅O₄: m/z 540.3 (M+H).

**Step C. 2-((3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrol-2(1*H*)-yl)-*N,N-*dimethylacetamide.** The title compound was prepared analogously to Step **B** in Example **13** as a TFA salt. LC/MS: C₂₁H₂₈F₃N₅O₂: m/z 440.2 (M+H).

**Step D. 2-((3a*R*,5*R*,6a*R*)-5-(((3*S*^{*},4*S*^{*})-3-Methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrol-2(1*H*)-yl)-*N,N*-dimethylacetamide.** The title compound was prepared analogously to Step **C** in Example **13** as a colorless gel. ¹H-NMR (400 MHz, CDCl₃): δ 1.54 - 1.87 (m, 5 H), 2.23 (br. s., 1 H), 2.52 - 2.86 (m, 5 H), 2.94 (s, 3 H), 3.04 (s, 3 H), 3.06 - 3.44 (m, 11 H), 3.48 - 3.59 (m, 1 H), 3.68 (d, *J*=4.8 Hz, 1 H), 3.73 - 3.97 (m, 3 H), 4.00 - 4.09 (m, 1 H), 4.77 (br. s., 2 H), 7.69 (s, 1 H), 8.70 (s, 1 H); LC/MS: C₂₇H₃₈F₃N₅O₄: m/z 554.5 (M+H).

### Example 107

### Methyl 2-((3aR,5R,6aR)-5-(((3S^{*},4S^{*})-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrol-2(1H)-yl)acetate

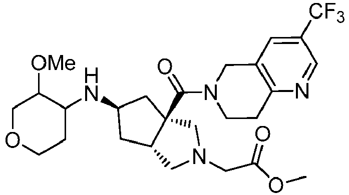

**Step A. Methyl 2-((3a*R*,5*R*,6a*R*)-5-((*tert*-butoxycarbonyl)amino)-3a-(3-(trifluoromethyl)-5,6,7,8-tetrahydro-1,6-naphthyridine-6-carbonyl)hexahydrocyclopenta[*c*]pyrrol-2(1*H*)-yl)acetate.** A solution of the product from Step A in Example **106** (52 mg, 0.101 mmol) in methanol (4 mL) was treated with (trimethylsilyl)diazomethane (2 *M* in hexanes, 0.304 mL, 0.609 mmol), and the resulting mixture was stirred at rt overnight. After removal of solvent, the residue was purified by CombiFlash (eluent: EtOAc) to give the product as a colorless gel. ¹H-NMR (400 MHz, CDCl₃): δ 1.31 - 1.46 (m, 9 H), 1.64 - 2.05 (m, 3 H), 2.29 (br. s., 1 H), 2.62 (dd, *J*=8.6, 3.0 Hz, 1 H), 2.67 - 2.84 (m, 2 H), 3.01 - 3.19 (m, 3 H), 3.20 - 3.37 (m, 2 H), 3.58 - 3.95 (m, 6 H), 4.24 (br. s., 1 H), 4.53 - 4.94 (m, 3 H), 7.70 (s, 1 H), 8.71 (s, 1 H); LC/MS: C₂₅H₃₃F₃N₄O₅: m/z 527.2 (M+H).

**Step B. Methyl 2-((3a*R*,5*R*,6a*R*)-5-amino-3a-(3-(trifluoromethyl)-5,6,7,8-tetrahydro-1,6-naphthyridine-6-carbonyl)hexahydrocyclopenta[*c*]pyrrol-2(1*H*)-yl)acetate.** The title compound was prepared analogously to Step **B** in Example **13** as a TFA salt. LC/MS: C₂₀H₂₅F₃N₄O₃: m/z 427.2 (M+H).

**Step C. Methyl 2-((3a*R*,5*R*,6a*R*)-5-(((3*S*^{*},4*S*^{*})-3-methoxytetrahydro-2*H*-pyran-4-yl)amino)-3a-(3-(trifluoromethyl)-5,6,7,8-tetrahydro-1,6-naphthyridine-6-carbonyl)hexahydrocyclopenta[*c*]pyrrol-2(1*H*)-yl)acetate.** The title compound was prepared analogously to Step **C** in Example **13** as a colorless gel. ¹H-NMR (400 MHz, CDCl₃): δ 1.85 - 2.08 (m, 3 H), 2.19 - 2.45 (m, 2 H), 2.49 - 2.82 (m, 4 H), 3.06 - 3.49 (m, 12 H), 3.56 - 3.75 (m, 5 H), 3.80 - 4.06 (m, 4 H), 4.17 (d, *J*=13.1 Hz, 1 H), 4.70 - 5.00 (m, 2 H), 7.73 (br. s., 1 H), 8.70 (br. s., 1 H); LC/MS: C₂₆H₃₅F₃N₄O₅: m/z 541.5 (M+H).

### Example 108

### ((3aR,5R,6aR)-5-(((3S^{*},4S^{*})-3-Methoxytetrahydro-2H-pyran-4-yl)amino)-2-phenyloctahydrocyclopenta[c]pyrrol-3a-yl)(3-(trifluoromethyl)-7,8-dihydro-1,6-naphthyridin-6(5H)-yl)methanone

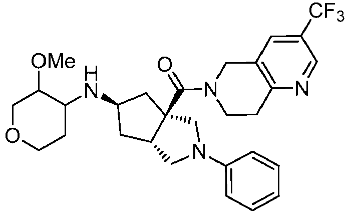

**Step A. *tert-*Butyl ((3a*R*,5*R*,6a*R*)-2-phenyl-3a-(3-(trifluoromethyl)-5,6,7,8-tetrahydro-1,6-naphthyridine-6-carbonyl)octahydrocyclopenta[*c*]pyrrol-5-yl)carbamate.** A mixture of Intermediate **1** (133 mg, 0.293 mmol), 2,6-lutidine (28.91 mg, 0.27 mmol), myristic acid (11.6 mg, 0.051 mmol), phenylboronic acid (52 mg, 0.427 mmol) and copper acetate (52 mg, 0.287 mmol) in toluene (6 mL) was stirred at rt for 2 days and diluted with toluene (25 mL), filtered. The filtrate was washed with saturated NaHCO₃ solution, brine and dried over Na₂SO₄. Filtration and evaporation to dryness gave the crude product as a clear oil. LC/MS: C₂₈H₃₃F₃N₄O₃: m/z 531 (M+H).

**Step B. ((3a*R*,5*R*,6a*R*)-5-Amino-2-phenyloctahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **B** in Example **13** as a TFA salt. LC/MS: C₂₃H₂₅F₃N₄O: m/z 431 (M+H).

**Step C. ((3a*R*,5*R*,6a*R*)-5-(((3*S*^{*},4*S*^{*})-3-Methoxytetrahydro-2*H*-pyran-4-yl)amino)-2-phenyloctahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **13** as a colorless gel. ¹H-NMR (400 MHz, CDCl₃): δ 1.80 (br. s., 1 H), 1.89 - 2.32 (m, 4 H), 2.76 (br. s., 1 H), 3.08 - 3.59 (m, 16 H), 3.76 - 4.08 (m, 5 H), 4.25 (d, *J*=13.2 Hz, 1 H), 6.66 - 6.81 (m, 3 H), 7.19 (t, *J*=7.9 Hz, 2 H), 8.05 (s, 1 H), 8.72 (s, 1 H); LC/MS: C₂₉H₃₅F₃N₄O₃: m/z 545 (M+H).

### Example 109

### ((3aR,5R,6aR)-5-(((3S^{*},4S^{*})-3-Methoxytetrahydro-2H-pyran-4-yl)amino)-2-phenyloctahydro-cyclopenta[c]pyrrol-3a-yl)(3-(trifluoromethyl)-7,8-dihydro-1,6-naphthyridin-6(5H)-yl)methanone

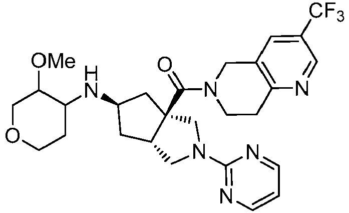

A mixture of Example **1** (32 mg, 0.0554 mmol), 2-bromopyrimidine (13.9 mg, 0.0831 mmol) in TEA (0.0385 mL, 0.277 mmol) and EtOH (2 mL) in a sealed tube was heated at 90 °C overnight. After removal of solvent by evaporation, the residue was purified by CombiFlash (eluent: 5% 7*N* NH₃ in methanol in DCM) to give the product as a colorless gel. ¹H-NMR (400 MHz, CDCl₃): δ 1.55 - 2.01 (m, 5 H), 2.39 (br. s., 1 H), 2.73 (m, 2 H), 3.05 - 3.67 (m, 11 H), 3.77 - 4.12 (m, 7 H), 4.63 - 5.00 (m, 2 H), 6.53 (t, *J*=4.7 Hz, 1 H), 7.70 (s, 1 H), 8.31 (d, *J*=4.5 Hz, 2 H), 8.72 (br. s., 1 H); LC/MS: C₂₇H₃₃F₃N₆O₃: m/z 547.2 (M+H).

### Example 110

### ((3aR,5R,6aR)-5-(((3S^{*},4S^{*})-3-Methoxytetrahydro-2H-pyran-4-yl)amino)-2-(thiazol-2-yl)octahydrocyclopenta[c]pyrrol-3a-yl)(3-(trifluoromethyl)-7,8-dihydro-1,6-naphthyridin-6(5H)-yl)methanone

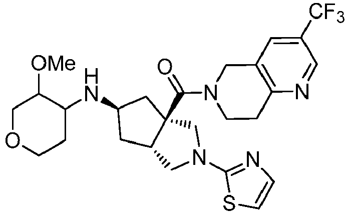

A mixture of Example **1** (HCl salt, 32 mg, 0.0554 mmol), 2-bromothiazole (13.62 mg, 0.0831 mmol), TEA (0.0385 mL, 0.277 mmol) and potassium fluoride (1.61 mg, 0.0277 mmol) in ethanol (1 mL) in a sealed tube was heated at 90 °C for 6 days. After aqueous work up, the residue was purified by CombiFlash (eluent: 5% methanol in DCM) to give the product. ¹H-NMR (400 MHz, CDCl₃): δ 1.50 - 1.93 (m, 5 H), 2.12 - 2.47 (m, 2 H), 2.65 - 2.80 (m, 2 H), 3.03 - 4.19 (m, 17 H), 4.64 - 5.01 (m, 2 H), 6.55 (d, *J*=3.5 Hz, 1 H), 7.20 (d, *J*=3.5 Hz, 1 H), 7.69 (br. s., 1 H), 8.71 (d, *J*=5.1 Hz, 1 H); LC/MS: C₂₆H₃₂F₃N₅O₃S: m/z 552.2 (M+H).

### Example 111

### ((3aR,5R,6aR)-5-(((3S^{*},4S^{*})-3-Methoxytetrahydro-2H-pyran-4-yl)amino)-2-(3-methyl-1,2,4-oxadiazol-5-yl)octahydrocyclopenta[c]pyrrol-3a-yl)(3-(trifluoromethyl)-7,8-dihydro-1,6-naphthyridin-6(5H)-yl)methanone.

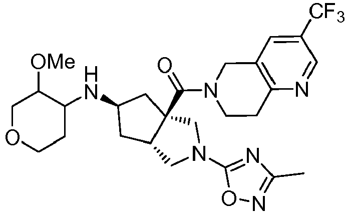

A mixture of 3-methyl-1,2,4-oxadiazol-5(4*H*)-one (21.36 mg, 0.213 mmol), DIPEA (0.184 mL, 1.067 mmol) and PyBrOP (99.5 mg, 0.213 mmol) in 1,4-dioxane (3 mL) was stirred at 50 °C for 1 h, followed by addition of Example **1** (HCl salt, 50 mg, 0.107 mmol). The resulting mixture was stirred at 50 °C overnight. After aqueous work up, the residue was purified by CombiFlash (eluent: 6% 7*N* NH₃ in methanol in DCM) to give the product. ¹H-NMR (400 MHz, CD₃OD): δ 1.55 - 1.96 (m, 8 H), 2.12 - 2.27 (5, 3 H), 2.30 - 2.45 (m, 1 H), 2.74 (d, *J*=1.0 Hz, 1 H), 3.11 - 3.56 (m, 10 H), 3.77 - 3.97 (m, 5 H), 4.01 - 4.13 (m, 1 H), 4.65 - 5.02 (m, 2 H), 7.70 (s, 1 H), 8.73 (s, 1 H); LC/MS: C₂₆H₃₃F₃N₆O₄: m/z 551 (M+H).

### Example 112

### ((3aR,5R,6aR)-2-(4,5-Dihydrothiazol-2-yl)-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

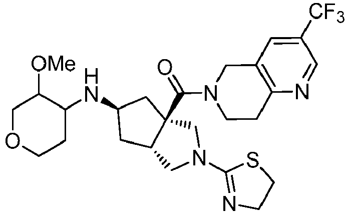

**Step A. *tert-*Butyl ((3a*R*,5*R*,6a*R*)-2-(4,5-dihydrothiazol-2-yl)-3a-(3-(trifluoromethyl)-5,6,7,8-tetrahydro-1,6-naphthyridine-6-carbonyl)octahydrocyclopenta[*c*]pyrrol-5-yl)carbamate.** To a solution of Intermediate **1** (0.114 mg, 0.251 mmol) and TEA (253 mg, 2.503 mmol) in DCM (5 mL) was added 2-bromoethyl isothiocyanate (50 mg, 0.301 mmol). The mixture was stirred at rt overnight. The reaction mixture was quenched by addition of brine, extracted with DCM, dried over Na₂SO₄. Filtration and evaporation to dryness gave the product as a tan solid. LC/MS: C₂₅H₃₂F₃N₅O₃S: m/z 540 (M+H).

**Step B. ((3a*R*,5*R*,6a*R*)-5-Amino-2-(4,5-dihydrothiazol-2-yl)octahydrocyclopenta-[*c*]pyrrol-3a-yl)(3-(trifluoromethyl)-7,8-dihydro-1,6-naphthyridin-6(5*H*)-yl)methanone ditrifluoroacetate.** The title compound was prepared analogously to Step **B** in Example **13** as a TFA salt. LC/MS: C₂₀H₂₄F₃N₅OS: m/z 440 (M+H).

**Step C. ((3a*R*,5*R*,6a*R*)-2-(4,5-Dihydrothiazol-2-yl)-5-(((3*S*^{*},4*S*^{*})-3-methoxytetrahydro-2*H*-pyran-4-yl)amino)octahydrocyclopenta[*c*]pyrrol-3a-yl)(3-(trifluoromethyl)-7,8-dihydro-1,6-naphthyridin-6(5*H*)-yl)methanone.** The title compound was prepared analogously to Step **C** in Example **13** as a yellow gel. ¹H-NMR (400 MHz, CD₃OD): δ 1.80 - 2.02 (m, 2 H), 2.09 - 2.36 (m, 3 H), 2.94 - 3.17 (m, 1 H), 3.21 - 3.75 (m, 13 H), 3.80 - 4.32 (m, 11 H), 4.85 - 5.05 (m, 2 H), 8.55 (br. s., 1 H), 9.03 (br. s., 1 H); LC/MS: C₂₆H₃₄F₃N₅O₃S: m/z 554 (M+H).

### Example 113

### ((3aR,5R,6aR)-2-(4,5-Dihydrooxazol-2-yl)-5-(((3S^{*},4S^{*})-3-methoxytetrahydro-2H-pyran-4-yl)amino)octahydrocyclopenta[c]pyrrol-3a-yl)(3-(trifluoromethyl)-7,8-dihydro-1,6-naphthyridin-6(5H)-yl)methanone

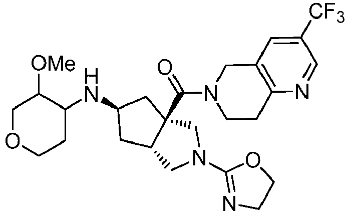

The title compound was prepared analogously to Example **112** while using 2-bromoethyl isocyanate instead of 2-bromoethyl isothiocyanate in Step **A**; the product was isolated as a white salt. ¹H-NMR (400 MHz, CD₃OD): δ 1.82 - 2.01 (m, 2 H), 2.06 - 2.38 (m, 3 H), 2.79 - 3.16 (m, 1 H), 3.23 - 4.30 (m, 23 H), 4.78 - 5.12 (m, 2 H), 8.77 (br. s., 1 H), 9.10 - 9.24 (m, 1 H); LC/MS: C₂₆H₃₄F₃N₅O₄: m/z 538 (M+H).

### Example 114

### (3aR,5R,6aR)-5-(((3S^{*},4S^{*})-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carbaldehyde.

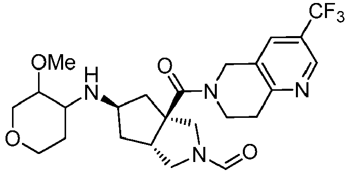

A mixture of Example **1** (70.28 mg, 0.15 mmol) in ethyl formate (1 mL) in a sealed tube was heated at 70 °C overnight. After cooling to rt, the mixture was concentrated and purified by CombiFlash (eluent: 8% methanol in DCM) gave the product as a yellowish gel. ¹H-NMR (400 MHz, CDCl₃): δ 1.50 - 1.97 (m, 5 H), 2.28 (br. s., 1 H), 2.72 (br. s., 1 H), 2.92 - 3.51 (m, 11 H), 3.59 - 4.16 (m, 8 H), 4.61 - 5.02 (m, 2 H), 7.70 (br. s., 1 H), 8.08 - 8.24 (m, 1 H), 8.73 (br. s., 1 H); LC/MS: C₂₄H₃₁F₃N₄O₄: m/z 497.2 (M+H).

### Example 115

### (3aR,5R,6aR,E)-N'-Cyano-5-(((3S^{*},4S^{*})-3-methoxytetrahydro-2H-pyran-4-yl)amino)-3a-(3-(trifluoromethyl)-5,6,7,8-tetrahydro-1,6-naphthyridine-6-carbonyl)hexahydrocyclopenta-[c]pyrrole-2(1H)-carboximidamide.

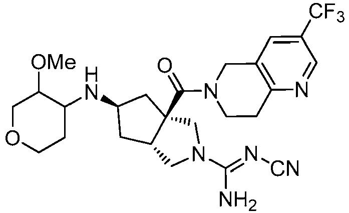

A mixture of Example **1** (HCl salt, 50 mg, 0.0865 mmol), sodium dicyanamide (10.32 mg, 0.116 mmol) in 5% water in *i*-PrOH (1.2 mL) in a sealed tube was flushed with Ar and heated at 120 °C for 5 h. After cooling to rt, the mixture was diluted with DCM, filtered and evaporated. The residue was purified by CombiFlash (eluent: 10% 7*N* NH₃ in methanol in DCM) to give the product as a colorless gel. ¹H-NMR (400 MHz, CDCl₃): δ 1.54 - 1.92 (m, 5 H), 2.37 (br. s., 1 H), 2.71 (br. s., 1 H), 3.07 - 4.23 (m, 19 H), 4.66 - 4.96 (m, 2 H), 5.80 (m, 2 H), 7.73 (s, 1 H), 8.71 (br. s., 1 H); LC/MS: C₂₅H₃₂F₃N₇O₃: m/z 536.2 (M+H).

### Example 116

### (3aR,5R,6aR,E)-N'-Hydroxy-5-(((3S^{*},4S^{*})-3-methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrole-2(1H)-carboximidamide

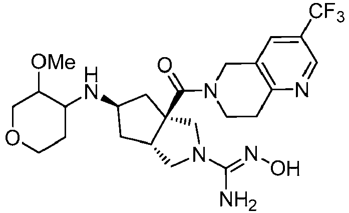

**Step A. *tert*-Butyl ((3a*R*,5*R*,6a*R*)-2-((*E*)-]*N*'-hydroxycarbamimidoyl)-3a-(3-(trifluoromethyl)-5,6,7,8-tetrahydro-1,6-naphthyridine-6-carbonyl)octahydrocyclopenta-[*c*]pyrrol-5-yl)carbamate.** A mixture of the product of Step **A** of Example **2** (0.12 g, 0.25 mmol), hydroxylamine hydrochloride (43.48 mg, 0.626 mmol) and TEA (0.087 mL, 0.626 mmol) in ethanol (1.2 mL) in a sealed tube in Ar was heated at 80 °C overnight. After removal of solvent, the residue was partitioned between EtOAc and brine, separated, dried over Na₂SO₄. Purification by CombiFlash (eluent: 8% methanol in DCM) gave the product as a white solid. ¹H-NMR (400 MHz, CDCl₃): δ 1.30 - 1.45 (m, 9 H), 1.70 - 1.99 (m, 3 H), 2.38 (br. s., 1 H), 3.09 (d, *J*=9.3 Hz, 3 H), 3.28 - 3.54 (m, 3 H), 3.65 - 3.89 (m, 3 H), 4.23 (d, *J*=5.1 Hz, 1 H), 4.44 - 4.93 (m, 5 H), 7.72 (br. s., 1 H), 8.69 (s, 1 H); LC/MS: C₂₃H₃₁F₃N₆O₄: m/z 513 (M+H).

**Step B. (3a*R*,5*R*,6a*R*,*E*)-5-Amino-*N*'-hydroxy-3a-(3-(trifluoromethyl)-5,6,7,8-tetrahydro-1,6-naphthyridine-6-carbonyl)hexahydrocyclopenta[*c*]pyrrole-2(1*H*)-carboximidamide.** The title compound was prepared analogously to Step **B** in Example **13** as TFA salt. LC/MS: C₁₈H₂₃F₃N₆O₂: m/z 413.0 (M+H).

**Step C. (3a*R*,5*R*,6a*R*,*E*)-*N*'-Hydroxy-5-(((3*S*^{*},4*S*^{*})-3-methoxytetrahydro-2*H*-pyran-4-yl)amino)-3a-(3-(trifluoromethyl)-5,6,7,8-tetrahydro-1,6-naphthyridine-6-carbonyl)hexahydro-cyclopenta[*c*]pyrrole-2(1*H*)-carboximidamide.** The title compound was prepared analogously to Step **C** in Example **13** as a colorless gel. ¹H-NMR (400 MHz, CDCl₃): δ 1.53 - 1.91 (m, 6 H), 2.28 (br. s., 1 H), 2.73 (br. s., 1 H), 2.95 - 3.58 (m, 14 H), 3.67 - 3.98 (m, 4 H), 4.00 - 4.14 (m, 1 H), 4.39 (br. s., 2 H), 4.63 - 4.95 (m, 2 H), 7.69 (s, 1 H), 8.70 (s, 1 H); LC/MS: C₂₄H₃₃F₃N₆O₄: m/z 527.2 (M+H).

### Example 117

### (3aR,5R,6aR)-5-(((3S^{*},4S^{*})-3-Methoxytetrahydro-2H-pyran-4-yl)amino)-3a-(3-(trifluoromethyl)-5,6,7,8-tetrahydro-1,6-naphthyridine-6-carbonyl)hexahydrocyclopenta[c]pyrrole-2(1H)-carbonitrile

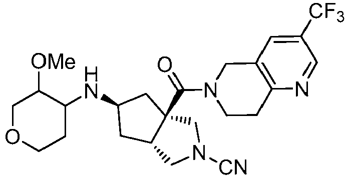

A mixture of Example **1** (0.05 g, 0.107 mmol) and potassium carbonate (17.7 mg, 0.128 mmol) in acetonitrile (1 mL) was added cyanogen bromide (5M in acetonitrile, 0.0213 mL, 0.107 mmol) and stirred at rt overnight. The mixture was filtered and the filtrate was concentrated in vacuo. The residue was purified by CombiFlash (eluent: 8% methanol in DCM) to give Example **117** as a colorless gel.

¹H-NMR (400 MHz, CDCl₃): δ 1.15 - 1.87 (m, 5 H), 2.22 - 2.38 (m, 2 H), 2.73 (br. s., 1 H), 3.06 - 4.22 (m, 18 H), 4.73 (br. s., 2 H), 7.70 (br. s., 1 H), 8.73 (br. s., 1 H); LC/MS: C₂₄H₃₀F₃N₅O₃: m/z 494.2 (M+H).

### Intermediate 9

### (3aR,5R,6aS)-tert-Butyl 5-((tert-butoxycarbonyl)amino)-1-oxo-6a-(3-(trifluoromethyl)-5,6,7,8-tetrahydro-1,6-naphthyridine-6-carbonyl)hexahydrocyclopenta-[c]pyrrole-2(1H)-carboxylate

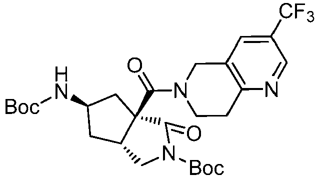

**Step A. (3a*R*,5*R*,6a*R*)-2-*tert*-Butyl 3a-methyl 5-((*tert*-butoxycarbonyl)amino)-hexahydrocyclopenta[*c*]pyrrole-2,3a(1*H*)-dicarboxylate.** A solution of the product from Step **D** in Intermediate **1** (1.314 g, 4.62 mmol), TEA (3 mL, 21.583 mmol) and di-*tert*-butyl dicarbonate (1.559 g, 6.93 mmol) in DCM (30 mL) was stirred at rt overnight. The reaction was quenched by addition of 0.5 N aqueous hydrochloric acid. The organic phase was washed with saturated NaHCO₃ solution, brine, and dried over Na₂SO₄. Purification by CombiFlash (eluent: 20% EtOAc in hexanes to 40%) gave the product as a colorless gel. ¹H-NMR (400 MHz, CDCl₃): δ 1.36 - 1.55 (m, 18 H), 1.83 - 1.98 (m, 2 H), 2.07 - 2.21 (m, 2 H), 2.96 (br. s., 1 H), 3.23 (br. s., 2 H), 3.49 - 3.67 (m, 1 H), 3.74 (s, 3 H), 3.81 - 3.95 (m, 1 H), 4.23 (d, *J*=6.1 Hz, 1 H), 4.85 (br. s., 1 H); LC/MS: C₁₉H₃₂N₂O₆: m/z 791 (2M+H).

Step **B. (3a*R*,5*R*,6a*R*)-2-*tert*-Butyl 3a-methyl 5-((*tert*-butoxycarbonyl)amino)-3-oxohexahydrocyclopenta[*c*]pyrrole-2,3a(1*H*)-dicarboxylate and (3a*R*,5*R*,6a*R*)-2-*tert*-butyl 3a-methyl 5-((*tert*-butoxycarbonyl)amino)-1-oxohexahydrocyclopenta[*c*]pyrrole-2,3a(1*H*)-dicarboxylate.** To a solution of the product from Step **A** (0.17 g, 0.442 mmol) in ACN (0.5 mL) was added RuCl₃ (1.834 mg, 0.00884 mmol), followed by a solution of sodium bromate (0.1 g, 0.663 mmol) in water (1.2 mL). The resulting biphasic mixture was stirred at rt for 12 h. The mixture was diluted with EtOAc and the organic phase was washed with Na₂S₂SO₃, brine, and dried over Na₂SO₄. Purification by CombiFlash (eluent: 20% EtOAc in hexanes to 30%) gave a mixture of two isomers as a colorless gel. LC/MS: C₁₉H₃₀N₂O₇: m/z 819.2 (2M+H).

**Step C. (3a*R*,5*R*,6a*R*)-2-(*tert*-Butoxycarbonyl)-5-((*tert*-butoxycarbonyl)amino)-3-oxooctahydrocyclopenta[c]pyrrole-3a-carboxylic acid and (3a*R*,5*R*,6a*R)*-2-(*tert-*butoxycarbonyl)-5-((*tert*-butoxycarbonyl)amino)-1-oxooctahydrocyclopenta[*c*]pyrrole-3a-carboxylic acid.** A solution of the products from Step **B** (0.46 g, 1.154 mmol) in THF (0.6 mL) and methanol (0.6 mL) was added 6 *N* KOH solution (0.577 mL, 3.463 mL). The mixture was stirred at rt for 4h. After concentration, the residue was acidified by a cooled 1*N* hydrochloric acid, extracted with EtOAc, and dried over Na₂SO₄. Filtration and evaporation to dryness gave the crude product as a mixture. LC/MS: C₁₈H₂₈N₂O₇: m/z 569.3 (2M+H-2Boc).

**Step D. (3a*R*,5*R*,6a*S*)-*tert*-Butyl 5-((*tert*-butoxycarbonyl)amino)-1-oxo-6a-(3-(trifluoromethyl)-5,6,7,8-tetrahydro-1,6-naphthyridine-6-carbonyl)hexahydrocyclopenta-[*c*]pyrrole-2(1*H*)-carboxylate and (3a*R*,5*R*,6a*R*)-*tert*-butyl 5-((tert-butoxycarbonyl)amino)-1-oxo-3a-(3-(trifluoromethyl)-5,6,7,8-tetrahydro-1,6-naphthyridine-6-carbonyl)hexahydrocyclopenta[*c*]pyrrole-2(1*H*)-carboxylate.** To a solution of the products from Step **C** (0.37 g, 0.962 mmol) and 3-(trifluoromethyl)-5,6,7,8-tetrahydro-1,6-naphthyridine dihydrochloride (0.318 g, 1.155 mmol) in DMF (5 mL) was added DIPEA (1.327 mL, 7.7 mmol), EDAC (0.24 g, 1.251 mol) and HOBt (0.26 g, 1.925 mmol). The resulting mixture was stirred at rt overnight. The reaction was quenched by addition of brine, extracted with EtOAc, and dried over Na₂SO₄. Evaporation and purification by column chromatography (eluent: 40% EtOAc in hexanes to 60%) gave the Intermediate **9** (less polar fraction) as a colorless gel and the Intermediate **10** (polar fraction). Spectra for **9:** ¹H-NMR (400 MHz, CDCl₃): δ 1.35 (br. s., 9 H), 1.46 - 1.58 (m, 9 H), 1.84 (dt, *J*=13.1, 9.0 Hz, 1 H), 2.00 - 2.19 (m, 1 H), 2.49 (dd, *J*=12.1, 6.3 Hz, 2 H), 3.17 (br. s., 2 H), 3.76 - 4.20 (m, 6 H), 4.52 - 5.02 (m, 3 H), 7.72 (br. s., 1 H), 8.73 (br. s., 1 H); LC/MS: C₂₇H₃₅F₃N₄O₆: m/z 469.2 (M-Boc).

### Intermediate 10

### (3aR,5R,6aR)-tert-Butyl 5-((tert-butoxycarbonyl)amino)-1-oxo-3a-(3-(trifluoromethyl)-5,6,7,8-tetrahydro-1,6-naphthyridine-6-carbonyl)hexahydrocyclopenta[c]pyrrole-2(1H)-carboxylate

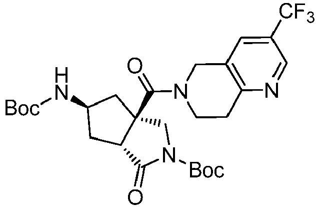

The title compound was prepared as described in the procedure for Intermediate **9.** ¹H-NMR (400 MHz, CDCl₃): δ 1.30 - 1.45 (m, 18 H), 1.84 (s, 1 H), 2.21 - 2.37 (m, 1 H), 2.52 - 2.67 (m, 1 H), 3.03 - 3.45 (m, 3 H), 3.56 - 3.98 (m, 1 H), 4.02 - 4.34 (m, 3 H), 4.52 - 4.64 (m, 1 H), 4.80 - 4.95 (m, 2 H), 5.10 (br. s., 1 H), 7.69 (br. s., 1 H), 8.70 (br. s., 1 H); LC/MS: C₂₇H₃₅F₃N₄O₆: m/z 469.2 (M-Boc).

### Example 118

### (3aR,5R,6aR)-5-(((3S^{*},4S^{*})-3-Methoxytetrahydro-2H-pyran-4-yl)amino)-3a-(3-(trifluoromethyl)-5,6,7,8-tetrahydro-1,6-naphthyridine-6-carbonyl)hexahydro-cyclopenta[c]pyrrol-1(2H)-one

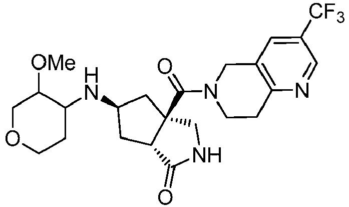

**Step A. (3a*R*,5*R*,6a*R*)-5-Amino-3a-(3-(trifluoromethyl)-5,6,7,8-tetrahydro-1,6-naphthyridine-6-carbonyl)hexahydrocyclopenta[*c*]pyrrol-1(2*H*)-one.** The title compound was prepared from Intermediate **10** analogously to Step **B** in Example **13** as a TFA salt. LC/MS: C₁₇H₁₉F₃N₄O₂: m/z 369.2 (M+H).

**Step B. (3a*R*,5*R*,6a*R*)-5-(((3*S*^{*},4*S*^{*})-3-Methoxytetrahydro-2*H*-pyran-4-yl)amino)-3a-(3-(trifluoromethyl)-5,6,7,8-tetrahydro-1,6-naphthyridine-6-carbonyl)hexahydro-cyclopenta[*c*]pyrrol-1(2*H*)-one.** The title compound was prepared analogously to Step **C** in Example **13.** ¹H-NMR (400 MHz, CDCl₃): δ 1.25 (s, 2 H), 1.63 (br. s., 5 H), 2.23 (t, *J*=5.3 Hz, 1 H), 2.82 (br. s., 1 H), 3.04 - 3.74 (m, 13 H), 3.89 - 4.31 (m, 5 H), 7.59 (s, 1 H), 8.68 (br. s., 1 H); LC/MS: C₂₃H₂₉F₃N₄O₄: m/z 483.3 (M+H).

### Example 119

### (3aR,5R,6aR)-5-(((3S^{*},4S^{*})-3-Methoxytetrahydro-2H-pyran-4-yl)amino)-6a-(3-(trifluoromethyl)-5,6,7,8-tetrahydro-1,6-naphthyridine-6-carbonyl)hexahydrocyclopenta-[c]pyrrol-1(2H)-one.

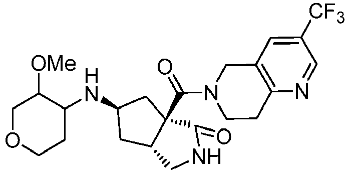

The title compound was prepared from Intermediate **9** analogously to Example **118.** ¹H-NMR (400 MHz, CDCl₃): δ 1.58 - 1.77 (m, 4 H), 1.91 - 2.05 (m, 1 H), 2.32 - 2.47 (m, 2 H), 2.69 - 2.84 (m, 1 H), 3.08 - 3.45 (m, 9 H), 3.53 (d, *J*=9.1 Hz, 1 H), 3.67 - 4.18 (m, 6 H), 4.80 (br. s., 2 H), 6.54 (d, *J*=13.6 Hz, 1 H), 7.70 (br. s., 1 H), 8.73 (br. s., 1 H); LC/MS: C₂₃H₂₉F₃N₄O₄: m/z 483.3 (M+H).

### Example 120: IN VITRO BIOLOGICAL DATA

Compounds of the invention were subjected to various representative biological tests.
The results of these tests are intended to illustrate the invention in a non-limiting fashion. MCP-1 Receptor Binding Assay in THP-1 Cells Human monocytic cell line THP-1 cells were obtained from American Type Culture Collection (Manassas, Va., USA). The THP-1 cells were grown in RPMI-1640 (RPMI: Roswell Park Memorial Institute Medium-cell culture growth media) supplemented with 10% fetal bovine serum in a humidified 5% CO₂ atmosphere at 37 °C. The cell density was maintained between 0.5×10⁶ cells/mL.

THP-1 (cells were incubated with 0.5 nM ¹²⁵I labeled MCP-1 (Perkin-Elmer Life Sciences, Inc. Boston, Mass.) in the presence of varying concentrations of either unlabeled MCP-1 (R & D Systems, Minneapolis, Minn.) or test compound for 2 hours at 30 °C. in a 96 well plate. Cells were then harvested onto a filter plate, dried, and 20 µL of Microscint 20 was added to each well. Plates were counted in a TopCount NXT, Microplate Scintillation & Luminescence Counter (Perkin-Elmer Life Sciences, Inc. Boston, Mass.). Blank values (buffer only) were subtracted from all values and drug treated values were compared to vehicle treated values. 1 µM cold MCP-1 was used for nonspecific binding.

IC₅₀ values for inhibition of MCP-1 binding to CCR2 were obtained for test compounds of the invention.

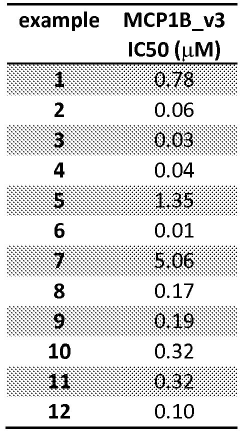

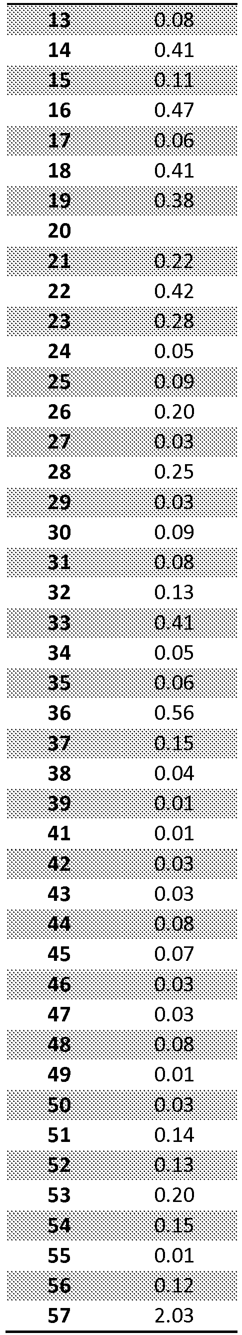

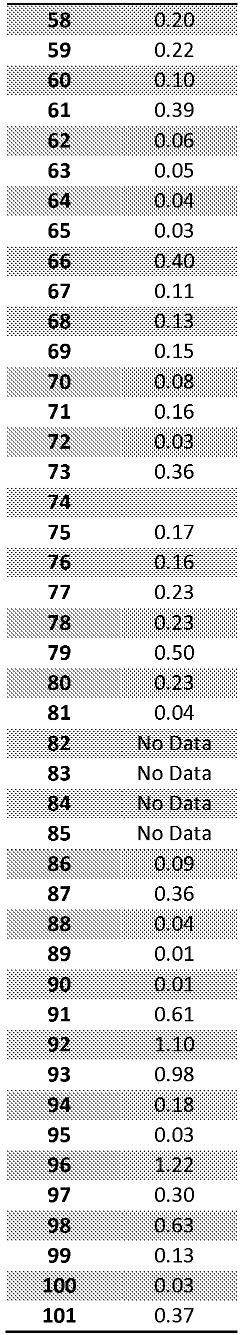

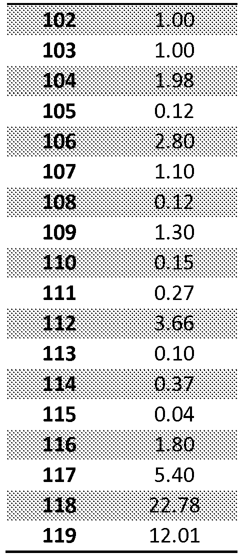

### Example 121: Animals.

Mouse CCR2 knock-out / human CCR2 knock-in mice are generated using targeted 129Sv/Evbrd embryonic stem cell clones injected into C57BL/6 mice. Expression of the *hCCR2* transcript is confirmed by quantitative reverse transcription-polymerase chain reaction performed on spleen and blood total RNA from homozygous hCCR2 knock-in mice. Backcrossing into C57BL/6 genetic background continued to the eighth generation. Transgenic mice are housed in a specific-pathogen-free, temperature-controlled facility that maintained a 12-hour light/12-hour dark cycle. Mice have free access to water and food. Experimental procedures are carried out in accordance with institutional standards for animal care and are approved by the institute's animal care and use committee.

### Example 122: Murine In vivo Cell Migration Assay.

Animals are orally dosed with vehicle or CCR2 antagonists at 3, 10 and 30 mg/kg bid. Animals undergo anesthesia and laparotomy. A distal loop of small bowel (5 cm in length) is gently eventrated onto moist sterile gauze. Synthetic human MCP-1 (1 mg/100 ml sterile PBS) or PBS alone is administered drop-wise onto the serosa of the eventrated loop. A suture knot is placed into the mesentery to mark the terminus of the treated area. Twenty-four hours later, the animal is sacrificed and the segment of bowel plus the adjacent region is removed. The tissue is opened along the mesenteric border, pinned flat and the mucosa removed. The remaining muscle layer is fixed briefly in 100% EtOH and then stained using Hanker-Yates reagent to detect myeloperoxidase-containing immune cells. At 10 mpk, P.O. bid, a compound is deemed efficacious if the inhibition of cell migration reaches 30% compared with vehicle-treated animals.

### Example 123: Thiolycollate-Induced Peritonitis in Mice.

Animals are orally dosed with vehicle or CCR2 antagonists at 3, 10, 30 and 100 mg/kg bid). One hour later, the animals are intraperiponeally injected with sterile thioglycollate (25 mL/kg, ip, Sigma) for induction of peritonitis. Animals are orally treated twice daily with vehicle or CCR2 antagonists. At the 72-hour time point, perinoteal cavities are lavaged with 10 mL of sterile saline. Total cell counts in the peritoneal lavage fluid are performed using a microscope and cell differentiation is performed using cytospin analysis after Giemsa staining (Hema Tek 2000). Percent inhibition of the thioglycollate-induced peritonitis is calculated by comparing the change in number of leukocytes of CCR2 antagonist treated mice to the vehicle-treated mice.

### Example 124: MCP-1-Induced Monocyte Recruitment to Airway of Mice.

Animals are orally treated with vehicle or CCR2 antagonists at 3, 10, and 30 mg/kg po bid). One hour later, the animals are intranasally dosed with 4 µg of MCP-1 in sterile saline. The animals are orally treated twice daily with vehicle or CCR2 antagonists. After 48 h, mice are euthanized by intraperitoneal injection of anesthesia solution (Sleepaway-Sodium pentobarbital). Whole bronchoalveolar lavage (BAL) is performed using 1.4 ml of ice-cold PBS containing 3 mM EDTA. Total cell counts in the BAL lavage fluid are performed using a microscope and cell differentiation is performed using cytospin analysis after Giemsa staining (Hema Tek 2000). Percent inhibition is calculated by comparing the change in number of total leukocyte counts (including monocytes/macrophages and lymphocytes) of compound-treated mice to the vehicle-treated mice. Compounds are deemed efficacious if percent inhibition reaches 30%.

### Example 125: High-fat Diet Induced Obesity and Insulin Resistance in Mice.

Obesity is induced by a high-fat diet that derived approximately 60% calories from lipids (D-12492; Research Diets Inc.) in animals for 10- 24 weeks at age of 7 weeks. Prior to age 7 weeks, animals are fed a standard pellet diet, in which 5% of calories were provided as fat. Obese animals were randomized by body weight and fat mass. The obese animals are orally treated with vehicle or CCR2 antagonists at 3, 10 and 30 mg/kg, po bid. Body weight and food intake and were fasting blood glucose levels monitored. Body mass was determined by a NMR analyzer (Burker MiniSpec). Insulin tolerance test is carried out in animals that were fasted for 3 hours. After an intraperitoneal bolus injection of recombinant human insulin (1.5 U/kg), blood glucose concentrations are measured using a Glucometer before and 15, 30, 45, 60, 90 and 120 minutes after injection. Glucose tolerance tests are performed after an overnight (17-hour) fast. Blood glucose concentrations are measured before and after 15, 30, 60, 90, 120 minutes after an oral dose of glucose dissolved in water (1g/kg). Energy expenditure analysis was monitored by a complete laboratory animal monitor system. After 40 days treatment with vehicle or CCR2 antagonists, the animals are sacrificed by CO₂ asphyxiation. Percent of weight loss is calculated by comparing the body weight changes of the compound-treated mice with the vehicle-treated mice.

### Example 126: Mouse Model of Allergic Asthma.

Animals are sensitized by intraperitoneal injection of 10 µg chicken egg albumin (OVA) absorbed to 1 mg Imject^{®} in 100 µL phosphate-buffered saline (PBS) on days 0 and 5. Control animals received PBS ip. OVA-immunized animals were challenged by inhalation of 0.5% OVA aerosol for 10 minutes by an ultrasonic nebulizer on days 12, 16 and 20. Control animals were challenged with PBS in similar fashion. The OVA-sensitized animals receive vehicle (0.5% Methocel) or CCR2 antagonists orally at 3, 10, 30 mg/kg twice daily from days 9-20 and once daily on Day 21,2 hours before sacrifice. Dexamethason (5 mg/kg) and Montelukast (1 mg/kg) are given orally once a day. On day 21,2 hours post the last dose of CCR2 compounds, bronchial reactivity to aerosolized methacholine is measured using a Buxco whole body plethysmograph. On day 21, the animals are sacrificed. Bronchoalveolar lavage fluid is collected (1 mL) and total cells counted. The numbers of eosinophils, lymphocytes, monocytes and neutrophils are determined using cytospin analysis after Giemsa staining (Hema Tek 2000). Percent inhibition of total BAL leukocyte count (and eosinophil count) is calculated by comparing the compound-treated mice with vehicle-treated mice. Compounds are deemed efficacious if the inhibition reaches 30%.

### Example 127: Oral Formulation - Prophetic Example

As a specific embodiment of an oral composition, 100 mg of the compound prepared as in any of Examples 1 to 119 is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims.

## Claims

1. The compounds of Formula I 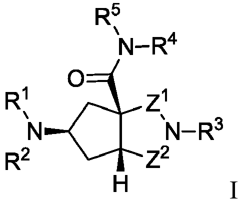 wherein
R¹ is C(₁₋₄)alkylOC(₁₋₄)alkyl, cyclohexyl, or tetrahydropyranyl, wherein said cyclohexyl or tetrahydropyranyl may be optionally substituted with one substituent selected from the group consisting of: OC(₁₋₄)alkyl, OH, CH₂CH₃, -CN, NH₂, NH(CH₃), N(CH₃)₂, or OCF₃;
R² is H, C(S)NHCH₂CH(CH₃)₂, or C(S)NHCH₃;
R³ is 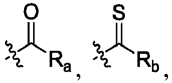 -SO₂-R_{c}, 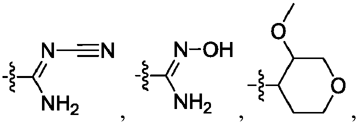 H, -CN, C(₁₋₄)alkyl, C₍₁₋₄₎alkylNA¹A², C₍₁₋₃₎alkylC(O)NA¹A², C₍₃₋₆₎cycloalkyl, oxetan-3-yl, -(CH₂)ₙPh-Rₐₐ, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl; wherein said 4,5 dihydro thiazolyl, 4,5 dihydro oxazolyl, thiazolyl, oxazolyl, pyrimidinyl, pyridyl, pyrazyl, and furyl may be optionally substituted with up to two substituent independently selected from Rₐₐ.
n is 0, 1, 2, or 3;
Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl, -CH₂NHBoc,-OCH₂CH=CH₂, -OCH₂CH₂CF₃, -OCH₂CH₂C(CH₃)₂OH, -OCH₂CH₂OC₍₁₋₄₎alkyl, -OCH₂CH₂CN, -OPh-Rₐₐ, 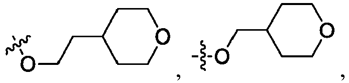 -OC₍₁₋₄₎alkyl-Ph-Rₐₐ, 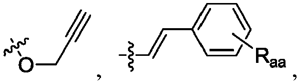 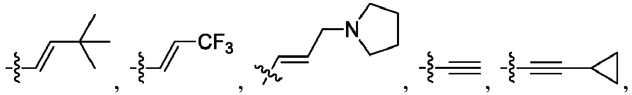 phenyl-Rₐₐ, oxazol-2-yl, oxazol-4-yl, isoxazol-5-yl, or thiazol-2-yl;
Rₐₐ is H, OC₍₁₋₄₎alkyl, OCF₃, -CO₂H, Cl, Br, F, or -CN;
R_{b} is NA¹A²;
R_{c} is NA¹A², CH₂Ph, CH₂CH₂Ph, or C₍₁₋₄₎alkyl;
A¹ is H, C₍₁₋₆₎alkyl, Ph-Rₐₐ, C(O)CH₃, CH₂Ph-Rₐₐ, or C₍₁₋₄₎alkylOC₍₁₋₄₎alkyl;
A² is H, C₍₁₋₆₎alkyl; or
A¹ and A² may be taken together with the nitrogen to which they are attached to form a ring selected from the group consisting of: 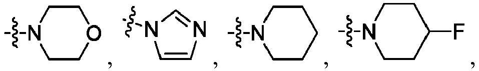 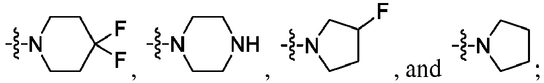
R⁴ is CH₂Ph, wherein said Ph is optionally substituted with up to two groups selected from CF₃, OCF₃, and F;
R⁵ is H; or
R⁴ and R⁵ are taken together with their attached nitrogen to form a pair of fused rings selected from the group consisting of: 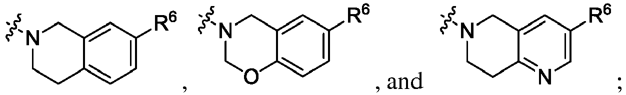 R⁶ is CF₃, or OCF₃;
Z¹ is CH₂ or C=O;
Z² is CH₂ or Z² may be C=O provided that Z¹ and Z² are not both simultaneously C=O;
and pharmaceutically acceptable salts thereof.

2. A compound of claim 1, wherein:
R¹ is C₍₁₋₄₎alkylOCH₃, cyclohexyl, 1-methoxy cyclohex-2-yl, tetrahydropyran-4-yl, or 3-C₍₁₋₄₎alkoxy tetrahydropyran-4-yl;
R³ is 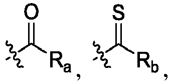 -SO₂-R_{c}, 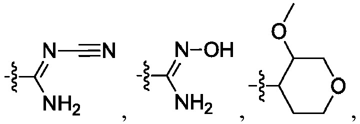 H, -CN, C₍₁₋₄₎alkyl, C₍₁₋₄₎alkylNA¹A², C₍₁₋₃₎alkylC(O)N(C₍₁₋₂₎alkyl)₂, C₍₃₋₆₎cycloalkyl, oxetan-3-yl, -(CH₂)ₙPh, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl;
Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl, -CH₂NHBoc,-OCH₂CH=CH₂, -OCH₂CH₂CF₃, -OCH₂CH₂C(CH₃)₂OH, -OCH₂CH₂OC₍₁₋₄₎alkyl, -OCH₂CH₂CN, -OPh, 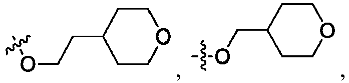 -OC₍₁₋₄₎alkyl-Ph-Rₐₐ, 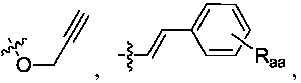 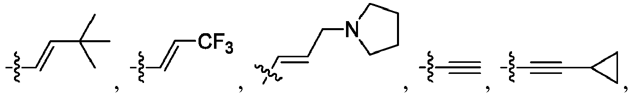 phenyl, oxazol-2-yl, oxazol-4-yl, isoxazol-5-yl, or thiazol-2-yl;
Rₐₐ is H, OC₍₁₋₄₎alkyl, -CO₂H, Cl, Br, F, or -CN;
R_{c} is NH₂, NHCH₂Ph, CH₂Ph, CH₂CH₂Ph, or C₍₁₋₄₎alkyl;
R⁴ is CH₂Ph, wherein said Ph is optionally substituted with up to two groups selected from CF₃, OCF₃, and F;
R⁵ is H; or
R⁴ and R⁵ are taken together with their attached nitrogen to form a pair of fused rings selected from the group consisting of: 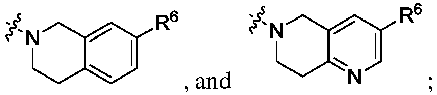
and pharmaceutically acceptable salts thereof.

3. A compound of claim 2, wherein:
R¹ is C₍₁₋₄₎alkylOCH₃, 3-C₍₁₋₄₎alkoxy tetrahydropyran-4-yl, or tetrahydropyran-4-yl;
R³ is 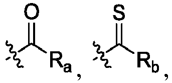 -SO₂-Rc, 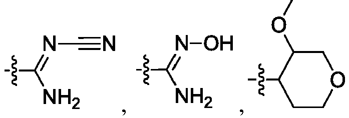 H, -CN, C₍₁₋₄₎alkyl, -C₍₁₋₃₎alkylC(O)N(C₍₁₋₂₎alkyl)₂, C₍₃₋₆₎cycloalkyl, oxetan-3-yl, -(CH₂)ₙPh, -C₍₁₋₄₎alkylCO₂C₍₁₋₄₎alkyl, 4,5 dihydro thiazolyl, 4,5 dihydro oxazolyl, thiazolyl, oxazolyl, pyrimidinyl, pyridyl, pyrazyl, furyl, or 3-methyl 1,2,4 oxadiazol-5-yl;
Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂OC(O)C₍₁₋₄₎alkyl, -CH₂OC₍₁₋₄₎alkyl, -CH₂NHBoc,-OCH₂CH=CH₂, -OCH₂CH₂CF₃, -OCH₂CH₂C(CH₃)₂OH, -OCH₂CH₂OCH₃, -OCH₂CH₂CN,-OPh, 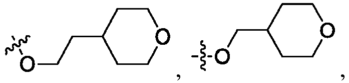 -OC₍₁₋₄₎alkyl-Ph-Rₐₐ, 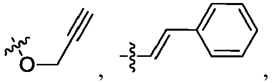 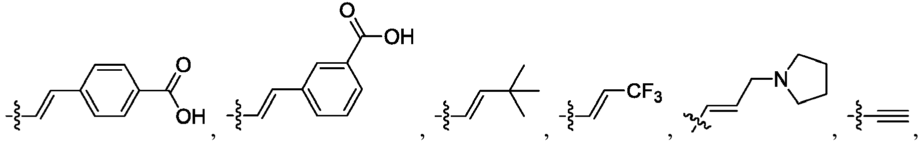 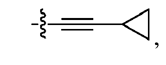 phenyl, oxazol-2-yl, oxazol-4-yl, isoxazol-5-yl, or thiazol-2-yl;
Rₐₐ is H, OC₍₁₋₄₎alkyl, or -CN;
R_{b} is 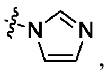 NH₂, NHCH₃, NHCH₂Ph, or NHCH₂CH(CH₃)₂;
R_{c} is NH₂, NHCH₂Ph, CH₂Ph, CH₂CH₂Ph, or CH₃;
A¹ is H, C₍₁₋₆₎alkyl, Ph, or C(O)CH₃, CH₂Ph, C₍₁₋₄₎alkylOC₍₁₋₄₎alkyl;
A² is H, C₍₁₋₆₎alkyl; or
A¹ and A² may be taken together with the nitrogen to which they are attached to form a ring selected from the group consisting of: 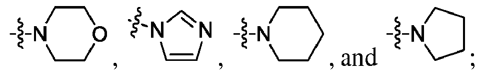
and pharmaceutically acceptable salts thereof.

4. A compound of claim 3, wherein:
R¹ is CH₂CH₂OCH₃, 3-C₍₁₋₄₎alkoxy tetrahydropyran-4-yl, or tetrahydropyran-4-yl;
R³ is 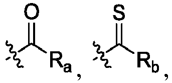 -SO₂-Rc, 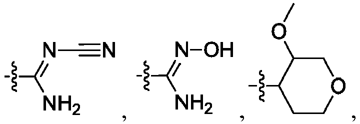 H, -CN, C₍₁₋₄₎alkyl, -C₍₁₋₃₎alkylC(O)N(C₍₁₋₂₎alkyl)₂, cyclopropyl, cyclobutanyl, oxetan-3-yl, -(CH₂)ₙPh, -C₍₁₋₄₎alkylCO₂C₍₁₋₄₎alkyl, 4,5 dihydro thiazol-2-yl, 4,5 dihydro oxazol-2-yl, thiazol-2-yl, pyrimidin-2-yl, or 3-methyl 1,2,4 oxadiazol-5-yl;
Rₐ is H, NA¹A², NHCH₂CH₂NA₁A₂, C₍₁₋₄₎alkylNA¹A², OC₍₁₋₄₎alkylNA¹A², C₍₁₋₆₎alkyl, OC₍₁₋₆₎alkyl, -CN, -CH₂CH₂Ph, -CH₂OPh, -CH₂NHBoc, -OCH₂Ph, -CH₂OC(O)C₍₁₋₄₎alkyl, -CH₂OC₍₁₋₄₎alkyl,-OCH₂Ph-CN, -OCH₂Ph-OCH₃, -OCH₂CH=CH₂, -OCH₂CH₂CF₃, - OCH₂CH₂C(CH₃)₂OH, -OCH₂CH₂OCH₃, -OCH₂CH₂CN, -OPh, 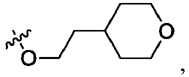 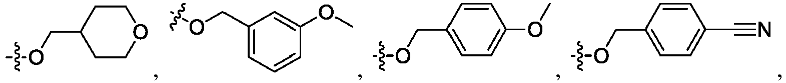 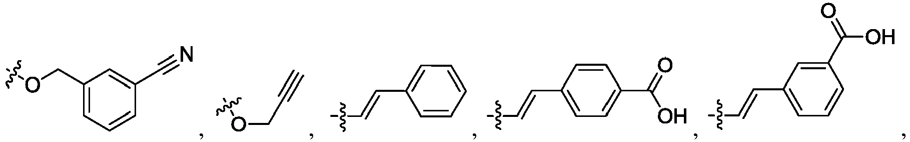 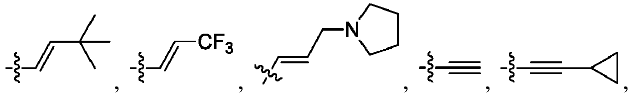 phenyl, oxazol-2-yl, oxazol-4-yl, isoxazol-5-yl, or thiazol-2-yl;
A¹ is H, C₍₁₋₆₎alkyl, Ph, or C(O)CH₃, CH₂Ph, C₍₁₋₄₎alkylOC₍₁₋₄₎alkyl;
A² is H, C₍₁₋₆₎alkyl; or
A¹ and A² may be taken together with the nitrogen to which they are attached to form a ring selected from the group consisting of: 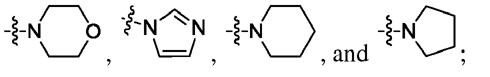
and pharmaceutically acceptable salts thereof.

5. A compound of claim 4, wherein:
R¹ is CH₂CH₂OCH₃, 3-C₍₁₋₂₎alkoxy tetrahydropyran-4-yl, or tetrahydropyran-4-yl;
R³ is 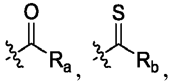 -SO₂-Rc, 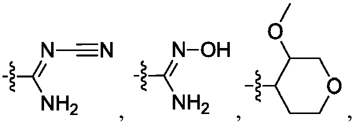 H, -CN, C₍₁₋₃₎alkyl, - CH₂C(O)N(CH₃)₂, cyclopropyl, oxetan-3-yl, -(CH₂)ₙPh, -CH₂CO₂CH₃, 4,5 dihydro thiazol-2-yl, 4,5 dihydro oxazol-2-yl, thiazol-2-yl, pyrimidin-2-yl, or 3-methyl 1,2,4 oxadiazol-5-yl;
Rₐ is H, NH₂, NHCH₃, N(CH₃)₂, NHCH₂CH(CH₃)₂, NHCH(CH₃)₂, NHCH₂C(CH₃)₃, NHCH₂CH₂OCH₃, NHCH₂CH₂-morpholinyl, NHPh, NHCH₂Ph, CH₂CH₂N(CH₃)₂, CH₂CH₂CH₂N(CH₃)₂, -CN, -CH₃, -CH₂CH₂Ph, -CH₂OPh, -CH₂OC(O)CH₃, -CH₂OCH₃,-CH₂OC(CH₃)₃, -CH₂NHC(O)CH₃, -CH₂N(CH₃)₂, -CH₂NHBoc, -OCH₃, -OCH₂C(CH₃)₃,-OCH₂CH(CH₃)₂, -OCH₂CH₂CH₃, -OCH₂Ph, -OCH₂Ph-CN, -OCH₂Ph-OCH₃, -OCH₂CH=CH₂,-OCH₂CH₂CF₃, -OCH₂CH₂C(CH₃)₂OH, -OCH₂CH₂OCH₃, -OCH₂CH₂CN, -OCH₂CH₂N(C₍₁₋₂₎alkyl)₂, -OCH₂CH₂NHC(O)CH₃, -OPh, 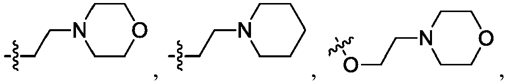 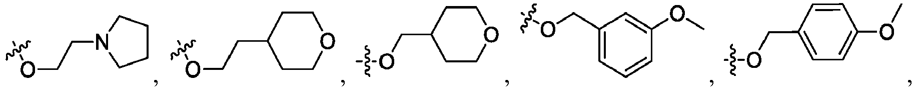 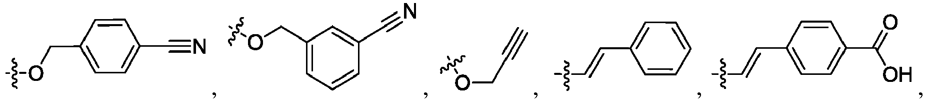 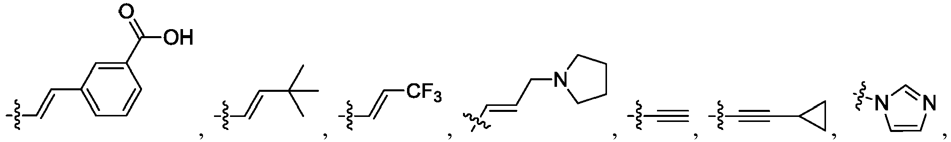 phenyl, oxazol-2-yl, oxazol-4-yl, isoxazol-5-yl, thiazol-2-yl, 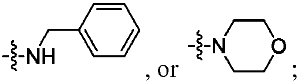
R⁴ is 1,3-bis(trifluoromethyl)benz-5-yl, 1-fluoro-3-(trifluoromethyl)benz-5-yl, or 1-(trifluoromethyl)benz-5-yl;
R⁵ is H; or
R⁴ and R⁵ are taken together with their attached nitrogen to form a pair of fused rings selected from the group consisting of: 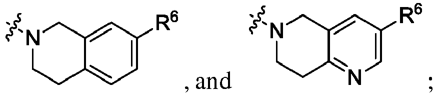
and pharmaceutically acceptable salts thereof.

6. A compound of claim 1, selected from the group consisting of: 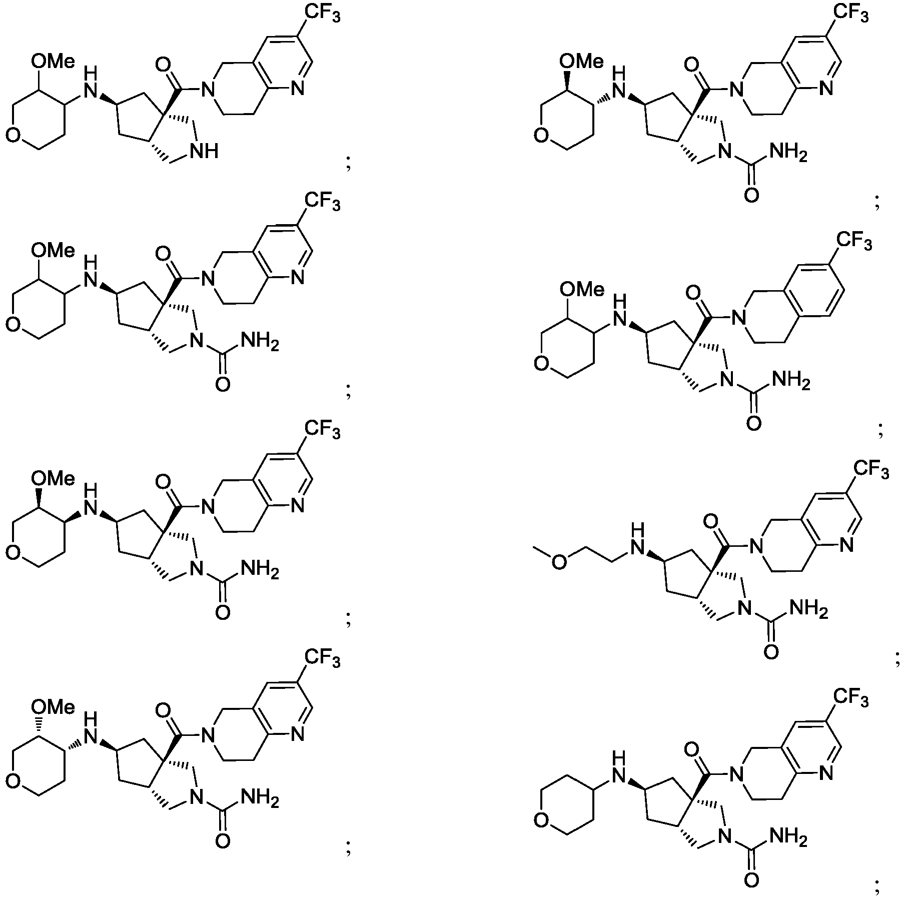 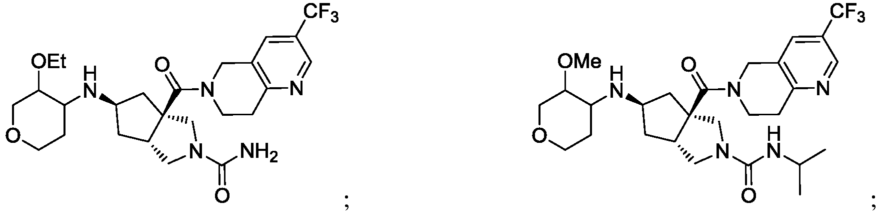 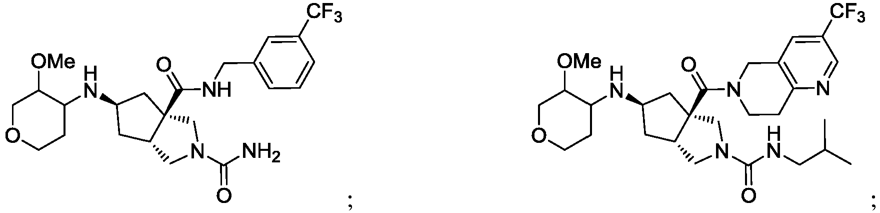 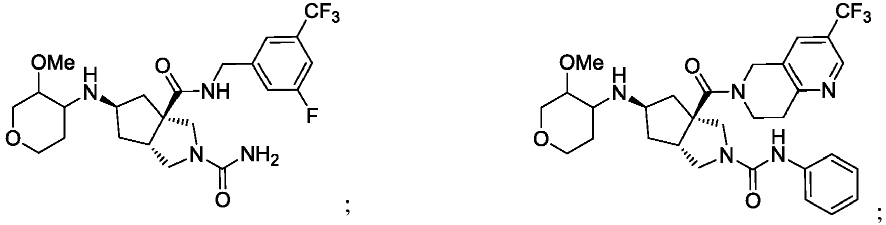 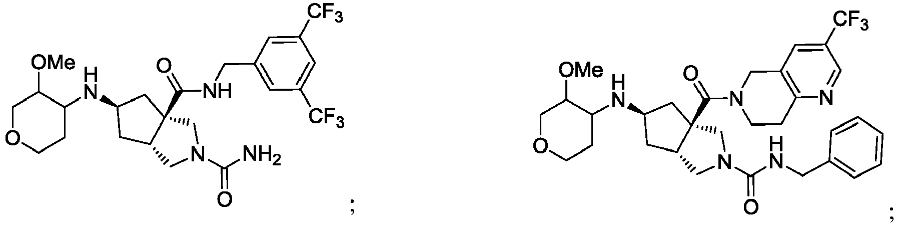 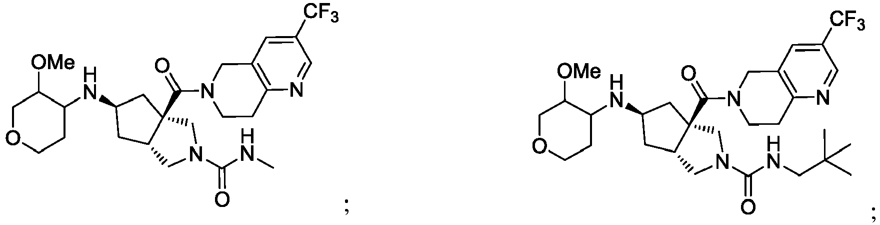 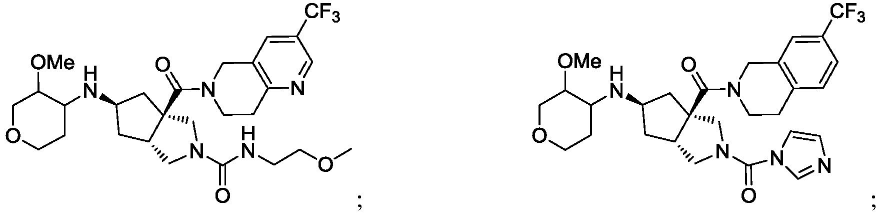 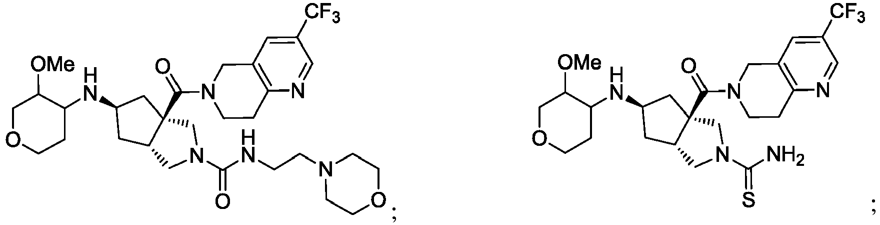 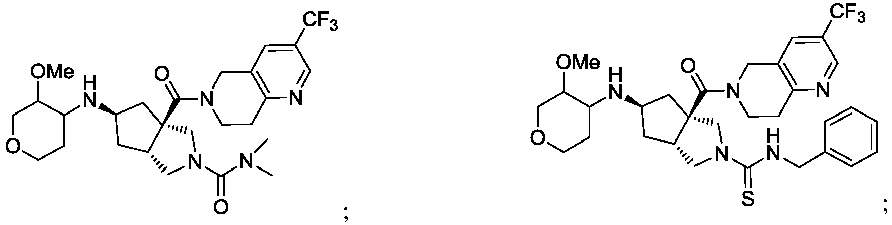 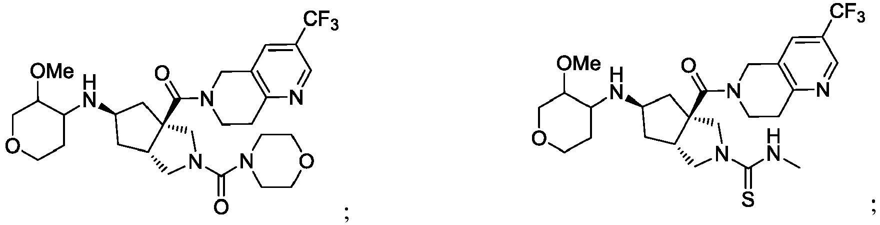 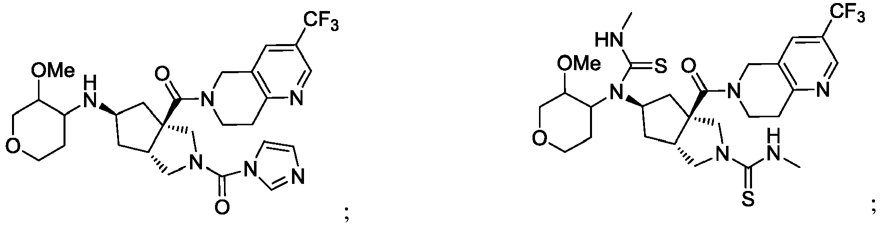 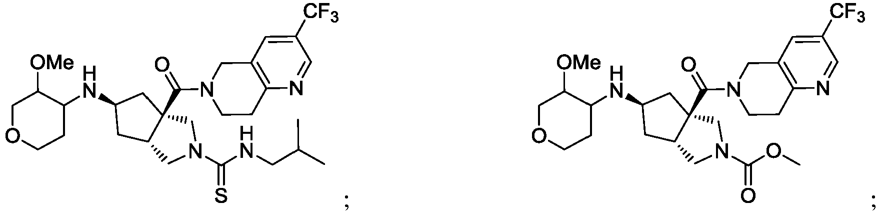 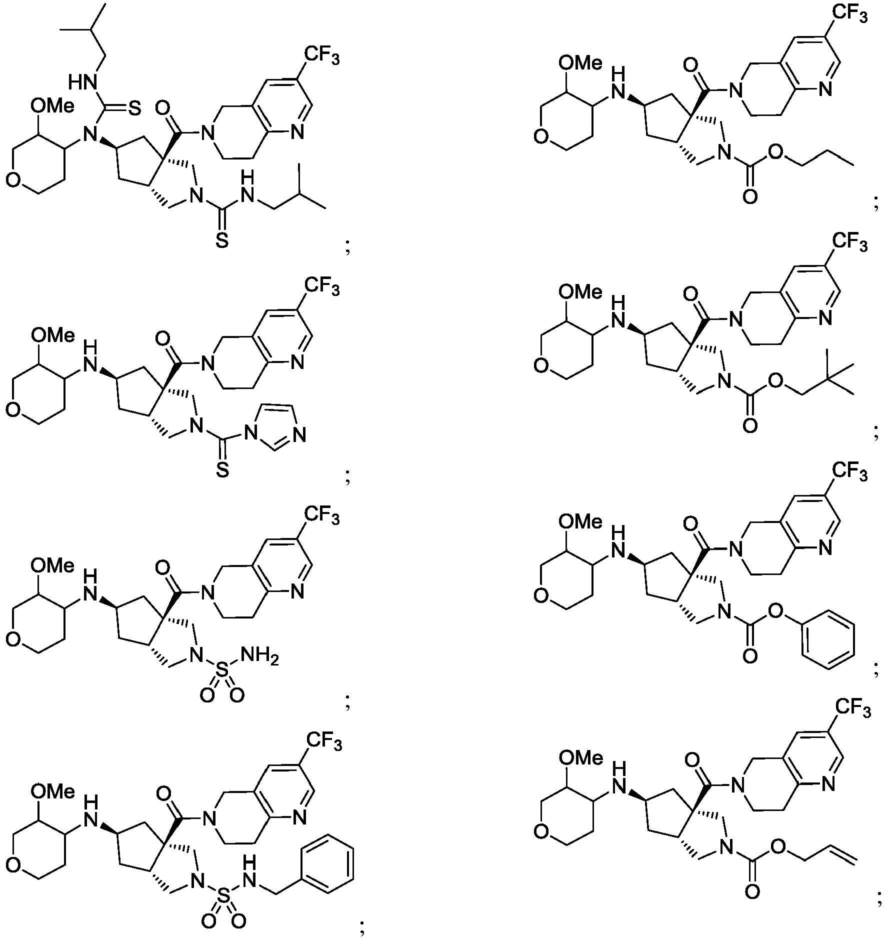 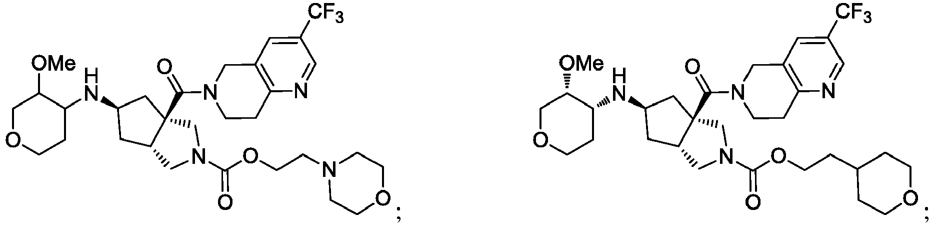 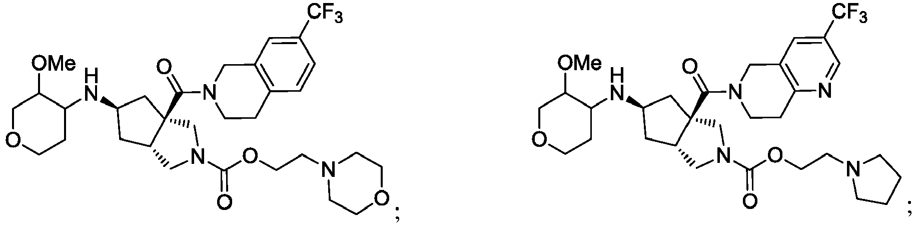 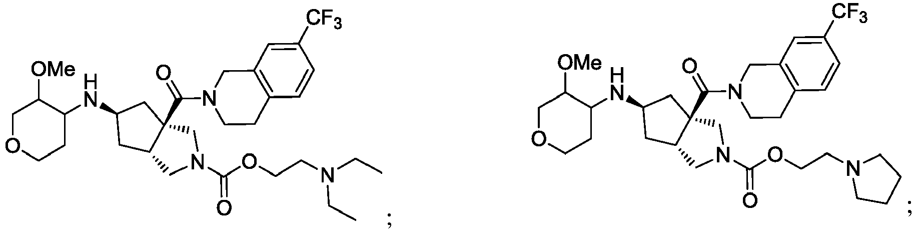 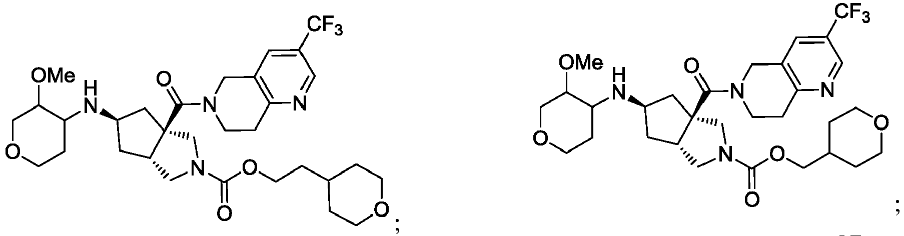 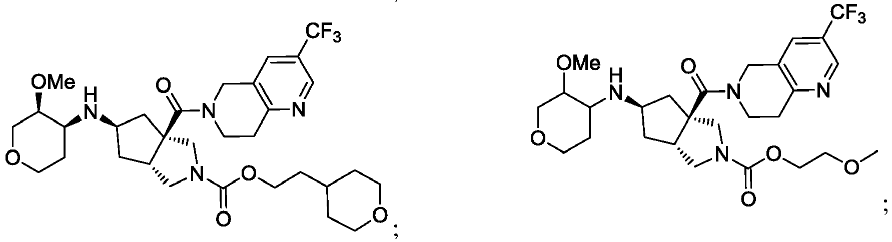 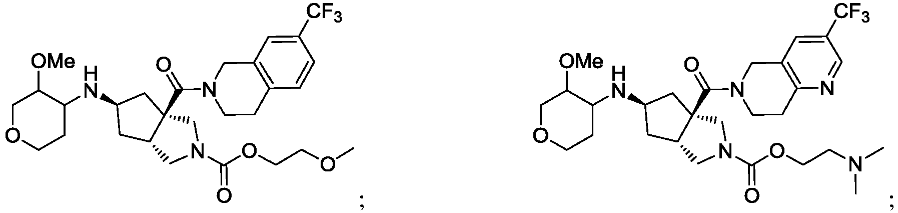 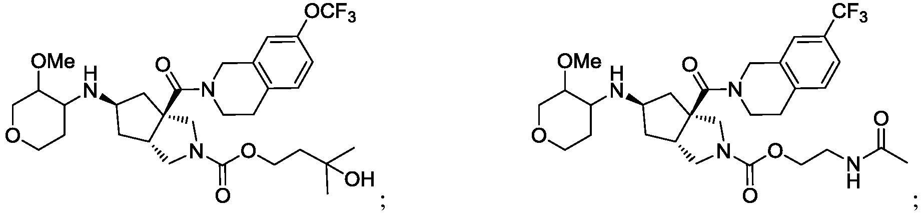 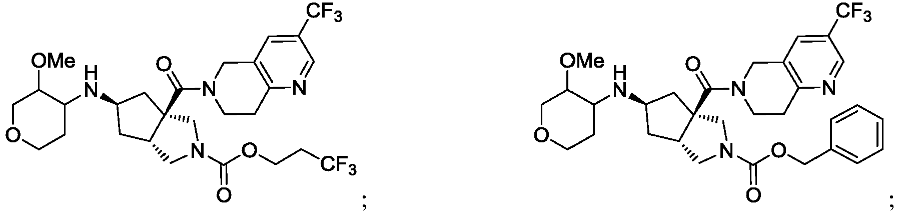 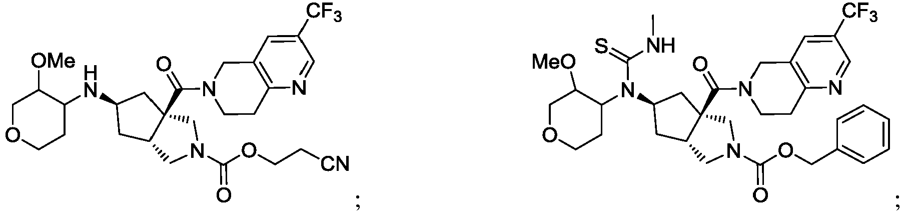 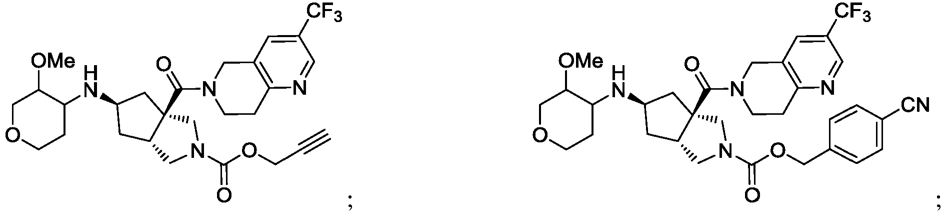 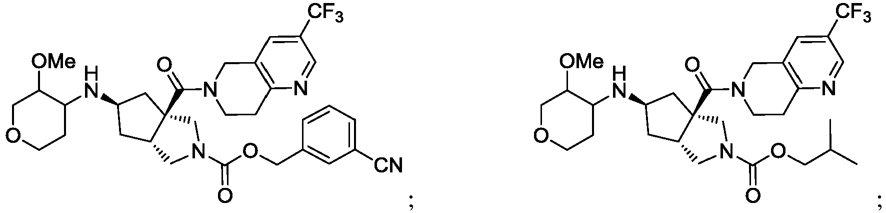 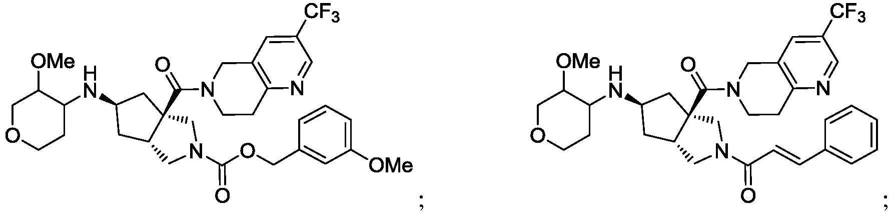 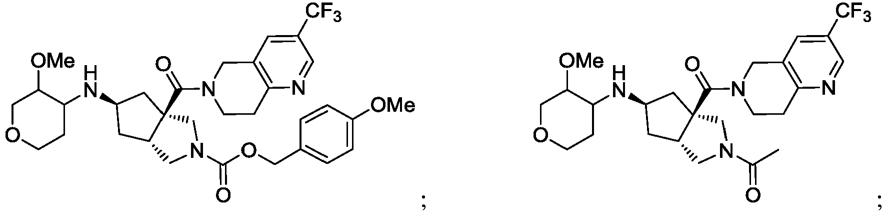 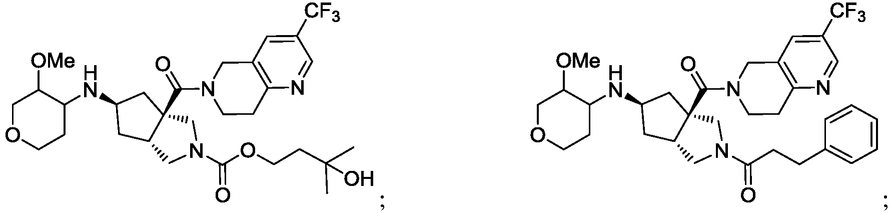 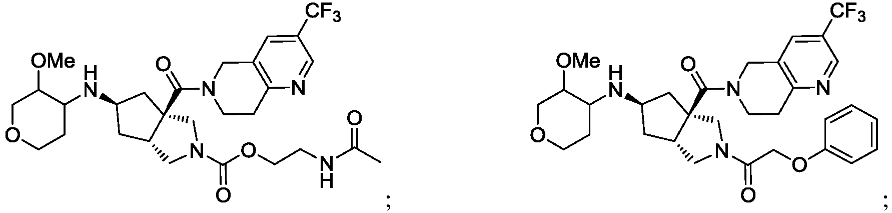 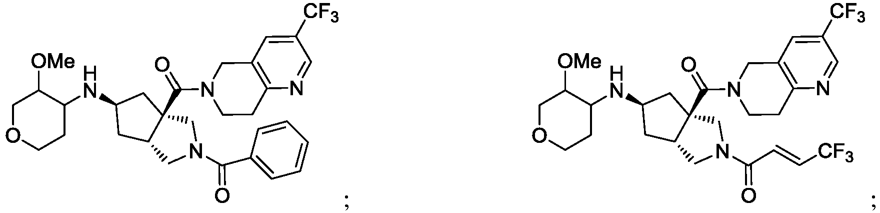 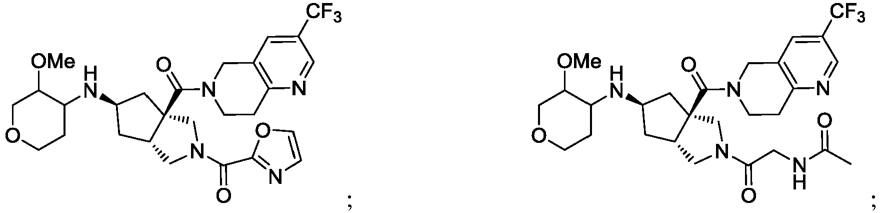 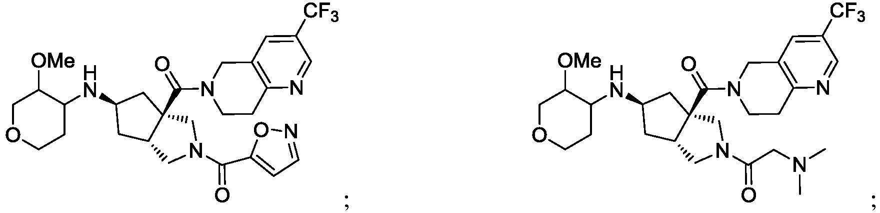 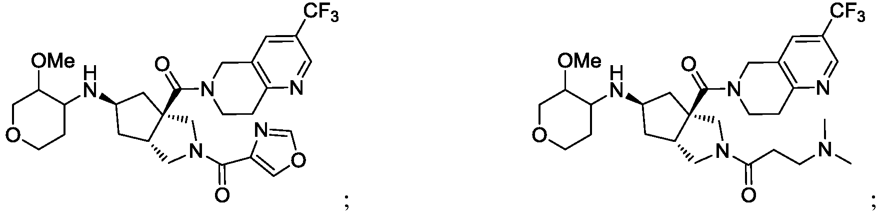 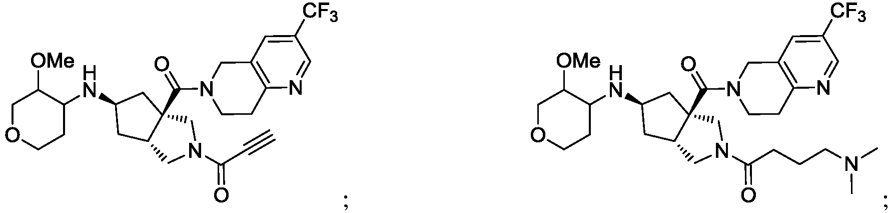 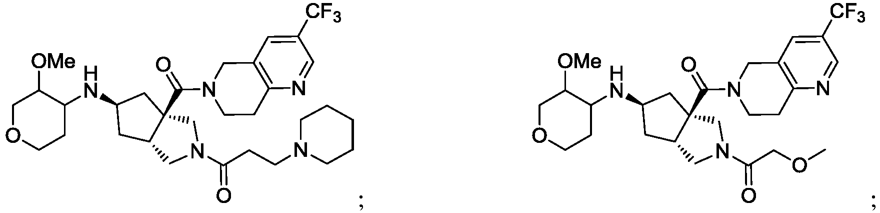 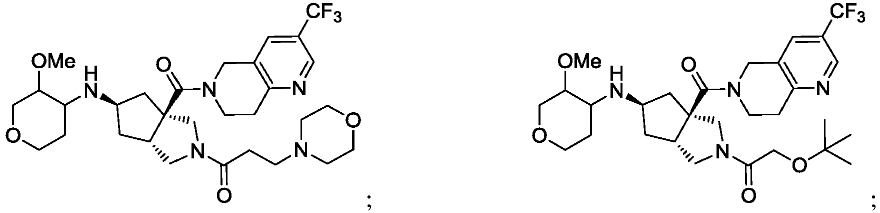 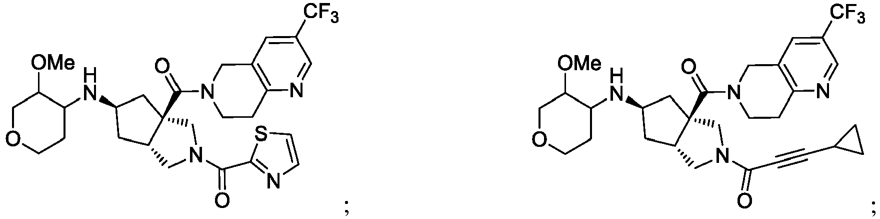 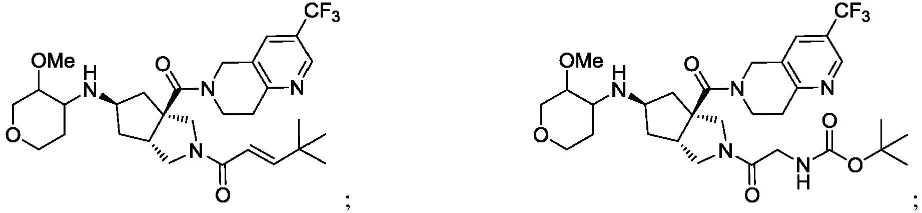 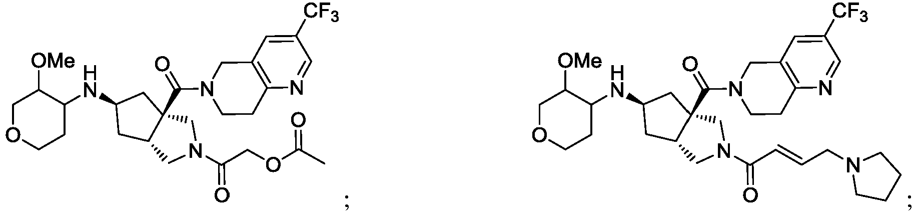 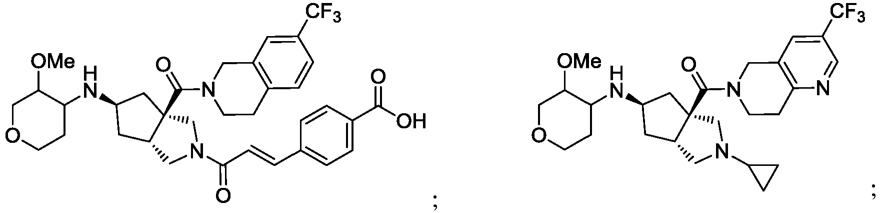 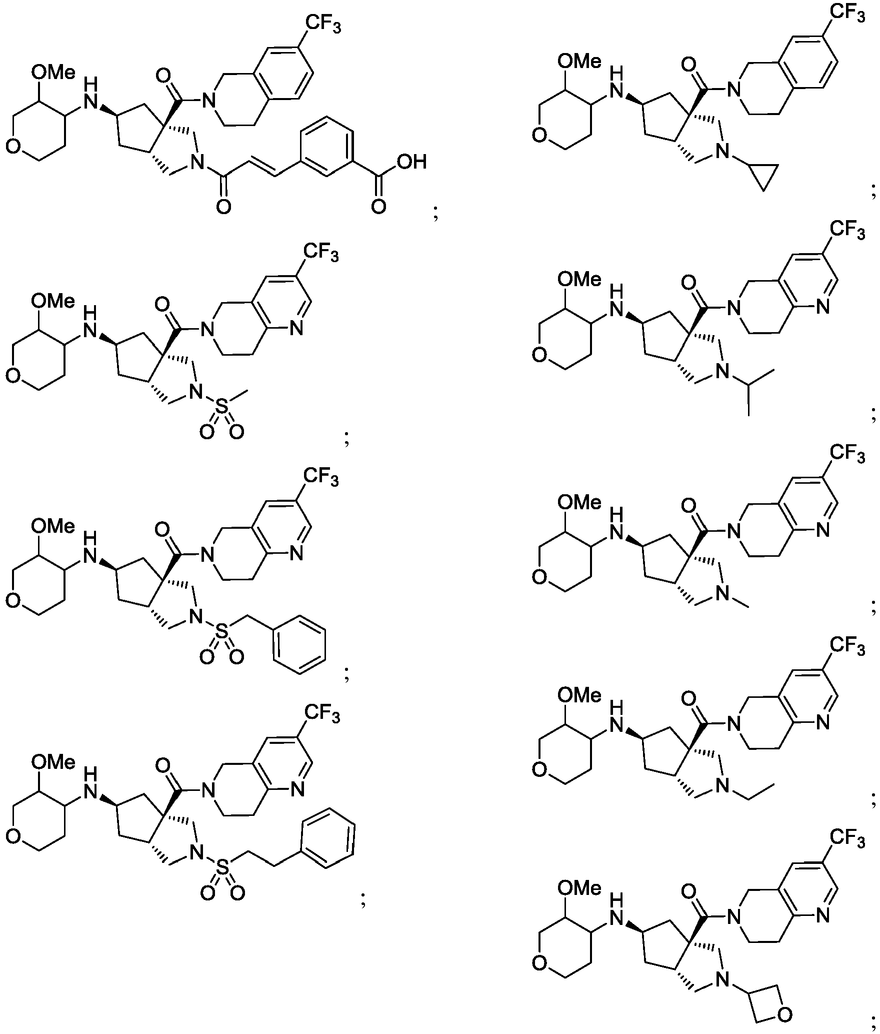 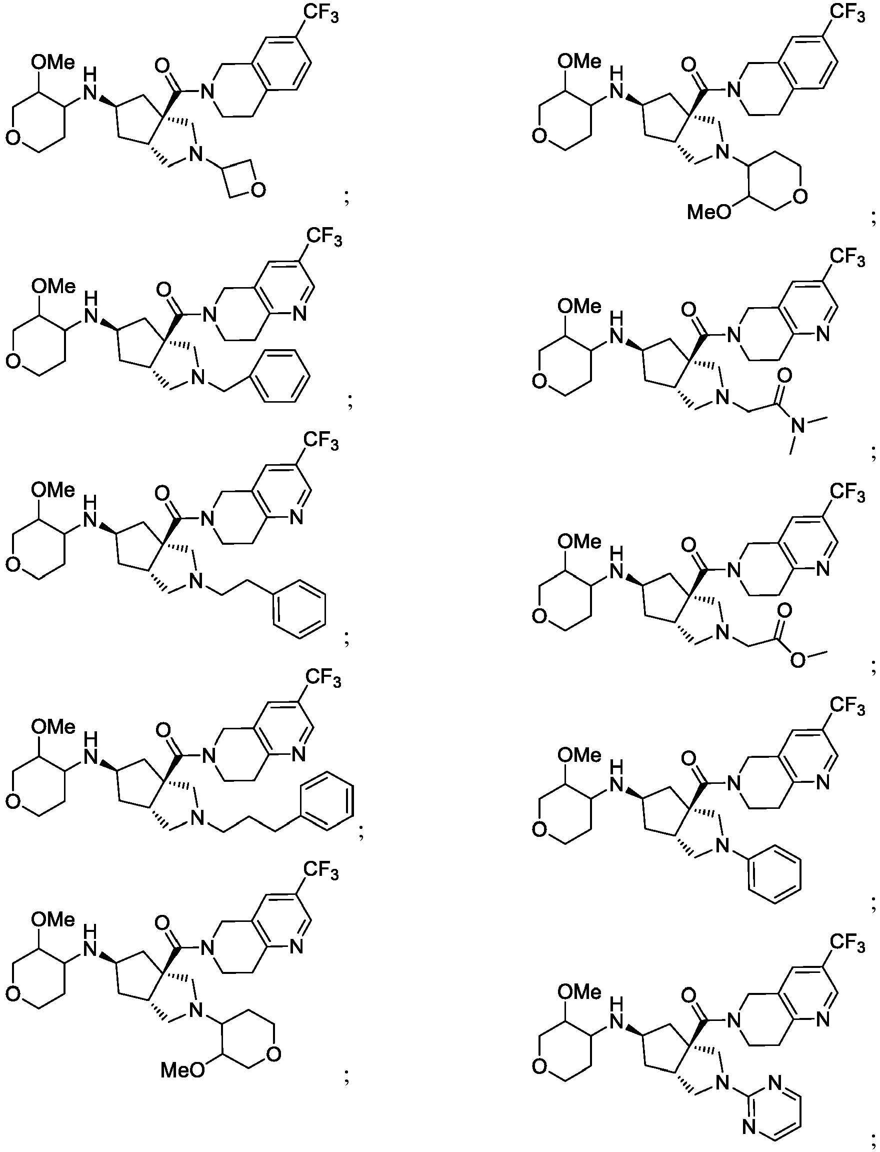 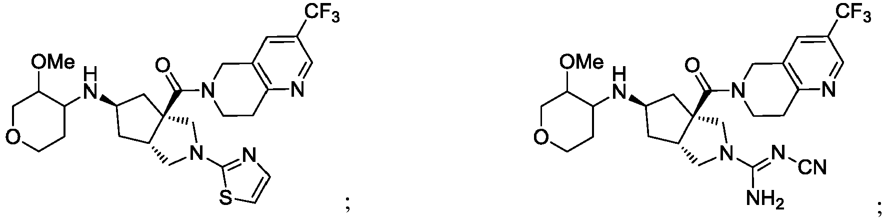 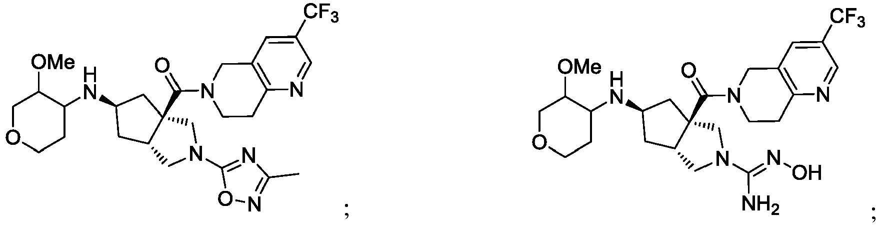 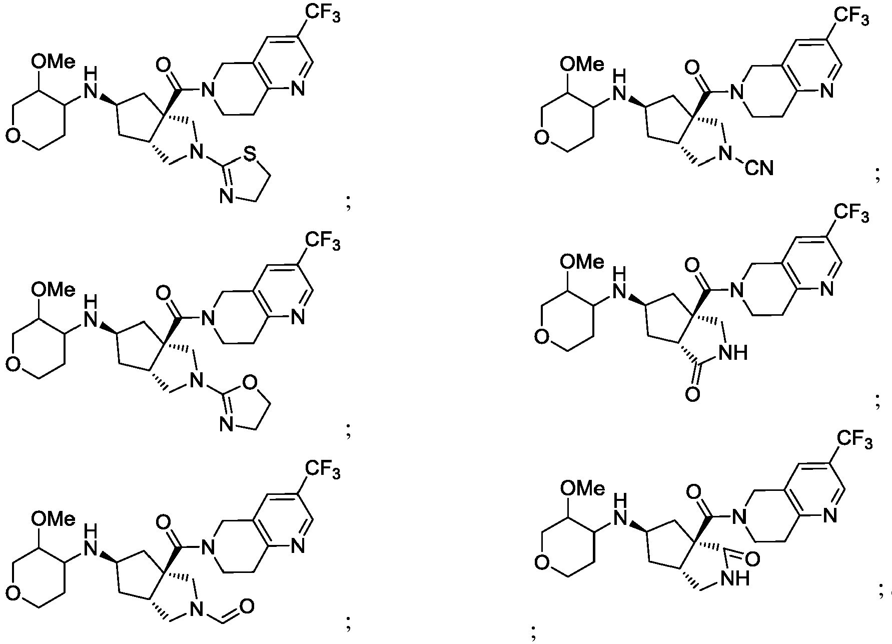 and and pharmaceutically acceptable salts thereof.

7. A pharmaceutical composition, comprising a compound of any one of claims 1 to 6 and a pharmaceutically acceptable carrier.

8. The pharmaceutical composition of claim 7, wherein the pharmaceutical composition is made by mixing the compound and the pharmaceutically acceptable carrier.

9. A process for making the pharmaceutical composition of claim 7, said process comprising mixing the compound and the pharmaceutically acceptable carrier.

10. A compound of any one of claims 1 to 6 for use in a method of preventing, treating or ameliorating a syndrome, disorder or disease, wherein said syndrome, disorder or disease is selected from the group consisting of: chronic obstructive pulmonary disorder (COPD), ophthalmic disorders, uveitis, atherosclerosis, rheumatoid arthritis, psoriasis, psoriatic arthritis, atopic dermatitis, multiple sclerosis, Crohn's Disease, ulcerative colitis, nephritis, organ allograft rejection, fibroid lung, renal insufficiency, type-I diabetes, type II diabetes, diabetic complications, diabetic nephropathy, diabetic retinopathy, diabetic retinitis, diabetic microangiopathy, overweight, obesity, obesity-associated insulin resistance, metabolic syndrome, tuberculosis, sarcoidosis, invasive *staphyloccocia,* inflammation after cataract surgery, allergic rhinitis, allergic conjunctivitis, chronic urticaria, asthma, allergic asthma, periodontal diseases, periodonitis, gingivitis, gum disease, diastolic cardiomyopathies, cardiac infarction, myocarditis, chronic heart failure, angiostenosis, restenosis, reperfusion disorders, aortic abdominal aneurism, glomerulonephritis, solid tumors and cancers, chronic lymphocytic leukemia, chronic myelocytic leukemia, multiple myeloma, malignant myeloma, Hodgkin's disease, and carcinomas of the bladder, breast, cervix, colon, lung, prostate, or stomach, and chronic neuroinflammatory disorders including, but not limited to, Alzheimer's disease, ischemic stroke, spinal cord injury, nerve crush injury and traumatic brain injury, wherein said method comprises administering to a subject in need thereof an effective amount of said compound.

11. A compound for use in the method of preventing, treating or ameliorating a syndrome, disorder or disease according to claim 10, wherein said syndrome, disorder or disease is selected from the group consisting of: type I diabetes, type II diabetes, diabetic complications, diabetic nephropathy, diabetic retinopathy, diabetic retinitis, diabetic microangiopathy, obesity, obesity-associated insulin resistance, metabolic syndrome, asthma, and allergic asthma, wherein said method comprises administering to a subject in need thereof a therapeutically effective amount of the compound.

12. A compound for use in the method of treating a disorder according to claim 10 selected from the group consisting of type II diabetes, obesity and asthma, wherein said method comprises administering to a subject in need thereof a therapeutically effective amount of the compound.

## Patentansprüche

1. Verbindungen der Formel I 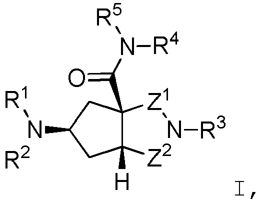 wobei
R¹ für C₍₁₋₄₎-Alkyl-O-C₍₁₋₄₎-alkyl, Cyclohexyl, oder Tetrahydropyranyl steht, wobei das Cyclohexyl oder Tetrahydropyranyl gegebenenfalls durch einen Substituenten ausgewählt aus der Gruppe bestehend aus O-C₍₁₋₄₎-Alkyl, OH, CH₂CH₃, -CN, NH₂, NH(CH₃), N(CH₃)₂ oder OCF₃ substituiert sein kann,
R² für H, C(S)NHCH₂CH(CH₃)₂ oder C(S)NHCH₃ steht,
R³ für 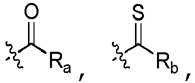 -SO₂-R_{c}, 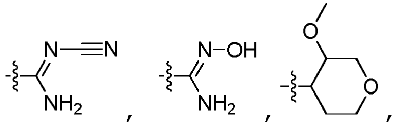 H, -CN, C₍₁₋₄₎-Alkyl, C₍₁₋₄₎-Alkyl-NA¹A², C₍₁₋₃₎-Alkyl-C(O)NA¹A², C₍₃₋₆₎-Cycloalkyl, Oxetan-3-yl, -(CH₂)ₙPh-Rₐₐ, -C₍₁₋₄₎-Alkyl-CO₂C₍₁₋₄₎-alkyl, 4, 5-Dihydrothiazolyl, 4,5-Dihydrooxazolyl, Thiazolyl, Oxazolyl, Pyrimidinyl, Pyridyl, Pyrazyl, Furyl oder 3-Methyl-1,2,4-oxadiazol-5-yl steht, wobei 4,5-Dihydrothiazolyl, 4,5-Dihydrooxazolyl, Thiazolyl, Oxazolyl, Pyrimidinyl, Pyridyl, Pyrazyl und Furyl gegebenenfalls durch bis zu zwei unabhängig voneinander aus Rₐₐ ausgewählte Substituenten substituiert sein können,
n für 0, 1, 2 oder 3 steht,
Rₐ für H, NA¹A², NHCH₂CH₂NA₁A₂, C₍₁₋₄₎-Alkyl-NA¹A², O-C₍₁₋₄₎-Alkyl-NA¹A², C₍₁₋₆₎-Alkyl, O-C₍₁₋₆₎-Alkyl, -CN, -CH₂CH₂Ph, -CH₂OPh, -CH₂OC(O)-C₍₁₋₄₎-Alkyl, -CH₂O-C₍₁₋₄₎-Alkyl, -CH₂NHBoc, -OCH₂CH=CH₂, -OCH₂CH₂CF₃, -OCH₂CH₂C(CH₃)₂OH, -OCH₂CH₂O-C₍₁₋₄₎-Alkyl, -OCH₂CH₂CN, -OPh-Rₐₐ, 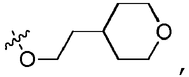 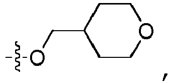 -O-C₍₁₋₄₎-Alkyl-Ph-Rₐₐ, 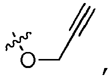 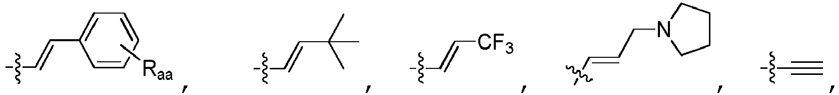 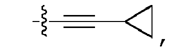 Phenyl-Rₐₐ, Oxazol-2-yl, Oxazol-4-yl, Isoxazol-5-yl oder Thiazol-2-yl steht,
Rₐₐ für H, O-C₍₁₋₄₎-Alkyl, OCF₃, -CO₂H, Cl, Br, F oder -CN steht,
R_{b} für NA¹A² steht,
R_{c} für NA¹A², CH₂Ph, CH₂CH₂Ph oder C₍₁₋₄₎-Alkyl steht,
A¹ für H, C₍₁₋₆₎-Alkyl, Ph-Rₐₐ, C(O)CH₃, CH₂Ph-Rₐₐ oder C₍₁₋₄₎-Alkyl-O-C₍₁₋₄₎-alkyl steht,
A² für H oder C₍₁₋₆₎-Alkyl steht oder
A¹ und A² zusammen mit dem Stickstoff, an den sie gebunden sind, einen Ring ausgewählt aus der Gruppe bestehend aus 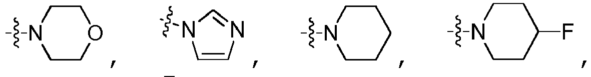 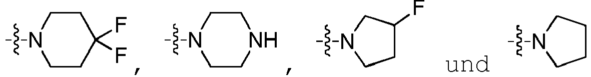 bilden können,
R⁴ für CH₂Ph steht, wobei Ph gegebenenfalls durch bis zu zwei Gruppen ausgewählt aus CF₃, OCF₃ und F substituiert ist,
R⁵ für H steht oder
R⁴ und R⁵ zusammen mit dem Stickstoff, an den sie gebunden sind, ein Paar kondensierter Ringe ausgewählt aus der Gruppe bestehend aus 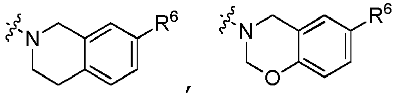 und 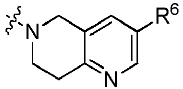 bilden, R⁶ für CF₃ oder OCF₃ steht,
Z¹ für CH₂ oder C=O steht,
Z² für CH₂ steht oder Z² für C=O stehen kann, mit der Maßgabe, dass Z¹ und Z² nicht beide gleichzeitig für C=O stehen,
und pharmazeutisch unbedenkliche Salze davon.

2. Verbindung nach Anspruch 1, wobei:
R¹ für C₍₁₋₄₎-Alkyl-OCH₃, Cyclohexyl, 1-Methoxycyclohex-2-yl, Tetrahydropyran-4-yl oder 3-C₍₁₋₄₎-Alkoxytetrahydropyran-4-yl steht,
R³ für 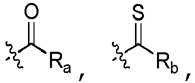 -SO₂-R_{c}, 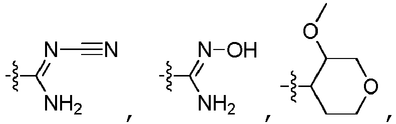 H, -CN, C(₁₋₄₎-Alkyl, C(₁₋₄)-Alkyl-NA¹A², C(₁₋₃)-Alkyl-C(O)N-(C(₁₋₂)-alkyl)₂, C(₃₋₆)-Cycloalkyl, Oxetan-3-yl, -(CH₂)ₙPh, -C(₁₋₄)-Alkyl-CO₂-C(₁₋₄)-alkyl, 4, 5-Dihydrothiazolyl, 4,5-Dihydrooxazolyl, Thiazolyl, Oxazolyl, Pyrimidinyl, Pyridyl, Pyrazyl, Furyl oder 3-Methyl-1,2,4-oxadiazol-5-yl steht,
Rₐ für H, NA¹A², NHCH₂CH₂NA₁A₂, C₍₁₋₄)-Alkyl-NA¹A², OC(₁₋₄)-Alkyl-NA¹A², C(₁₋₆)-Alkyl, O-C(₁₋₆)-Alkyl, -CN, -CH₂CH₂Ph, -CH₂OPh, -CH₂OC (O-C₍₁₋₄₎-Alkyl, -CH₂O-C₍₁₋₄₎-Alkyl, -CH₂NHBoc, -OCH₂CH=CH₂, -OCH₂CH₂CF₃, -OCH₂CH₂C (CH₃)₂OH, -OCH₂CH₂O-C₍₁₋₄₎-Alkyl, -OCH₂CH₂CN, -OPh, 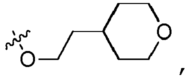 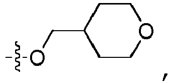 -O-C₍₁₋₄₎ -Alkyl-Ph-Rₐₐ, 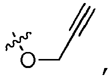 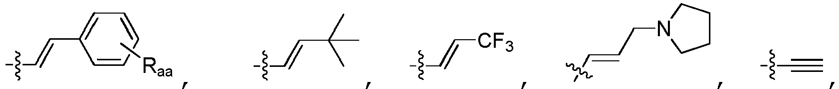 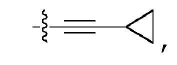 Phenyl, Oxazol-2-yl, Oxazol-4-yl, Isoxazol-5-yl oder Thiazol-2-yl steht,
Rₐₐ für H, O-C₍₁₋₄₎-Alkyl, -CO₂H, Cl, Br, F oder -CN steht,
R_{c} für NH₂, NHCH₂Ph, CH₂Ph, CH₂CH₂Ph oder C₍₁₋₄₎-Alkyl steht,
R⁴ für CH₂Ph steht, wobei Ph gegebenenfalls durch bis zu zwei Gruppen ausgewählt aus CF₃, OCF₃ und F substituiert ist,
R⁵ für H steht oder
R⁴ und R⁵ zusammen mit dem Stickstoff, an den sie gebunden sind, ein Paar kondensierter Ringe ausgewählt aus der Gruppe bestehend aus 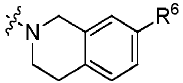 und 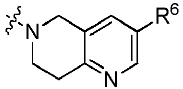 bilden,
und pharmazeutisch unbedenkliche Salze davon.

3. Verbindung nach Anspruch 2, wobei:
R¹ für C₍₁₋₄₎-Alkyl-OCH₃, 3-C₍₁₋₄₎-Alkoxytetrahydropyran-4-yl oder Tetrahydropyran-4-yl steht,
R³ für 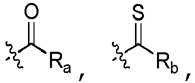 -SO₂-R_{c}, 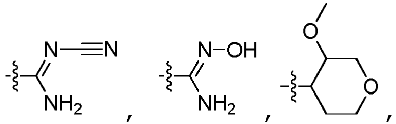 H, -CN, C₍₁₋₄₎ -Alkyl, -C₍₁₋₃₎-Alkyl-C(O) N-C₍₁₋₂₎-alkyl)₂, C₍₃₋₆₎-Cycloalkyl, Oxetan-3-yl, -(CH₂)ₙPh, -C₍₁₋₄₎-Alkyl-CO₂-C₍₁₋₄₎-alkyl, 4, 5-Dihydrothiazolyl, 4,5-dihydrooxazolyl, Thiazolyl, Oxazolyl, Pyrimidinyl, Pyridyl, Pyrazyl, Furyl oder 3-Methyl-1,2,4-oxadiazol-5-yl steht,
Rₐ für H, NA¹A², NHCH₂CH₂NA₁A₂, C₍₁₋₄₎-Alkyl-NA¹A², O-C₍₁₋₄₎-Alkyl-NA¹A², C₍₁₋₆₎-Alkyl, OC₍₁₋₆₎-Alkyl, -CN, -CH₂CH₂Ph, -CH₂OPh, -CH₂OC(O)-C₍₁₋₄₎-Alkyl, -CH₂O-C₍₁₋₄₎-Alkyl, -CH₂NHBoc, -OCH₂CH=CH₂, -OCH₂CH₂CF₃, -OCH₂CH₂C(CH₃)₂OH, -OCH₂CH₂OCH₃, -OCH₂CH₂CN, -OPh, 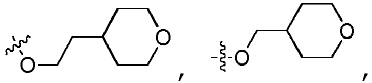 -O-C₍₁₋₄₎-Alkyl-Ph-Rₐₐ, 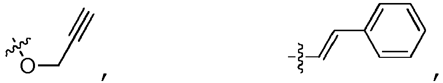 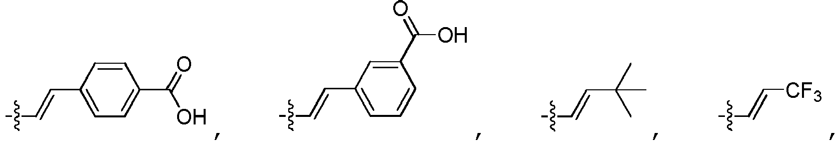 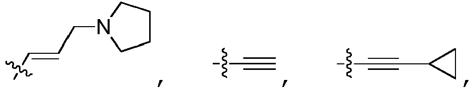 Phenyl, Oxazol-2-yl, Oxazol-4-yl, Isoxazol-5-yl oder Thiazol-2-yl steht,
Rₐₐ für H, O-C₍₁₋₄₎-Alkyl oder -CN steht,
R_{b} für 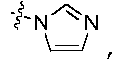 NH₂, NHCH₃, NHCH₂Ph oder NHCH₂CH(CH₃)₂ steht,
R_{c} für NH₂, NHCH₂Ph, CH₂Ph, CH₂CH₂Ph oder CH₃ steht,
A¹ für H, C₍₁₋₆₎-Alkyl, Ph oder C(O)CH₃, CH₂Ph, C₍₁₋₄₎-Alkyl-O-C₍₁₋₄₎-alkyl steht,
A² für H oder C₍₁₋₆₎-Alkyl steht oder
A¹ und A² zusammen mit dem Stickstoff, an den sie gebunden sind, einen Ring ausgewählt aus der Gruppe bestehend aus 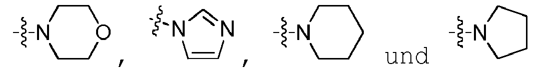 bilden können,
und pharmazeutisch unbedenkliche Salze davon.

4. Verbindung nach Anspruch 3, wobei:
R¹ für CH₂CH₂OCH₃, 3-C₍₁₋₄₎-Alkoxytetrahydropyran-4-yl oder Tetrahydropyran-4-yl steht,
R³ für 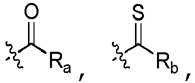 -SO₂-R_{c,} 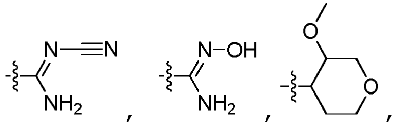 H, -CN, C₍₁₋₄₎-Alkyl, -C₍₁₋₃₎-Alkyl-C(O)N-(C₍₁₋₂₎-alkyl)₂, Cyclopropyl, Cyclobutanyl, Oxetan-3-yl, -(CH₂)ₙPh, -C₍₁₋₄₎-Alkyl-CO₂-C₍₁₋₄₎-alkyl, 4,5-dihydrothiazol-2-yl, 4,5-Dihydrooxazol-2-yl, Thiazol-2-yl, Pyrimidin-2-yl oder 3-Methyl-1,2,4-oxadiazol-5-yl steht,
Rₐ für H, NA¹A², NHCH₂CH₂NA₁A₂, C₍₁₋₄₎-Alkyl-NA¹A², O-C₍₁₋₄₎ -Alkyl-NA¹A², C₍₁₋₆₎-Alkyl, O-C₍₁₋₆₎-Alkyl, -CN, -CH₂CH₂Ph, -CH₂OPh, -CH₂NHBoc, -OCH₂Ph, -CH₂OC(O)-C₍₁₋₄₎-Alkyl, -CH₂O-C₍₁₋₄₎-Alkyl, -OCH₂Ph-CN, -OCH₂Ph-OCH₃, -OCH₂CH=CH₂, -OCH₂CH₂CF₃, -OCH₂CH₂C(CH₃)₂OH, -OCH₂CH₂OCH₃, -OCH₂CH₂CN, -OPh, 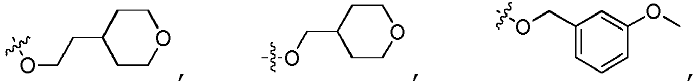 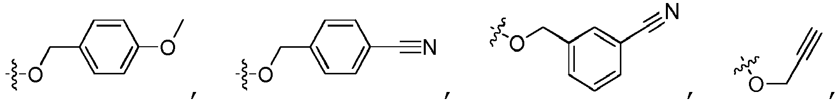 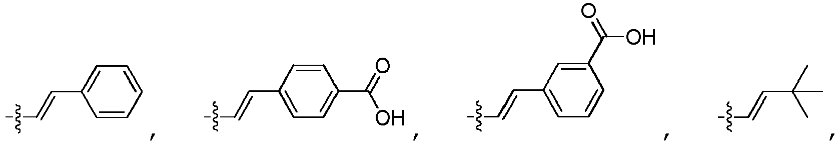 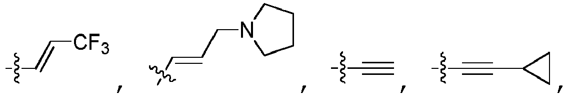 Phenyl, Oxazol-2-yl, Oxazol-4-yl, Isoxazol-5-yl oder Thiazol-2-yl steht,
A¹ für H, C₍₁₋₆₎-Alkyl, Ph oder C(O)CH₃, CH₂Ph, C₍₁₋₄₎-Alkyl-O-C₍₁₋₄₎-alkyl steht,
A² für H oder C₍₁₋₆₎-Alkyl steht oder
A¹ und A² zusammen mit dem Stickstoff, an den sie gebunden sind, einen Ring ausgewählt aus der Gruppe bestehend aus 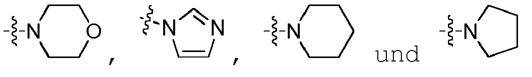 bilden können,
und pharmazeutisch unbedenkliche Salze davon.

5. Verbindung nach Anspruch 4, wobei:
R¹ für CH₂CH₂OCH₃, 3-C₍₁₋₂₎-Alkoxytetrahydropyran-4-yl oder Tetrahydropyran-4-yl steht,
R³ für 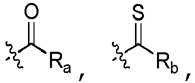 -SO₂-R_{c,} 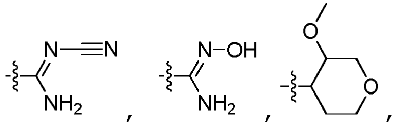 H, -CN, C₍₁₋₃₎-Alkyl, -CH₂C(O)N(CH₃)₂, Cyclopropyl, Oxetan-3-yl, -(CH₂)ₙPh, -CH₂CO₂CH₃, 4,5-Dihydrothiazol-2-yl, 4,5-Dihydrooxazol-2-yl, Thiazol-2-yl, Pyrimidin-2-yl oder 3-Methyl-1,2,4-oxadiazol-5-yl steht,
Rₐ für H, NH₂, NHCH₃, N(CH₃)₂, NHCH₂CH(CH₃)₂, NHCH(CH₃)₂, NHCH₂C(CH₃)₃, NHCH₂CH₂OCH₃, NHCH₂CH₂-Morpholinyl, NHPh, NHCH₂Ph, CH₂CH₂N(CH₃)₂, CH₂CH₂CH₂N(CH₃)₂, -CN, -CH₃, -CH₂CH₂Ph, -CH₂OPh, -CH₂OC(O)CH₃, -CH₂OCH₃, -CH₂OC(CH₃), -CH₂NHC(O)CH₃, -CH₂N(CH₃)₂, -CH₂NHBoc, -OCH₃, -OCH₂C(CH₃)₃, -OCH₂CH(CH₃)₂, -OCH₂CH₂CH₃, -OCH₂Ph, -OCH₂Ph-CN, -OCH₂Ph-OCH₃, -OCH₂CH=CH₂, -OCH₂CH₂CF₃, -OCH₂CH₂C(CH₃)₂OH, -OCH₂CH₂OCH₃, -OCH₂CH₂CN, -OCH₂CH₂N-(C₍₁₋₂₎Alkyl)₂, -OCH₂CH₂NHC(O)CH₃, -OPh, 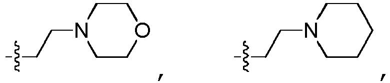 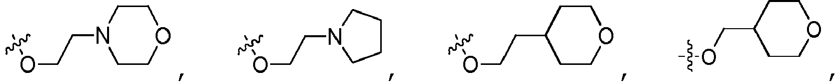 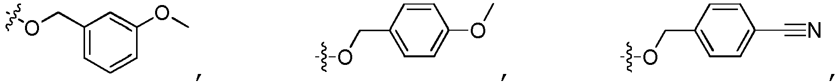 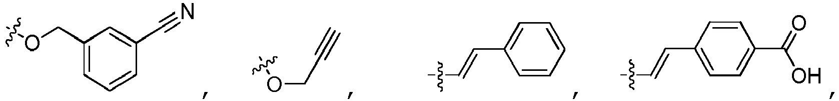 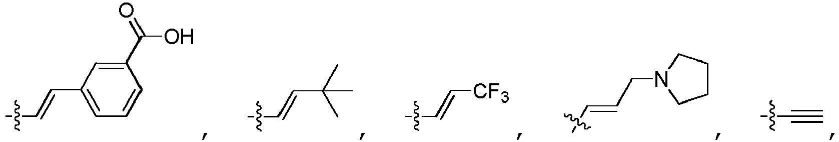 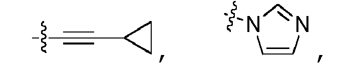 Phenyl, Oxazol-2-yl, Oxazol-4-yl, Isoxazol-5-yl, Thiazol-2-yl, 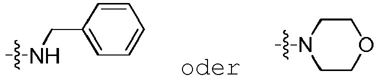 steht,
R⁴ für 1,3-Bis(trifluormethyl)benz-5-yl, 1-Fluor-3-(trifluormethyl)benz-5-yl oder 1-(Trifluoromethyl)benz-5-yl steht,
R⁵ für H steht oder
R⁴ und R⁵ zusammen mit dem Stickstoff, an den sie gebunden sind, ein Paar kondensierter Ringe ausgewählt aus der Gruppe bestehend aus 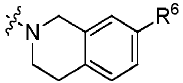 und 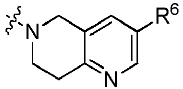 bilden,
und pharmazeutisch unbedenkliche Salze davon.

6. Verbindung nach Anspruch 1, ausgewählt aus der Gruppe bestehend aus: 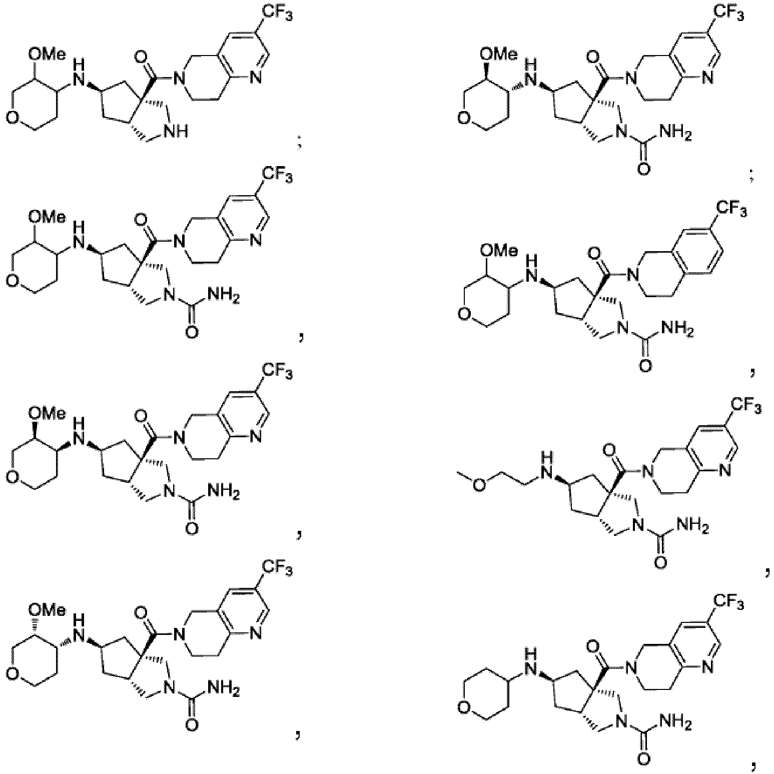 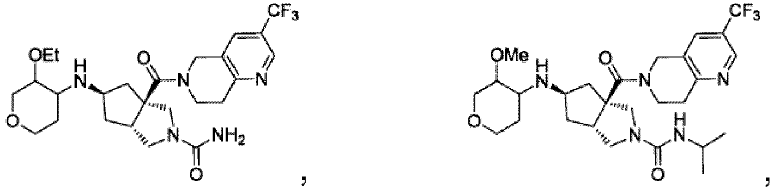 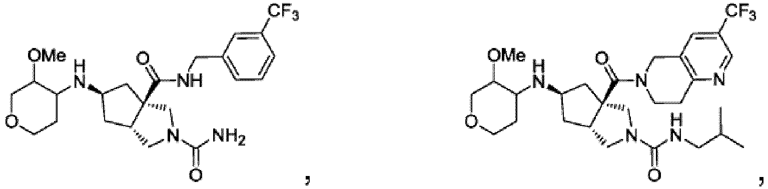 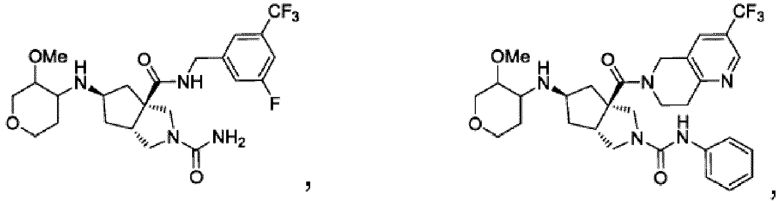 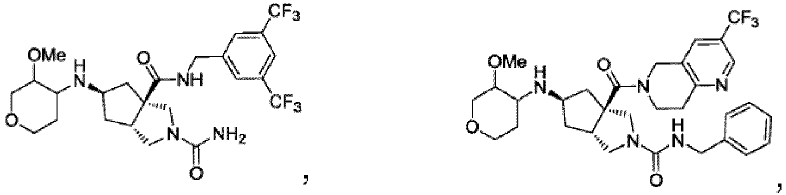 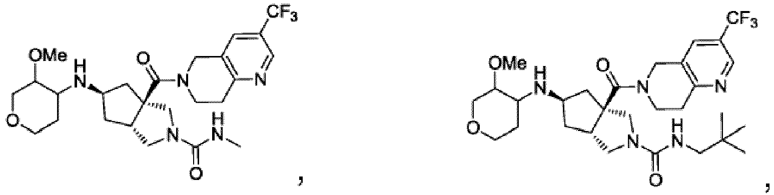 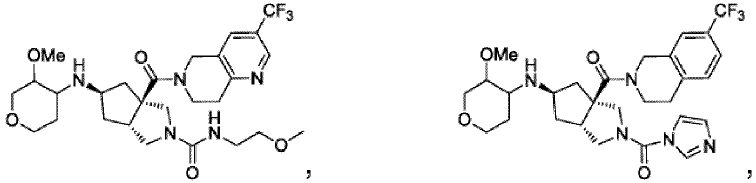 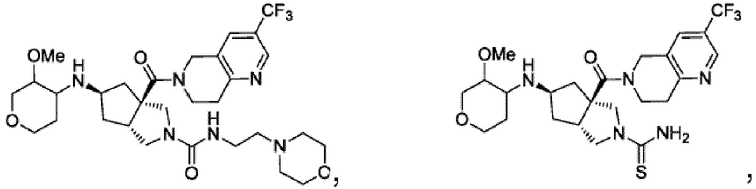 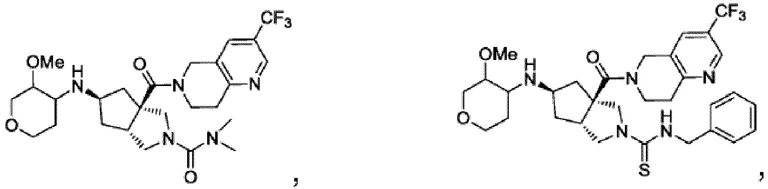 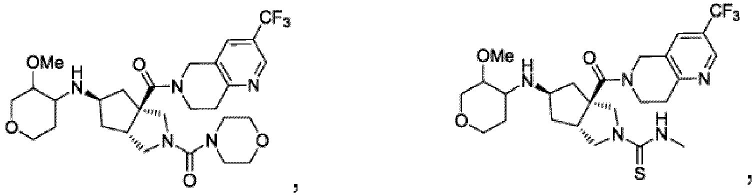 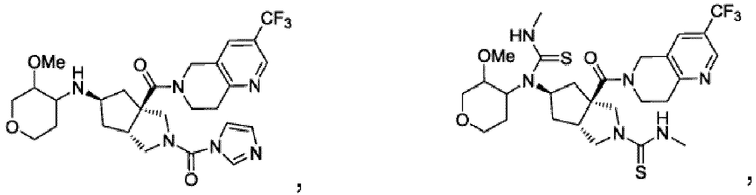 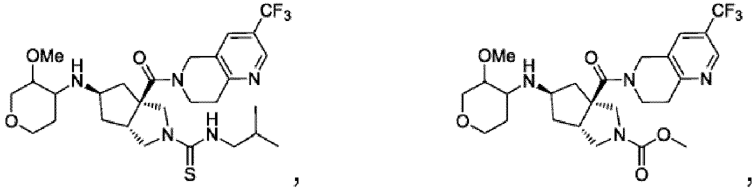 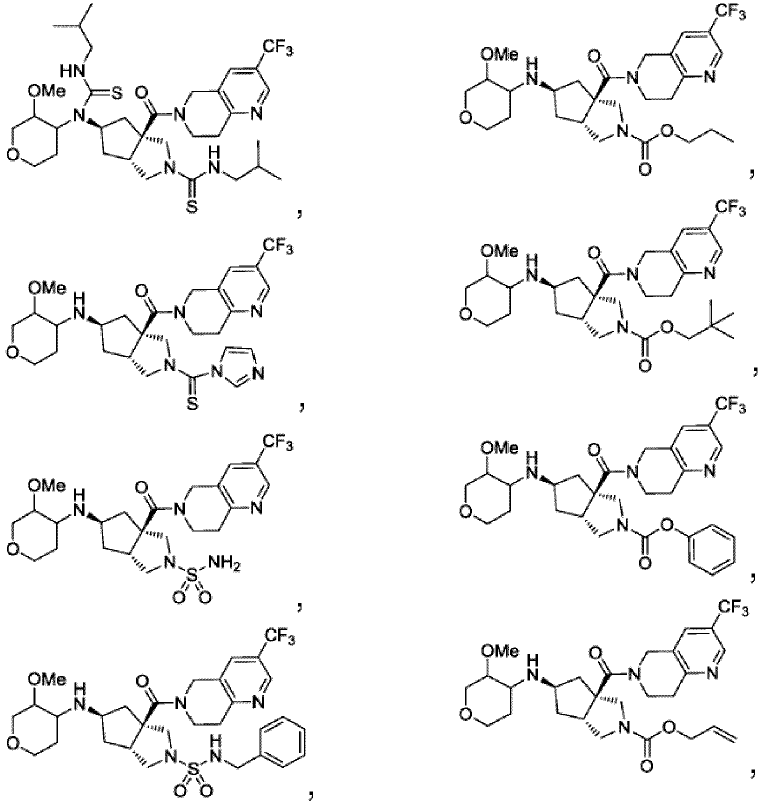 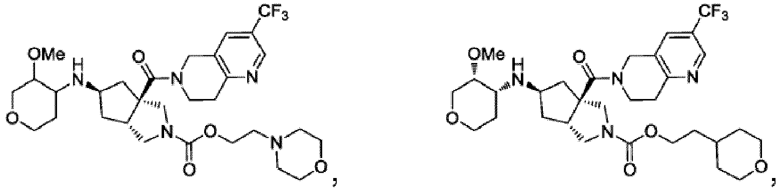 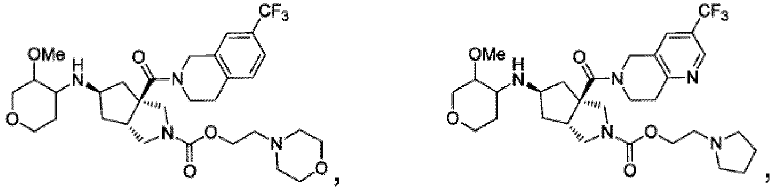 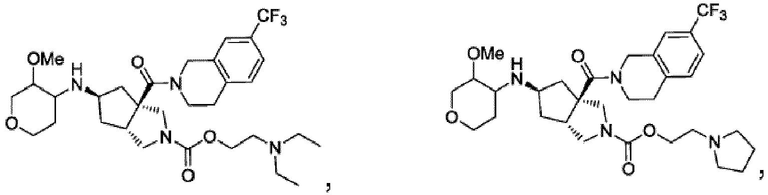 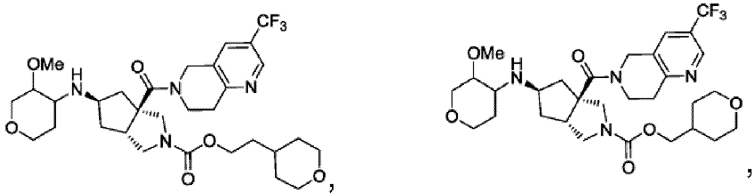 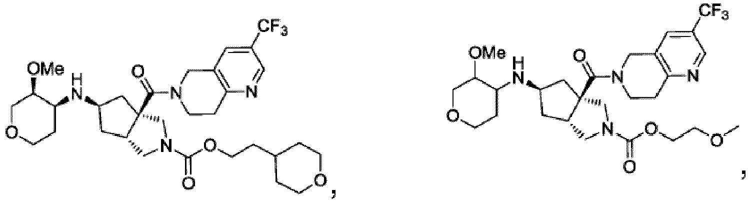 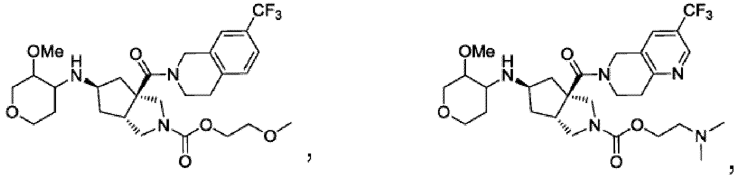 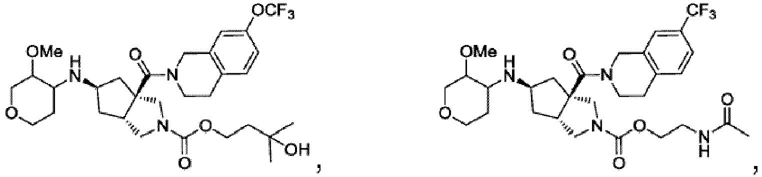 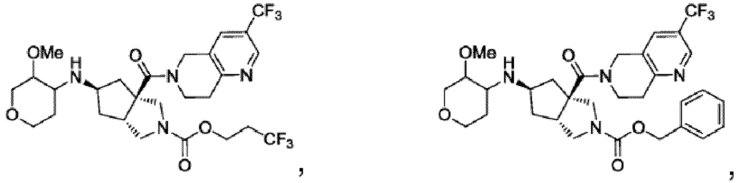 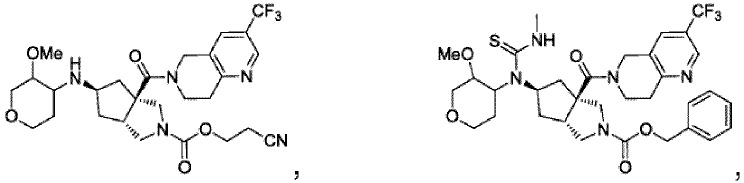 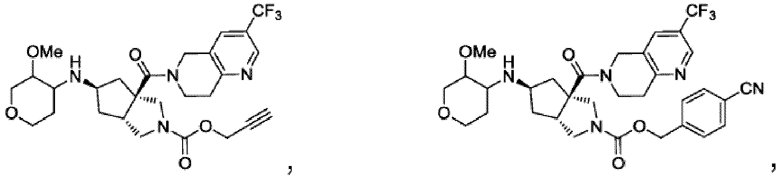 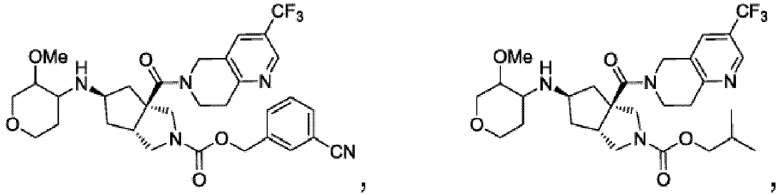 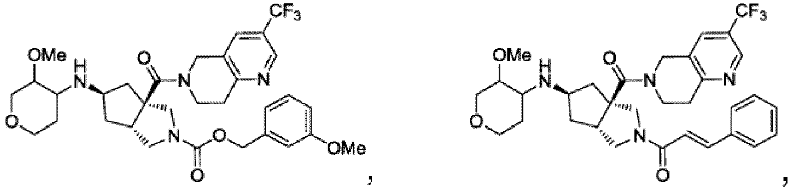 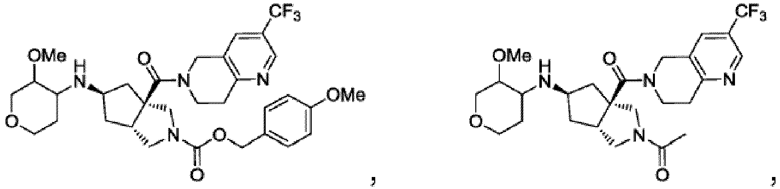 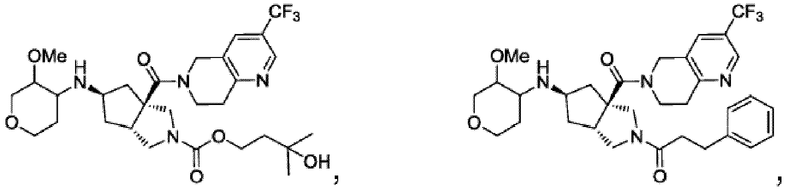 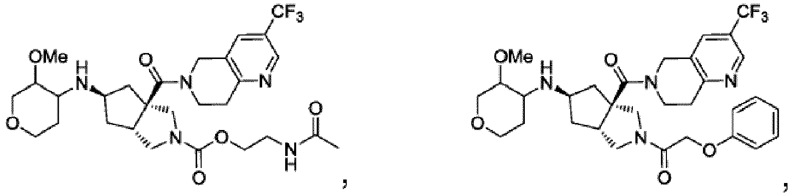 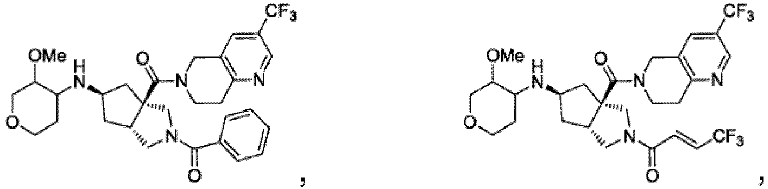 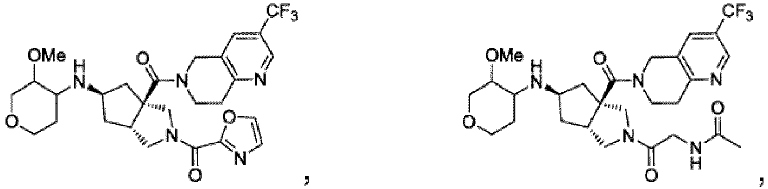 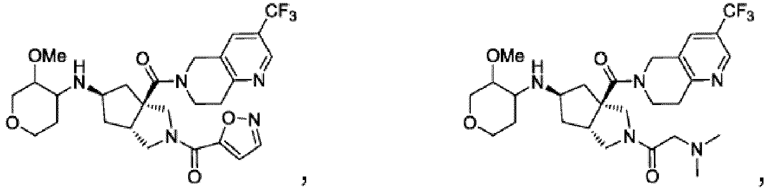 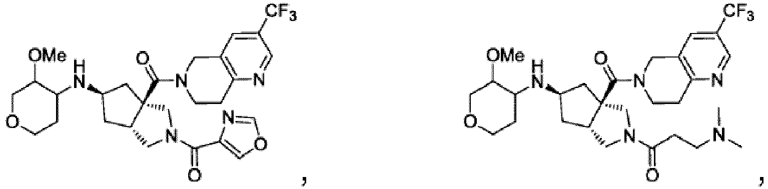 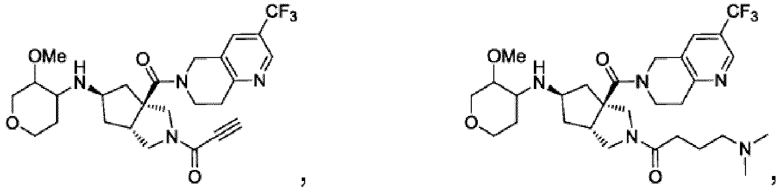 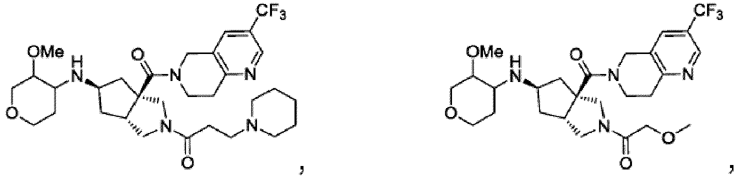 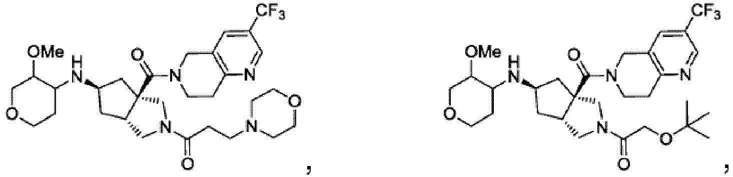 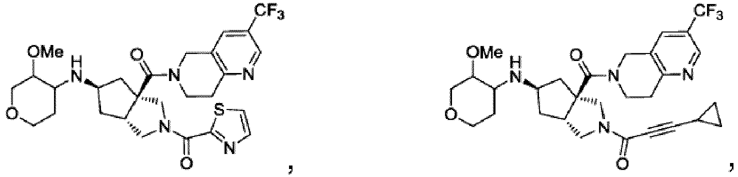 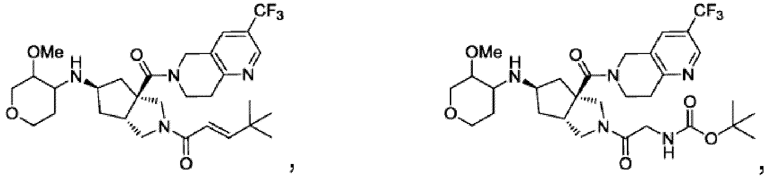 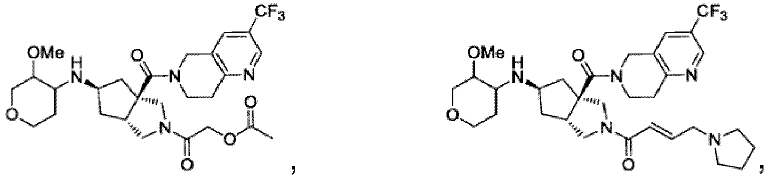 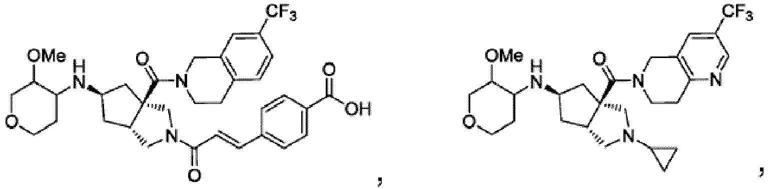 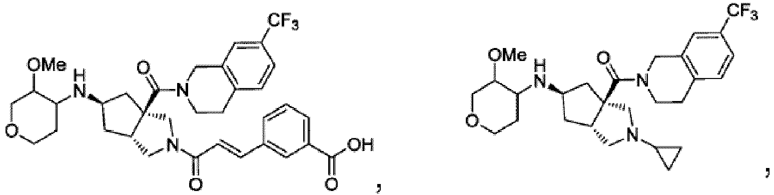 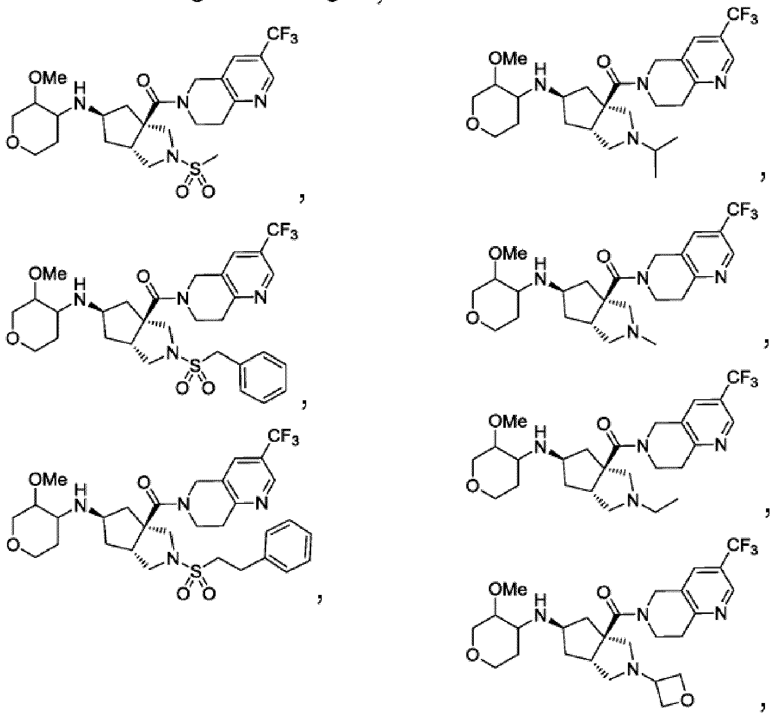 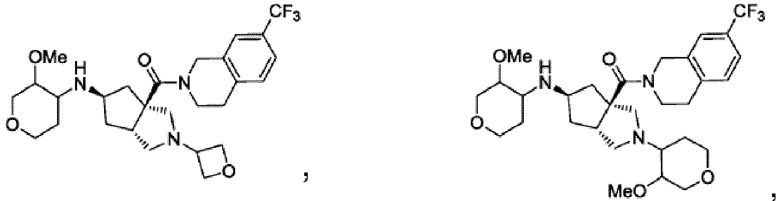 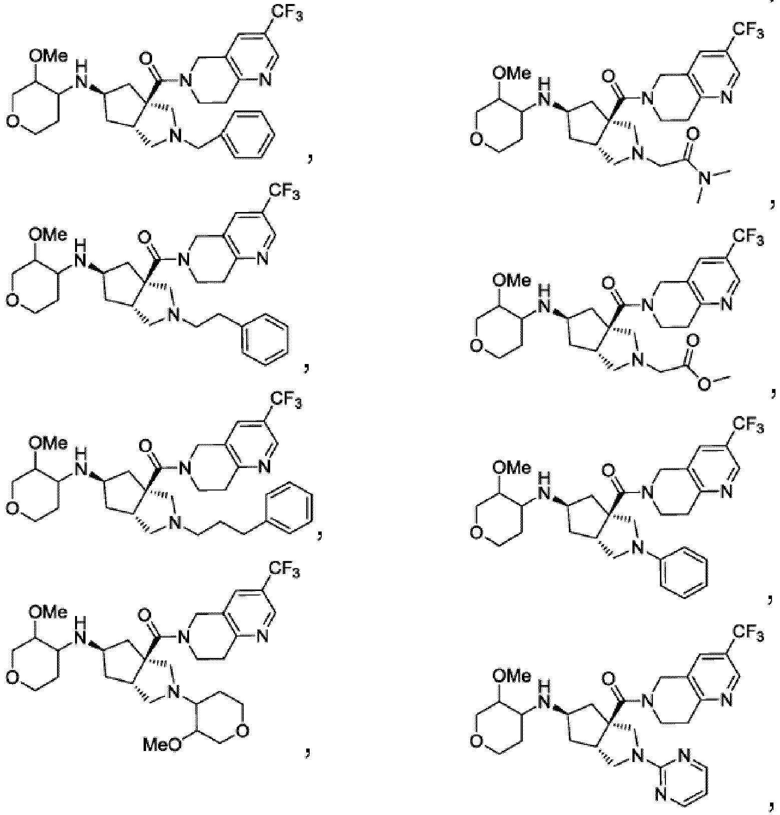 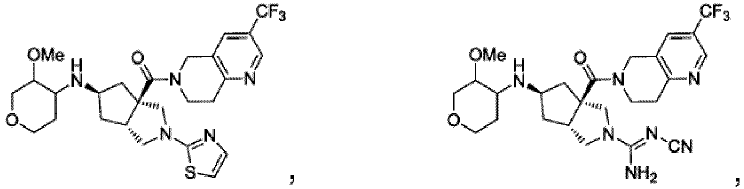 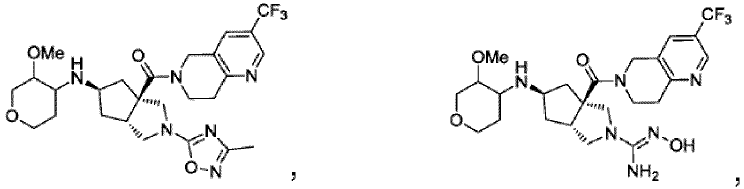 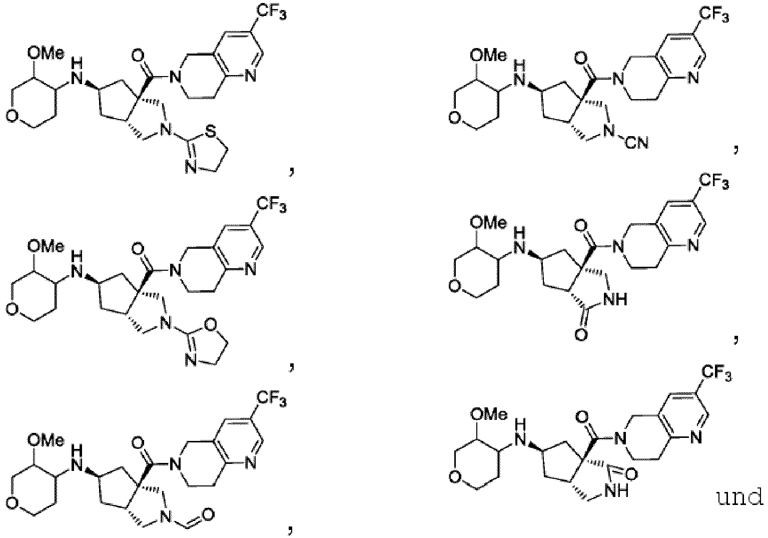 und pharmazeutisch unbedenkliche Salze davon.

7. Pharmazeutische Zusammensetzung, umfassend eine Verbindung nach einem der Ansprüche 1 bis 6 und einen pharmazeutisch unbedenklichen Träger.

8. Pharmazeutische Zusammensetzung nach Anspruch 7, wobei die pharmazeutische Zusammensetzung durch Mischen der Verbindung und des pharmazeutisch unbedenklichen Trägers hergestellt wird.

9. Verfahren zur Herstellung der pharmazeutischen Zusammensetzung nach Anspruch 7, wobei das Verfahren das Mischen der Verbindung und des pharmazeutisch unbedenklichen Trägers umfasst.

10. Verbindung nach einem der Ansprüche 1 bis 6 zur Verwendung in einem Verfahren zur Prävention, Behandlung oder Linderung eines Syndroms, einer Erkrankung oder einer Krankheit, wobei das Syndrom, die Erkrankung bzw. die Krankheit ausgewählt ist aus der Gruppe bestehend aus: chronischer obstruktiver Atemwegserkrankung (Chronic Obstructive Pulmonary Disorder, COPD), Augenerkrankungen, Uveitis, Atherosklerose, rheumatoider Arthritis, Psoriasis, Psoriasisarthritis, atopischer Dermatitis, multipler Sklerose, Morbus Crohn, Colitis ulcerosa, Nephritis, Organtransplantatabstoßung, Lungenfibrose, Niereninsuffizienz, Typ-I-Diabetes, Typ-II-Diabetes, diabetischen Komplikationen, diabetischer Nephropathie, diabetischer Retinopathie, diabetischer Retinitis, diabetischer Mikroangiopathie, Übergewicht, Obesitas, mit Obesitas assoziierter Insulinresistenz, metabolischem Syndrom, Tuberkulose, Sarkoidose, invasiven Staphylokokken, Entzündung nach Kataraktoperation, allergischer Rhinitis, allergischer Konjunktivitis, chronischer Nesselsucht, Asthma, allergischem Asthma, Parodontalkrankheiten, Parodontitis, Gingivitis, Zahnfleischerkrankung, diastolischen Kardiomyopathien, Herzinfarkt, Myokarditis, chronischer Herzinsuffizienz, Angiostenose, Restenose, Reprefusionserkrankungen, Bauchaortenaneurysma, Glomerulonephritis, festen Tumoren und Krebserkrankungen, chronicher lymphatischer Leukämie, chronischer myeloischer Leukämie, mutliplem Myelom, malignem Myelom, Hodgkin-Krankheit und Karzinomen der Blase, der Brust, des Gebärmutterhalses, des Kolons, der Lunge, der Prostata oder des Magens, und chronischen neuroinflammatorischen Erkrankungen einschließlich, jedoch nicht darauf beschränkt, Alzheimer-Krankheit, ischämischen Schlaganfall, Rückenmarksverletzung, Nervenquetschverletzung und traumatischer Hirnverletzung, wobei das Verfahren die Verabreichung einer wirksamen Menge der Verbindung an ein dessen bedürftiges Subjekt umfasst.

11. Verbindung zur Verwendung in dem Verfahren zur Prävention, Behandlung oder Linderung eines Syndroms, einer Erkrankung oder einer Krankheit nach Anspruch 10, wobei das Syndrom, die Erkrankung bzw. die Krankheit ausgewählt ist aus der Gruppe bestehend aus: Typ-I-Diabetes, Typ-II-Diabetes, diabetischen Komplikationen, diabetischer Nephropathie, diabetischer Retinopathie, diabetischer Retinitis, diabetischer Mikroangiopathie, Obesitas, mit Obesitas assoziierter Insulinresistenz, metabolischem Syndrom, Asthma und allergischem Asthma, wobei das Verfahren die Verabreichung einer therapeutisch wirksamen Menge der Verbindung an ein dessen bedürftiges Subjekt umfasst.

12. Verbindung zur Verwendung in dem Verfahren zur Behandlung einer Erkrankung nach Anspruch 10, ausgewählt aus der Gruppe bestehend aus Typ-II-Diabetes, Obesitas und Asthma, wobei das Verfahren die Verabreichung einer therapeutisch wirksamen Menge der Verbindung an ein dessen bedürftiges Subjekt umfasst.

## Revendications

1. Composés de formule I 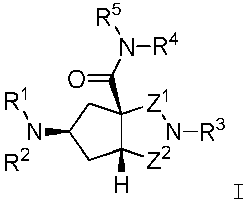 dans laquelle
R¹ est (alkyle en C₁₋₄)O (alkyle en C₁₋₄), cyclohexyle, ou tétrahydropyranyle, où lesdits cyclohexyle ou tétrahydropyranyle peuvent être facultativement substitués par un substituant choisi dans le groupe constitué de : O(alkyle en C₁₋₄), OH, CH₂CH₃, -CN, NH₂, NH(CH₃), N(CH₃)₂, ou OCF₃ ;
R² est H, C(S)NHCH₂CH(CH₃)₂, ou C(S)NHCH₃ ;
R³ est 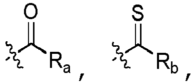 -SO₂-R_{c,} 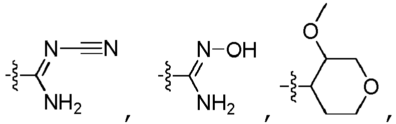 H, -CN, (alkyle en C₁₋₄), (alkyle en C₁₋₄)NA¹A², (alkyle en C₁₋₃₎C(O)NA¹A², cycloalkyle en C₃₋₆, oxétan-3-yle, -(CH₂)ₙPh-Rₐₐ, -(alkyle en C₁₋₄)CO₂(alkyle en C₁₋₄), 4,5-dihydro-thiazolyle, 4,5-dihydro-oxazolyle, thiazolyle, oxazolyle, pyrimidinyle, pyridyle, pyrazyle, furyle, ou 3-méthyl-1,2,4-oxadiazol-5-yle ; où lesdits 4,5-dihydro-thiazolyle, 4,5-dihydro-oxazolyle, thiazolyle, oxazolyle, pyrimidinyle, pyridyle, pyrazyle et furyle peuvent être facultativement substitués par jusqu'à deux substituants indépendamment choisis parmi Rₐₐ
n est 0, 1, 2, ou 3 ;
Rₐ est H, NA¹A², NHCH₂CH₂NA₁A₂, (alkyle en C₁₋₄)NA¹A², O(alkyle en C₁₋₄)NA¹A², (alkyle en C₁₋₆), O(alkyle en C₁₋₆), -CN, -CH₂CH₂Ph, -CH₂OPh, -CH₂OC(O)(alkyle en C₁₋₄), -CH₂O(alkyle en C₁₋₄)_{,} -CH₂NHBoc, -OCH₂CH=CH₂, -OCH₂CH₂CF₃, -OCH₂CH₂C(CH₃)₂OH, -OCH₂CH₂O (alkyle en C₁₋₄), -OCH₂CH₂CN, -OPh-Rₐₐ, 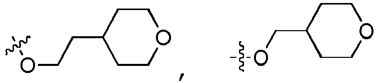 , -O(alkyle en C₁₋₄)-Ph-Rₐₐ, 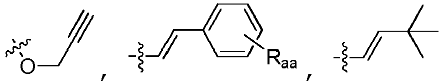 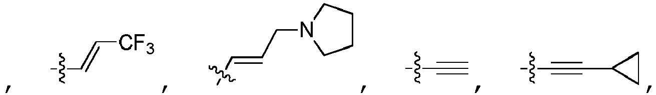 phényl-Rₐₐ, oxazol-2-yle, oxazol-4-yle, isoxazol-5-yle, ou thiazol-2-yle ;
Rₐₐ est H, 0 (alkyle en C₁₋₄), OCF₃, -CO₂H, Cl, Br, F, ou - CN ;
R_{b} est NA¹A² _{;}
R_{c} est NA¹A², CH₂Ph, CH₂CH₂Ph, ou (alkyle en C₁₋₄) ;
A¹ est H, (alkyle en C₁₋₆), Ph-Rₐₐ, C(O)CH₃, CH₂Ph-Rₐₐ, ou (alkyle en C₁₋₄)O(alkyle en C₁₋₄) ;
A² est H, alkyle en C₁₋₆ ; ou
A¹ et A² peuvent former conjointement avec l'atome d'azote auquel ils sont liés un cycle choisi dans le groupe constitué de : 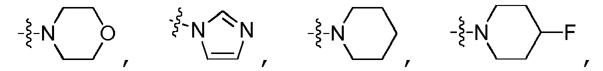 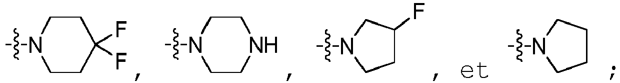
R⁴ est CH₂Ph, où ledit Ph est facultativement substitué par jusqu'à deux groupes choisis parmi CF₃, OCF₃, et F ;
R⁵ est H ; ou
R⁴ et R⁵ forment conjointement avec leur azote lié une paire de cycles condensés choisis dans le groupe constitué de : 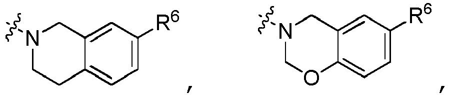 et 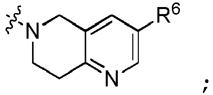 R⁶ est CF₃, ou OCF₃ ;
Z¹ est CH₂ ou C=O ;
Z² est CH₂ ou Z² peut être C=O à condition que Z¹ et Z² ne soient pas tous deux simultanément C=O ;
et sels pharmaceutiquement acceptables de ceux-ci.

2. Composé de la revendication 1, dans lequel :
R¹ est (alkyle en C₁₋₄)OCH₃, cyclohexyle, 1-méthoxy cyclohex-2-yle, tétrahydropyran-4-yle, ou 3-(alcoxy en C₁₋₄) tétrahydropyran-4-yle ;
R³ est 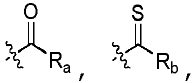 -SO₂₋R_{c}, 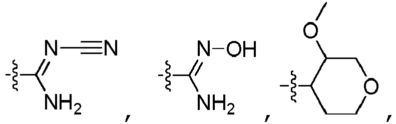 H, -CN, (alkyle en C₁₋₄), (alkyle en C₁₋₄)NA¹A², (alkyle en C₁₋₃)C(O)N(alkyle en C₁₋₂)₂, cycloalkyle en C₃₋₆, oxétan-3-yle, -(CH₂)ₙPh, -(alkyle en C₁₋₄) CO₂ (alkyle en C₁₋₄), 4,5-dihydro-thiazolyle, 4,5-dihydro-oxazolyle, thiazolyle, oxazolyle, pyrimidinyle, pyridyle, pyrazyle, furyle, ou 3-méthyl-1,2,4-oxadiazol-5-yle ; Rₐ est H, NA¹A², NHCH₂CH₂NA₁A₂, (alkyle en C₁₋₄)NA¹A², 0 (alkyle en C₁₋₄)NA¹A², (alkyle en C₁₋₆), 0 (alkyle en C₁₋₆), -CN, -CH₂CH₂Ph, -CH₂OPh, -CH₂OC(O)(alkyle en C₁₋₄), -CH₂O(alkyle en C₁₋₄), -CH₂NHBoc, -OCH₂CH=CH₂, -OCH₂CH₂CF₃, -OCH₂CH₂C(CH₃)₂OH, -OCH₂CH₂O(alkyle en C₁₋₄), -OCH₂CH₂CN, -OPh, 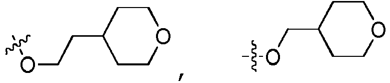 , -O(alkyle en C₁₋₄)-Ph-Rₐₐ, 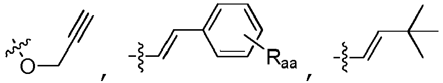 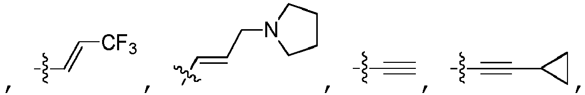 phényle, oxazol-2-yle, oxazol-4-yle, isoxazol-5-yle, ou thiazol-2-yle ;
Rₐₐ est H, 0(alkyle en C₁₋₄), -CO₂H, Cl, Br, F, ou -CN ;
R_{c} est NH₂, NHCH₂Ph, CH₂Ph, CH₂CH₂Ph, ou (alkyle en C₁₋₄) ;
R⁴ est CH₂Ph, où ledit Ph est facultativement substitué par jusqu'à deux groupes choisis parmi CF₃, OCF₃, et F ; R⁵ est H ; ou
R⁴ et R⁵ forment conjointement avec leur azote lié une paire de cycles condensés choisis dans le groupe constitué de : 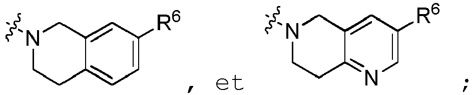
et sels pharmaceutiquement acceptables de celui-ci.

3. Composé de la revendication 2, dans lequel :
R¹ est (alkyle en C₁₋₄)OCH₃, 3-(alcoxy en C₁₋₄)tétrahydropyran-4-yle, ou tétrahydropyran-4-yle ;
R³ est 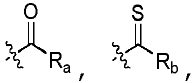 -SO₂-R_{c} 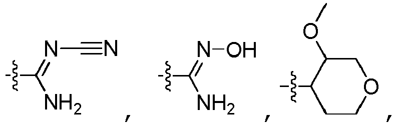 H, -CN, (alkyle en C₁₋₄), -(alkyle en C₁₋₃)C(O)N(alkyle en C₁₋₂)₂, cycloalkyle en C₃₋₆, oxétan-3-yle, -(CH₂)ₙPh, -(alkyle en C₁₋₄)CO₂(alkyle en C₁₋₄), 4,5-dihydro-thiazolyle, 4,5-dihydro-oxazolyle, thiazolyle, oxazolyle, pyrimidinyle, pyridyle, pyrazyle, furyle, ou 3-méthyl-1,2,4-oxadiazol-5-yle ;
Rₐ est H, NA¹A², NHCH₂CH₂NA₁A₂, (alkyle en C₁₋₄)NA¹A², 0 (alkyle en C₁₋₄)NA¹A², (alkyle en C₁₋₆), O(alkyle en C₁₋₆), -CN, -CH₂CH₂Ph, -CH₂OPh, -CH₂OC(O)(alkyle en C₁₋₄), -CH₂O(alkyle en C₁₋₄), -CH₂NHBoc, -OCH₂CH=CH₂, -OCH₂CH₂CF₃, -OCH₂CH₂C(CH₃)₂OH, -OCH₂CH₂OCH₃, -OCH₂CH₂CN, -OPh, 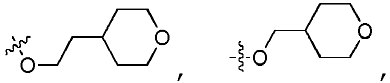 -0 (alkyle en C₁₋₄)-Ph-R_{aa,} 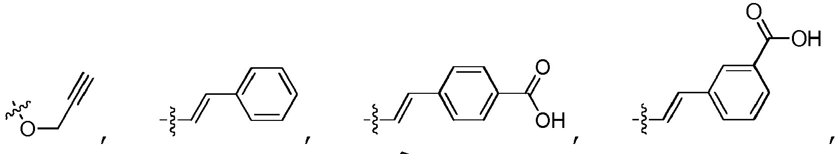 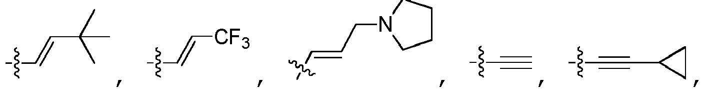 phényle, oxazol-2-yle, oxazol-4-yle, isoxazol-5-yle, ou thiazol-2-yle ;
Rₐₐ est H, O(alkyle en C₁₋₄), ou -CN ;
R_{b} est 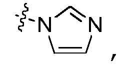 NH₂, NHCH₃, NHCH₂Ph, ou NHCH₂CH(CH₃)₂ ;
R_{c} est NH₂, NHCH₂Ph, CH₂Ph, CH₂CH₂Ph, ou CH₃ ;
A¹ est H, (alkyle en C₁₋₆) , Ph, ou C(O)CH₃, CH₂Ph, (alkyle en C₁₋₄)O (alkyle en C₁₋₄) ;
A² est H, alkyle en C₁₋₆ ; ou
A¹ et A² peuvent former conjointement avec l' atome d' azote auquel ils sont liés un cycle choisi dans le groupe constitué de : 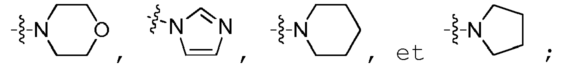 et sels pharmaceutiquement acceptables de celui-ci.

4. Composé de la revendication 3, dans lequel :
R¹ est CH₂CH₂OCH₃, 3- (alcoxy en C₁₋₄) tétrahydropyran-4-yle, ou tétrahydropyran-4-yle ;
R³ est 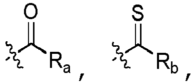 -SO₂-R_{c}, 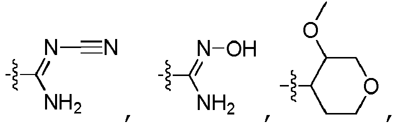 H, -CN, (alkyle en C₁₋₄), -(alkyle en C₁₋₃)C(O)N (alkyle en C₁₋₂)₂, cyclopropyle, cyclobutanyle, oxétan-3-yle, -(CH₂)ₙPh, -(alkyle en C₁₋₄)CO₂(alkyle en C₁₋₄), 4,5-dihydro-thiazol-2-yle, 4,5-dihydro-oxazol-2-yle, thiazol-2-yle, pyrimidin-2-yle, ou 3-méthyl-1,2,4-oxadiazol-5-yle ;
Rₐ est H, NA¹A², NHCH₂CH₂NA₁A₂, (alkyle en C₁₋₄)NA¹A², 0 (alkyle en C₁₋₄)NA¹A², (alkyle en C₁₋₆), 0 (alkyle en C₁₋₆), -CN, -CH₂CH₂Ph, -CH₂OPh, -CH₂NHBoc, -OCH₂Ph, -CH₂OC (0) (alkyle en C₁₋₄), -CH₂O(alkyle en C₁₋₄), -OCH₂Ph-CN, -OCH₂Ph-OCH₃, -OCH₂CH=CH₂, -OCH₂CH₂CF₃, -OCH₂CH₂C(CH₃)₂OH, -OCH₂CH₂ OCH₃, -OCH₂CH₂CN, -OPh, 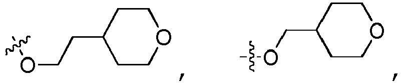 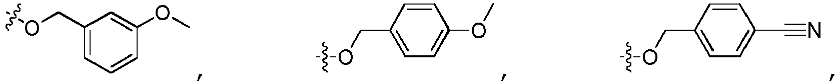 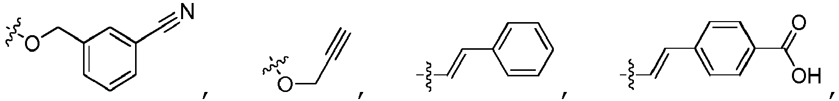 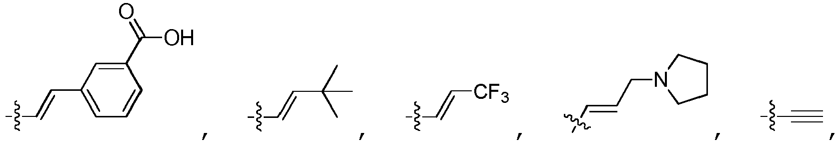 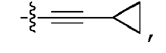 phényle, oxazol-2-yle, oxazol-4-yle, isoxazol-5-yle, ou thiazol-2-yle ;
A¹ est H, (alkyle en C₁₋₆), Ph, ou C(O)CH₃, CH₂Ph, (alkyle en C₁₋₄)O(alkyle en C₁₋₄) ;
A² est H, alkyle en C₁₋₆ ; ou
A¹ et A² peuvent former conjointement avec l'atome d'azote auquel ils sont liés un cycle choisi dans le groupe constitué de : 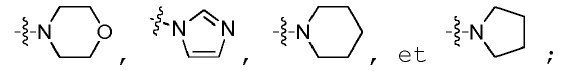
et sels pharmaceutiquement acceptables de celui-ci.

5. Composé de la revendication 4, dans lequel :
R¹ est CH₂CH₂OCH₃, 3-(alcoxy en C₁₋₂) tétrahydropyran-4-yle, ou tétrahydropyran-4-yle ;
R³ est 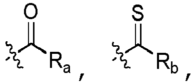 -SO₂-R_{c}, 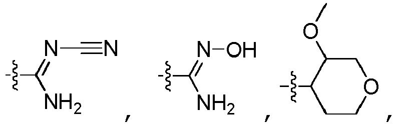 H, -CN, alkyle en C₁₋₃, -CH₂C(O)N(CH₃)₂, cyclopropyle, oxétan-3-yle, -(CH₂)ₙPh, -CH₂CO₂CH₃, 4,5-dihydro-thiazol-2-yle, 4,5-dihydro-oxazol-2-yle, thiazol-2-yle, pyrimidin-2-yle, ou 3-méthyl-1,2,4-oxadiazol-5-yle ;
Rₐ est H, NH₂, NHCH₃, N(CH₃)₂, NHCH₂CH(CH₃)₂, NHCH(CH₃)₂, NHCH₂C(CH₃)₃, NHCH₂CH₂OCH₃, NHCH₂CH₂-morpholinyle, NHPh, NHCH₂Ph, CH₂CH₂N(CH₃)₂, CH₂CH₂CH₂N(CH₃)₂, -CN, -CH₃, -CH₂CH₂Ph, -CH₂OPh, -CH₂OC(O)C H₃, -CH₂OCH₃, -CH₂OC(CH₃)₃, -CH₂NHC(O)CH₃, -CH₂N(CH₃)₂, -CH ₂NHBoc, -OCH₃, -OCH₂C(CH₃)₃, -OCH₂CH(CH₃)₂, -OCH₂CH₂CH₃, -OC H₂Ph, -OCH₂Ph-CN, -OCH₂Ph-OCH₃, -OCH₂CH=CH₂, -OCH₂CH₂CF₃, -OCH₂CH₂C(CH₃)₂OH, -OCH₂CH₂ OCH₃, -OCH₂CH₂CN, -OCH₂CH₂N(alkyle en C₁₋₂)₂, -OCH₂CH₂NHC(O)CH₃, -OPh, 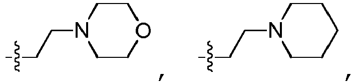 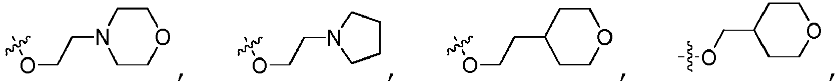 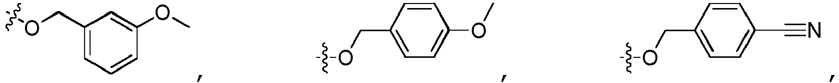 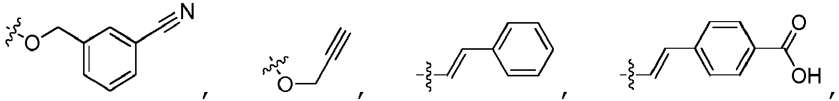 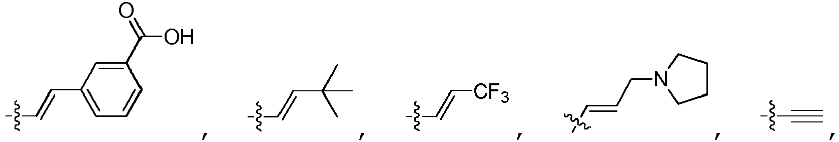 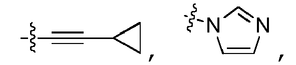 phényle, oxazol-2-yle, oxazol-4-yle, isoxazol-5-yle, thiazol-2-yle, 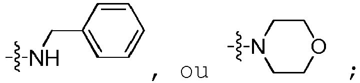
R⁴ est 1,3-bis(trifluorométhyl)benz-5-yle, 1-fluoro-3-(trifluorométhyl)benz-5-yle, ou 1-(trifluorométhyl)benz-5-yle ;
R⁵ est H ; ou
R⁴ et R⁵ forment conjointement avec leur azote lié une paire de cycles condensés choisis dans le groupe constitué de : 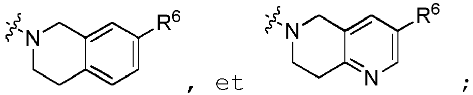
et sels pharmaceutiquement acceptables de celui-ci.

6. Composé de la revendication 1, choisi dans le groupe constitué de : 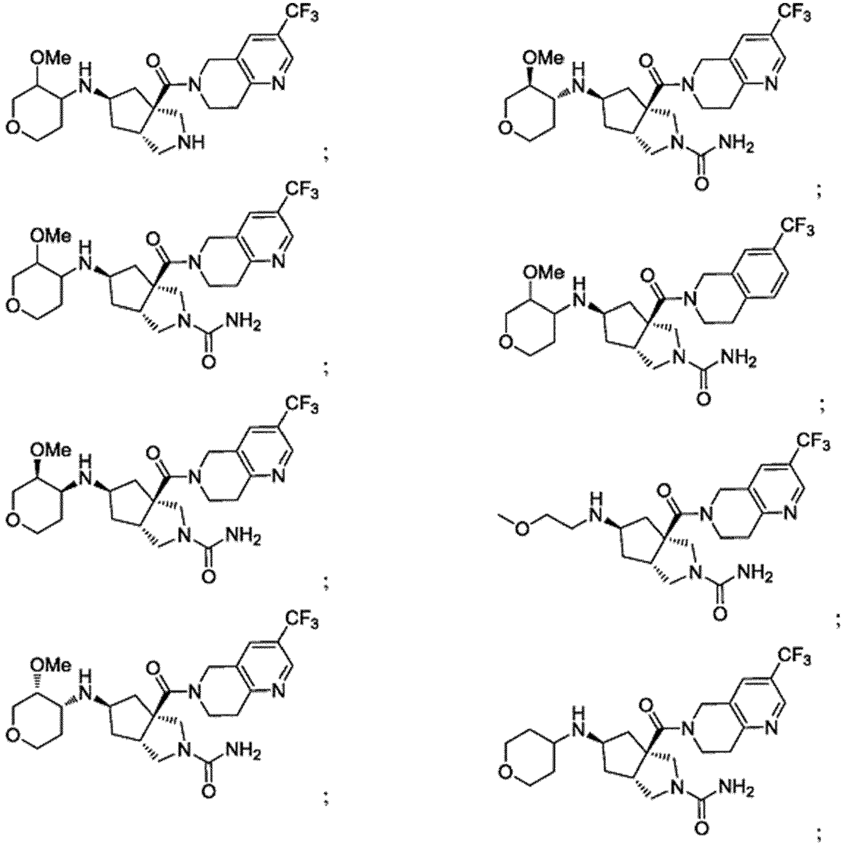 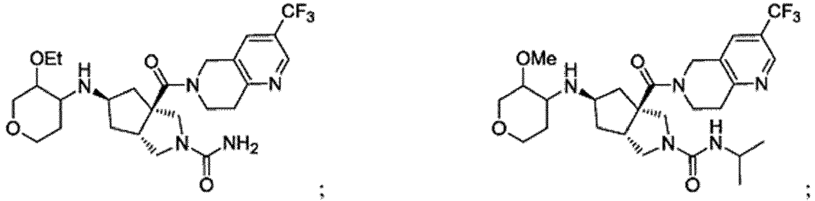 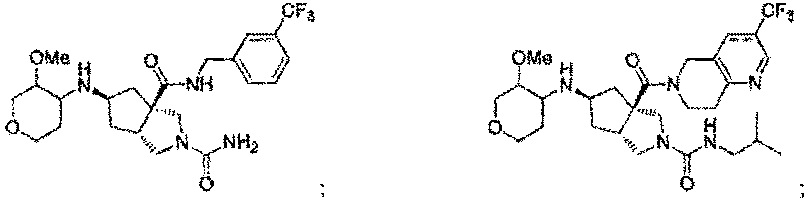 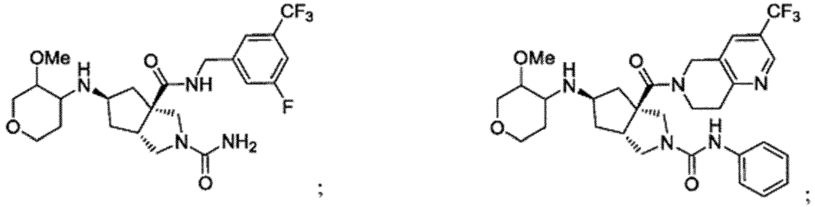 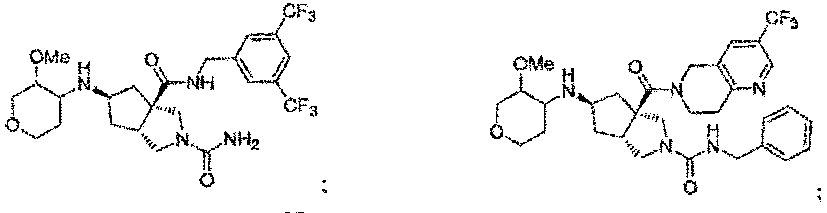 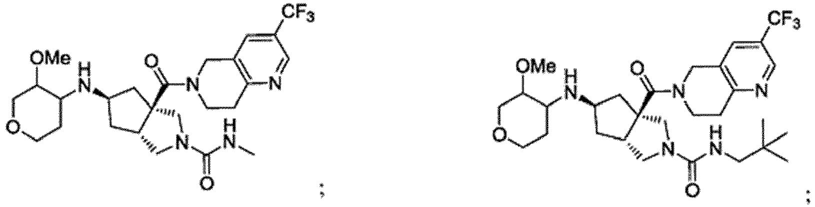 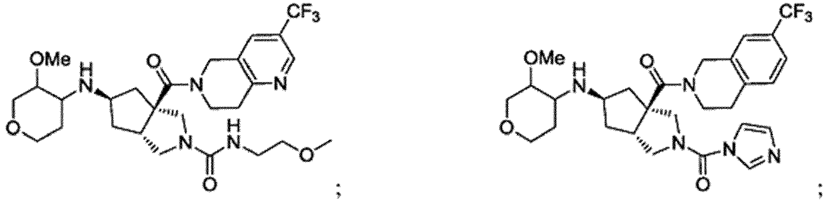 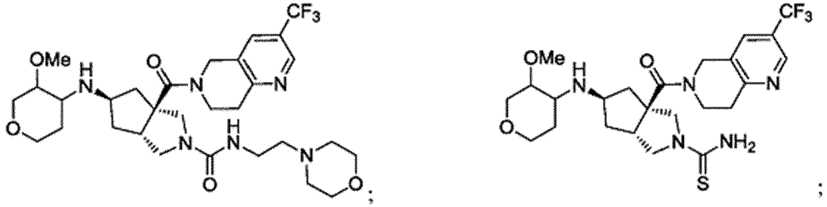 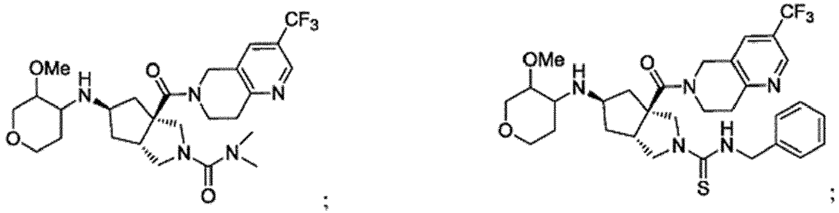 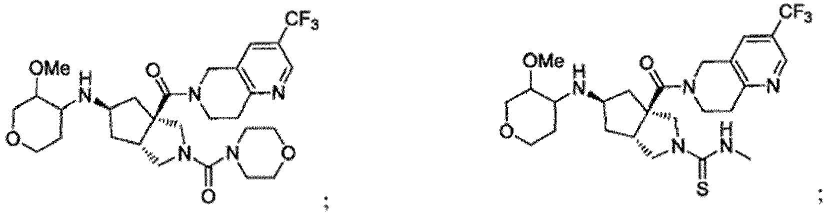 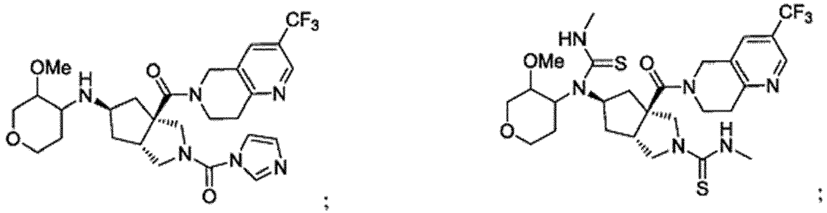 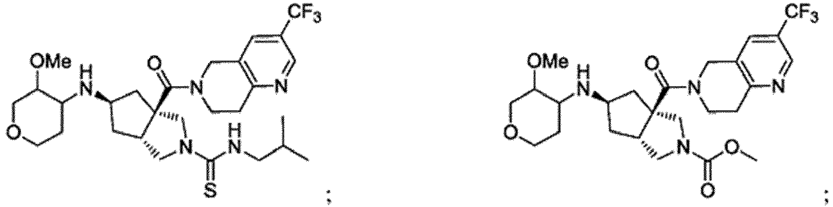 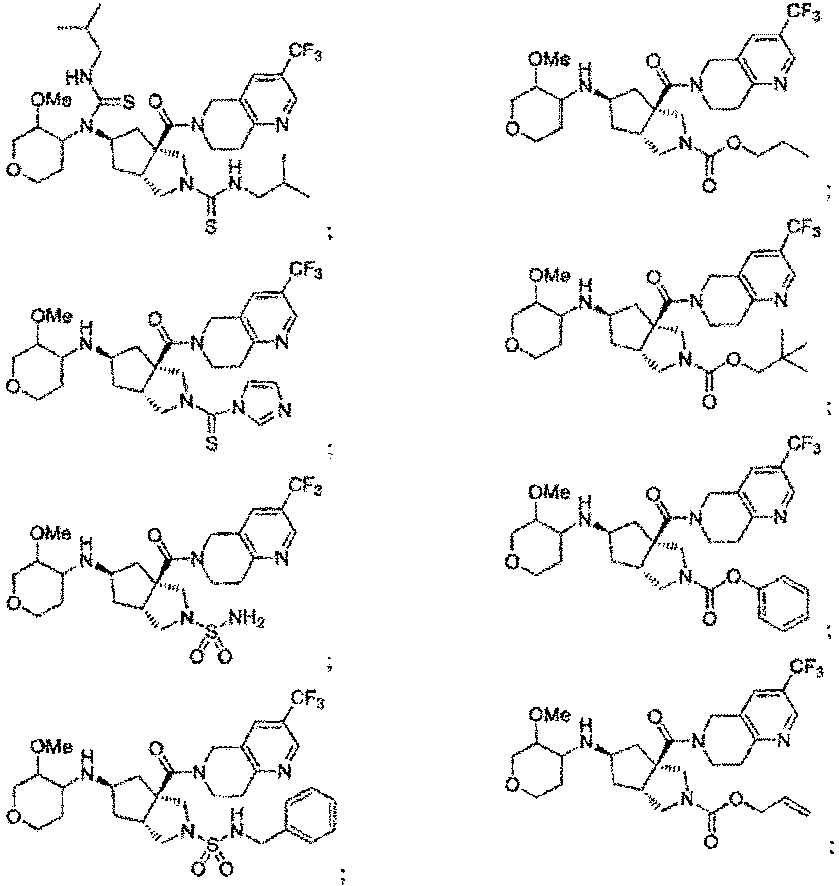 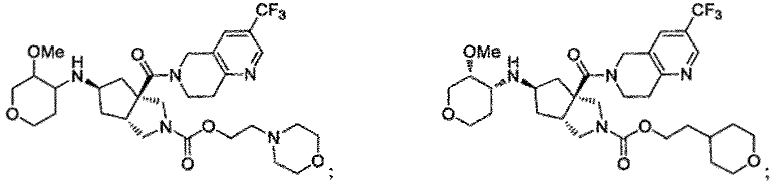 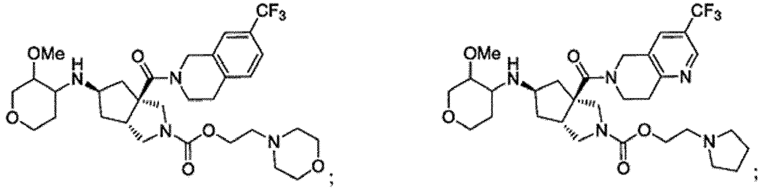 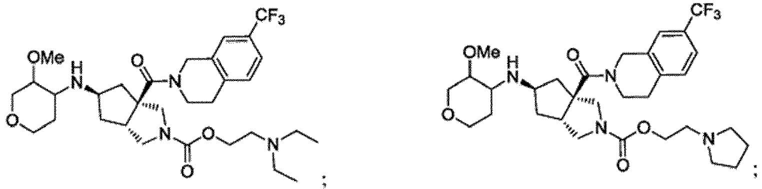 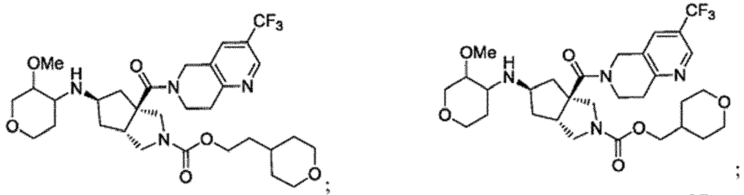 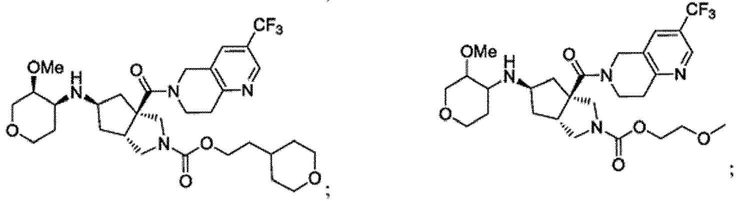 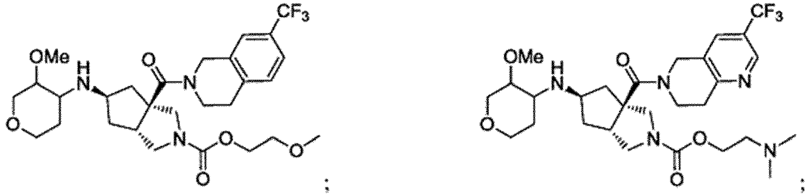 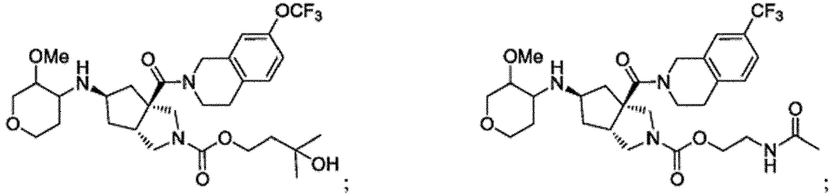 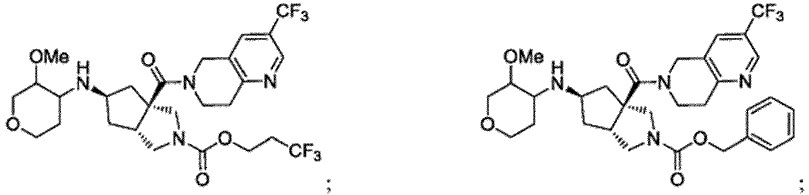 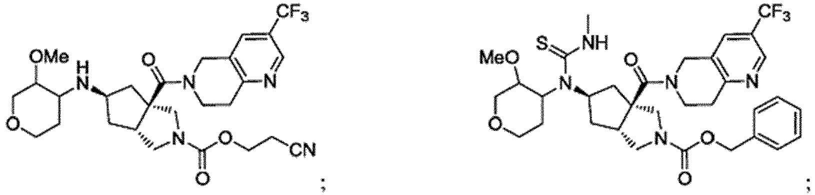 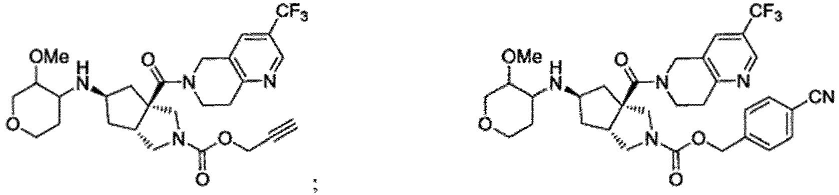 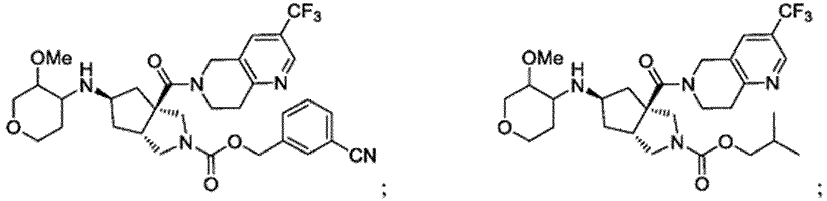 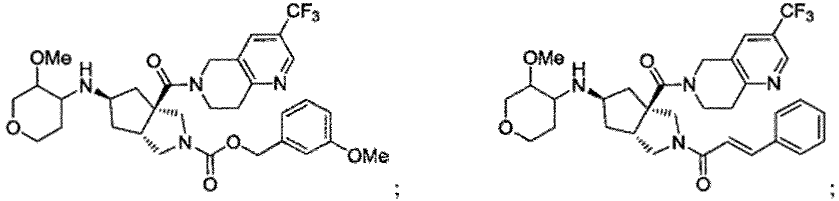 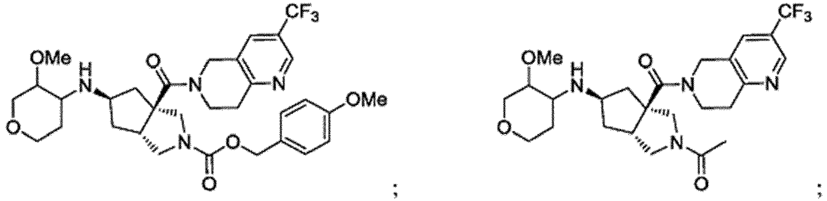 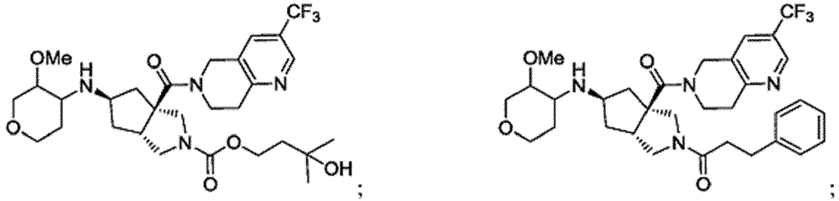 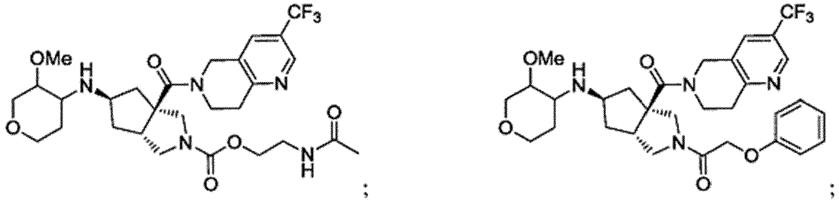 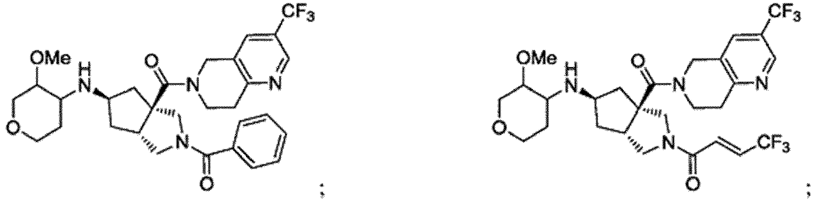 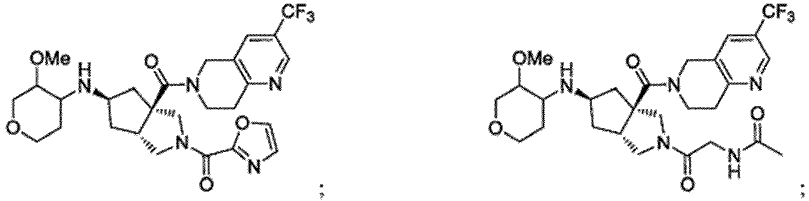 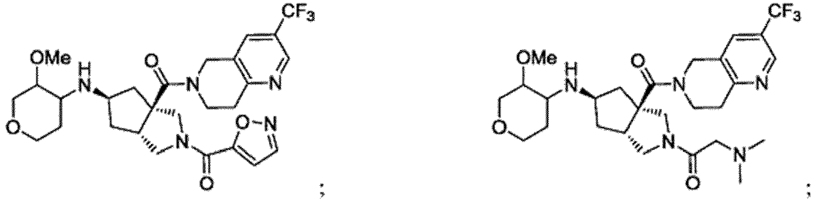 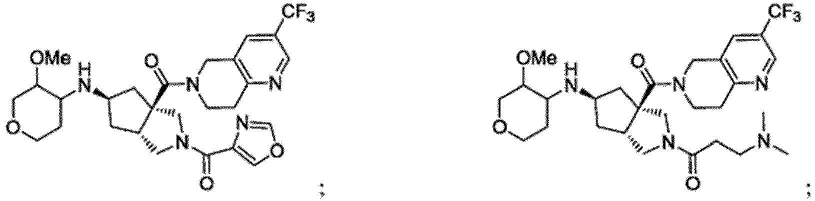 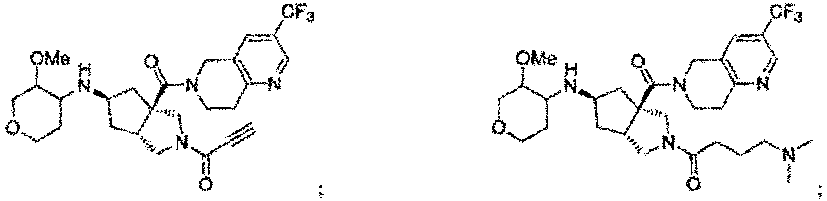 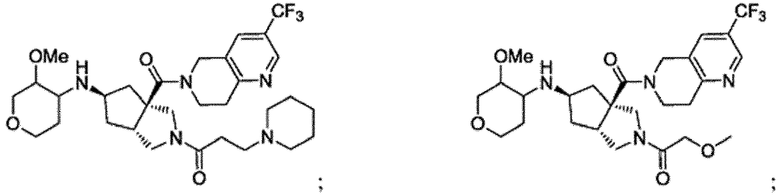 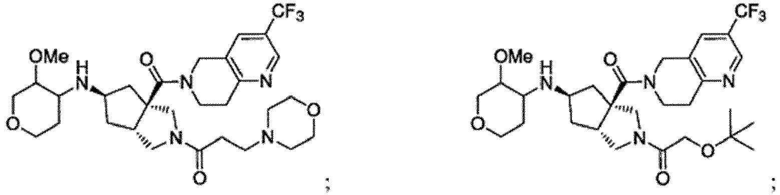 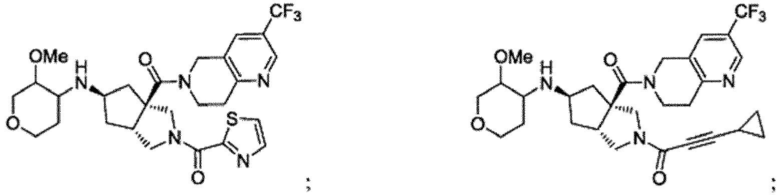 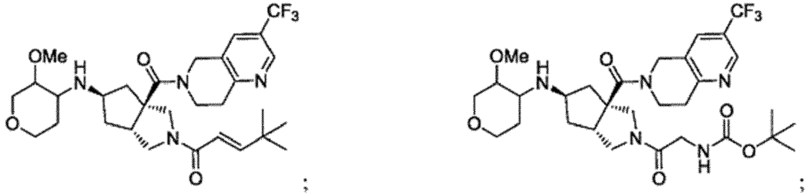 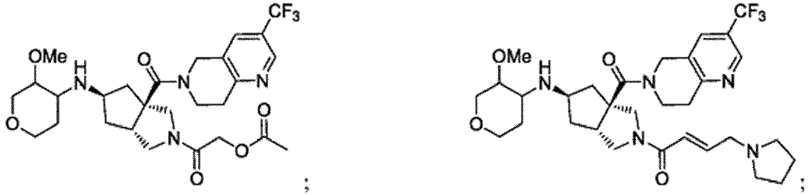 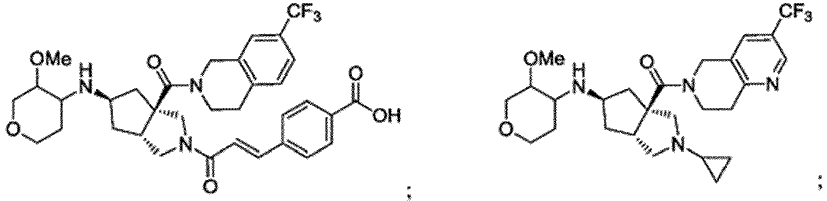 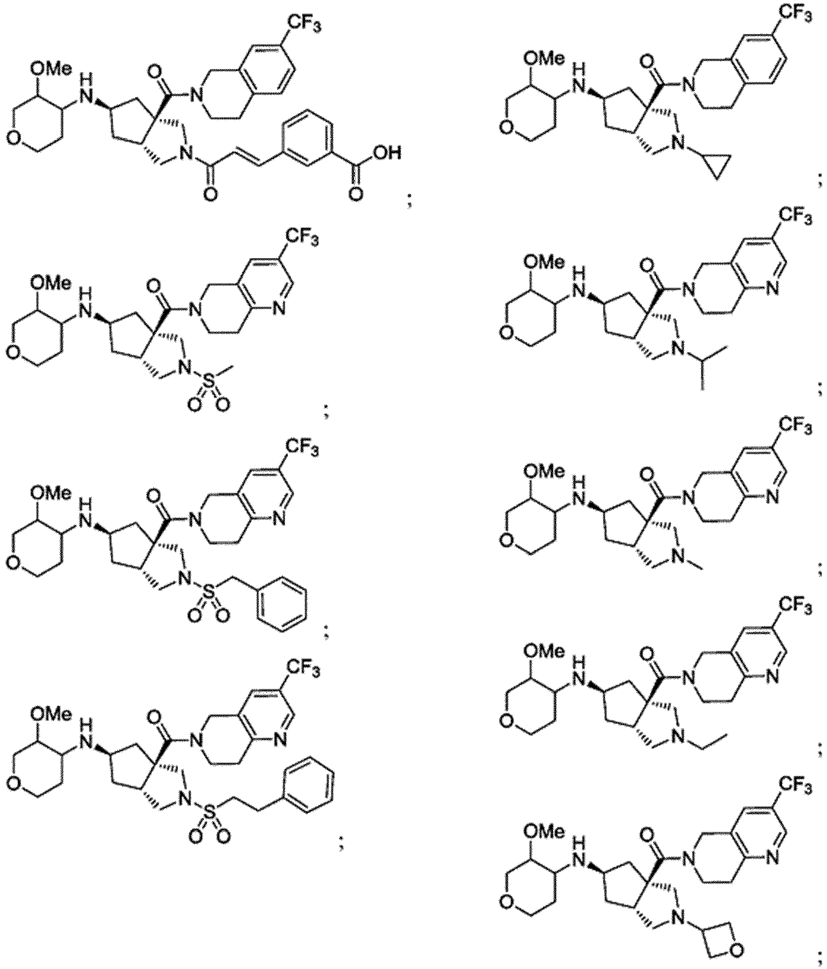 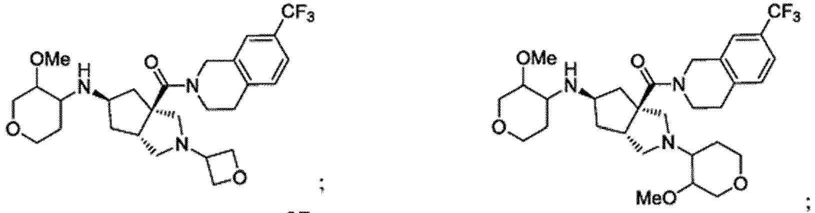 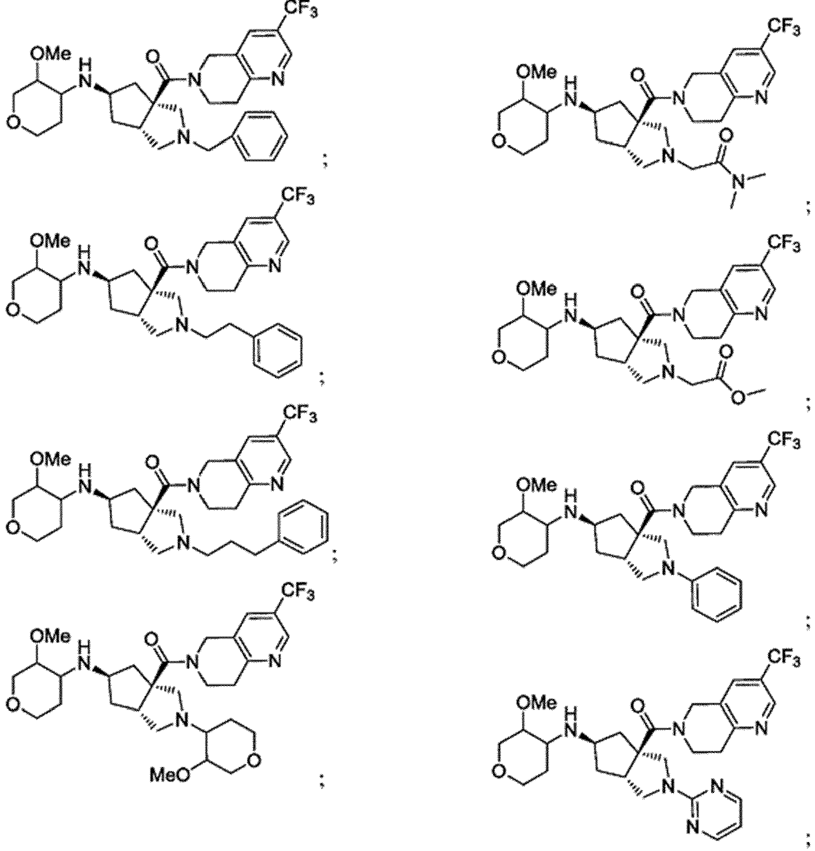 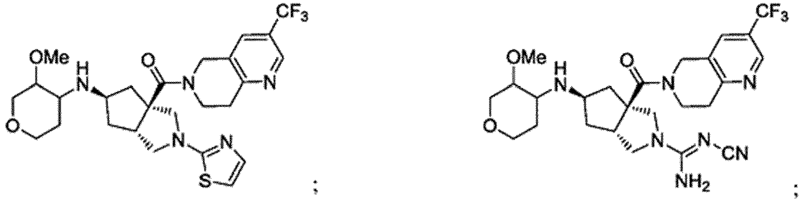 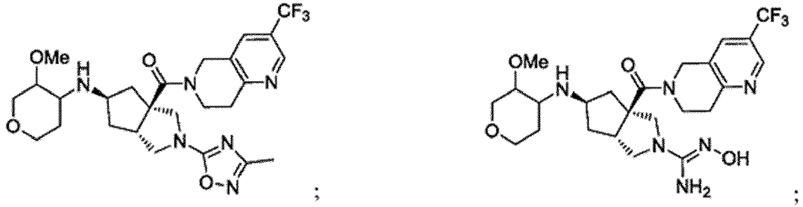 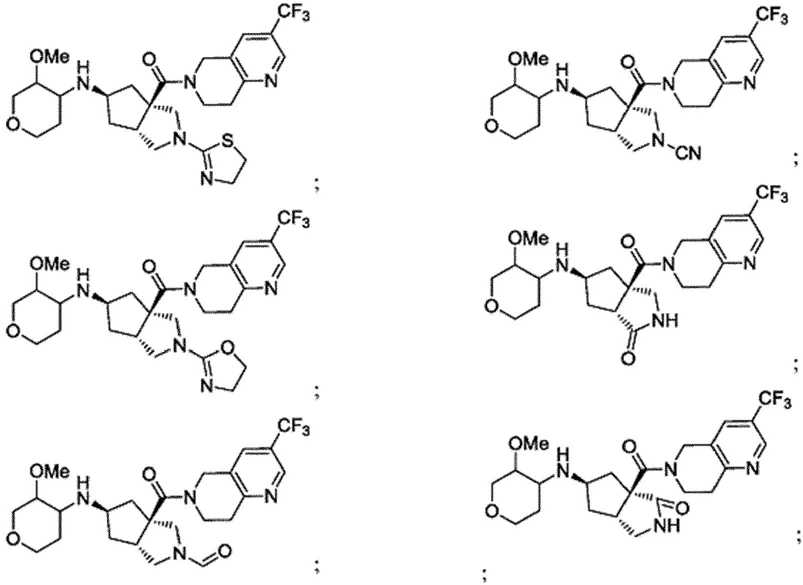 et et sels pharmaceutiquement acceptables de celui-ci.

7. Composition pharmaceutique, comprenant un composé de l'une quelconque des revendications 1 à 6 et un véhicule pharmaceutiquement acceptable.

8. Composition pharmaceutique de la revendication 7, la composition pharmaceutique étant préparée par mélange du composé et du véhicule pharmaceutiquement acceptable.

9. Procédé de fabrication de la composition pharmaceutique de la revendication 7, ledit procédé comprenant le mélange du composé et du véhicule pharmaceutiquement acceptable.

10. Composé de l'une quelconque des revendications 1 à 6 pour utilisation dans un procédé de prévention, traitement ou amélioration d'un syndrome, d'un trouble ou d'une maladie, ledit syndrome, trouble ou maladie étant choisi dans le groupe constitué de : la bronchopneumopathie chronique obstructive (BPCO), des troubles ophtalmiques, l'uvéite, l'athérosclérose, la polyarthrite rhumatoïde, le psoriasis, le rhumatisme psoriasique, la dermatite atopique, la sclérose en plaques, la maladie de Crohn, la recto-colite hémorragique, la néphrite, le rejet d'allogreffe d'organe, la fibrose pulmonaire, l'insuffisance rénale, le diabète de type I, le diabète de type II, les complications diabétiques, la néphropathie diabétique, la rétinopathie diabétique, la rétinite diabétique, la micro-angiopathie diabétique, le surpoids, l'obésité, l'insulinorésistance liée à l'obésité, le syndrome métabolique, la tuberculose, la sarcoïdose, les staphylocoques invasifs, l'inflammation après une chirurgie de la cataracte, la rhinite allergique, la conjonctivite allergique, l'urticaire chronique, l'asthme, l'asthme allergique, les maladies parodontales, la parodontite, la gingivite, une maladie des gencives, des cardiomyopathies diastoliques, un infarctus cardiaque, la myocardite, l'insuffisance cardiaque chronique, l'angiosténose, la resténose, des troubles de reperfusion, un anévrisme abdominal aortique, la glomérulonéphrite, des tumeurs solides et des cancers, la leucémie lymphocytaire chronique, la leucémie myélocytaire chronique, le myélome multiple, le myélome malin, la maladie de Hodgkin et des carcinomes de la vessie, du sein, du col de l'utérus, du côlon, du poumon, de la prostate ou de l'estomac, et des troubles neuro-inflammatoires chroniques comprenant, mais non limités à, la maladie d'Alzheimer, un accident vasculaire cérébral ischémique, une lésion de la moelle épinière, une lésion par écrasement de nerf et une lésion cérébrale traumatique, ledit procédé comprenant l'administration à un sujet en ayant besoin d'une quantité efficace dudit composé.

11. Composé pour utilisation dans le procédé de prévention, traitement ou amélioration d'un syndrome, d'un trouble ou d'une maladie selon la revendication 10, ledit syndrome, trouble ou maladie étant choisi dans le groupe constitué de : le diabète de type I, le diabète de type II, les complications diabétiques, la néphropathie diabétique, la rétinopathie diabétique, la rétinite diabétique, la micro-angiopathie diabétique, l'obésité, l'insulinorésistance liée à l'obésité, le syndrome métabolique, l'asthme, et l'asthme allergique, ledit procédé comprenant l'administration à un sujet en ayant besoin d'une quantité thérapeutiquement efficace du composé.

12. Composé pour utilisation dans le procédé de traitement d'un trouble selon la revendication 10 choisi dans le groupe constitué du diabète de type II, de l'obésité et de l'asthme, ledit procédé comprenant l'administration à un sujet en ayant besoin d'une quantité thérapeutiquement efficace du composé.
